# EUROPEAN PATENT APPLICATION

(11) **EP 1 382 970 A1**
(43) Date of publication of application: **21.01.2004**
(21) Application number: 02015609.7
(22) Date of filing: 15.07.2002
(51) Int. Cl.: G01N 33/68, C07K 14/335

(54) **Novel mucin binding polypeptides**

(71) Applicant: SOCIETE DES PRODUITS NESTLE S.A., 1800 Vevey (CH)
(72) Inventor: Pridmore, Raymond David, 1005 Lausanne (CH)
(74) Representative: Becker Kurig Straus

(57) **Abstract**

The present invention relates to novel genes of Lactobacillus johnsonii encoding polypeptides involved in binding of the bacteria to mucins. In particular, the invention pertains to the use of the present genes in the preparation of polypeptides having the capacity to bind to mucins.

## Description

The present invention relates to novel genes of Lactobacillus johnsonii encoding polypeptides involved in binding of the bacteria to mucins. In particular, the invention pertains to the use of the present genes in the preparation of polypeptides having the capacity to bind to mucins.

Due to their widespread employment in the preparation and preservation of food products, lactic acid bacteria have become a focus of intensive investigation. In nature, these bacteria are usually found in milk of mammals and plants, but are also prevalent in the intestine of human beings and other animals, where they represent a major constituent of the micro-flora.

In the recent past some particular strains of lactic acid bacteria have been found to exhibit favorable properties to man and animals upon ingestion. In particular, specific strains of the genus Lactobacillus or Bifidobacterium have been established to pass the gastro-intestinal tract in a viable and live form and to be able to adhere to and colonize the intestinal mucosa, with their temporary or sustained maintenance in the gut bringing about numerous positive effects on the health of the living beings. These kinds of strains are generically referred to as probiotics.

In EP 0 768 375, such a specific strain of the genus Bifidobacterium is disclosed, which is reported to become implanted in the intestinal flora and to assist in a modulation of the host's immune system, being capable to competitively exclude adhesion of pathogenic bacteria to intestinal cells, thus supporting the maintenance of the individual's health.

Further, in EP 0 577 903 the use of such a lactic acid bacteria having the ability of replacing Helicobacter pylori, an approved origin for the development of ulcer, for the therapeutic or prophylactic treatment of ulcer associated with the action of Helicobacter pylori is disclosed.

In general, the activity of said probiotic strains may reside either in competitively excluding pathogens from the intestinal micro-flora, but also in producing and releasing particular metabolic agents, inducing or implementing the beneficial activities observed. Yet, one of the prerequisites of the favorable activities such probiotic strains may exert in the host is their capability to at least transiently remain in the intestinal tract, which the micro-organisms may achieve by adhering to intestinal tissues, so as to prolong their residence time therein and to compete with other bacteria for region to grow on.

In general, in view of the known valuable properties of probiotic strains obtaining a more detailed information relating to the biology of these strains is desired, in particular as regards the interaction with the hosts, that is the capability to adhere to the intestine's mucosa, which information will allow a better understanding of these mechanisms.

Consequently, a problem of the present invention is to provide means with which probiotic strains attach to intestinal structures.

This problem has been solved by providing novel genes of Lactobacillus johnsonii which encode surface proteins, i.e. a cell wall anchored polypeptides, allowing the micro-organism to bind and adhere to mucins. Mucins are large glycoprotein molecules having a molecular weight of more than about 1,000,000, being present on the surface of a number of epithelial cells, including those of the gastrointestinal tract, the lung or the uterine cervicex. Their main biological function resides protecting and lubricating the epithelial cells thereunder, inhibiting bacteria from infestation of the tissue and rendering waterproof capability to the mucosa. The polypeptide portion of mucins contains threonine, serine and proline as a major constituent and often exhibits a tandemly repeated unit of amino acids. The oligosaccharide side chains, which comprise up to 80% of the molecular weight of the mucin glycoproteins, consist of hexose, fucose, hexosamine, sialic acid and sulphate ester and show a high variability. In humans at least four mucin families have been found so far, known as MUC1, MUC2, MUC3 and MUC4.

In the figures,

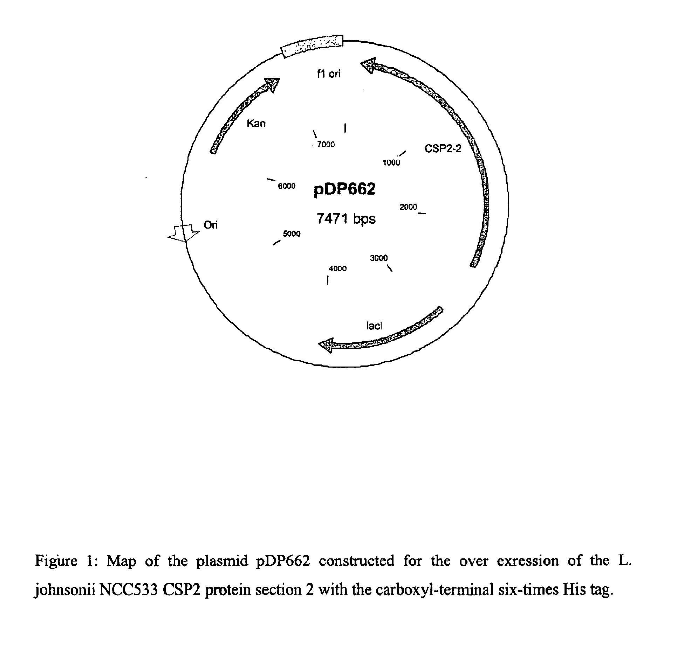

Fig. 1 shows a map of the plasmid pDP662 constructed for the over-expression of the L. johnsonii NCC533 CSP2 fragment with the carboxyl-terminal six-times His tag;

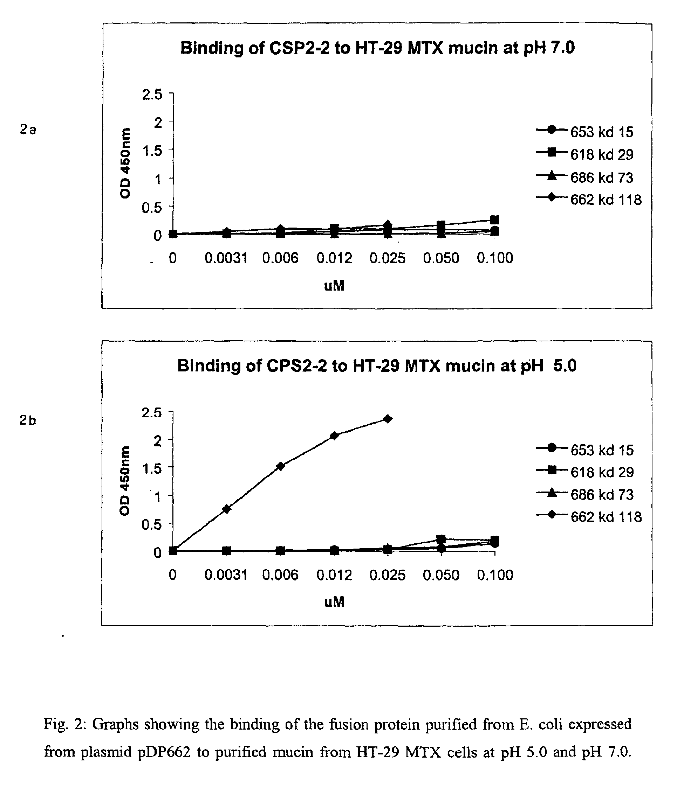

Figs. 2A and 2B show the results of binding experiments of the CSP2 fragment 2 fusion protein to purified mucin from HT-29 MTX cells at pH 5.0 and pH 7.0;

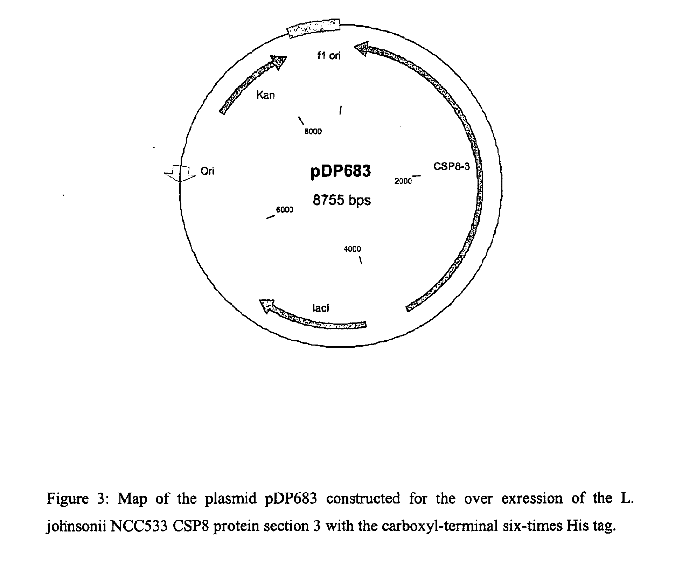

Fig. 3 shows a map of the plasmid pDP683 constructed for the over-expression of the L. johnsonii NCC533 CSP8 fragment with the carboxyl-terminal six-times His tag;

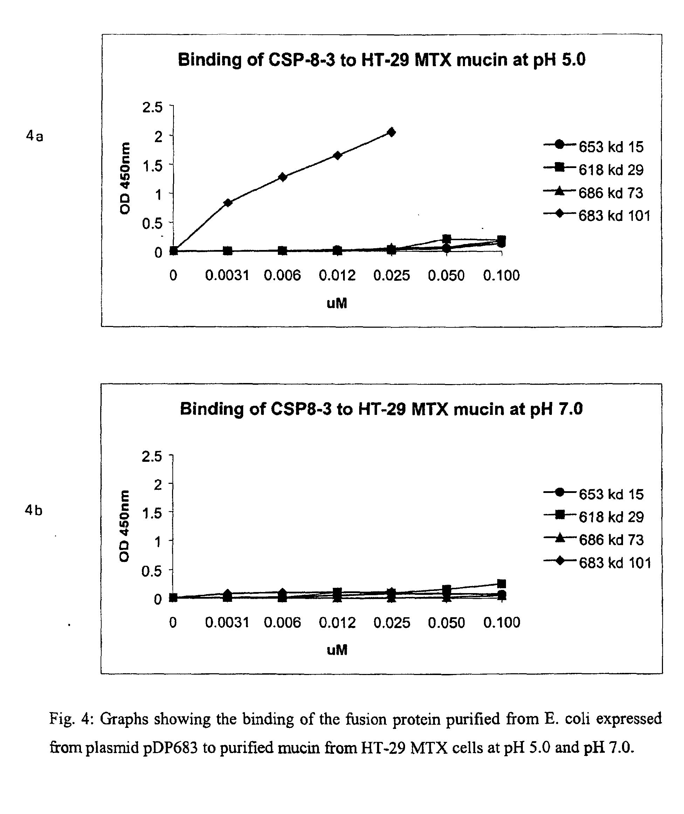

Figs. 4A and 4B show the results of binding experiments of the CSP8 fragment 3 fusion protein to purified mucin from HT-29 MTX cells at pH 5.0 and pH 7.0;

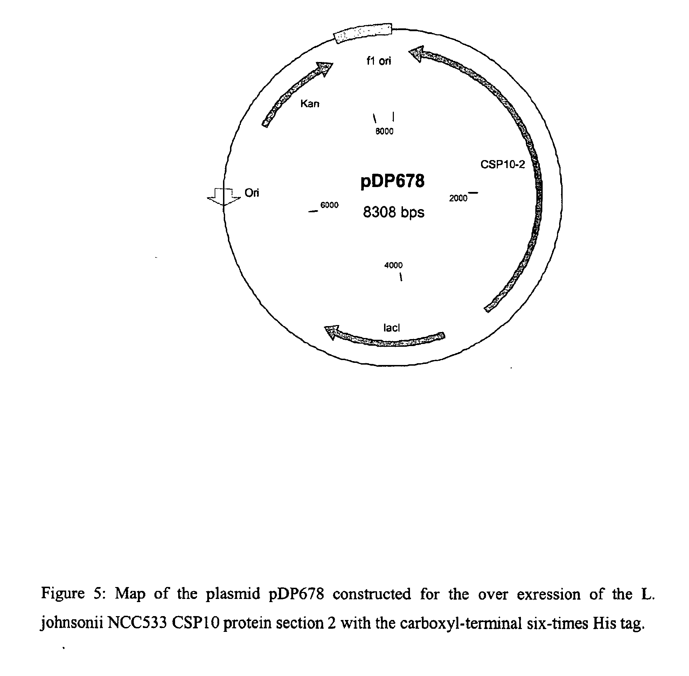

Fig. 5 shows a map of the plasmid pDP678 constructed for the over-expression of the L. johnsonii NCC533 CSP10 fragment with the carboxyl-terminal six-times His tag;

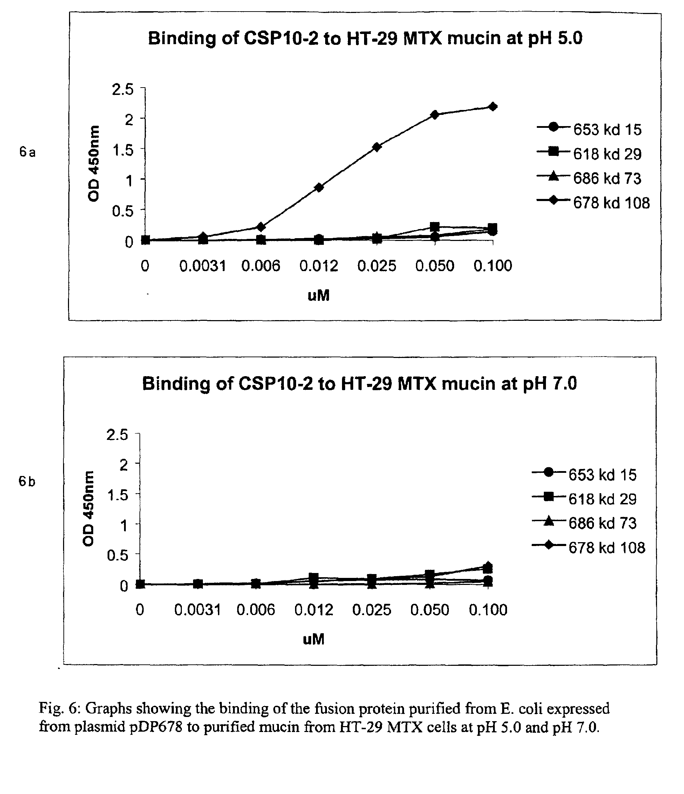

Figs. 6A and 6B show the results of binding experiments of the CSP10 fragment 2 fusion protein to purified mucin from HT-29 MTX cells at pH 5.0 and pH 7.0;

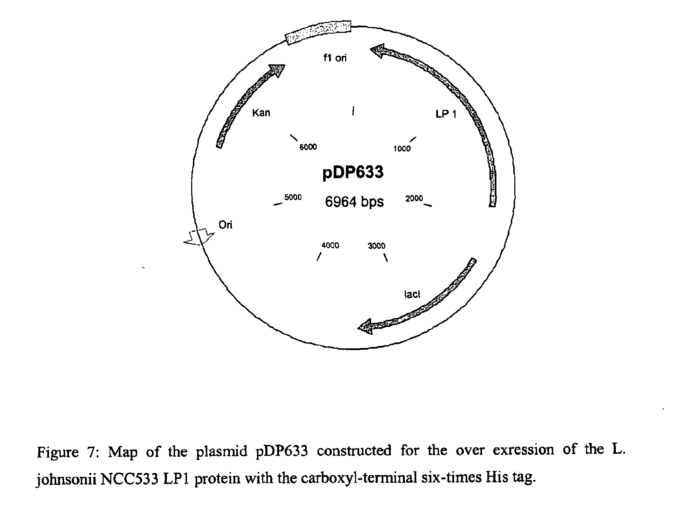

Fig. 7 shows a map of the plasmid pDP633 constructed for the over-expression of the L. johnsonii NCC533 LP1 fragment with the carboxyl-terminal six-times His tag;

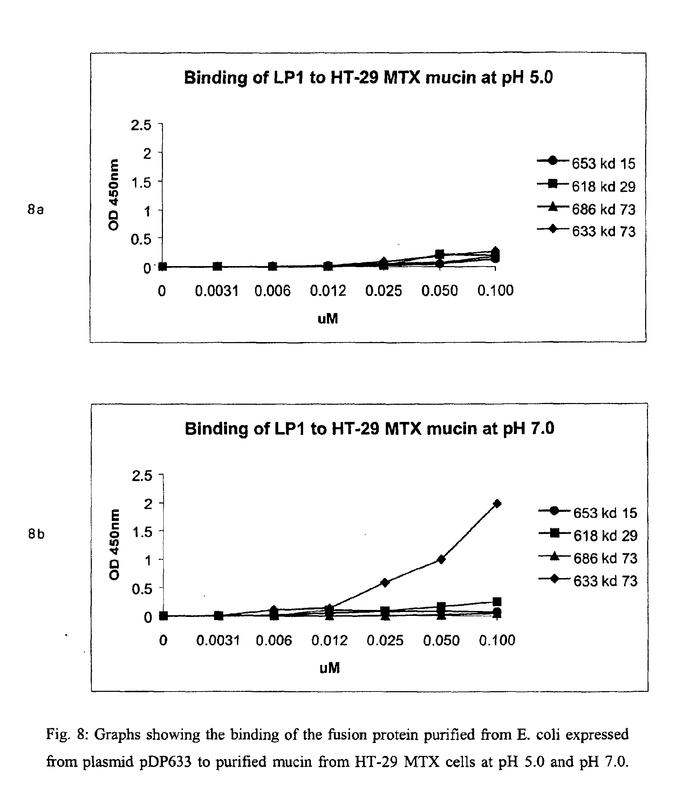

Figs. 8A and 8B show the results of binding experiments of the LP fusion protein to purified mucin from HT-29 MTX cells at pH 5.0 and pH 7.0;

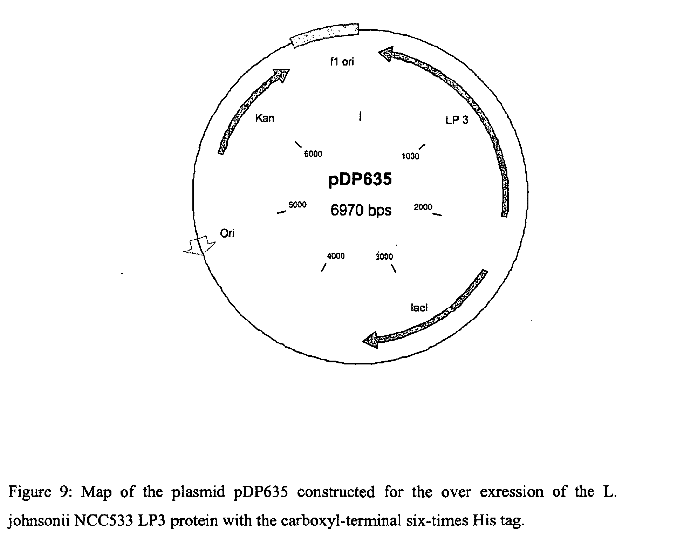

Fig. 9 shows a map of the plasmid pDP635 constructed for the over-expression of the L. johnsonii NCC533 LP3 protein with the carboxyl-terminal six-times His tag;

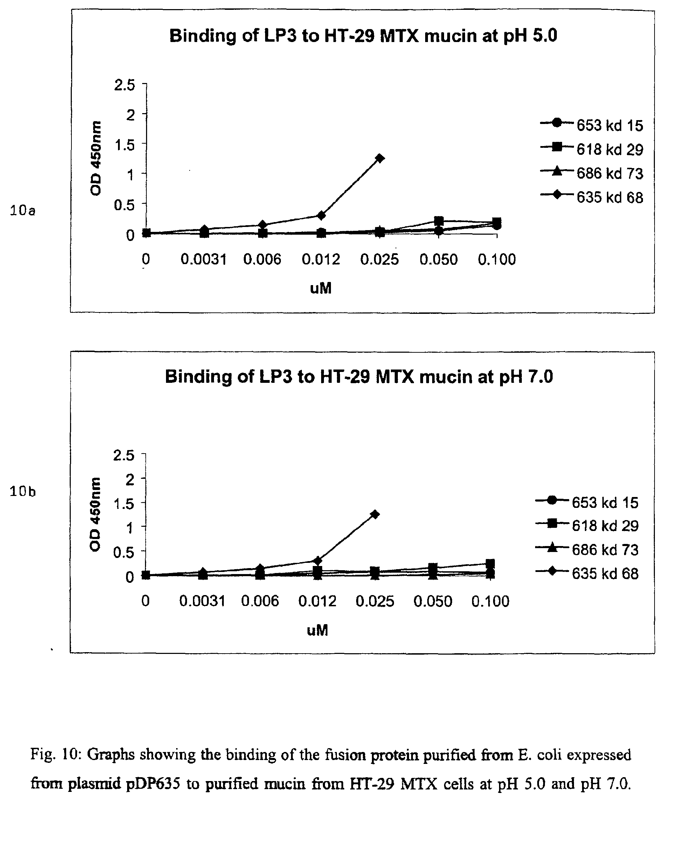

Figs. 10A and 10B show the results of binding experiments of the LP3 fusion protein to purified mucin from HT-29 MTX cells at pH 5.0 and pH 7.0;

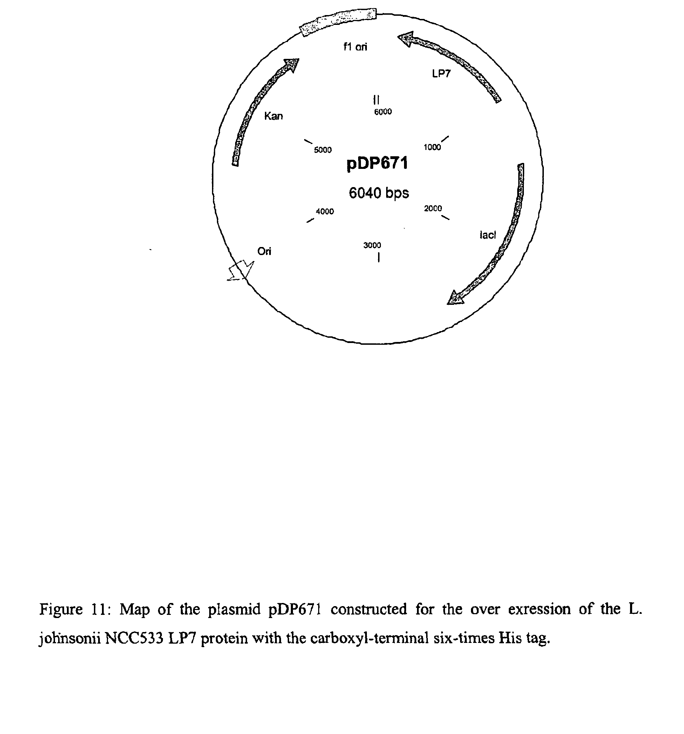

Fig. 11 shows a map of the plasmid pDP671 constructed for the over-expression of the L. johnsonii NCC533 LP7 protein with the carboxyl-terminal six-times His tag;

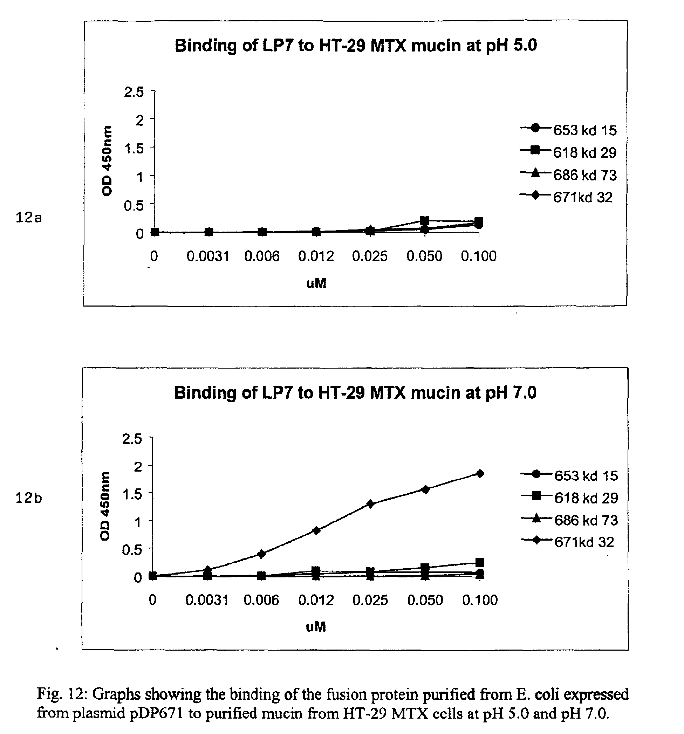

Figs. 12A and 12B show the results of binding experiments of the LP7 fusion protein to purified mucin from HT-29 MIX cells at pH 5.0 and pH 7.0;

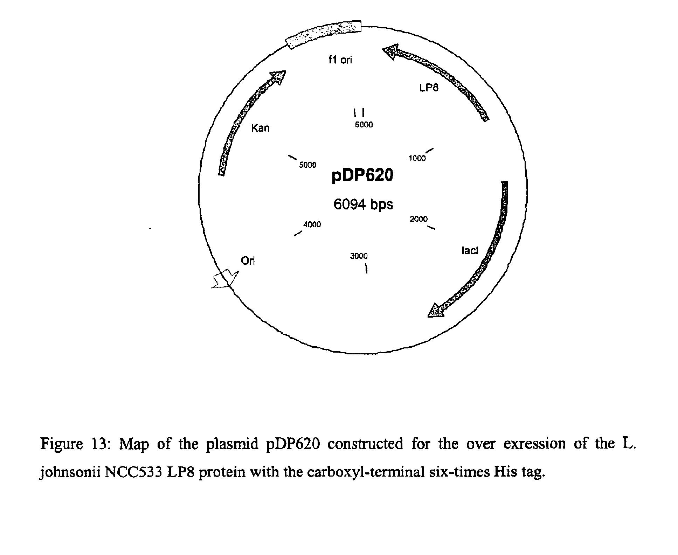

Fig. 13 shows a map of the plasmid pDP620 constructed for the over-expression of the L. johnsonii NCC533 LP8 protein with the carboxyl-terminal six-times His tag;

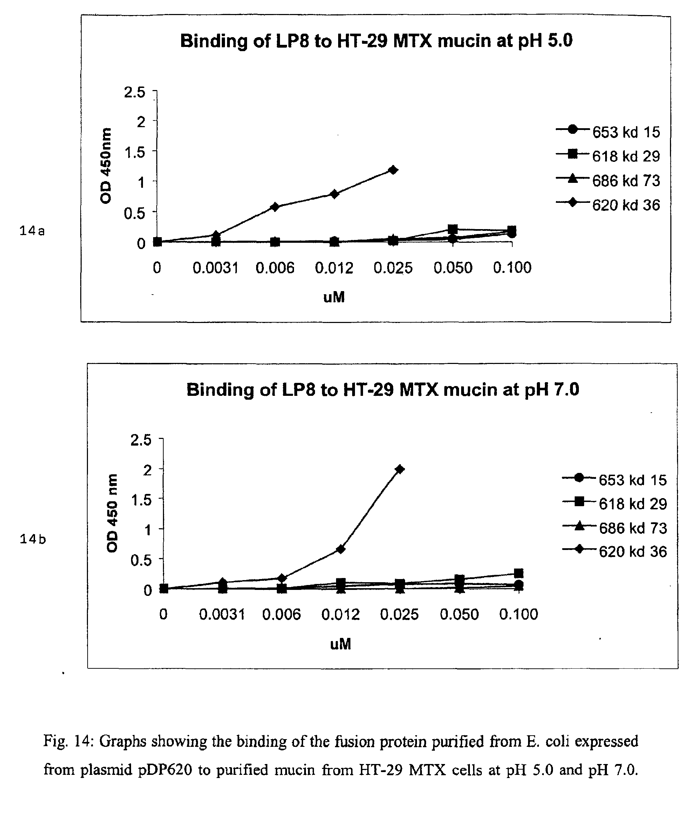

Figs. 14A and 14B show the results of binding experiments of the LP8 fusion protein to purified mucin from HT-29 MIX cells at pH 5.0 and pH 7.0;

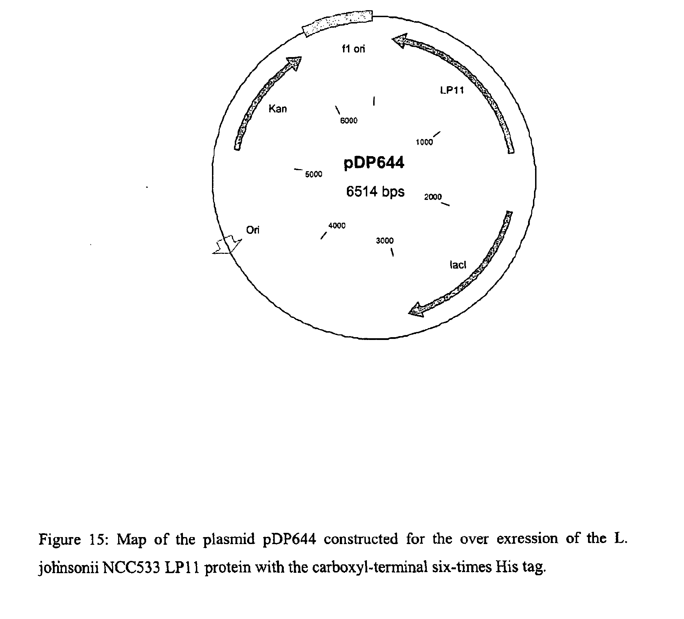

Fig. 15 shows a map of the plasmid pDP644 constructed for the over-expression of the L. johnsonii NCC533 LP11 protein with the carboxyl-terminal six-times His tag;

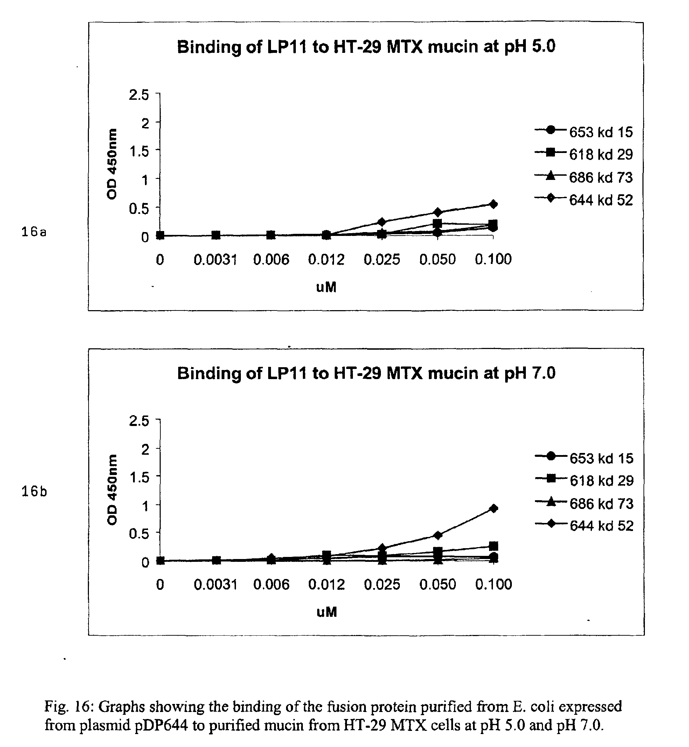

Figs. 16A and 16B show the results of binding experiments of the LP11 fusion protein to purified mucin from HT-29 MTX cells at pH 5.0 and pH 7.0;

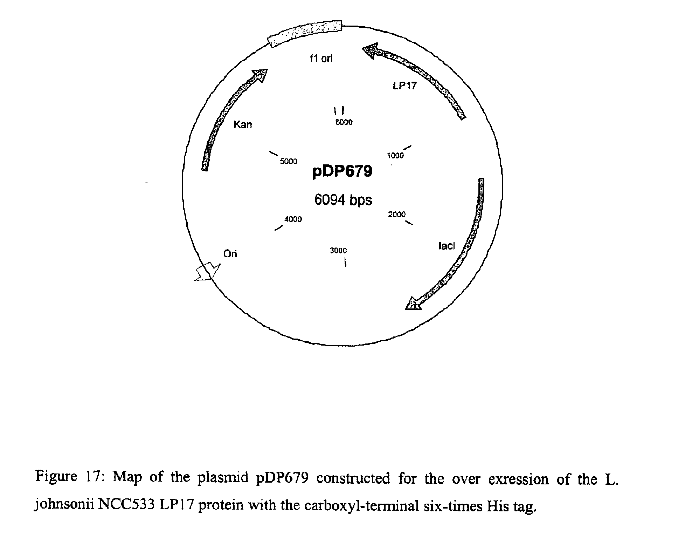

Fig. 17 shows a map of the plasmid pDP679 constructed for the over-expression of the L. johnsonii NCC533 LP17 protein with the carboxyl-terminal six-times His tag;

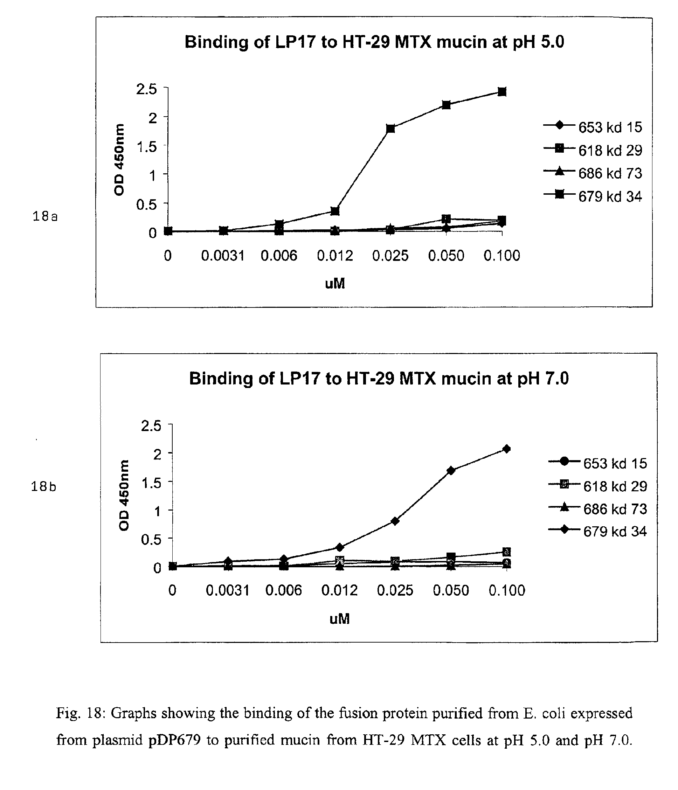

Figs. 18A and 18B show the results of binding experiments of the LP17 fusion protein to purified mucin from HT-29 MTX cells at pH 5.0 and pH 7.0;

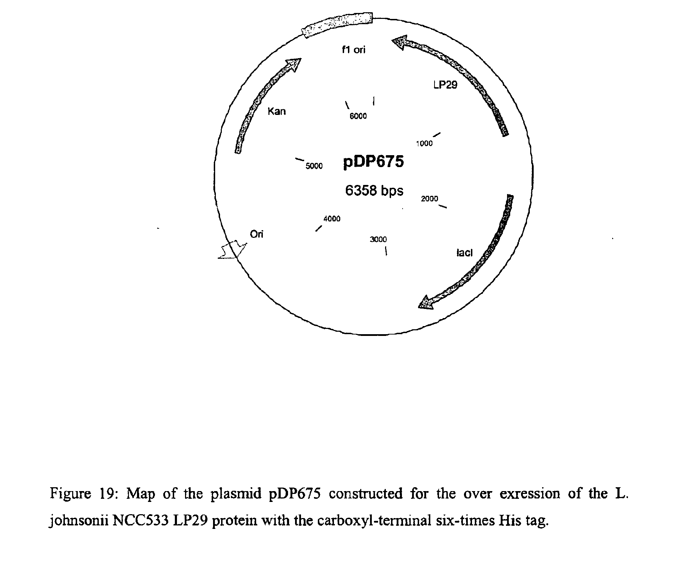

Fig. 19 shows a map of the plasmid pDP675 constructed for the over-expression of the L. johnsonii NCC533 LP29 protein with the carboxyl-terminal six-times His

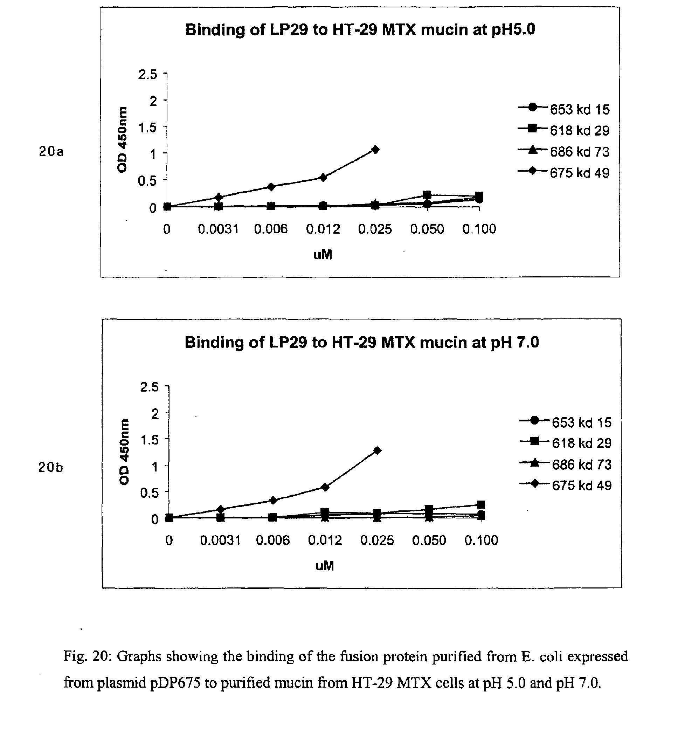

Figs. 20A and 20B show the results of binding experiments of the LP29 fusion protein to purified mucin from HT-29 MTX cells at pH 5.0 and pH 7.0.

In the line of sequencing the genome of the probiotic strain Lactobacillus johnsonii Lal a number of open reading frames was detected that did not show any substantial homology to known polypeptides as accessible in public data bases. In some of the open reading frames a leader sequence and a cell wall anchor motif could be noted, so that the resulting putative polypeptide was assumed to be involved in an interaction of the micro-organism with the environment. Consequently, a these open reading frames were selected for a subsequent analysis.

To identify the function of said new polypeptides the respective nucleic acids encoding the putative polypeptides were cloned into expression vectors and the genes were recombinantly expressed in E.coli and collected (purified). The resulting polypeptides were then subjected to a variety of different experimental conditions, such as e.g. binding assays to collagen, fibronectin, or cleavage assays to various purified proteins.

Surprisingly, an experiment, wherein the recombinant polypeptides were contacted with mucins, showed a substantial binding affinity giving rise to the notion that the present gene products are involved in allowing Lactobacillus johnsonii to adhere to the mucosal tissue in the intestinal tract.

Therefore, according to an embodiment the present invention provides a polynucleotide selected from the group consisting of SEQ. ID.. No. 1 to SEQ. ID.. No. 20 or variants thereof, which variants will yield a biological active polypeptide, that is a polypeptide having the capacity to bind to mucins. According to most preferred embodiments the polynucleotides are identified by a sequence as identified by SEQ. ID.. No. 1 to 20

The polynucleotides referred to in this application as SEQ. ID. Nos. 1 to 20 are:

The polynucleotides SEQ. ID.. Nos. 1, 5, 9, 13, 17, 21, 25, 27, 31 and 35 represent sequences embracing the open reading frame and 5' and 3' regions of the genes termed CSP 2, 8, 10, LP 1, 3, 7, 8, 11, 17 and 29.

The polynucleotides SEQ. ID. Nos. 2, 6, 10, 14, 18, 22, 26, 30, 34 and 36 represent sequences of the corresponding genes comprising the open reading frame from the start methionine up to the last amino acid;

The peptide SEQ. ID. Nos. 3, 7, 11, 15, 19, 23, 27, 31, 35 and 37 represent nucleic acid sequences of the corresponding expressed proteins from the start methionine up to the last amino acid;

The peptide SEQ. ID. Nos. 21 to 40 represent sequences of the corresponding proteins expressed in E. coli with the his-tag from the start methionine up to the last amino acid;

In the context of the present application, the term "homology" shall designate a polynucleotide sequence if at least 90%, preferably at least 95 %, more preferably at least 97 %, even more preferably at least 98 %, of its nucleotide composition and nucleotide sequence corresponds or has identity to any of the sequences as identified by SEQ. ID. Nos. 1 to 20. Likewise, the term "variant" shall designate a polynucleotide having the above degree of homology to a polynucleotide as identified by any of the SEQ. ID. Nos. 1 to 20, or fragments thereof. The term "polynucleotide" as used in the present specification refers in general to polyribonucleotides and polydeoxyribonucleotides, and denotes an unmodified RNA or DNA or a modified RNA or DNA. In the context of the present invention, the term "fragment" is to be understood to refer to polynucleotides shorter than the polynucleotide as identified by SEQ. ID. Nos. 1 and 2.

The homology or sequence similarity or sequence identity of nucleotide sequences may easily be determined according to techniques well known in the art, e.g. by using established software or computer programs, e.g. the BestFit or Gap pairwise (GCG Wisconsin Package, Genetics Computer Group, 575 Science Drive, Madison, Wis. 53711). BestFit uses the local homology algorithm of Smith and Waterman, Advances in Applied Mathematics 2: 482-489 (1981), to find the best segment of identity or similarity between two sequences. Gap performs global alignments, all of one sequence with all of another similar sequence using the method of Needleman et al. (J. Mol. Biol. 48: (1970), 443-453). When using a sequence alignment program such as BestFit, to determine the degree of sequence homology, similarity or identity, the default setting may be used, or an appropriate scoring matrix may be selected to optimize identity, similarity or homology scores. Similarly, when using a program such as BestFit to determine sequence identity, similarity or homology between two different amino acid sequences, the default settings may be used, or an appropriate scoring matrix, such as blosum45 or blosum80, may be selected to optimize identity, similarity or homology scores.

Accordingly, the present invention also encompasses polynucleotides that hybridize, preferably under stringent conditions, to any of the nucleotide sequences as identified by SEQ. ID.. Nos. 1 to 20, or their complements. Hybridization procedures are well known in the art and instructions for identifying DNA sequences by means of hybridization may be found in the Handbook by Sambrook et al.: Molecular Cloning, A Laboratory Manual (Cold Spring Harbor Laboratory Press, 1989). The hybridization may take place under stringent conditions, that is to say only hybrids in which the probe and target sequence, i.e. the polynucleotides contacted with the probe, are at least 90% identical are formed. It is generally accepted that the stringency of the hybridization, including the washing steps, is influenced or determined by varying the buffer composition, the temperature, and the salt concentration. The hybridization reaction is preferably carried out under a relatively low stringency compared with the washing steps.

The polynucleotide sequences according to the invention may suitably be utilized as hybridization probes for RNA, cDNA and DNA, in order to isolate DNAs or genes from other species which exhibit a high degree of similarity to the sequence of the present gene. Moreover, oligonucleotides, derived from the present polynucleotide sequences do also represent suitable primers for performing a polymerase chain reaction (PCR) in order to synthesize DNA encoding a mucin binding polypeptides. Such oligonucleotides may contain of from 5 to 50 nucleotides, preferably of from 8 to 30, more preferably of from 10 to 20 nucleotides.

According to an embodiment, the present invention also provides vectors containing one or more copies of any of the polynucleotides as described herein and allowing propagation and amplification of the present polynucleotides. Suitable vectors are e.g. those, which are replicated in the micro-organisms, into which they have been introduced, at a high copy number. Also suitable are those vectors by means of which the process of gene amplification by integration in the chromosome can be employed. Preferably the vector shall also allow propagation in different micro-organisms (shuttle vector) and/or expression of the desired polypeptide. In order to enable a stable transmission to the progeny the vectors will also contain a selection marker, such as a gene conferring resistance to a specific agent, e.g. an antibiotic. Alternatively, a stable transmission may also be achieved in integrating the present polynucleotides into the host's chromosome. Preferably the vector will also allow expression of the polynucleotide contained therein, and includes regulatory regions, such as e.g. promotors or repressors, which allow transcription of the insert under specific conditions only. Thus, a desirable promotor may be inducible, so that the genetic material harboring the polynucleotide according to the present invention is amplified within the micro-organism, used for propagation and expression, while expression of the desired polypeptide is only effected, when the micro-organism has been cultivated to a certain extent. To this end a great number of polypeptides may be produced in the micro-organism, which might otherwise represent a burden of the growth thereof.

Examples of suitable vectors include e.g. pBR322, pUC, pNZ124, pGK12, pMG36, pSA3, pGKV110 and pGKV210.

According to another embodiment the present invention also pertains to a host cell containing one or more polynucleotides or vectors according to the present invention. In the context of the present invention a "host cell" as claimed shall designate a cell, that contains a polynucleotide or a vector as described herein, which polynucleotide or vector, respectively has not been originally endogenous. That is, the present invention embraces any kind of cell, into which a polynucleotide or vector according to the present invention has been introduced, which cell did not originally contain the present polynucleotides or vectors. Likewise, a host cell according to the present invention also includes a micro-organism, from which the present polynucleotide has been derived, but which now contains additional copies thereof.

Suitable host cells for use in the present invention comprise any kind of cell, in which the present polynucleotide may conveniently be propagated and/or expressed. Examples of such host cells are e.g. Lactococcus lactis, Lactobacillus delbruekii subsp. lactis and bulgaricus, Streptococcus thermophilus and other LAB that may be genetically transformed.

The host cells may be produced by inserting the polynucleotide according to well known techniques, such as is described in the Handbook by Sambrook et al.: Molecular Cloning, A Laboratory Manual. (Cold Spring Harbor Laboratory Press, 1989; Kullen MJ., Klaenhammer TR. Genetic modification of intestinal lactobacilli and bifidobacteria. Curr. Issues Mol Biol. 2000;2(2):41-50). The host cells containing at least an endogenous copy of the polynucleotide according to the present invention may be cultivated batch-wise or continuously, e.g. by a batch process (batch cultivation) or by a fed batch process (feed process) or by a repeated fed batch process (repetitive feed process), for the purpose of propagating the genetic material or for synthesizing the polypeptide encoded by the present polynucleotides. A review of known methods of cultivation is given in the textbook by Chmiel (Bioprozesstechnik 1. Einfuhrung in die Bioverfahrenstechnik (Gustav Fischer Verlag, Stuttgart, 1991)) and in the textbook by Storhas (Bioreaktoren und periphere Einrichtungen (Vieweg Verlag, Brunswick/Wiesbaden, 1994)).

The culture medium which is used must fulfil the requirements of the respective micro-organisms used. Descriptions of culture media for various microorganisms are given in the Handbook "Manual of Methods for General Bacteriology" published by the American Society for Bacteriology (Washington D.C., USA, 1981). Suitable sources of carbon include sugar and carbohydrates such as glucose, saccharose, lactose, fructose, maltose, molasses, starch and cellulose, oils and fats such as soya oil, sunflower oil, peanut oil and cocoa fat, fatty acids such as palmitic acid, stearic acid and linoleic acid, alcohols such as glycerol and ethanol, and organic acids such as acetic acid. These substances can be used individually or in admixture. Suitable sources of nitrogen include compounds which contain organic nitrogen, such as peptone, yeast extract, meat extract, malt extract, corn steep liquor, soya bean flour and urea, and inorganic compounds such as ammonium sulphate, ammonium chloride, ammonium phosphate, ammonium carbonate and ammonium nitrate. These sources of nitrogen can be used individually or in admixture. Phosphoric acid, potassium dihydrogen phosphate or dipotassium hydrogen phosphate, or the corresponding sodium-containing salts, can be used as sources of phosphorus. In addition, the culture medium must contain salts of metals such as magnesium sulphate or iron sulphate which are necessary for growth. Finally, essential growth promoting substances such as amino acids and vitamins can be used in addition to the above substances. Moreover, suitable precursors can be added to the culture medium. The aforementioned substances which are used can be added to the culture in the form of a single batch or can be supplied in a suitable manner during cultivation. Basic compounds such as sodium hydroxide, potassium hydroxide, ammonia or aqueous ammonia, or acidic compounds such as phosphoric acid or sulphuric acid are used in a suitable manner in order to control the pH of the culture. Anti-foaming agents such as polyglycol esters of fatty acids can be used to control the generation of foam. In order to maintain the stability of vectors, suitable substances with a selective activity, such as antibiotics, can be added to the medium. In order to maintain aerobic conditions, oxygen or oxygen-containing gas mixtures such as air are passed into the culture. The temperature of the culture normally ranges from 20 °C. to 45 °C. and is preferably 25 °C to 40 °C. Cultivation is continued until a maximum of genetic or protein material has been formed. This target is normally reached within 10 hours to 160 hours.

According to another aspect the present invention also provides polypeptides as identified by SEQ. ID.. Nos. 21 to 30, or 31 to 40, or variants thereof, which have a degree of homology to any of the polypeptide of SEQ. ID.. Nos. 21 to 30 or 31 to 40 of at least 90 % and are functional, mucin binding polypeptides.

The variants according to invention include polypeptides having the biological activity of binding to mucins and having a homology of at least 90 %, preferably at least 95 %, more preferably at least 98 % to the said polypeptides as identified by SEQ. ID.. Nos. 21 to 40. These variant polypeptides as used herein shall be understood to mean polypeptides or a fragment thereof which has the above mentioned homology to the sequences shown and which are produced by nucleic acids according to the present invention. These variants may be obtained by deleting, adding or substituting one or more amino acids. Preferably, in case of substituting amino acids, the new amino acids will have properties corresponding or similar to the original ones, particularly with respect to the charge, hydrophobic character, steric properties, etc.

The variant polypeptides of the present invention having mucin binding capacity and having a homology to the sequences as identified by SEQ. ID. NOs. 21 and 40 may be produced by conventional mutagenesis or directed evolution procedures. Random mutagenesis of the nucleotide sequences of SEQ. ID. Nos. 1 to 20 may be effected by several techniques known in the art, such as by chemical mutagenesis, UV or X-ray irradiation, insertion of an oligonucleotide linker randomly into a vector comprising SEQ. ID. Nos. 1 to 20, or by techniques such as error-prone PCR mutagenesis.

The present invention also comprises chimeric or fusion proteins containing a polypeptide, variants or fragments thereof as described herein. Methods of engineering fragments using recombinant DNA techniques, protein engineering techniques, such as proteolytic digestion, or by protein synthesis are well known in the art. In particular, these fusion proteins may contain, apart from the polypeptide of the present invention, a short sequence of specific amino acids, which will allow a convenient isolation of the polypeptide. Such a tag is e.g. the so-called "His-tag" or another short amino acid sequence having a high antigeneicity and to which antibodies are available having a high binding affinity thereto.

It will be appreciated that the fusion polypeptides according to the present invention may also contain another amino acid sequence having a desired biological activity. Since the polypeptides according to the present invention are surface proteins, i.e. anchored in the micro-organism's cell wall and extending into the environment, the present polypeptides may likewise be utilized for expressing a given polypeptide or peptide having a given biological function in a host cell according to the present invention. To his end, the fusion polypeptide may contain, apart from the amino acid sequence according to he present invention, short peptide sequences, designed for immunizing the host against a given antigen. Likewise, the additional polypeptide part of the present fusion polypeptide may exhibit an enzymatic activity, e.g. a protease activity, which may assist the host in digesting the food material ingested and occasionally also to decrease the allergeneicity of the said food material.

The polynucleotides according to the present invention may be utilized for preparing any of the polypeptides according to the present invention and also a micro-organism having improved mucin binding capacity.

To this end, a micro-organism of interest is selected and one or more polynucleotides according to the present invention is/are inserted therein by recombinant techniques. Preferably, in order to enable a stable propagation of the genetic material introduced, the present constructs) or polynucleotide(s) is/are inserted into the hosts chromosome, preferably in a higher copy number. The micro-organism is then subjected to culture conditions allowing expression of the desired polypeptide(s).

The micro-organisms are preferably micro-organisms, having a beneficial effect on the host incorporating them. Since the inherent task of the gene described herein is to allow a micro-organism to adhere to mucosal tissues, the capability of a micro-organism, expressing such polypeptides, to attach and adhere to the mucosa in a host's organism will be improved. The micro-organism utilized, is preferably a probiotic micro-organism, with the effect that the residence time of the said probiotic micro-organism in the host will be prolonged, while it may exert its beneficial actions for the host for a longer period of time.

Alternatively, in case of a fusion polypeptide having an additional biological function, the micro-organism expressing the present polypeptide will have a novel activity beneficial to the host incorporating them, such as the capacity to display antigens, i.e. its aptitude for being used as a vaccine, or the capacity to degrade proteins, i.e. a protease activity. It will be appreciated that in case of a micro-organism, that is inherently not substantially resistant to conditions in the stomach and therefore not expected to arrive in the gut of an individual in an essentially live and viable form, the skilled person will formulate said micro-organism in a suitable carrier, that enables such a passage. Alternatively, the mucin binding domain could be used to selectively target bioactive molecules, such as IL-10, to the mucus layer.

The present invention also provides a method for the production of a polypeptide according to the present invention which essentially comprises the step of culturing a micro-organism, containing one or more gene(s) or gene construct(s) as described herein under conditions suitable for growth of the micro-organism and expression of the gene(s) and collecting the polypeptide(s). This may be achieved by centrifuging the culture broth to recover the micro-organisms as a pellet. The pellet is the subjected to well known treatments in order to isolate the membrane fraction and the proteins contained therein are collected. In order to purify the polypeptide(s) according to the invention antibodies raised against the present polypeptides or antibodies raised against a portion of the fusion protein (e.g. the "tag") may be utilized. Alternatively, in case the polypeptide is synthesized in the micro-organism without its membrane binding domain, the intracellular fraction of the micro-organism is isolated and the proceedings for isolation as exemplarily indicated above are followed. For a more detailed information as regards the isolation of polypeptides from micro-organism reference is made to the Handbook by Sambrook et al.: Molecular Cloning, A Laboratory Manual (Cold Spring Harbor Laboratory Press, 1989)

The present invention also provides a method for detecting a polynucleotide sequence encoding a mucin binding polypeptide and having a polynucleotide sequence homologous to that as identified by SEQ. ID.. Nos. 1 to 20. Such method comprises the steps of collecting a biological sample, such as e.g. selecting a particular micro-organism. In order to enable good results in the present method, the DNA and/or the RNA of said micro-organism may be isolated prior to performing the detection step. Methods for isolating DNA and/or RNA are described in the Handbook by Sambrook et al.: Molecular Cloning, A Laboratory Manual (Cold Spring Harbor Laboratory Press, 1989). The detection step may be performed by e.g. hybridizing the sample and/or the DNA isolated therefrom with a polynucleotide according to the present invention or a fragment thereof, and detecting the hybridization complex, wherein the presence of the hybridization complex correlates with the presence of a polynucleotide encoding a mucin binding proteins in the biological sample. The detection may be achieved by labeling the polynucleotides used as a probe with e.g. radioactive nucleotides or fluorescence markers and identifying the presence of the label by means of a autoradiography. Alternatively, also the well known technique of the polymerase chain reaction may be utilized. To this end, two oligonucleotides, derived from the present polynucleotide, are chosen such that one oligonucleotide is chosen from the sense and the other from the anti-sense strand such that a fragment, lying between them is defined. The oligonucleotides are then hybridized with the biological sample or the DNA or RNA, respectively isolated therefrom and the fragment lying between them is amplified. The presence of a polynucleotide according to the present invention may be detected by applying an aliquot of the PCR-reaction on a gel and visualizing the fragment obtained, wherein the presence of a fragment, having the appropriate size is indicative of the presence of a mucin binding protein in the biological sample investigated. In order to confirm the results obtained by PCR, the fragment amplified may be sequenced and the nucleic acid sequence thus obtained may be compared with the sequence according to the present invention.

According to an alternative embodiment the invention also provides a method of detecting the presence of a polypeptide according to the present invention in a micro-organism, wherein an antibody raised against the present polypeptides is utilized. The presence of a bound antibody is then indicative of the presence of a mucin binding protein in the micro-organism investigated. It will be appreciated that the skilled person is well aware of raising polyclonal or monoclonal antibodies against a given antigen. In this respect reference is made to Granato D.A., Piguet P.-F. Clin. Exp. Immunol. 1986, 63:703-710, which document is incorporated herein by way of reference.

The following examples illustrate the invention without limiting it thereto.

### Example 1

Cloning of the CSP2 , CSP8 and CSP10 fragments and the LP1, LP3, LP7, LP8. LP11, LP17 and LP29 genes:

To order to elucidate the biological activity of the CSP2 , CSP8 and CSP10 fragments and the LP1, LP3, LP7, LP8, LP11, LP17 and LP29 genes or the gene products thereof, respectively, the respective nucleic acids were expressed in the heterologous host E. coli in sections of approximately 1000 amino acids or as the complete genes. The CSP8 gene was modified to remove the secretion signal sequence at the amino terminus of the produced protein to reduce toxic effects of overexpression, while the cell wall anchor domain was also eliminated and replaced by a peptide linker plus six times histidine tag to aid purification and detection of the recombinant protein.

To achieve this objective, CSP8 carboxyl-terminal region was PCR amplified from L. johnsonii NCC533 chromosomal DNA using the oligonucleotide primers 5'ATATATCCATGGCTTATCGCGTGACTGGTACGG3', introducing a NcoI restriction site (underlined), and 5'ATATATGGATCCTTCCCTACTAATGTGCTGC3' introducing a BamHI restriction site (underlined).

Also, CSP2 section 2 was PCR amplified from L. johnsonii NCC533 chromosomal DNA using the oligonucleotide primers 5'ATATATCCATGGCTAACACAAATGCTGC3', introducing a NcoI restriction site (underlined), and 5'ATATATGGATCCCTTACAAGTACGTAACCC3' introducing a BamHI restriction site (underlined).

Also, CSP10 section 2 was PCR amplified from L. johnsonii NCC533 chromosomal DNA using the oligonucleotide primers 5'ATATATCCATGGTTGCTCGTGCTATTGCTGCC3', introducing a NcoI restriction site (underlined), and 5'ATATATGGATCCCCTTCATCTTGTGCAGTC3' introducing a BamHI restriction site (underlined).

Also, the LP1 region encoding the mature protein was PCR amplified from L. johnsonii NCC533 chromosomal DNA using the oligonucleotide primers 5'TAACATCCATGGCATGTGGTAACAATTCAAATTCAAAAGTAGATAACCC3', introducing a NcoI restriction site (underlined), and 5'ATATATGGATCCTTTGCAATTCCTACATTGTACC3' introducing a BamHI restriction site (underlined).

Also, the LP3 region encoding the mature protein was PCR amplified from L. johnsonii NCC533 chromosomal DNA using the oligonucleotide primers 5'TAACTTCCATGGCCTGTGGTAATAGTAATAGCAATAATACTAGTC3', introducing a NcoI restriction site (underlined), and 5'ATATATGGATCCTTTGCATAGCCAACGTAGTACCAG3' introducing a BamHI restriction site (underlined)

Also, the LP7 region encoding the mature protein was PCR amplified from L. johnsonii NCC533 chromosomal DNA using the oligonucleotide primers 5'ATATATCCATGGCTTGTTCAAATAAAAGCAGTAAACAAGC3', introducing a NcoI restriction site (underlined), and 5'ATATATGGATCCTGATTATTTGTTCTTCTTGTGC3' introducing a BamHI restriction site (underlined).

Also, the LP8 region encoding the mature protein was PCR amplified from L. johnsonii NCC533 chromosomal DNA using the oligonucleotide primers 5'ATATATCCATGGCATGCTCAAAAAAGAAAATTCATC3', introducing a Ncol restriction site (underlined), and 5'ATATATGGATCCTCTAGTTTTTATTAATATTTGC3' introducing a BamHI restriction site (underlined).

Also, the LP11 region encoding the mature protein was PCR amplified from L. johnsonii NCC533 chromosomal DNA using the oligonucleotide primers 5'ATATATCCATGGCTTGCTCAAATTCTAAGAGTAATGATAGCTC3', introducing a NcoI restriction site (underlined), and 5'ATATATGGATCCTGATTCCAAGCACTTTCGAACTTAGTC3' introducing a BamHI restriction site (underlined).

Also, the LP17 region encoding the mature protein was PCR amplified from L. johnsonii NCC533 chromosomal DNA using the oligonucleotide primers 5'ATATATCCATGGGTTGTCAAAGTAATGAAAACC3', introducing a NcoI restriction site (underlined), and 5'ATATATGGATCCTCATCGTCACTATTATCATC3' introducing a BamHI restriction site (underlined).

Also, the LP29 region encoding the mature protein was PCR amplified from L. johnsonii NCC533 chromosomal DNA using the oligonucleotide primers 5'ATATATCCATGGCATGTGGCAATTTAAAAAATTCTG3', introducing a NcoI restriction site (underlined), and 5'ATATATCCATGGCATGTGGCAATTTAAAAAATTCTG3' introducing a BamHI restriction site (underlined).

PCR reactions were performed using the Pwo thermostable polymerase (Roche Molecular Biochemicals) on an Applied Biosystems 9700 thermocycler using the following amplification protocol for each experiment: incubate at 95°C for 5 min, followed by 30 cycles of 95°C for 30 sec, 50°C for 30 sec, 68°C for 4 min and finally incubated at 68°C for 7 min before holding at 4°C. The respective amplicons obtained were purified using the Millipore spin column system and the fragments were eluted in 50 µl of TE buffer (10 mM Tris pH 8.0, 1 mM EDTA). A 25 µl sample was each digested to completion with the restriction enzymes Ncol plus BamHI, the desired fragments were purified over a 1% agarose gel, extracted from the agarose using the Life Technologies Concert gel extraction system and eluted in a 50 µl volume of TE.

The fragments each obtained were ligated into the E. coli expression vector pET24d (Novagen) previously digested with the restriction enzymes Ncol plus BamHI and dephosphorylated. The ligation was transformed into the E. coli strain XL1 blue (Stratagene) and transformants were selected on LB plates supplemented with Kanamycin. Individual colonies were screened by small-scale plasmid preparation and restriction enzyme analysis. plasmids showing the expected restriction pattern were subjected to DNA sequence analysis to confirm the integrity of the construction. The plasmids were designated pDP662 (CSP2), pDP683 (CSP8), pDP678 (CSP10), pDP633 (LP1), pDP635 (LP3), pDP671 (LP7), pDP620 (LP8), pDP644 (LP11), pDP679 (LP17), pDP675 (LP29).

### Example 2

### Expression-purification:

The plasmids pDP662 (CSP2), pDP683 (CSP8), pDP678 (CSP10), pDP633 (LP1), pDP635 (LP3), pDP671 (LP7), pDP620 (LP8), pDP644 (LP11), pDP679 (LP17), pDP675 (LP29) were each transformed into the expression host BL21 (DE3) codon plus RIL obtained from Stratagene Inc and transformants were selected on LB agar plates supplemented with 50 µg/ml Kanamycin and 30 µg/ml chloramphenicol. The host BL21 (DE3) codon plus RIL contains a plasmid carrying the chloramphenicol resistance selectable marker plus the arginine (AGA), isoleucine (ATT) and leucine (TTA) tRNA genes to promote the over expression of *L. johnsonii* Lal genes which is relatively A/T rich and hence containing these potentially problematic anticodons.

10 ml of a respective 16 hr pre-culture in LB medium supplemented with Kanamycin and chloramphenicol was inoculated into 500 ml of the same medium and incubated at 37°C to an OD₆₀₀ of 0.6 with vigorous shaking. IPTG was added to a final concentration of 1 mM and the culture incubated for a further four hours. The bacteria were each harvested by centrifugation and the wet weight of the pellet was determined. The respective pellets were frozen, thawed, suspended in 5 ml lysis buffer (50 mM NaH₂PO₄, 300 mM NaCl, 5 mM imidazole at pH 8.0) containing 1mg/ml lysozyme per 2.5 gm bacterial pellet and incubated for 30 min on ice. The suspensions obtained were then sonicated six times for 15 seconds at 50% power setting (with intervals of cooling on ice) and the cell debris was pelleted by cenrifugation at 10'000 g for 30 min at 4°C. The soluble fractions were removed from the pellets, mixed 2 ml soluble fraction per one ml of Ni-agarose (Roche Molecular Biochemicals) and gently agitated at 4°C for 16 hr. The soluble fractions/Ni-agarose were then loaded onto an empty column (BioRad) and washed with 'wash buffer' (50 mM NaH₂PO₄, 300 mM NaCl, 10 mM imidazole at pH 8.0) until no more protein was eluted (OD₂₈₀reading at 0). The respective bound proteins were eluted in 500 µl fraction in 'elution buffer' (50 mM NaH₂PO₄, 300 mM NaCl, 125 mM imidazole at pH 8.0) and the protein concentration was determined for each fraction (and sample) using the BioRad Bradford protein determination system and the four most concentrated fractions were pooled. A PD10 column (Amersham Pharmacia Biotech) was equilibrated with 20 ml PBS (20 mM KPO₄, 150 mM NaCl, pH 7.0), the protein samples were added and eluted in four ml PBS buffer. The concentration of the purified protein was each determined, mixed with glycerol to 10% and frozen away in aliquots at -20°C. Protein purification was controlled at all step by SDS-PAGE.

### Example 3:

### Mucin isolation:

HT29-MTX cells (cf. Lesuffleur et al. Growth adaptation to methotrexate of HT-29 human colon carcinoma cells is associated with their ability to differentiate into columnar absorptive and mucus-secreting. *Cancer Res* (1990) 50: 6334-43,) were cultured for 3 weeks in DMEM cell medium (Gibco) supplemented with 10% fetal calf serum (Gibco) containing 4 µCi/ml D-(6-³H) glucosamine (to facilitate monitoring of mucin purification). Cell culture supernatants and mucus were collected by repeated gentle pipetting over cells without disturbing the monolayer. Samples were centrifuged 10 min at 1'200 rpm to remove dead cells and the supernatants then further centrifuged 30 min at 15'000 rpm to remove cell debris. The supernatants were recovered and treated withNaN₃ (0.01%), EDTA (5 mM) and PMSF (2 mM) to avoid bacterial contamination and protein degradation. The mucus samples were then lyophilised, suspended in 400 µl of 100 mM ammonium bicarbonate pH 7.6 per HT29-MTX plate and loaded onto a Sepharose CL-4B column (Pharmacia) equilibrated in the same buffer. The column was washed with the same buffer at a flow rate of 0.5 ml/min, the fractions collected and tested for the presence of mucin which eluted in the void volume. The samples were pooled and lyophilised.

Total protein concentration of the mucus enriched preparation was determined using the Bio-Rad protein assay (Bio-Rad) according to manufacturers instructions, while total sugars were determined as described (cf. Dubois et al. Colorimetric method for determination of sugars and related substances. Anal Chem. (1956) 68: 350-6). Mucus enriched preparations were also analyzed by SDS/PAGE by loading 10-20 µg sample onto a 5% polyacrylamide gel and revealed by silver staining. Sugars were analyzed by loading 10-20 µg sample onto a 4-20% polyacrylamide gel and revealed by PAS (Periodic acid Schiff).

### Mucin binding assay:

Polyvinyl 96 wells Nunc-Immuno^{TM} Plates Maxisorb^{TM} Surface were coated with 50 µl of 5 µg/ml of purified mucins from HT29 MTX cells in 0.05 M carbonate bicarbonate buffer pH 9.6 overnight at 4 °C. The wells were saturated by adding 220 µl of 10 mg/ml of animal gelatin (gelatin alba Golddrück, Siegfried) in PBS 0.02% sodium azide for 1 hour at 37 °C.

Samples diluted in PBS or acetate buffer containing 1% BSA were added to the wells and incubated for 2 hours at 37 °C with slow agitation. After 3 washings of 220 µl with the buffers at the respective pH's, the samples were fixed by incubating with 220 µl of 2.5% of paraformaldehyde in PBS pH 7.2 buffer for 10 minutes at room temperature. After 3 washings with PBS, 100 µl of 1/2000 anti penta histag antibody coupled to peroxydase (Qiagen) in PBS 1% BSA were added and incubated for 1 hr at 37 °C with a slow agitation in the dark. After 3 washings with PBS, revelation of the bound antibody was done by addition of 100 µl of a 1:1 mixture of the peroxydase substrate and H₂O₂ (TMB kit, Pierce). The reaction was stopped with 1M of H₂SO₄ and the absorbance of the wells was read at 450 nm in Dynatech MR5000 ELISA reader.

SEQUENCE LISTING

<110>  Société des Produits Nestlé S. A.

<120>  Novel mucin binding polypeptides

<130>  80371 EP

<140>  EP 02 015 609.7
<141>  2002-07-15

<160>  59

<170>  PatentIn version 3.1

<210>  1
<211>  6920
<212>  DNA
<213>  Lactobacillus johnsonii

<400>  1
catttttaat gattttagtg tataaaataa tatacaaatt tttgaatatt atcatataat      60

aaacacatta ttttatatat aaaaacaatg aaatacagta aaaatcagca attaagcaag     120

aaaaatactg attaaataac ttctagacct attcttgtaa tgaaaaatat ttgttataat     180

aacactaaag atccgatagt attactaaaa tatattaatt atataacagt taaatggagg     240

aaaataagga atgctatcta agaataacta ccaagaacgc ttaagaaaaa tggatgacaa     300

acaagaacgt ttttcaatcc gtaaattttc tgtcggtgca gcttctgttt tagtcggaac     360

agctgtatta actatgcaaa atgttcaaac agcacgcgcc gatactactt caaatactac     420

aactaagaca acagatttat cttcagaagc tgaagaacaa aacaagcaaa atgcttatga     480

taaaactctt aatgaggcac aacctacaac taagcctgca gatgctcctg tggagagtca     540

agatagtaag gtggcctctt ttagtgtgcc taaaaatgaa ggaacatttg tagaaaaaac     600

actaacgcct aatattactg aagagacaga ggcaatgcag gataaggctg agttagctca     660

agtaaatagt gaggataagt cagctgaaaa caataaaact aatgtaaagg aagaaagtca     720

gactgttgaa gagaatcaaa tggataagcg atcagctgaa gcgaatatat cggatacaga     780

aaacacatta gtaagtaaca caactttaaa tgtccctaag acaactccag ctactacacg     840

cttttcagca acagcactaa gtgagagcaa atttcaagtc aatgctccac gcgtgggcca     900

agatactcct gatacacaag ttaagaaaaa taattataaa cttgtaacca atgctactgc     960

acttcaacaa gcaattaata ctggtgttgc aggtgttaat attgatagaa gtattgatgc    1020

ttctaatgtt gatttggcta ttagaaatac ttttacaatt gttggtctta acgatgcagc    1080

agcacttaat ttaggtcaaa aatctcttaa taattccggt aatttaactc tccaagatat    1140

taagattaat ggatcaatct ctggtagcgg tacggtaaat attaagggaa atgttacttc    1200

taatgtaaat agtactaatt cttctgttcc aactcaggat caatataatg ctcaaaatta    1260

tactggtagt agaactaact ttaagaactc taacattgct gctaggaacg taaatattga 1320

aaatggtgct tccttaacta ttaacagttc tgaaattaat gatggaatta acttagctga 1380

tggcggtact gttaaagttg gcgataatgc tactttaaat gttaacctga ctaatacatc 1440

aactactgct acccgttacc atgttgctgg tgtgtttgcc aagaatggtg gtaactttat 1500

tagtggttac aagtcaaatg ttaattttaa tacaggatta ggccaagcta ttgcgatcgg 1560

agcaacccga ccaacaagta ctgacgccga tagatacggt ggttatggta cacgctcaag 1620

aaatgatggt ccaacattag ttcaattagg tgactcatcg acttttaact ttactggacg 1680

tgacggtatt atcttaggta ataatgctaa ctttatttct ggagagaatt caaatgttca 1740

ttttgaaaac aagggacgag gagttgcttt agatttagct aataactcaa atatcgaaat 1800

ttctaaacat tccattactt ctttccattc tgttggcaag acaggaacgt ctggtagctt 1860

tgatggctat aactatattg gtgttaacga aggtggtaat attactgttg acgaatatgc 1920

taccttccgt gtaattcttg aaggtcgtgg cgataatcca tgggatgacg taatttcctt 1980

agattctaga aatgctaaca caaatgctgc tttcacttct aagacaggag caattgttga 2040

cattcgtgat gataatacta acttttacgc agaacttatt tcattcccac tagggagtgc 2100

aaactcacgc attgatattc aagatcccct aatgcttaac ctgcaacgct actctaatgg 2160

tggagcaacg actggttgga tggctactgg tggtgacaaa atcaacacaa cttcggcaca 2220

atatactgct aacttaattt atatgagtgg taataaggga gtatttagtg ttagtggtgg 2280

ggattacgat ccatctaatc ctaatagttc cggctttgtt gtttaccaac aaattaagtc 2340

agatggctca aaacaaatct ggctcaatgt aaatagcgtt gatattccaa tgaatggatt 2400

ccaaactaaa gatatttgga ataatcaagc aaacccagac gtatcaattg caggtaatgg 2460

tttaactgca ggtattaaag cgaaccaagt tcataacttt gatgggtctc cactaactgg 2520

tccgaatgca ccatactatg gaatttcaac ccaacgtgct agtcatcaaa tttggattcc 2580

tcatagaaca tcattggaag ttactggtga acacaagagt actattaagt acgttgatga 2640

aaatggaaaa gaaatttttc cagaaaatac tcaatcatta gatatgaaac gtagtctgat 2700

acttgatatt acacaagata agattcaaga tattcagaat tatgctttaa gtcatactgc 2760

agatgaaact ttagagtaca ttaaaaacag tcaagcagta gttcaagatt caggttggga 2820

tttcactgat gctaatggta aagctgttat tgatccatat agtacaattg actcacctaa 2880

attggctggc tatgctgcaa cgatccaatc aacaaatgtt tcaactttaa aggttggagc 2940

agatgcttct gcagtaactg ctaaatttat ggttgaacca tctcaagata tggttcaaaa 3000

tggcgaatta tcaactgctt acaagcaaaa tggcactact ggaattcctg cagattatgt 3060

aacagtagtc gtttataata aacaagcaac ttatcaggca ggtaaaactg atagtaagag 3120

tgttgttcgt gatattaact acttagatgg taagacaggc aaaaagattc cagctaacct 3180

aattaaagat aatccagttt ctcaaactgt tactatgcac cgtactgaaa ttcttgataa 3240
ttcaggcaag gtaataggtt atgggactat tagtgaagat ggtaagtcgt acacacttaa 3300
caacgattgg attattgatg gtaactggga agctgtaaaa tcaccagact taaccactaa 3360
tggttacaag acaccacgtt ttgaaaatgg tgatatagcc aaagtagttc cagcagaaac 3420
tgtaaatgaa aatacccctg atactactgt aaatgtttac tacgaccaca atgaaatccc 3480
agttggtcca gatactccag ataagcatgg ggtagattta aacgaattaa ctaaagatgt 3540
taacgaaact gttcactacg tatacagtga tggaactaag gcagctcctg atgttgttca 3600
aacttctaag tggactagaa ctttaactat tgatgcagta actaatgaag taatttccga 3660
tggtcaatat accactgatt gggcaattcc taagggtgaa aagactgttt acgatcaagt 3720
agatactcca gtaatcaaag gctatcacgc agataagcgt caaatcaatg ctacagtggt 3780
tactcaaaat aatattgaag agactgttac ttaccaacca aatggtaaga tcattccagt 3840
tgatccaaat ggagatccaa ttccaaatgt accaacccca acttacccaa cagatccaac 3900
agacccaact aaggtagtac cggatgaacc agtaccagaa attccaggat tgattccaag 3960
tgttccaaca gtaacaccag aaaatccagg taaggatacg ccagtaccat ataccccaga 4020
agttccagct aaggatcaag tagcagttgt taactacatt gatgcagatg acaacaacgc 4080
aatcatcact agctcaggca acttaactgg taaagctggc gaaaagattg actacagtac 4140
tgcttcaacg attgaagaac tagaaaacaa gggttacgta cttgtaagtg atggcttccc 4200
agcaggagca acgtttgata atgatgacaa tacaactcaa atctacacag ttgtattgaa 4260
gcacggtaca gttccagtaa caccagaaaa cccaggcaaa ccaggtgaac caattaatcc 4320
taacgatcca gatggtccta agtggccaga aggcactggt gaaaatggtg taaagagaac 4380
aggaactcaa actattcact acgaaggagc aggcgacaag actcctagtg atgatgtaca 4440
aacattcgac ttcactaaga agatgctggt agataaggta actggtaaga tcattgatgg 4500
aggcgaatgg aatgtaacta gtcacacatt tggctacaag gatactccag taattgaagg 4560
ctaccacgca gataagcgta atgccggagg atctgtggta acaccagatg acttgaacaa 4620
gactatcaca gtaagttacc aaccaaatgg taagatcatt ccagttgatc caaatggaga 4680
tccaattcca aatgtaccaa ccccaactta cccaacagat ccaacagacc caactaaggt 4740
agtaccggat gaaccagtac cagaaattcc aggattgatt ccaagtgttc caacagtaac 4800
accagaaaat ccaggtaagg atacgccagt accatatacc ccagaagttc cagctaagga 4860
tcaagtagca gttgttaact acattgatgc agatgacaac aacgcaatca tcactagctc 4920
agacaactta actggtaaag ctggcgaaaa gattgactac agtactgctt caacgattga 4980
agaactagaa aacaagggtt acgtacttgt aagtgatggc ttcccagcag gagcaacgtt 5040

tgataatgat gacaatacaa ctcaaatcta cacagttgta ttgaagcacg gtacagttcc 5100

agtaacacca gaaaacccag gcaaaccagg tgaaccaatt aatcctaacg atccagatgg 5160

tcctaagtgg ccagaaggca ctggtgaaaa tggtgtaaag agaacaggaa ctcaaactat 5220

tcactacgaa ggagcaggcg acaagactcc tagtgatgat gtacaaacat tcgacttcac 5280

taagaagatg ctggtagata aggtaactgg taagatcatt gatggaggcg aatggaatgt 5340

aactagtcac acatttggct acaaggatac tccagtaatt gaaggctacc acgcagataa 5400

gcgtaatgcc ggaggatctg tggtaacacc agatgacttg aacaagacta tcacagtaag 5460

ttaccaacca aatggtaaga tcattccagt tgatccaaat ggagatccaa ttccaaatgt 5520

accaacccca acttacccaa cagatccaac agacccaact aaggtagtac cggatgaacc 5580

agtaccagaa attccaggat tgattccaag tgttccaaca gtaacaccag aaaatccagg 5640

taaggataca tcagtaccat ataacccagt taaggatcca gataaggtaa ctacagtaga 5700

aggtaaacaa attattcact ttgtagatgg cgataataac aagccactta aggatccaaa 5760

tattcaaact tatgaattca agattactaa tggtgtgcca aacgaatcca gtcatacctt 5820

tactctagtt aatgtaccgg tgattccagg atatgttgca gaaattaagt ctgctggtgg 5880

aaagacggta actccagata agcctcttgc agaagtaaca gttgtttacc ataagatcgg 5940

taagatcgtt ccagttgatc caaatggaaa tccaattcca aatgtaccaa ctccaagcta 6000

cactaacgat ccgacagatc caactaaggt agtaccaaat gaaccggtgc cagctattcc 6060

tggaaagaca ccagataaga cgacagtaac tccagttgat ccaactaagg atacacctgt 6120

tgtttacaag gatgataaag taccacctgc tccaaataca caaaaagctg tagttaactt 6180

tattgatgtt aatactggaa aaattatcag tacttctggt attttaagtg gtcgcccagg 6240

agaagatatt aataagttat tctcgtctgc tgaagtaatt aagcaattag aggctgcagg 6300

ttacgaagtt gtttataatg catttgacgg cgatggtgtg actaagtact ttgacaatga 6360

tggcaatacg gtacaacaat ttactattgc tttgaaactg aaagaaaaag ctaagacgcc 6420

agatcccgtt cttcctgatc cagaagttcc tgtagaaaag gaaactcgat ctgaacaacc 6480

agttaagcaa aatgttgctg ttcctactcc agaaaaacct gtagaaaaga agactactga 6540

caataaggaa gtcttaccgc aaacaggtgc tgatagtaat gaagcggcta ctattttagg 6600

tgccgtagca acagcaattg gcatgacaag cttgattggc gcaaagagac gcaagaaaga 6660

tgataaataa ttttttaaat taaatatctt ttttcaaaat tagctagtaa aaaggcgttg 6720

agacttgatt ctcagcgcct ttttttaata cattctattt ataatagtgg atccatataa 6780

gaaaagacat acaatatata aagtaattca cgttaacaag ggggcatgaa atatatggat 6840

aatttaagtg agaaagttgc atcagtagct gtaaaatata cggatttaga taataatctg 6900

gctgaacttt ttacttctga 6920

<210> 2
<211> 6420
<212> DNA
<213> Lactobacillus johnsonii

<400> 2
atgctatcta agaataacta ccaagaacgc ttaagaaaaa tggatgacaa acaagaacgt 60

ttttcaatcc gtaaattttc tgtcggtgca gcttctgttt tagtcggaac agctgtatta 120

actatgcaaa atgttcaaac agcacgcgcc gatactactt caaatactac aactaagaca 180

acagatttat cttcagaagc tgaagaacaa aacaagcaaa atgcttatga taaaactctt 240

aatgaggcac aacctacaac taagcctgca gatgctcctg tggagagtca agatagtaag 300

gtggcctctt ttagtgtgcc taaaaatgaa ggaacatttg tagaaaaaac actaacgcct 360

aatattactg aagagacaga ggcaatgcag gataaggctg agttagctca agtaaatagt 420

gaggataagt cagctgaaaa caataaaact aatgtaaagg aagaaagtca gactgttgaa 480

gagaatcaaa tggataagcg atcagctgaa gcgaatatat cggatacaga aaacacatta 540

gtaagtaaca caactttaaa tgtccctaag acaactccag ctactacacg cttttcagca 600

acagcactaa gtgagagcaa atttcaagtc aatgctccac gcgtgggcca agatactcct 660

gatacacaag ttaagaaaaa taattataaa cttgtaacca atgctactgc acttcaacaa 720

gcaattaata ctggtgttgc aggtgttaat attgatagaa gtattgatgc ttctaatgtt 780

gatttggcta ttagaaatac ttttacaatt gttggtctta acgatgcagc agcacttaat 840

ttaggtcaaa aatctcttaa taattccggt aatttaactc tccaagatat taagattaat 900

ggatcaatct ctggtagcgg tacggtaaat attaagggaa atgttacttc taatgtaaat 960

agtactaatt cttctgttcc aactcaggat caatataatg ctcaaaatta tactggtagt 1020

agaactaact ttaagaactc taacattgct gctaggaacg taaatattga aaatggtgct 1080

tccttaacta ttaacagttc tgaaattaat gatggaatta acttagctga tggcggtact 1140

gttaaagttg gcgataatgc tactttaaat gttaacctga ctaatacatc aactactgct 1200

acccgttacc atgttgctgg tgtgtttgcc aagaatggtg gtaactttat tagtggttac 1260

aagtcaaatg ttaattttaa tacaggatta ggccaagcta ttgcgatcgg agcaacccga 1320

ccaacaagta ctgacgccga tagatacggt ggttatggta cacgctcaag aaatgatggt 1380

ccaacattag ttcaattagg tgactcatcg acttttaact ttactggacg tgacggtatt 1440

atcttaggta ataatgctaa ctttatttct ggagagaatt caaatgttca ttttgaaaac 1500

aagggacgag gagttgcttt agatttagct aataactcaa atatcgaaat ttctaaacat 1560

tccattactt ctttccattc tgttggcaag acaggaacgt ctggtagctt tgatggctat 1620

aactatattg gtgttaacga aggtggtaat attactgttg acgaatatgc taccttccgt 1680

gtaattcttg aaggtcgtgg cgataatcca tgggatgacg taatttcctt agattctaga 1740

aatgctaaca caaatgctgc tttcacttct aagacaggag caattgttga cattcgtgat 1800

gataatacta acttttacgc agaacttatt tcattcccac tagggagtgc aaactcacgc 1860

attgatattc aagatcccct aatgcttaac ctgcaacgct actctaatgg tggagcaacg 1920

actggttgga tggctactgg tggtgacaaa atcaacacaa cttcggcaca atatactgct 1980

aacttaattt atatgagtgg taataaggga gtatttagtg ttagtggtgg ggattacgat 2040

ccatctaatc ctaatagttc cggctttgtt gtttaccaac aaattaagtc agatggctca 2100

aaacaaatct ggctcaatgt aaatagcgtt gatattccaa tgaatggatt ccaaactaaa 2160

gatatttgga ataatcaagc aaacccagac gtatcaattg caggtaatgg tttaactgca 2220

ggtattaaag cgaaccaagt tcataacttt gatgggtctc cactaactgg tccgaatgca 2280

ccatactatg gaatttcaac ccaacgtgct agtcatcaaa tttggattcc tcatagaaca 2340

tcattggaag ttactggtga acacaagagt actattaagt acgttgatga aaatggaaaa 2400

gaaatttttc cagaaaatac tcaatcatta gatatgaaac gtagtctgat acttgatatt 2460

acacaagata agattcaaga tattcagaat tatgctttaa gtcatactgc agatgaaact 2520

ttagagtaca ttaaaaacag tcaagcagta gttcaagatt caggttggga tttcactgat 2580

gctaatggta aagctgttat tgatccatat agtacaattg actcacctaa attggctggc 2640

tatgctgcaa cgatccaatc aacaaatgtt tcaactttaa aggttggagc agatgcttct 2700

gcagtaactg ctaaatttat ggttgaacca tctcaagata tggttcaaaa tggcgaatta 2760

tcaactgctt acaagcaaaa tggcactact ggaattcctg cagattatgt aacagtagtc 2820

gtttataata aacaagcaac ttatcaggca ggtaaaactg atagtaagag tgttgttcgt 2880

gatattaact acttagatgg taagacaggc aaaaagattc cagctaacct aattaaagat 2940

aatccagttt ctcaaactgt tactatgcac cgtactgaaa ttcttgataa ttcaggcaag 3000

gtaataggtt atggactatt tagtgaagat ggtaagtcgt acacacttaa caacgattgg 3060

attattgatg gtaactggga agctgtaaaa tcaccagact taaccactaa tggttacaag 3120

acaccacgtt ttgaaaatgg tgatatagcc aaagtagttc cagcagaaac tgtaaatgaa 3180

aatacccctg atactactgt aaatgtttac tacgaccaca atgaaatccc agttggtcca 3240

gatactccag ataagcatgg ggtagattta aacgaattaa ctaaagatgt taacgaaact 3300

gttcactacg tatacagtga tggaactaag gcagctcctg atgttgttca aacttctaag 3360

tggactagaa ctttaactat tgatgcagta actaatgaag taatttccga tggtcaatat 3420

accactgatt gggcaattcc taagggtgaa aagactgttt acgatcaagt agatactcca 3480

gtaatcaaag gctatcacgc agataagcgt caaatcaatg ctacagtggt tactcaaaat 3540

aatattgaag agactgttac ttaccaacca aatggtaaga tcattccagt tgatccaaat 3600

ggagatccaa ttccaaatgt accaacccca acttacccaa cagatccaac agacccaact 3660

aaggtagtac cggatgaacc agtaccagaa attccaggat tgattccaag tgttccaaca 3720

gtaacaccag aaaatccagg taaggatacg ccagtaccat ataccccaga agttccagct 3780

aaggatcaag tagcagttgt taactacatt gatgcagatg acaacaacgc aatcatcact 3840

agctcaggca acttaactgg taaagctggc gaaaagattg actacagtac tgcttcaacg 3900

attgaagaac tagaaaacaa gggttacgta cttgtaagtg atggcttccc agcaggagca 3960

acgtttgata atgatgacaa tacaactcaa atctacacag ttgtattgaa gcacggtaca 4020

gttccagtaa caccagaaaa cccaggcaaa ccaggtgaac caattaatcc taacgatcca 4080

gatggtccta agtggccaga aggcactggt gaaaatggtg taaagagaac aggaactcaa 4140

actattcact acgaaggagc aggcgacaag actcctagtg atgatgtaca aacattcgac 4200

ttcactaaga agatgctggt agataaggta actggtaaga tcattgatgg aggcgaatgg 4260

aatgtaacta gtcacacatt tggctacaag gatactccag taattgaagg ctaccacgca 4320

gataagcgta atgccggagg atctgtggta acaccagatg acttgaacaa gactatcaca 4380

gtaagttacc aaccaaatgg taagatcatt ccagttgatc caaatggaga tccaattcca 4440

aatgtaccaa ccccaactta cccaacagat ccaacagacc caactaaggt agtaccggat 4500

gaaccagtac cagaaattcc aggattgatt ccaagtgttc caacagtaac accagaaaat 4560

ccaggtaagg atacgccagt accatatacc ccagaagttc cagctaagga tcaagtagca 4620

gttgttaact acattgatgc agatgacaac aacgcaatca tcactagctc agacaactta 4680

actggtaaag ctggcgaaaa gattgactac agtactgctt caacgattga agaactagaa 4740

aacaagggtt acgtacttgt aagtgatggc ttcccagcag gagcaacgtt tgataatgat 4800

gacaatacaa ctcaaatcta cacagttgta ttgaagcacg gtacagttcc agtaacacca 4860

gaaaacccag gcaaaccagg tgaaccaatt aatcctaacg atccagatgg tcctaagtgg 4920

ccagaaggca ctggtgaaaa tggtgtaaag agaacaggaa ctcaaactat tcactacgaa 4980

ggagcaggcg acaagactcc tagtgatgat gtacaaacat tcgacttcac taagaagatg 5040

ctggtagata aggtaactgg taagatcatt gatggaggcg aatggaatgt aactagtcac 5100

acatttggct acaaggatac tccagtaatt gaaggctacc acgcagataa gcgtaatgcc 5160

ggaggatctg tggtaacacc agatgacttg aacaagacta tcacagtaag ttaccaacca 5220

aatggtaaga tcattccagt tgatccaaat ggagatccaa ttccaaatgt accaacccca 5280

acttacccaa cagatccaac agacccaact aaggtagtac cggatgaacc agtaccagaa 5340

attccaggat tgattccaag tgttccaaca gtaacaccag aaaatccagg taaggataca 5400

tcagtaccat ataacccagt taaggatcca gataaggtaa ctacagtaga aggtaaacaa 5460

attattcact ttgtagatgg cgataataac aagccactta aggatccaaa tattcaaact 5520

tatgaattca agattactaa tggtgtgcca aacgaatcca gtcatacctt tactctagtt 5580

aatgtaccgg tgattccagg atatgttgca gaaattaagt ctgctggtgg aaagacggta 5640

actccagata agcctcttgc agaagtaaca gttgtttacc ataagatcgg taagatcgtt 5700

ccagttgatc caaatggaaa tccaattcca aatgtaccaa ctccaagcta cactaacgat 5760

ccgacagatc caactaaggt agtaccaaat gaaccggtgc cagctattcc tggaaagaca 5820

ccagataaga cgacagtaac tccagttgat ccaactaagg atacacctgt tgtttacaag 5880

gatgataaag taccacctgc tccaaataca caaaaagctg tagttaactt tattgatgtt 5940

aatactggaa aaattatcag tacttctggt attttaagtg gtcgcccagg agaagatatt 6000

aataagttat tctcgtctgc tgaagtaatt aagcaattag aggctgcagg ttacgaagtt 6060

gtttataatg catttgacgg cgatggtgtg actaagtact ttgacaatga tggcaatacg 6120

gtacaacaat ttactattgc tttgaaactg aaagaaaaag ctaagacgcc agatcccgtt 6180

cttcctgatc cagaagttcc tgtagaaaag gaaactcgat ctgaacaacc agttaagcaa 6240

aatgttgctg ttcctactcc agaaaaacct gtagaaaaga agactactga caataaggaa 6300

gtcttaccgc aaacaggtgc tgatagtaat gaagcggcta ctattttagg tgccgtagca 6360

acagcaattg gcatgacaag cttgattggc gcaaagagac gcaagaaaga tgataaataa 6420

<210> 3
<211> 7130
<212> DNA
<213> Lactobacillus johnsonii

<400> 3
atttatttaa taatttaaac agttatttat ttgtaaatca atgttaattt tttgctacat 60

tattagtacg atttggaaga aagcagtaaa tactgtacat ttgatagtta tttttttgta 120

taaaaaagca ttgaacttta atataaattg gatataatat taaaataaaa atatatcaaa 180

ttaaatatat tcttatttta ttataagtta gttacaaggg tcagttaatc aattctttaa 240

ggaggattta aatgaaaaag aaaagagaaa aatatacttg gttattactc agtacagctc 300

ttataactag tggtcaaata ctaggtggag gagaacaaat tataaaggca agtgtagaaa 360

gccaaactaa cagcgttaaa aaatctaaga cagctgtaga acattctacg acagctctct 420

cgagacaagc tgttgaggcc caacttgccg ctcaaggtgt taattttgaa cgattaacac 480

ccgaggaaca gcaagaggtc tatgtagatg tgattgtcca actcgaggct ctaccagctt 540

ctgaaaatgg atcaattgat tcacaaacag caagtagagc agaaattgag caagcatcga 600

ataaagtaat tgctgctcaa tctggaatta aggatgaagt tcaaaaaatt acgaatcaag 660

ccattgataa aagttatggg tatgtagtta atggttttgc aacaaaagca aaagttggag 720

atattaaaaa attaagagag attaaaggtg taaaatcagt tactttagct aaggtatatt 780
ttgcagccga tacctcagca aataacatgg ctaatgtttc gacggtttgg tcaaactatc 840
aatataaggg tgaaggaaca gttgtttcaa ttattgatac gggaattgat ccaaaccata 900
aagatttacg attaagtgat gaatctaagg ttaaattgac tgctaaggat attgatggat 960
ttactgaaaa tagtggatat ggacgctact ttaccagtaa agttccattt ggacataatt 1020
attcagataa taacgatatt attacggatg atgatccaaa ggaacaacat gggatgcacg 1080
ttgcgggaat tgttgctgct aatggtactg ggaagaattc agcaagctca gtagttggag 1140
ttgcgcctga agcacaatta cttgcgatga aggctttttc taattcagat agctcctcca 1200
caaccgattc aacaagcgta atcggagcag ttgatgactc agctaaattg ggagcagacg 1260
tccttaatat gtcattagga tctgtatctg gtgaacaaac tgaggatgat ccagaaatcg 1320
cggctgtaga aaaagctgtt aagcatggaa cagcagccgt aatttcagcc ggaaactctg 1380
gaacttcaac ctctaatcaa gagggagtta ataaagactt ttatggtaat cctgatatgg 1440
aaactatcgg aagtcctggt actagtagaa gtgctacaac tgttgcatct gctgaaaata 1500
ctaaggttac taccgatggg atgactgttt ctaccgctga tggcaaaaag atatttggac 1560
caagtgtaac gcaactttct ccaaatacat ctcacgatgc tttcgatagt aaaaaattct 1620
atattgtaaa agatgcatct ggaaaactag gaatgggaac tcctagtcaa tatacagctg 1680
atgtaaaagg aaaagttgca gttgtttcgc gaggtgaaat tacattcact gataaacaaa 1740
aatatgccca agcagctggc gccgcaggac ttattattgt aaataatgct ggaggaaata 1800
cgccattaac gtcagtttta tacaatgaag gttttcctac agctggttta tcaactgacg 1860
atggtaataa gcttgttgct tatgttgaag ctcatccaga tgaattatta cgagtaaacg 1920
ttgaagttca accattaaac aatgttattc gtgaagaaga tttaatgtct agctttacgt 1980
cttatggacc agtatctgat ctatcattta agcctgatat tacagctccc ggaggaaata 2040
tttggtctct ccaaaataat aatggttata tcaatatgtc aggtacatca atggcttcac 2100
catttattgc tggatcacaa gctttacttg tacaagctat gaatgataag acaggtaagt 2160
tttatgaaac ataccaaaag atgtctggaa gtgaacgtgc agctttaata aaaaatattc 2220
agatgaatac tgctaatatt gaagttgacg ttgatcatgg ctcagttatt gaatcacctc 2280
gtagacaggg agccggctta gtcaatgtag aggctgcaat taatgctatt ttgcataatc 2340
cttcaactgt tagtggaagt aatggttatc ctggtgttga attgaaagat ttccaggatc 2400
gccaacatca atttactatt aaatttacaa atcggaccaa taaagacatt gaatatggtt 2460
taaatgagaa cggtaagttt tctgatgtat atacttcaga aaccgatcca aaaacaggtg 2520
tcttattcga aaagaagatt gatggtgctt cacttacacc aagtgaaaag atagttgttc 2580

cagcaaactc tactaaagaa gtaactatta acttatcatt acctgataac tttaaagaaa 2640

atcaatatgt agaaggattt atggcattta ctgggtccga taactcacat ttaaagattc 2700

catacatggg attctttggt gattgggctg cacctgctat ttttgatggt ttaaacggat 2760

tagcatttaa cccaggaaat aataacctcg gaactattgt gactgcagga aataaaaatg 2820

gagcagttgg ttatgccggc ttaaaccaag atgaagatgg aaattataga gttgatccag 2880

atgcaattgc tttatctaca gcagatggag catctgtatc gtgggtgaga ccacaatact 2940

tcttgtttag aaatgctaat gatgttaagg ctgaaatctt aaatcaagat ggtgaagtaa 3000

ttaatacgct agtttcattg gcacatgtta ctaagtcata ttgggcagca agtagtcaaa 3060

gatatgctaa gtttaattat gcacctgctt gggatggtac atatttcaat caacaaacaa 3120

ataagaccga aaaagtacca gatggaactt atacttatcg cgtgactggt acggttgatg 3180

gtactaagaa gcagcaacat tatgatatta aagttaaagt tgatagtgtg aaaccagaag 3240

ttaaaaactt gaaattagga tcacataagg atcaaactgg aaaagtatca tatgttctta 3300

aagcagaagc gaaagataac ttcagtggat taaatggaca agcaaatact tatgtaaatg 3360

gagaacttaa tagaagtgtc gcatatgata ttgttggttc atctagtgat ggttatcaaa 3420

agattgaagt tccacttagc gatgagcaag ttaaaactct tagggctgga aaaaatgatt 3480

tagctattgc tgtatttgat aatgctacta acgctggtac taattcaggg acatcaaata 3540

aaccaggtga aattaacttt ggactaatta ttgataataa cttgccgcaa aagatcacaa 3600

cggtaagcga tggttatgat atgactgatg attcatatac tatttcaggt acatatcccg 3660

agaaagtata tggtacatat acagataaag atggaaaaga acacgatctt aacataagtt 3720

atgatgaagc tagtgaacgg tttgttacta agttgccatt atcagtaagt gactatgaca 3780

ccaatgttaa attctatgct gatgaagaac atgaaacact aattactcaa aaacgtatta 3840

atgttagtct tgttccacca aaacttgaaa gtcttaaagt agatgatcaa gaaacatata 3900

ctggaaatga agaagctaaa ctaagtcaaa cttctgaaga cacagtagaa gtatcaggaa 3960

aagttagtga tgatactgat aaagttgctg taaaagttgc aggtaagact tatagtgcta 4020

aaccaactaa ggaacacacc tttaaagtta aagttccagt aagctacggc gaaaatacaa 4080

tgaatattgt tcttactgac aaagatggga atagttcatc tgttaagcaa attgttaagt 4140

caagtgatcg tggtaagact gttgtttccg caaaggatgt tacctttgat aatggaatta 4200

agtttggaac aactagcgtt aataccgaga ccgaaaacta cgatcctaag actggtaagt 4260

taactttaac tggaaaagta aatcgcccaa ctactactgt tagaattggt gatcatacag 4320

ttaaagttaa agcagatgga acatttaaac tagtacttga tcttggtaag catggagcta 4380

aagttttccc agttttaatt ggtgatacta ctgttaatga tactgttcaa gaacgcttaa 4440

cattctacgt agattcaaat aatccagaat taacccttaa ccaagagaag gatcaatcag 4500

gttatgtacc tgtttataca aataaagaag aatttaagct tcaaggtaca attagtgatg 4560

attatccata ttatagtttg ctcataaatg ataataatgt tgatgcaaat tgggatgatg 4620

ttgattacaa cggtaataag aatctgaaga agagctttag ccattctgtt aaacttaaag 4680

aaggcaaaaa tacttttaat gttgtagtag tcgacaacaa tgataaccgc agtgaagtcc 4740

aaactttagt tgtgtactat aagaaagctc agaaattggc ctcaccgcaa attactgcta 4800

caacagcgtc agataaaaaa tcagtaactg taactggaaa ggctaaagat ggcaacgttt 4860

tatacagtac tgataatgga aataaataca atgttcttcc agaagatggt gtaactgtca 4920

aaaataatgg caagcttcta tttaagactg tagataaata cggtaatgaa tccgaagtag 4980

ttgagtatga tgtcaaaact attggaaaag aagagagtac tgtagataaa agcgttgcac 5040

aagcaagaaa agatttaaga aagaagcttg atcaagcacg tgctttagga aatacaggta 5100

agtacacaca tgaatcagct aaaaaattag ctcaagctcg ccaagaagca agtaaagcgt 5160

tgaaagataa gaatgctact ctccaagaat tgaagcaggc aagcgaacaa ctagaagaag 5220

caattaagaa tttggttgaa aagcctgtag atcaaaataa agataaagac aaagtagaag 5280

ataaagactc acaagtaaac gcacttaaag aaaaattgga agagacagtt aaggctggtg 5340

aaaaattcga taaggataaa tatactgatg attcagtaga aaaagtaact aaagctcttg 5400

atgaagctaa agtagtttta gctaataaag atgcaaattc aactgatgtt caagatgcga 5460

tagattctat cgttaatgca acgaaatctc ttaaggaaaa gcaagtatct ccagaaaaaa 5520

caactcagca agaagataca ccaaaggaaa ataaagatac cgaggaagtc cttgctgcaa 5580

agaatgcttt aaaagaaaag gcagacaaac ttagtcattt ggataccacg aagtatactt 5640

ctgagagtgc agaaaacttg agtaatgcac tgaagaaagt aaatcaggtt ttgactaata 5700

aagatgctaa taaagctcaa gttcaagaag cacttgatag tttatctcaa gctgagaaag 5760

atttagttga aaaaacagaa tcaaataaag atgttgaaac agctaaagaa caattaagag 5820

aagaacttaa taagcataaa gatgaagata aatctcagta tactgatgat tctgctaaag 5880

tgaaggataa tgctgaaaag atttcagaag gagtactcga ttcaaaagat gcacaagcag 5940

acgaggttaa taaagcaaaa gatagtcttg ttgaagcaga aaaaggctta gttaagaaag 6000

aagaaaataa gcctgctgaa tctgatactc aagaagttga taaagctaga gaggcattag 6060

aacaagaggt taataagaat gccaatgtaa acttagacgg atatacgcca gaatcgcaag 6120

ataaattcaa agaaatattg aatggtgtcc gggatgtttt aaatgataaa aatgcatctg 6180

caagcagctt agaaaaagca gaaaaagtac ttgaaactgc tacaggtgtt ttaacacagg 6240

tagaacatca agtggaattg cctaaggttg aacaaccagt tgtaactcct gagaagaagc 6300

agactgaaca agaagaagct aaaaaatctg aatctagttc tgtatcaacg tctaaagatg 6360

aagtgaaaga accagaagaa aagaaacaag atgctcactc tgtatctgat aaaggaactg 6420

gaacttcagt agagaagaag gggcaaacac ctgcagatgc tctatcacaa gtagaacatc 6480

aggcagaatt atctaagaat gaacaagaag aagctaaaaa atctgaatct cattctgcat 6540

caacgtctaa agatgaagtg aaagaaccag aagaaaagaa acaagatgct gaatctgtat 6600

ctgataaaga gagcagcaca ttagtaggga aaaagcatca agttgcaaga agtgctcaag 6660

aatcttcaaa gaagaatgct gataataagg tgccaactca taaaaatagc aataaagctc 6720

aaaatactac aaatagttca actcgaagta ctaagagtag caataataaa gaattaccta 6780

agactggaga aagagaaaca ttctttggat tactagttac aggaataact gccttatttg 6840

ctagtttagg aacagtactg agaataaaga ataaaaagta gttatttaag gaatatgttt 6900

ttaattaaaa tccattttct atgagcaaag aaaaatgcgt taagatattt aatcttagcg 6960

cattttttat tcgcttaaag ttaagctatt ctttcttctt ccgatcaccg attaatccaa 7020

gtaagaagct aataccagct actgctaatc cgattagttg agcttggttt tcttttcttc 7080

cagtttgtgg aagctcttcc ttcttcttgt tactttcaac gttatttgca 7130

<210> 4
<211> 6630
<212> DNA
<213> Lactobacillus johnsonii

<400> 4
   atgaaaaaga aaagagaaaa atatacttgg ttattactca gtacagctct tataactagt 60
   ggtcaaatac taggtggagg agaacaaatt ataaaggcaa gtgtagaaag ccaaactaac 120
   agcgttaaaa aatctaagac agctgtagaa cattctacga cagctctctc gagacaagct 180
   gttgaggccc aacttgccgc tcaaggtgtt aattttgaac gattaacacc cgaggaacag 240
   caagaggtct atgtagatgt gattgtccaa ctcgaggctc taccagcttc tgaaaatgga 300
   tcaattgatt cacaaacagc aagtagagca gaaattgagc aagcatcgaa taaagtaatt 360
   gctgctcaat ctggaattaa ggatgaagtt caaaaaatta cgaatcaagc cattgataaa 420
   agttatgggt atgtagttaa tggttttgca acaaaagcaa aagttggaga tattaaaaaa 480
   ttaagagaga ttaaaggtgt aaaatcagtt actttagcta aggtatattt tgcagccgat 540
   acctcagcaa ataacatggc taatgtttcg acggtttggt caaactatca atataagggt 600
   gaaggaacag ttgtttcaat tattgatacg ggaattgatc caaaccataa agatttacga 660
   ttaagtgatg aatctaaggt taaattgact gctaaggata ttgatggatt tactgaaaat 720
   agtggatatg gacgctactt taccagtaaa gttccatttg gacataatta ttcagataat 780
   aacgatatta ttacggatga tgatccaaag gaacaacatg ggatgcacgt tgcgggaatt 840
   gttgctgcta atggtactgg gaagaattca gcaagctcag tagttggagt tgcgcctgaa 900
   gcacaattac ttgcgatgaa ggctttttct aattcagata gctcctccac aaccgattca 960
   acaagcgtaa tcggagcagt tgatgactca gctaaattgg gagcagacgt ccttaatatg 1020
   tcattaggat ctgtatctgg tgaacaaact gaggatgatc cagaaatcgc ggctgtagaa 1080
   aaagctgtta agcatggaac agcagccgta atttcagccg gaaactctgg aacttcaacc 1140
   tctaatcaag agggagttaa taaagacttt tatggtaatc ctgatatgga aactatcgga 1200
   agtcctggta ctagtagaag tgctacaact gttgcatctg ctgaaaatac taaggttact 1260
   accgatggga tgactgtttc taccgctgat ggcaaaaaga tatttggacc aagtgtaacg 1320
   caactttctc caaatacatc tcacgatgct ttcgatagta aaaaattcta tattgtaaaa 1380
   gatgcatctg gaaaactagg aatggaaact cctagtcaat atacagctga tgtaaaagga 1440
   aaagttgcag ttgtttcgcg aggtgaaatt acattcactg ataaacaaaa atatgcccaa 1500
   gcagctggcg ccgcaggact tattattgta aataatgctg gaggaaatac gccattaacg 1560
   tcagttttat acaatgaagg ttttcctaca gctggtttat caactgacga tggtaataag 1620
   cttgttgctt atgttgaagc tcatccagat gaattattac gagtaaacgt tgaagttcaa 1680
   ccattaaaca atgttattcg tgaagaagat ttaatgtcta gctttacgtc ttatggacca 1740
   gtatctgatc tatcatttaa gcctgatatt acagctcccg gaggaaatat ttggtctctc 1800
   caaaataata atggttatat caatatgtca ggtacatcaa tggcttcacc atttattgct 1860
   ggatcacaag ctttacttgt acaagctatg aatgataaga caggtaagtt ttatgaaaca 1920
   taccaaaaga tgtctggaag tgaacgtgca gctttaataa aaaatattca gatgaatact 1980
   gctaatattg aagttgacgt tgatcatggc tcagttattg aatcacctcg tagacaggga 2040
   gccggcttag tcaatgtaga ggctgcaatt aatgctattt tgcataatcc ttcaactgtt 2100
   agtggaagta atggttatcc tggtgttgaa ttgaaagatt tccaggatcg ccaacatcaa 2160
   tttactatta aatttacaaa tcggaccaat aaagacattg aatatggttt aaatgagaac 2220
   ggtaagtttt ctgatgtata tacttcagaa accgatccaa aaacaggtgt cttattcgaa 2280
   aagaagattg atggtgcttc acttacacca agtgaaaaga tagttgttcc agcaaactct 2340
   actaaagaag taactattaa cttatcatta cctgataact ttaaagaaaa tcaatatgta 2400
   gaaggattta tggcatttac tgggtccgat aactcacatt taaagattcc atacatggga 2460
   ttctttggtg attgggctgc acctgctatt tttgatggtt taaacggatt agcatttaac 2520
   ccaggaaata ataacctcgg aactattgtg actgcaggaa ataaaaatgg agcagttggt 2580
   tatgccggct taaaccaaga tgaagatgga aattatagag ttgatccaga tgcaattgct 2640
   ttatctacag cagatggagc atctgtatcg tgggtgagac cacaatactt cttgtttaga 2700
   aatgctaatg atgttaaggc tgaaatctta aatcaagatg gtgaagtaat taatacgcta 2760
   gtttcattgg cacatgttac taagtcatat tggcagcaag gtagtcaaag atatgctaag 2820
   tttaattatg cacctgcttg ggatggtaca tatttcaatc aacaaacaaa taagaccgaa 2880
   aaagtaccag atggaactta tacttatcgc gtgactggta cggttgatgg tactaagaag 2940
   cagcaacatt atgatattaa agttaaagtt gatagtgtga aaccagaagt taaaaacttg 3000
   aaattaggat cacataagga tcaaactgga aaagtatcat atgttcttaa agcagaagcg 3060
   aaagataact tcagtggatt aaatggacaa gcaaatactt atgtaaatgg agaacttaat 3120
   agaagtgtcg catatgatat tgttggttca tctagtgatg gttatcaaaa gattgaagtt 3180
   ccacttagcg atgagcaagt taaaactctt agggctggaa aaaatgattt agctattgct 3240
   gtatttgata atgctactaa cgctggtact aattcaggga catcaaataa accaggtgaa 3300
   attaactttg gactaattat tgataataac ttgccgcaaa agatcacaac ggtaagcgat 3360
   ggttatgata tgactgatga ttcatatact atttcaggta catatcccga gaaagtatat 3420
   ggtacatata cagataaaga tggaaaagaa cacgatctta acataagtta tgatgaagct 3480
   agtgaacggt ttgttactaa gttgccatta tcagtaagtg actatgacac caatgttaaa 3540
   ttctatgctg atgaagaaca tgaaacacta attactcaaa aacgtattaa tgttagtctt 3600
   gttccaccaa aacttgaaag tcttaaagta gatgatcaag aaacatatac tggaaatgaa 3660
   gaagctaaac taagtcaaac ttctgaagac acagtagaag tatcaggaaa agttagtgat 3720
   gatactgata aagttgctgt aaaagttgca ggtaagactt atagtgctaa accaactaag 3780
   gaacacacct ttaaagttaa agttccagta agctacggcg aaaatacaat gaatattgtt 3840
   cttactgaca aagatgggaa tagttcatct gttaagcaaa ttgttaagtc aagtgatcgt 3900
   ggtaagactg ttgtttccgc aaaggatgtt acctttgata atggaattaa gtttggaaca 3960
   actagcgtta ataccgagac cgaaaactac gatcctaaga ctggtaagtt aactttaact 4020
   ggaaaagtaa atcgcccaac tactactgtt agaattggtg atcatacagt taaagttaaa 4080
   gcagatggaa catttaaact agtacttgat cttggtaagc atggagctaa agttttccca 4140
   gttttaattg gtgatactac tgttaatgat actgttcaag aacgcttaac attctacgta 4200
   gattcaaata atccagaatt aacccttaac caagagaagg atcaatcagg ttatgtacct 4260
   gtttatacaa ataaagaaga atttaagctt caaggtacaa ttagtgatga ttatccatat 4320
   tatagtttgc tcataaatga taataatgtt gatgcaaatt gggatgatgt tgattacaac 4380
   ggtaataaga atctgaagaa gagctttagc cattctgtta aacttaaaga aggcaaaaat 4440
   acttttaatg ttgtagtagt cgacaacaat gataaccgca gtgaagtcca aactttagtt 4500
   gtgtactata agaaagctca gaaattggcc tcaccgcaaa ttactgctac aacagcgtca 4560
   gataaaaaat cagtaactgt aactggaaag gctaaagatg gcaacgtttt atacagtact 4620
   gataatggaa ataaatacaa tgttcttcca gaagatggtg taactgtcaa aaataatggc 4680
   aagcttctat ttaagactgt agataaatac ggtaatgaat ccgaagtagt tgagtatgat 4740
   gtcaaaacta ttggaaaaga agagagtact gtagataaaa gcgttgcaca agcaagaaaa 4800
   gatttaagaa agaagcttga tcaagcacgt gctttaggaa atacaggtaa gtacacacat 4860
   gaatcagcta aaaaattagc tcaagctcgc caagaagcaa gtaaagcgtt gaaagataag 4920
   aatgctactc tccaagaatt gaagcaggca agcgaacaac tagaagaagc aattaagaat 4980
   ttggttgaaa agcctgtaga tcaaaataaa gataaagaca aagtagaaga taaagactca 5040
   caagtaaacg cacttaaaga aaaattggaa gagacagtta aggctggtga aaaattcgat 5100
   aaggataaat atactgatga ttcagtagaa aaagtaacta aagctcttga tgaagctaaa 5160
   gtagttttag ctaataaaga tgcaaattca actgatgttc aagatgcgat agattctatc 5220
   gttaatgcaa cgaaatctct taaggaaaag caagtatctc cagaaaaaac aactcagcaa 5280
   gaagatacac caaaggaaaa taaagatacc gaggaagtcc ttgctgcaaa gaatgcttta 5340
   aaagaaaagg cagacaaact tagtcatttg gataccacga agtatacttc tgagagtgca 5400
   gaaaacttga gtaatgcact gaagaaagta aatcaggttt tgactaataa agatgctaat 5460
   aaagctcaag ttcaagaagc acttgatagt ttatctcaag ctgagaaaga tttagttgaa 5520
   aaaacagaat caaataaaga tgttgaaaca gctaaagaac aattaagaga agaacttaat 5580
   aagcataaag atgaagataa atctcagtat actgatgatt ctgctaaagt gaaggataat 5640
   gctgaaaaga tttcagaagg agtactcgat tcaaaagatg cacaagcaga cgaggttaat 5700
   aaagcaaaag atagtcttgt tgaagcagaa aaaggcttag ttaagaaaga agaaaataag 5760
   cctgctgaat ctgatactca agaagttgat aaagctagag aggcattaga acaagaggtt 5820
   aataagaatg ccaatgtaaa cttagacgga tatacgccag aatcgcaaga taaattcaaa 5880
   gaaatattga atggtgtccg ggatgtttta aatgataaaa atgcatctgc aagcagctta 5940
   gaaaaagcag aaaaagtact tgaaactgct acaggtgttt taacacaggt agaacatcaa 6000
   gtggaattgc ctaaggttga acaaccagtt gtaactcctg agaagaagca gactgaacaa 6060
   gaagaagcta aaaaatctga atctagttct gtatcaacgt ctaaagatga agtgaaagaa 6120
   ccagaagaaa agaaacaaga tgctcactct gtatctgata aaggaactgg aacttcagta 6180
   gagaagaagg ggcaaacacc tgcagatgct ctatcacaag tagaacatca ggcagaatta 6240
   tctaagaatg aacaagaaga agctaaaaaa tctgaatctc attctgcatc aacgtctaaa 6300
   gatgaagtga aagaaccaga agaaaagaaa caagatgctg aatctgtatc tgataaagag 6360
   agcagcacat tagtagggaa aaagcatcaa gttgcaagaa gtgctcaaga atcttcaaag 6420
   aagaatgctg ataataaggt gccaactcat aaaaatagca ataaagctca aaatactaca 6480
   aatagttcaa ctcgaagtac taagagtagc aataataaag aattacctaa gactggagaa 6540
   agagaaacat tctttggatt actagttaca ggaataactg ccttatttgc tagtttagga 6600
   acagtactga gaataaagaa taaaaagtag 6630

<210> 5
<211> 14710
<212> DNA
<213> Lactobacillus johnsonii

<400> 5
atactattta taggaagatt taattctaaa gaaagtcata taaattatct aagagtatag 60

aataagaaaa gattacatgt tactatatca cttgtaatta attttagata atagtacatt 120

aacagtgatg acgagcattt atgttaagta gtaaaaggga tgataaaagt aaggttaaat 180

agatttaaaa tttaagctaa tttaaataaa taacttgtct aattaaacaa acttaaattt 240

tgaagttgga ggaaaatatg gtttcaaaaa acaatattga aatgaaatat aaaaaaatga 300

gagacgaaaa acaacgtttc tcaattcgta aatttagtgt gggagctgca tccgttttaa 360

ttgggctaag ttttagttta tataacggac aacaagcatt tgctgatact gttgataatg 420

gcaataagtc tgttgtagca aatgcacaag ataaagaaca aacagacaat aaacaaaata 480

ctgatactaa tcctcaaaat aacgaagata aaacaaataa taatcaaact caaaatacag 540

taactgctga tcttgatcac agtactgttg gggctagtta caaggctgat actactaatg 600

ctagcgaagc taacaagagt agtgataagt cagatgaaaa gcaagctgca actactgaaa 660

ctgctaaaac tgaagaagct gctactaagg aagtagaagc aaagaatact gcaaatgatg 720

atgtagctaa gaaaactgaa gaggcagctg ctaagacaga tactaaggtc gatgttaata 780

aaacagtagt agaaaatgta aagactgaag ctgctactcc taatgtagag actaggaaca 840

ttgaaactca aactgcaaag actgatgaag aaaaagtaga aacagcagtt gacaatactt 900

cttctgattt aaaaaacgca gtagagaatg caaatactga agtttcaact aatacattta 960

acgtaaataa agcagttcga aatgcattat tccttagtgc aaataatctt gctgaacgta 1020

aggtagcagc tatagcactt ccagctgaag cagaagatcc taatgccgta acagttagtg 1080

atgcgaaggg atttattgat gcaattcaaa atgggactgc tactactatt aatgttgcaa 1140

gtgatcttaa tttagcagac caaacagata agaagtatac agaaattaat atcaagaata 1200

agcgtaatat tgtaattcaa tcagataatc cagaagaaaa acgtacaatt gatttctctg 1260

gatattcatt tgatatgaat acagagaatt ccgtaacctt taaagatttg aatatttatg 1320

ctcgtagtta ctggggcctt gtttataatg ctggtggcta cacatttaat aatgttaact 1380

ttactggttc acaacttatc tatactaaac caagtattaa ttcaacctta acatttaaga 1440

ataatattac tgcaaattct gtttcctctt atgtaggacc acttgatggt aagacccgcg 1500

atactcaaca aaacggtggg cagcaaattc ttcaatttga aggtggaaca aatcaaatta 1560

tttttgatga aaacagtaat gtgaccttaa caacagaaga tgctaatgct cttgaaattg 1620

atggtgggac cacaacaatt gatgtaaaag acggtgcaaa tgtagcaatt aatccacatt 1680

ctaagggtaa tcctgaaagt cgtaatggaa ttggaactgg gtatgttgct cgtgctattg 1740

ctgccaatgc aaagatgaca attaatattg atccaaattc aactttaact attaatactg 1800

aaaaggtaga tgacgataaa gatgttgcag gagcattata tttaaattct gatgctgctc 1860

ttaatgtaaa tggtaaatta gttatcaatt caaatggcac accaagtgca agcaaaagca 1920

atggtgttcc agtatacatc aatggaagtg caaatattaa tgttggaaat ggtggtagtt 1980

tcgtattaga tgctacaaat cttggagact atagttcaag tctaatttct gttaatggta 2040

aggggacggt taagttagat cctcactcta ctttcaagat tagcggtgac ggtactggag 2100

ctgtaactgc aattaatctt tcaagcggta gtactttcac ttctgaccaa ccagattcat 2160

ttactattga tctctccgca aataccagta atggtaagtc actaattaaa aatggtacta 2220

ttaatttcag tcgtgttaag acggttactg atggaaacga atcacagcct ttgggcaaaa 2280

ttgatgtaac ttatgataga aatggtaatg ctacttctta cacaattact gcacaagata 2340

aaaatacggt taatcaagta gcagagggac tggctaacaa gagcttgatt agcttagtta 2400

aagctggtga ggacgttacc ctatctaacc ttcatttaag taagaataat gtattgactg 2460

gtactgtagc ttcaagtggt agtgacaatc caatttatgt aactgttacc gttggtgggg 2520

taagcactaa tgtgccagta gtaggacatt acactgtgta caccaaaact aataaaacag 2580

taacttcaaa caatgttgat tacgctgcac aaactgcttc aactggtggt aatttctcta 2640

ttgatttatc aaaacttgca agtagcttga ctaatgatgc acaagttaca gttactgcta 2700

caaaagattt cgttgaatcc gctcaaacta aaagcgtagc agcattacgt gcacttaata 2760

ctactactct taaagaatta gtagatgctg ctccagcaga agaagcaaag ccaagttact 2820

acaatgctac tgaagaagct caaaaggcct atactgatgc aatttcaaaa ggtaatgaca 2880

ttcttgccga tccaaataat tacgatcaag ttgacgttga taatgccgta actgccattc 2940

aaaatgctca gaaggcttta actggtaaag aaactgataa gaaagccctt caaaaagcag 3000

ttgatgacgc atcaaaagta gaatcaagcg ataactacac taatgcggat gctaggcttc 3060

aaaatgatta ccaagctgca attaaagcag gtcaaggtat tttaagtaaa gaaaatgcta 3120

ctcaaactga ggtagataat gcattaacta ccattaataa tgcaaaagat gctttaaatg 3180

gggatgctaa gaaagctgca agtaaggaag ctcttcaaaa ggcagttgat gaagcaccta 3240

ctgtaaaatc agatgatgca gcatactaca atggttctgc tgaagctaaa actgcttatg 3300

acgatgcaat cagtgcaggt caaatagttt tagataatcc agatgcaact gctactcaaa 3360

ttactgatgc tttaaatgcc attaatactg ctaagggtaa tcttaagggt gaagcaactg 3420

ataaagcagc gcttcaaaca gcagttgaca attctgcaac tgtaaaagaa agcaataact 3480

atactaatgc agaccaaact caaaagactg catatgataa agcagttaca gctgcacaaa 3540

cagttttaga taaaactaat gcaactcaag cagaagtgaa ccaagctctt caagatcttg 3600

aaactgcaaa tcgtaaccta aatggtgatg ctaagactga agctgcaaac aaggctgcct 3660

tagaagctgc tgttaaggat gcgccaaatg taagaaatac acctgcatac tacaacggta 3720

gtgaagaaac ccaaacagca tataataatg caattactgc tggtcaaaca gttttgaatg 3780

aagcaaatcc atctgcaagt gaagttaaga atgctttaga tgcaattaat gcagcaaaag 3840

ataaccttaa aggtaaggct actaacactg aagctttgga aacggcttta accaatgcta 3900

ataatgctaa ggaaactggt aattacacta atgccgacca agcaaatcaa gaagccctta 3960

ataatgcgat tattgctggt caagagattt taaagaatac tagtgcaact caggcgcaag 4020

tagacagtgc agcaaaggct attactgatg caattagtgg tttgaatggg gatactaacc 4080

ttgcaaatgc taagactgct gctactgaag atattcaaaa ggctttagat actaagacta 4140

ccgaaatcac tgacgctact aatattgatc aagctactaa ggatcaatta attgccgatg 4200

ctaagaaggc agcagaggat gctaatactg cgattaatca atcaaccgat actgatacag 4260

ttaatactgc taaaactgaa gggattgcta acatcaataa ggtaacagtt ccaagtttag 4320

ccgatgctaa aactaaggca gccaaagaaa ttgatcaagc attaactgat aagaccaagg 4380

aaatcaataa cgctgaaaat attgatcaaa caacaaaaga tcagttaatt aaagaagcaa 4440

ctgatgcagc aaatacagca aaggatgcta ttgaaaaatc tacaactaat gacgaagcaa 4500

ctaaagcagg ccaagctggt gtagatgcaa ttaccaatgt aaaggtacca agcgttactg 4560

atagtcaaaa tgctgcaaaa gaagtaattg atgatgctct taacgctaag actaaggaaa 4620

tcaacgatgc taacaatatt gatcaaacaa ctaaggatca attaatcaaa gaggcaaccg 4680

atgcagctaa taaggctaaa gaagccattg ataaagcaac tacagctgac gcaattaaga 4740

ctgcacaaga tgaaggaaca actaatatta ataatgttac tgttccaagc ttggaggatg 4800

ccaagaaggc agctaataaa gcagtagatg atgctcttac tgctcaaact gaggtaatta 4860

ataaagctaa taatcttagt gatgcagaaa agaaagattt aattgatcaa gtaactgctg 4920

aagccaataa ggctaaagaa aatattgaaa ctgctacaac aaacaatgag gcagctcaag 4980

ctggtcaagc tggtgtggat gcaattaaga agattgttcc aacaagctta gatactgtga 5040

aatctgatgc aaataaagca attgacgatg ctctaactaa gaaattagaa gagatcaatt 5100

ccgctaatga tttaactact gatgaaaaga ctgcattgac acaagaagcc aatacagctg 5160

cagataaggc aaaagaagaa attaccaatg caacgactaa tgatgctgta attgaagcac 5220

aaaataatgg tgttactgca attgatggca ttaaagttcc aactgaatca gcagtcaaag 5280

aagcagctaa gcaggcagta gccgacgcag caactgcaaa gaatcaagca attgatgctt 5340

caaacttaac agatgaagaa aaagctgcct tgaagcaaaa ggttactgat gcccaaaatg 5400

ctgcagatca agcaattgac aatgcaacaa caaatgcggc agtaactgaa gcacaaacca 5460

atggtattaa ggcaattaat ggtattgaag taactacctc aactgttaaa gaagcagcta 5520

agaaggcagt agccgacgca gcaactgcaa agaacaatgc aattgatgct tcaaacttaa 5580

cagatgaaga aaaagctgcc ttaaagcaaa aagttacaga tgcccaaaat gctgcagatc 5640

aagcaattga caacgcaaca acaaatgtag cagtgactga agcacaaacc aatggtgtca 5700

atgcaattaa tggtattgaa gtaccaacta catcagctac taaggaacac gccataactg 5760

accttaatgc ggctgttgat gatgctaaga aagcaattga tcaagatagt aacttgactg 5820

atgaagagaa gcaagcagct aaggatcaaa ttgataggga tgctactaaa gcacaagaag 5880

caatcaataa tgccaaaact aacgatgatg ttaagaaggc gggtgacgct ggtacattag 5940

caattgataa agacgttgct aatgcagcca ttgataacgc agttgctggg aagaaggccg 6000

aaatttccaa gaccccatta acagatgaag aaaagactgc tttgaacaat gaagtagatc 6060

aaaaagctca agaggctaaa gaagcaatca ataatgccac tactccagaa gcagtaacaa 6120

ctgctcaaga cagtggtgtt aataacatta atgaaacttc agtgccatct gaatcagcag 6180

caaaacaagc agctaaggaa gctgtggcta aggcagttga tgaaaagaat gcagcaattg 6240

attcttcaaa cttaaccgaa gaagaaaaag aagctttgaa gcaaaaggtt actgaagcac 6300

aaacagctgc agatcaagca attgataatg caactaccaa cgctggagta actgaagcac 6360

aaaccaatgg tgtgaatgca attaatggta ttgaagtacc gaataaatca gatgctaaag 6420

aacaagccat aactgatctt aatactgctg ttgataatgc taagaaagca attgatcaag 6480

atagcaactt aacagatgaa gaaaaacaag cagctaaaga tcaaattgat tctgatgcta 6540

agaatgctca agatgccatc aacaatgcaa agactaatga tgatgttaag aaggcagctt 6600

atgacggtac attagcaatt gataaagacg ttgctaatgc agccattgat aacgcagtcg 6660

ctggtaagaa agcagaaatt tctaactcat cattaacaga cgaagaaaag actgctttaa 6720

acaatgaagt cgatcaaaag gcaaatagtg ctaaagatgc aattaacaat gcaactactc 6780

cagaagcagt aactactgca caaggaaatg gcattaagaa tatcaatgca acttccgtac 6840

caactacatc aacagctaaa gaagcagcta agaaggcagt agcagaagct gcagaagcca 6900

agaacagtgc aatcgactcg tcaaacttaa cagatgaaga aaaagctgca ttgaaacaaa 6960

aagttactga agcacaaaat ggcgcagatc acgctatcga taatgcaact accaacgcag 7020

ccgtaactga agctaaagac aatggtatta gtgcaattga cggcattgaa gttccaatta 7080

catcagctac taaggaacaa gccataactg atcttaatac ggctgttgat gacgctaaga 7140

agacaattga tcaagataac aacttaactg atgaacaaaa acaagcagct aaagatcaaa 7200

ttgattctga tgctaagact gcccaagatg ccatcaacaa tgctaagact gataatgacg 7260

taaacaatgc agttaacagt ggtaaggttg ccattaacaa agatgttgct aatgcagcta 7320

tcgacaatgc agttgctggt aagcttaagg agatccaaga tccattaact actgaagaaa 7380

aacaagctta cactgattta attaactctg aagcaactaa tgcaaaacaa aatattgcta 7440

atgcaactac tgttgaagca gtaactactg cacaaacaaa tggtgtaaat gaaattacta 7500

ataccgaaat tccaactact tcttccgcca aagaaaaggc aattgctgcc atcaatgatg 7560

ctcttcaaac gaagaccgac gaaatcaaca acgcatctaa tatcaacact caagaaaaga 7620

ctgatttaat taatcaagca actgaagcag ccaatgcagc taagaacaat atcaacaacg 7680

ccactactaa tgctgatgtt gatactgcac aaataaaggg tgaaaaagca attgctgatg 7740

taacagttcc aaacttgtct gatgttaaga aagaaagtat tgatctaatt aacaaagctt 7800

tagatgccaa gactaatgaa atcaataatg cttctaacct ttctcaagat gaaaagcaag 7860

gtttgatcaa tgatgcaact aatgccgcaa ctgaggcaat taacaacgtc aaccaagctc 7920

aaacaaatga cgatgctaaa gctgctgcaa ctactggcgt tcaaaacatc gaaaacatta 7980

ctattccaac tcttgatgaa gcgaagaaga acgctaacca agctattgat gatgcattga 8040

actctaaagt aaatgagatt aacaatgctt ctaacttgaa tgaaactgaa aagcaaaaat 8100

tagttgatca ggcaaatgaa gcagcaacta ctgctaagaa taatgttgaa aaggccacta 8160

ccaatgatga tgctcgcgat gcagctaatg ctggaattga taacattaaa ggtattacct 8220

ttacttcact agaagatgct aaaaatgcag caaacactgc aattgacaat gctttgcaag 8280

ttaagacaga tgaaattaat aatgcatcta acttaagtac tgaagaaaag caagacttaa 8340

tcaatcaagc aagtgaagta gctaaaaatg caaaagacaa tatcaacaat gcaactacca 8400

atgatgctgt aactgaagca caaaataagg gtattgcaga tattgccaat gtgactgttc 8460

caagcttaga tcaagttaaa caagatgcaa tcaatgcaat taagcaagtt caagatgcta 8520

agaataagca aatctctgct gcatctaact taagcactga agagcaaaaa gaattaagtg 8580

atcaagtaga taaaattgct aacgatgcaa ttgctcagat taatgattca tcaactacta 8640

caaatgatgc agttactgca acccgtgatg atgcaatcaa gcaaattact gatctattta 8700

ttccaactct agacggtgca caaacagatg ctcttaatgc aattgaaagt gctaagaatg 8760

ctaaactaaa tgacattaac aacgctactc acttaactga tcaagaaaag caagctttag 8820

ttgatcaaac caataaagtg gctgatgatg caactaagga aattaaggct gctcaaacaa 8880

atgacgcagt caagagtgca gaaacagctg gcttagataa cattaataac attgctattc 8940

caactttagt tcagaagcaa caagaagcta ttgaagaatt gaatgtagca cgtgatgcta 9000

agagtagtgc aattgataat gctactgatt taactactga tgaaaagaac agcttgaaag 9060

ataaagttca agcagaatac agtaacgcgg tatcaaatat caccagtgca acaactgatg 9120

aagcagtaac aactgctaaa gaaaacggca tcgcagcaat taaagacatt caaattccaa 9180

ctaagtcacc tgctaaggaa caagcaacaa gtgaccttaa tactgcagtt gatgaagcta 9240

agaacgcaat cgatcaagac aacaacttga ctgatgaaga gaagcaagca gctaaggatc 9300

aaattgattc tgatgctaag aaggcgcaag aagcaattga taatgctacg actgatgatg 9360

aggttaatag tgctgttgat aatggtaagc ttgccattga taaagatatt gctaatgcag 9420

caatcgacaa tgcagttgct ggtaagaaag cagaaatttc taaatcatcc ttaacatctg 9480

aagaaaaagc tgatttaaac aatgaagtcg atcaaaaggc taaagatgct aaagaagcaa 9540

ttaaggctgc cactactcca gaaactgtaa ctactgccca agaaaatggt attaagaata 9600

tcaatgatac taaagtacca actgagtcag cagctaaaga agctgctaag aaggcagtgg 9660

cagaagctgc agaagccaag aacaatgcaa tcgattcttc agacttaaca aatgaagaaa 9720

aggctgcatt gaagcaagaa gttgctgatg cacaaaacgc agcagataga gcaatagatg 9780

ctgcaactac aaacactgca gtaactgaag cgcaagacaa tggtattaaa gcaattgaga 9840

atgttactgt acctaccgaa tcagcagcca aagaaactgc taagaaggca gtagcagagg 9900

ctgcagaagc taaaaacaat gcaattgatt cttcaaactt aacatctgaa gaaaaagctg 9960

cattgaaaca agaagtagca gaagcacaaa atgctgccaa taccgcaatt gataacgcaa 10020

ctaccaatgc cgcagtaacc gaagcagaag ataatggtat taaagcaatt aattctattg 10080

aagtaccaac taagtcagat gctaaggaac aagcaacaag cgatcttaat agtgctgttg 10140

atgaagcgaa gaaggcaatc gatcaagata gcaacttgac tgatgaagag aaacaagtag 10200

ctaaagatca aattgactct gatgctaaga aagcgcaaga agcaattgat actgctaaga 10260

ctaacgatga cgttaagaag gcaatcgatg acggtacatt agcaattgat aaagacgttg 10320

ctaatgcagc aatcgataac gcagttgcag gcaagaaggc tgaaatttct aagtcaccat 10380

taactgatga agaaaagact gctttgaaca atgaagtaga tgaaaaggca aatagtgcta 10440

aagatgctat caataaggca actactccag agggagtaac tgaagcacaa aatagtggta 10500

ttaaatccat tgatgatgtc aatgtaccaa ctgagtcagt agctaaggaa gcagctaaga 10560

aggcagtagc cgaagctgca gaagctaaga acaatgcaat tgattcttca aacttaacag 10620

acgaagaaaa agctgcattg aagcaagaag ttagtgatgc acaaactgct gctaatactg 10680

ccattgataa tgcaattacc aacgcagaag taactgaagc tgaagacaat ggtgttaaga 10740

caattaatgg tattgaagta ccaactaaat caactactaa agaacaagca acaaatgatc 10800

ttaacaacga agttgaaaat gctaagaagg caattgatca agataacaac ttaactgatg 10860

agcaaaaaca agcagccaag gatcaaattg attcggacgc taagaaagct caagatgcca 10920

tcaacaatgc taagaataat gatgatgtta agaaggcagt tgatgatgga aaactagtaa 10980

ttgacaaaga tgttgccaac gctgccatcg ataacgcagt tgctggcaag aaggatgaaa 11040

tttctaaatc accattaact gatgaagaaa aagctgcttt aaacaatgaa gttgatcaaa 11100

aggcagaaga agctaaggaa gcaattaagg ctgccactac tccaggagct gtaactactg 11160

cacaagaaaa tggtattaag aatatcaatg agactgaagt accaactgag tccgtagcta 11220

aggaagcagc taagagggca gtagctgaag ctgcagacgc aaagaataaa gcaattgatt 11280

cttcaaactt aacagacgaa gaaaaagctg cattgaagca agaagttagt gatgcacaaa 11340

ctgctgctaa tactgccatt gataatgcaa ctaccaatgc cgcagtaacc gaagcagaag 11400

ataatggtat taaagcaatt aattctattg aagtaccaac taagtcagat gctaaggaac 11460

aagcaacaag cgatcttaat agtgctgttg atgaagcgaa gaaggcaatc gatcaagata 11520

gcaacttgac tgatgaagag aaacaagtag ctaaagatca aattgactct gatgctaaga 11580

aagcgcaaga agcaattgat actgctaaga ctaacgatga cgttaagaag gcaatcgatg 11640

acggtacatt agcaattgat aaagacgttg ctaatgcagc aatcgataac gcagttgcag 11700

gcaagaaggc tgaaatttct aagtcaccat taactgatga agaaaagact gctttgaaca 11760

atgaagtaga tgaaaaggca aatagtgcta aagatgctat caataaggca actactccag 11820

agggagtaac tgaagcacaa aatagtggta ttaaatccat tgatgatgtc aatgtaccaa 11880

ctgaatcagt agctaaagaa gcagctaaga aggcagtagc cgaagctgca gaagctaaga 11940

acaatgcaat tgattcttca aacttaacag acgaagaaaa agctgcattg aagcaagaag 12000

ttagtgatgc acaaactgct gctaatactg ccattgataa tgcaattacc aacgcagaag 12060

taactgaagc tgaagacaat ggtgttaaga caattaatgg tattgaagta ccaactaaat 12120

caactactaa agaacaagca acaaatgatc ttaacaacga agttgaaaat gctaagaagg 12180

caattgatca agatagcaac ttgactgatg aagagaaaca agtagctaaa gatcaaattg 12240

actctgatgc taagaaagct caagatgcca tcaacaatgc taagactaac gatgacgtta 12300

agaaggcaat cgatgatggt acattagcaa ttgataaaga cgttgctaat gcagctatcg 12360

acaatgcagt tgctggtaag cttaaggaaa ttaatgattc attaacaaat gaagaaaaac 12420

aagcttacac tgatctaatc aataatgaag cagataatgc aaaacaaaag attgcagaag 12480

caactactcc tgaagaagta actcgtgctc aagaagaagg agttaagaac attaataata 12540

ttaacgttcc aactacttct ccagctaagg acgcagcgaa tgctgcaatt gatcaggctt 12600

tgaagaacaa gaaggatgaa atcaataatg caactaacat ttcttctgaa gaaaagactg 12660

atttaattaa acaagcaact gaagctgcaa atattgctaa agacaacatc aacaatgcaa 12720

ctactaattc agaagttgaa actgctcaag ttgatggtga aaaagctatt gcggacgtta 12780

ctggtccagg tttatctgac atcaagaagg aaagtattga tcttatcaac aaagccttaa 12840

atgaaaagca agatgaaatc aataacgctt ctaacttgag tcaagatgaa agcacagagc 12900

ttattgatca agcaaagaag attgctactg aagcaattaa tgaaatcaat aatgctcaaa 12960

ctaatgatga agctaaggaa gctgcagata ctggagttaa gaacattgaa aatgtttcaa 13020

ttccaagtat tgaagatgct aagaagaatg caactcaagc aattgatgat gctttgaatt 13080

ctaagaagaa tgaaattaac aacgcatcta acttaactga ttcagaaaag actgatttaa 13140

tcaatcaagc aactgaaatt gccaatgctg ctaaggatgc tatcaacagt gcaacgacta 13200

atactgcagt tgaagctgcc gaatataaag gtgttgctga tattaacaac attcacttca 13260

ctaacttaga cgattctaag aaggcagcaa atagtgcgat tgaagatgct ttgaccacta 13320

agaaggatga aattaataat gcatctaact tgagtgattc agaaaaggct aagttaatca 13380

atcaagcaac tgaaattgcc aatgctgcta aggctgcaat taacaatgca actactaatt 13440

cggctgttac tgcggcagaa aacaagggaa ttgaagatat tgcaaatatt aatgttccaa 13500

gtttagctga aactaaacag gcagcaattg acgcaattca gcaagttcaa aaagctaaga 13560

acagtcaaat tgaagaagca aagaatctaa gtgcagacga gcaaaagaac ttaattgatc 13620

aagtaaataa gattgctcag gacgcaatta ataagcttaa cgatccagca acaactacaa 13680

atgaagttat tactgacact cgtgataaag caattgatca aattactaac ttgttcattc 13740

caaccttaga tagtgtccaa aaagatgctc aggaagcaat taactctgct caggaaacta 13800

agattgacga aattaataag gctgataatt taactgatca aatgaagcaa aacttaattg 13860

accaagtaga tcaagttgct gataaggcta ctaaggccat taataatgct caaacaaacg 13920

atgatgttaa agaagcagaa atcgagggct tagaagatat tgatagcatt aaagttccaa 13980

gcttagttga aagtaaggat gatgctatta aggaaatcaa tgatgctttg aagaagaaga 14040

ctgatgaaat caatgcagct gatcttgatc aaaagcaaaa agacgaatta atcagtcaaa 14100

ttactgatat tgctactgag actaagacca aggtattcaa tgctacaact aatgcagaag 14160

ttgatgctga agccgaagct ggtattaaag ccatcgaagc tgtgaagatt ccagctcgta 14220

ctgctgacaa cagcaatact gagagtgaaa gtaaggaaca aactgtaatt acaaattcag 14280

ttcaacctaa gagaaatgct gttcatcata agaatggcac tccagtaaac aagaaggcta 14340

ctttaccaca aactggtaag aaggataatc taactttaac gttagcagga gctgcacttc 14400

ttggtttagc aggtgtattc tcattgtttg gattaggaga caagagaaaa aagaataaat 14460

agtcaacatc gttgatatga aaaagagcga tactccctaa agtggaatat cgcctttttt 14520

gtttggaaat gtaataaagg cagaagcaca attaaatgct tctgccttta ttacatgatt 14580

ttattccgct gtgactggga ataaaaatga aattactggt ataactacat aaagaatcat 14640

ggcaagtcta ggaagaacaa acgatagccc aatgagaaca atatttaaaa ataaggtgac 14700

agcaaccttt 14710

<210> 6

<211>  14206
<212>  DNA
<213>  Lactobacillus johnsonii

<400>  6
atggtttcaa aaaacaatat tgaaatgaaa tataaaaaaa tgagagacga aaaacaacgt      60

ttctcaattc gtaaatttag tgtgggagct gcatccgttt taattgggct aagttttagt     120

ttatataacg gacaacaagc atttgctgat actgttgata atggcaataa gtctgttgta     180

gcaaatgcac aagataaaga acaaacagac aataaacaaa atactgatac taatcctcaa     240

aataacgaag ataaaacaaa taataatcaa actcaaaata cagtaactgc tgatcttgat     300

cacagtactg ttggggctag ttacaaggct gatactacta atgctagcga agctaacaag     360

agtagtgata agtcagatga aaagcaagct gcaactactg aaactgctaa aactgaagaa     420

gctgctacta aggaagtaga agcaaagaat actgcaaatg atgatgtagc taagaaaact     480

gaagaggcag ctgctaagac agatactaag gtcgatgtta ataaaacagt agtagaaaat     540

gtaaagactg aagctgctac tcctaatgta gagactagga acattgaaac tcaaactgca     600

aagactgatg aagaaaaagt agaaacagca gttgacaata cttcttctga tttaaaaaac     660

gcagtagaga atgcaaatac tgaagtttca actaatacat ttaacgtaaa taaagcagtt     720

cgaaatgcat tattccttag tgcaaataat cttgctgaac gtaaggtagc agctatagca     780

cttccagctg aagcagaaga tcctaatgcc gtaacagtta gtgatgcgaa gggatttatt     840

gatgcaattc aaaatgggac tgctactact attaatgttg caagtgatct taatttagca     900

gaccaaacag ataagaagta tacagaaatt aatatcaaga ataagcgtaa tattgtaatt     960

caatcagata atccagaaga aaaacgtaca attgatttct ctggatattc atttgatatg    1020

aatacagaga attccgtaac ctttaaagat ttgaatattt atgctcgtag ttactggggc    1080

cttgtttata atgctggtgg ctacacattt aataatgtta actttactgg ttcacaactt    1140

atctatacta aaccaagtat taattcaacc ttaacattta agaataatat tactgcaaat    1200

tctgtttcct cttatgtagg accacttgat ggtaagaccc gcgatactca acaaaacggt    1260

gggcagcaaa ttcttcaatt tgaaggtgga acaaatcaaa ttatttttga tgaaaacagt    1320

aatgtgacct taacaacaga agatgctaat gctcttgaaa ttgatggtgg gaccacaaca    1380

attgatgtaa aagacggtgc aaatgtagca attaatccac attctaaggg taatcctgaa    1440

agtcgtaatg gaattggaac tgggtatgtt gctcgtgcta ttgctgccaa tgcaaagatg    1500

acaattaata ttgatccaaa ttcaacttta actattaata ctgaaaaggt agatgacgat    1560

aaagatgttg caggagcatt atatttaaat tctgatgctg ctcttaatgt aaatggtaaa    1620

ttagttatca attcaaatgg cacaccaagt gcaagcaaaa gcaatggtgt tccagtatac    1680

atcaatggaa gtgcaaatat taatgttgga aatggtggta gtttcgtatt agatgctaca    1740

aatcttggag actatagttc aagtctaatt tctgttaatg gtaaggggac ggttaagtta 1800
gatcctcact ctactttcaa gattagcggt gacggtactg gagctgtaac tgcaattaat 1860
ctttcaagcg gtagtacttt cacttctgac caaccagatt catttactat tgatctctcc 1920
gcaaatacca gtaatggtaa gtcactaatt aaaaatggta ctattaattt cagtcgtgtt 1980
aagacggtta ctgatggaaa cgaatcacag cctttgggca aaattgatgt aacttatgat 2040
agaaatggta atgctacttc ttacacaatt actgcacaag ataaaaatac ggttaatcaa 2100
gtagcagagg gactggctaa caagagcttg attagcttag ttaaagctgg tgaggacgtt 2160
accctatcta accttcattt aagtaagaat aatgtattga ctggtactgt agcttcaagt 2220
ggtagtgaca atccaattta tgtaactgtt accgttggtg gggtaagcac taatgtgcca 2280
gtagtaggac attacactgt gtacaccaaa actaataaaa cagtaacttc aaacaatgtt 2340
gattacgctg cacaaactgc ttcaactggt ggtaatttct ctattgattt atcaaaactt 2400
gcaagtagct tgactaatga tgcacaagtt acagttactg ctacaaaaga tttcgttgaa 2460
tccgctcaaa ctaaaagcgt agcagcatta cgtgcactta atactactac tcttaaagaa 2520
ttagtagatg ctgctccagc agaagaagca aagccaagtt actacaatgc tactgaagaa 2580
gctcaaaagg cctatactga tgcaatttca aaaggtaatg acattcttgc cgatccaaat 2640
aattacgatc aagttgacgt tgataatgcc gtaactgcca ttcaaaatgc tcagaaggct 2700
ttaactggta aagaaactga taagaaagcc cttcaaaaag cagttgatga cgcatcaaaa 2760
gtagaatcaa gcgataacta cactaatgcg gatgctaggc ttcaaaatga ttaccaagct 2820
gcaattaaag caggtcaagg tattttaagt aaagaaaatg ctactcaaac tgaggtagat 2880
aatgcattaa ctaccattaa taatgcaaaa gatgctttaa atggggatgc taagaaagct 2940
gcaagtaagg aagctcttca aaaggcagtt gatgaagcac ctactgtaaa atcagatgat 3000
gcagcatact acaatggttc tgctgaagct aaaactgctt atgacgatgc aatcagtgca 3060
ggtcaaatag ttttagataa tccagatgca actgctactc aaattactga tgctttaaat 3120
gccattaata ctgctaaggg taatcttaag ggtgaagcaa ctgataaagc agcgcttcaa 3180
acagcagttg acaattctgc aactgtaaaa gaaagcaata actatactaa tgcagaccaa 3240
actcaaaaga ctgcatatga taaagcagtt acagctgcac aaacagtttt agataaaact 3300
aatgcaactc aagcagaagt gaaccaagct cttcaagatc ttgaaactgc aaatcgtaac 3360
ctaaatggtg atgctaagac tgaagctgca aacaaggctg ccttagaagc tgctgttaag 3420
gatgcgccaa atgtaagaaa tacacctgca tactacaacg gtagtgaaga aacccaaaca 3480
gcatataata atgcaattac tgctggtcaa acagttttga atgaagcaaa tccatctgca 3540
agtgaagtta agaatgcttt agatgcaatt aatgcagcaa aagataacct taaaggtaag 3600
gctactaaca ctgaagcttt ggaaacggct ttaaccaatg ctaataatgc taaggaaact 3660

ggtaattaca ctaatgccga ccaagcaaat caagaagccc ttaataatgc gattattgct 3720
ggtcaagaga ttttaaagaa tactagtgca actcaggcgc aagtagacag tgcagcaaag 3780
gctattactg atgcaattag tggtttgaat ggggatacta accttgcaaa tgctaagact 3840
gctgctactg aagatattca aaaggcttta gatactaaga ctaccgaaat cactgacgct 3900
actaatattg atcaagctac taaggatcaa ttaattgccg atgctaagaa ggcagcagag 3960
gatgctaata ctgcgattaa tcaatcaacc gatactgata cagttaatac tgctaaaact 4020
gaagggattg ctaacatcaa taaggtaaca gttccaagtt tagccgatgc taaaactaag 4080
gcagccaaag aaattgatca agcattaact gataagacca aggaaatcaa taacgctgaa 4140
aatattgatc aaacaacaaa agatcagtta attaaagaag caactgatgc agcaaataca 4200
gcaaaggatg ctattgaaaa atctacaact aatgacgaag caactaaagc aggccaagct 4260
ggtgtagatg caattaccaa tgtaaaggta ccaagcgtta ctgatagtca aaatgctgca 4320
aaagaagtaa ttgatgatgc tcttaacgct aagactaagg aaatcaacga tgctaacaat 4380
attgatcaaa caactaagga tcaattaatc aaagaggcaa ccgatgcagc taataaggct 4440
aaagaagcca ttgataaagc aactacagct gacgcaatta agactgcaca agatgaagga 4500
acaactaata ttaataatgt tactgttcca agcttggagg atgccaagaa ggcagctaat 4560
aaagcagtag atgatgctct tactgctcaa actgaggtaa ttaataaagc taataatctt 4620
agtgatgcag aaaagaaaga tttaattgat caagtaactg ctgaagccaa taaggctaaa 4680
gaaaatattg aaactgctac aacaaacaat gaggcagctc aagctggtca agctggtgtg 4740
gatgcaatta agaagattgt tccaacaagc ttagatactg tgaaatctga tgcaaataaa 4800
gcaattgacg atgctctaac taagaaatta gaagagatca attccgctaa tgatttaact 4860
actgatgaaa agactgcatt gacacaagaa gccaatacag ctgcagataa ggcaaaagaa 4920
gaaattacca atgcaacgac taatgatgct gtaattgaag cacaaaataa tggtgttact 4980
gcaattgatg gcattaaagt tccaactgaa tcagcagtca aagaagcagc taagcaggca 5040
gtagccgacg cagcaactgc aaagaatcaa gcaattgatg cttcaaactt aacagatgaa 5100
gaaaaagctg ccttgaagca aaaggttact gatgcccaaa atgctgcaga tcaagcaatt 5160
gacaatgcaa caacaaatgc ggcagtaact gaagcacaaa ccaatggtat taaggcaatt 5220
aatggtattg aagtaactac ctcaactgtt aaagaagcag ctaagaaggc agtagccgac 5280
gcagcaactg caaagaacaa tgcaattgat gcttcaaact taacagatga agaaaaagct 5340
gccttaaagc aaaaagttac agatgcccaa aatgctgcag atcaagcaat tgacaacgca 5400
acaacaaatg tagcagtgac tgaagcacaa accaatggtg tcaatgcaat taatggtatt 5460
gaagtaccaa ctacatcagc tactaaggaa cacgccataa ctgaccttaa tgcggctgtt 5520

gatgatgcta agaaagcaat tgatcaagat agtaacttga ctgatgaaga gaagcaagca 5580

gctaaggatc aaattgatag ggatgctact aaagcacaag aagcaatcaa taatgccaaa 5640

actaacgatg atgttaagaa ggcgggtgac gctggtacat tagcaattga taaagacgtt 5700

gctaatgcag ccattgataa cgcagttgct gggaagaagg ccgaaatttc caagacccca 5760

ttaacagatg aagaaaagac tgctttgaac aatgaagtag atcaaaaagc tcaagaggct 5820

aaagaagcaa tcaataatgc cactactcca gaagcagtaa caactgctca agacagtggt 5880

gttaataaca ttaatgaaac ttcagtgcca tctgaatcag cagcaaaaca agcagctaag 5940

gaagctgtgg ctaaggcagt tgatgaaaag aatgcagcaa ttgattcttc aaacttaacc 6000

gaagaagaaa aagaagcttt gaagcaaaag gttactgaag cacaaacagc tgcagatcaa 6060

gcaattgata atgcaactac caacgctgga gtaactgaag cacaaaccaa tggtgtgaat 6120

gcaattaatg gtattgaagt accgaataaa tcagatgcta aagaacaagc cataactgat 6180

cttaatactg ctgttgataa tgctaagaaa gcaattgatc aagatagcaa cttaacagat 6240

gaagaaaaac aagcagctaa agatcaaatt gattctgatg ctaagaatgc tcaagatgcc 6300

atcaacaatg caaagactaa tgatgatgtt aagaaggcag cttatgacgg tacattagca 6360

attgataaag acgttgctaa tgcagccatt gataacgcag tcgctggtaa gaaagcagaa 6420

atttctaact catcattaac agacgaagaa aagactgctt taaacaatga agtcgatcaa 6480

aaggcaaata gtgctaaaga tgcaattaac aatgcaacta ctccagaagc agtaactact 6540

gcacaaggaa atggcattaa gaatatcaat gcaacttccg taccaactac atcaacagct 6600

aaagaagcag ctaagaaggc agtagcagaa gctgcagaag ccaagaacag tgcaatcgac 6660

tcgtcaaact taacagatga agaaaaagct gcattgaaac aaaaagttac tgaagcacaa 6720

aatggcgcag atcacgctat cgataatgca actaccaacg cagccgtaac tgaagctaaa 6780

gacaatggta ttagtgcaat tgacggcatt gaagttccaa ttacatcagc tactaaggaa 6840

caagccataa ctgatcttaa tacggctgtt gatgacgcta agaagacaat tgatcaagat 6900

aacaacttaa ctgatgaaca aaaacaagca gctaaagatc aaattgattc tgatgctaag 6960

actgcccaag atgccatcaa caatgctaag actgataatg acgtaaacaa tgcagttaac 7020

agtggtaagg ttgccattaa caaagatgtt gctaatgcag ctatcgacaa tgcagttgct 7080

ggtaagctta aggagatcca agatccatta actactgaag aaaaacaagc ttacactgat 7140

ttaattaact ctgaagcaac taatgcaaaa caaaatattg ctaatgcaac tactgttgaa 7200

gcagtaacta ctgcacaaac aaatggtgta aatgaaatta ctaataccga aattccaact 7260

acttcttccg ccaaagaaaa ggcaattgct gccatcaatg atgctcttca aacgaagacc 7320

gacgaaatca acaacgcatc taatatcaac actcaagaaa agactgattt aattaatcaa 7380

gcaactgaag cagccaatgc agctaagaac aatatcaaca acgccactac taatgctgat 7440

gttgatactg cacaaataaa gggtgaaaaa gcaattgctg atgtaacagt tccaaacttg 7500

tctgatgtta agaaagaaag tattgatcta attaacaaag ctttagatgc caagactaat 7560

gaaatcaata atgcttctaa cctttctcaa gatgaaaagc aaggtttgat caatgatgca 7620

actaatgccg caactgaggc aattaacaac gtcaaccaag ctcaaacaaa tgacgatgct 7680

aaagctgctg caactactgg cgttcaaaac atcgaaaaca ttactattcc aactcttgat 7740

gaagcgaaga agaacgctaa ccaagctatt gatgatgcat tgaactctaa agtaaatgag 7800

attaacaatg cttctaactt gaatgaaact gaaaagcaaa aattagttga tcaggcaaat 7860

gaagcagcaa ctactgctaa gaataatgtt gaaaaggcca ctaccaatga tgatgctcgc 7920

gatgcagcta atgctggaat tgataacatt aaaggtatta cctttacttc actagaagat 7980

gctaaaaatg cagcaaacac tgcaattgac aatgctttgc aagttaagac agatgaaatt 8040

aataatgcat ctaacttaag tactgaagaa aagcaagact taatcaatca agcaagtgaa 8100

gtagctaaaa atgcaaaaga caatatcaac aatgcaacta ccaatgatgc tgtaactgaa 8160

gcacaaaata agggtattgc agatattgcc aatgtgactg ttccaagctt agatcaagtt 8220

aaacaagatg caatcaatgc aattaagcaa gttcaagatg ctaagaataa gcaaatctct 8280

gctgcatcta acttaagcac tgaagagcaa aaagaattaa gtgatcaagt agataaaatt 8340

gctaacgatg caattgctca gattaatgat tcatcaacta ctacaaatga tgcagttact 8400

gcaacccgtg atgatgcaat caagcaaatt actgatctat ttattccaac tctagacggt 8460

gcacaaacag atgctcttaa tgcaattgaa agtgctaaga atgctaaact aaatgacatt 8520

aacaacgcta ctcacttaac tgatcaagaa aagcaagctt tagttgatca aaccaataaa 8580

gtggctgatg atgcaactaa ggaaattaag gctgctcaaa caaatgacgc agtcaagagt 8640

gcagaaacag ctggcttaga taacattaat aacattgcta ttccaacttt agttcagaag 8700

caacaagaag ctattgaaga attgaatgta gcacgtgatg ctaagagtag tgcaattgat 8760

aatgctactg atttaactac tgatgaaaag aacagcttga aagataaagt tcaagcagaa 8820

tacagtaacg cggtatcaaa tatcaccagt gcaacaactg atgaagcagt aacaactgct 8880

aaagaaaacg gcatcgcagc aattaaagac attcaaattc caactaagtc acctgctaag 8940

gaacaagcaa caagtgacct taatactgca gttgatgaag ctaagaacgc aatcgatcaa 9000

gacaacaact tgactgatga agagaagcaa gcagctaagg atcaaattga ttctgatgct 9060

aagaaggcgc aagaagcaat tgataatgct acgactgatg atgaggttaa tagtgctgtt 9120

gataatggta agcttgccat tgataaagat attgctaatg cagcaatcga caatgcagtt 9180

gctggtaaga aagcagaaat ttctaaatca tccttaacat ctgaagaaaa agctgattta 9240

aacaatgaag tcgatcaaaa ggctaaagat gctaaagaag caattaaggc tgccactact 9300

ccagaaactg taactactgc ccaagaaaat ggtattaaga atatcaatga tactaaagta 9360
ccaactgagt cagcagctaa agaagctgct aagaaggcag tggcagaagc tgcagaagcc 9420
aagaacaatg caatcgattc ttcagactta acaaatgaag aaaaggctgc attgaagcaa 9480
gaagttgctg atgcacaaaa cgcagcagat agagcaatag atgctgcaac tacaaacact 9540
gcagtaactg aagcgcaaga caatggtatt aaagcaattg agaatgttac tgtacctacc 9600
gaatcagcag ccaaagaaac tgctaagaag gcagtagcag aggctgcaga agctaaaaac 9660
aatgcaattg attcttcaaa cttaacatct gaagaaaaag ctgcattgaa acaagaagta 9720
gcagaagcac aaaatgctgc caataccgca attgataacg caactaccaa tgccgcagta 9780
accgaagcag aagataatgg tattaaagca attaattcta ttgaagtacc aactaagtca 9840
gatgctaagg aacaagcaac aagcgatctt aatagtgctg ttgatgaagc gaagaaggca 9900
atcgatcaag atagcaactt gactgatgaa gagaaacaag tagctaaaga tcaaattgac 9960
tctgatgcta agaaagcgca agaagcaatt gatactgcta agactaacga tgacgttaag 10020
aaggcaatcg atgacggtac attagcaatt gataaagacg ttgctaatgc agcaatcgat 10080
aacgcagttg caggcaagaa ggctgaaatt tctaagtcac cattaactga tgaagaaaag 10140
actgctttga acaatgaagt agatgaaaag gcaaatagtg ctaaagatgc tatcaataag 10200
gcaactactc cagagggagt aactgaagca caaaatagtg gtattaaatc cattgatgat 10260
gtcaatgtac caactgagtc agtagctaag gaagcagcta agaaggcagt agccgaagct 10320
gcagaagcta agaacaatgc aattgattct tcaaacttaa cagacgaaga aaaagctgca 10380
ttgaagcaag aagttagtga tgcacaaact gctgctaata ctgccattga taatgcaatt 10440
accaacgcag aagtaactga agctgaagac aatggtgtta agacaattaa tggtattgaa 10500
gtaccaacta aatcaactac taaagaacaa gcaacaaatg atcttaacaa cgaagttgaa 10560
aatgctaaga aggcaattga tcaagataac aacttaactg atgagcaaaa acaagcagcc 10620
aaggatcaaa ttgattcgga cgctaagaaa gctcaagatg ccatcaacaa tgctaagaat 10680
aatgatgatg ttaagaaggc agttgatgat ggaaaactag taattgacaa agatgttgcc 10740
aacgctgcca tcgataacgc agttgctggc aagaaggatg aaatttctaa atcaccatta 10800
actgatgaag aaaaagctgc tttaaacaat gaagttgatc aaaaggcaga agaagctaag 10860
gaagcaatta aggctgccac tactccagga gctgtaacta ctgcacaaga aaatggtatt 10920
aagaatatca atgagactga agtaccaact gagtccgtag ctaaggaagc agctaagagg 10980
gcagtagctg aagctgcaga cgcaaagaat aaagcaattg attcttcaaa cttaacagac 11040
gaagaaaaag ctgcattgaa gcaagaagtt agtgatgcac aaactgctgc taatactgcc 11100
attgataatg caactaccaa tgccgcagta accgaagcag aagataatgg tattaaagca 11160
attaattcta ttgaagtacc aactaagtca gatgctaagg aacaagcaac aagcgatctt 11220

aatagtgctg ttgatgaagc gaagaaggca atcgatcaag atagcaactt gactgatgaa 11280

gagaaacaag tagctaaaga tcaaattgac tctgatgcta agaaagcgca agaagcaatt 11340

gatactgcta agactaacga tgacgttaag aaggcaatcg atgacggtac attagcaatt 11400

gataaagacg ttgctaatgc agcaatcgat aacgcagttg caggcaagaa ggctgaaatt 11460

tctaagtcac cattaactga tgaagaaaag actgctttga acaatgaagt agatgaaaag 11520

gcaaatagtg ctaaagatgc tatcaataag gcaactactc cagagggagt aactgaagca 11580

caaaatagtg gtattaaatc cattgatgat gtcaatgtac caactgaatc agtagctaaa 11640

gaagcagcta agaaggcagt agccgaagct gcagaagcta agaacaatgc aattgattct 11700

tcaaacttaa cagacgaaga aaaagctgca ttgaagcaag aagttagtga tgcacaaact 11760

gctgctaata ctgccattga taatgcaatt accaacgcag aagtaactga agctgaagac 11820

aatggtgtta agacaattaa tggtattgaa gtaccaacta aatcaactac taaagaacaa 11880

gcaacaaatg atcttaacaa cgaagttgaa aatgctaaga aggcaattga tcaagatagc 11940

aacttgactg atgaagagaa acaagtagct aaagatcaaa ttgactctga tgctaagaaa 12000

gctcaagatg ccatcaacaa tgctaagact aacgatgacg ttaagaaggc aatcgatgat 12060

ggtacattag caattgataa agacgttgct aatgcagcta tcgacaatgc agttgctggt 12120

aagcttaagg aaattaatga ttcattaaca aatgaagaaa aacaagctta cactgatcta 12180

atcaataatg aagcagataa tgcaaaacaa aagattgcag aagcaactac tcctgaagaa 12240

gtaactcgtg ctcaagaaga aggagttaag aacattaata atattaacgt tccaactact 12300

tctccagcta aggacgcagc gaatgctgca attgatcagg ctttgaagaa caagaaggat 12360

gaaatcaata atgcaactaa catttcttct gaagaaaaga ctgatttaat taaacaagca 12420

actgaagctg caaatattgc taaagacaac atcaacaatg caactactaa ttcagaagtt 12480

gaaactgctc aagttgatgg tgaaaaagct attgcggacg ttactggtcc aggtttatct 12540

gacatcaaga aggaaagtat tgatcttatc aacaaagcct taaatgaaaa gcaagatgaa 12600

atcaataacg cttctaactt gagtcaagat gaaagcacag agcttattga tcaagcaaag 12660

aagattgcta ctgaagcaat taatgaaatc aataatgctc aaactaatga tgaagctaag 12720

gaagctgcag atactggagt taagaacatt gaaaatgttt caattccaag tattgaagat 12780

gctaagaaga atgcaactca agcaattgat gatgctttga attctaagaa gaatgaaatt 12840

aacaacgcat ctaacttaac tgattcagaa aagactgatt taatcaatca agcaactgaa 12900

attgccaatg ctgctaagga tgctatcaac agtgcaacga ctaatactgc agttgaagct 12960

gccgaatata aaggtgttgc tgatattaac aacattcact tcactaactt agacgattct 13020

aagaaggcag caaatagtgc gattgaagat gctttgacca ctaagaagga tgaaattaat 13080

aatgcatcta acttgagtga ttcagaaaag gctaagttaa tcaatcaagc aactgaaatt 13140

gccaatgctg ctaaggctgc aattaacaat gcaactacta attcggctgt tactgcggca 13200

gaaaacaagg gaattgaaga tattgcaaat attaatgttc caagtttagc tgaaactaaa 13260

caggcagcaa ttgacgcaat tcagcaagtt caaaaagcta agaacagtca aattgaagaa 13320

gcaaagaatc taagtgcaga cgagcaaaag aacttaattg atcaagtaaa taagattgct 13380

caggacgcaa ttaataagct taacgatcca gcaacaacta caaatgaagt tattactgac 13440

actcgtgata aagcaattga tcaaattact aacttgttca ttccaacctt agatagtgtc 13500

caaaaagatg ctcaggaagc aattaactct gctcaggaaa ctaagattga cgaaattaat 13560

aaggctgata atttaactga tcaaatgaag caaaacttaa ttgaccaagt agatcaagtt 13620

gctgataagg ctactaaggc cattaataat gctcaaacaa acgatgatgt taaagaagca 13680

gaaatcgagg gcttagaaga tattgatagc attaaagttc caagcttagt tgaaagtaag 13740

gatgatgcta ttaaggaaat caatgatgct ttgaagaaga agactgatga aatcaatgca 13800

gctgatcttg atcaaaagca aaaagacgaa ttaatcagtc aaattactga tattgctact 13860

gagactaaga ccaaggtatt caatgctaca actaatgcag aagttgatgc tgaagccgaa 13920

gctggtatta aagccatcga agctgtgaag attccagctc gtactgctga caacagcaat 13980

actgagagtg aaagtaagga acaaactgta attacaaatt cagttcaacc taagagaaat 14040

gctgttcatc ataagaatgg cactccagta aacaagaagg ctactttacc acaaactggt 14100

aagaaggata atctaacttt aacgttagca ggagctgcac ttcttggttt agcaggtgta 14160

ttctcattgt ttggattagg agacaagaga aaaaagaata aatagt 14206

<210> 7
<211> 2320
<212> DNA
<213> Lactobacillus johnsonii

<400> 7
ggatgctcgt caacgtgaaa attaaatttg attatttaaa tattaaaaag caactcataa 60

ttgagttgct tttttgcatg tctttgtata aaaactaaag aatgtaggaa atattgatat 120

tgtaaatggg cataaaataa gttttttatg gtaaacataa tagtgaacaa acgttaatat 180

atcagcgctt tatagcaatt tagtcgaatt aaaattaaaa aatattaaaa ataattgagt 240

gaaaacattg tattatgagt ttgatgatga taatattaaa tcatcaactt gtcccaaata 300

gatggaggaa tataactatg aagaaagcca agctatttgg aagtttaact ttactttctg 360

gtgttgcttt aacattagca gcatgtggta acaattcaaa ttcaaaagta gataacccaa 420

ctaagaactt taagtcagct actccaaaga aagctgttaa aaagggtggt acagtaagtg 480

ttgcacttga atctgataca ccatttactg gtatcttttt aaatgaactt tctgatactc 540

agaatgactc tgatgctatg gcacctgcta atgaagggtt atttgatact gatgatactt 600

atcaaattaa tgacaaagga ccagctacat taaaattaga taatgataac aaaactgcta 660

ctattaaaat taagaatggt gttaagtggt cagatggtaa gcaagtaact gctaaggatg 720

ttgaatattc ttacgaaatt attgctaaca aggcaactaa gtcatcacgt tatactgact 780

cacttcaaaa tattgttggt atgagtgaat accacgatgg caaatctaac actatttctg 840

gtattgaaat gccggatggt gaaaatggaa gaaccgttgt tattcacttc aaagaaatga 900

agccaggtat gaagttcagt ggtaatggct acttctggga agctgcagct ccatatcact 960

atttgaagga tatcccattt gataagctac aatcatctga tcaagtaaga aagaagccac 1020

tattctttgg tccgtataag atgagtaagg ttgtacgtgg tcaagcagta acatttgttc 1080

ctaacaagta ctactggaga ggtacaccaa aattagataa agtaacaatt caagttttga 1140

accctaactc agcttctcaa gccattaaga gtcataagta tgatattgcc ggtgtaatca 1200

atgttcaatg gaagaacgtt gccaatacta ataatgttaa ctggatcgct aagattcctt 1260

tatcttatcg ttacctaggt tttaaggtag gaaagtggga tgcagctaaa ggcgaaaacg 1320

taatggataa gaatgcaaaa atgaataata aagcattacg tcaagctatt gcttatggta 1380

tgaatatctc cgctgtaaac aaacgttata ccaacggttt gactttccat attccaactt 1440

taattccagc acaatttggt aagtactttg ataaaaatgt taagggctac gattacaata 1500

tcaaaaaggg taatgaacta ttagataaag ctggatataa gaagaagggt aagtaccgtg 1560

ttcaacccaa cggtaagcct ttgactatcc attttgcagc tatgagtggt gactctactc 1620

aagaaccaat tattcaaaac tatatccaac aatggaagaa attaggtttg aatgttaagt 1680

taactggtgg tcgtttgatg gaaatgaact ccttctatga caaggtccaa aatgattcta 1740

aagacgtaga tatgttcatg gttgcatggt cattatcttc agaaccatca ccaaatgact 1800

tatacaatgc aaaagcacca tacaacttta gtcgttttgt aactgctaag aacactaagt 1860

tacttcaaga aatggattca caaaaggcat ttaatactaa ttaccgtgtt aagaaattcc 1920

atgaatggca aaaatatatg gatgatgaag cctacgttgt tccagtatct aacagttata 1980

gtattgatgc agttaacagc aagattacag gttactcatc taaaccatca gcagcaaaca 2040

gcttatggta caatgtagga attgcaaaat aatattagat aaatttcttc ataaagatcg 2100

tcttaagtga cggccttttt tttatttaag ttgttttaat cgttttcagt gctaaaatta 2160

aatatatcaa cttgtccaat gttattggag gaaaaattaa tgaagaaagg caaattaatt 2220

ggtacattaa ctttgctttc aggtgtagct ttaactttag cagcctgtgg taatagtaat 2280

agcaataata ctagtcatcc taatttcaag gagtcaactc 2320

<210> 8
<211> 1818

<212>  DNA
<213>  Lactobacillus johnsonii

<400>  8
atgagtttga tgatgataat attaaatcat caacttgtcc caaatagatg gaggaatata      60

actatgaaga aagccaagct atttggaagt ttaactttac tttctggtgt tgctttaaca     120

ttagcagcat gtggtaacaa ttcaaattca aaagtagata acccaactaa gaactttaag     180

tcagctactc caaagaaagc tgttaaaaag ggtggtacag taagtgttgc acttgaatct     240

gatacaccat ttactggtat ctttttaaat gaactttctg atactcagaa tgactctgat     300

gctatggcac ctgctaatga agggttattt gatactgatg atacttatca aattaatgac     360

aaaggaccag ctacattaaa attagataat gataacaaaa ctgctactat taaaattaag     420

aatggtgtta agtggtcaga tggtaagcaa gtaactgcta aggatgttga atattcttac     480

gaaattattg ctaacaaggc aactaagtca tcacgttata ctgactcact tcaaaatatt     540

gttggtatga gtgaatacca cgatggcaaa tctaacacta tttctggtat tgaaatgccg     600

gatggtgaaa atggaagaac cgttgttatt cacttcaaag aaatgaagcc aggtatgaag     660

ttcagtggta atggctactt ctgggaagct gcagctccat atcactattt gaaggatatc     720

ccatttgata agctacaatc atctgatcaa gtaagaaaga agccactatt ctttggtccg     780

tataagatga gtaaggttgt acgtggtcaa gcagtaacat ttgttcctaa caagtactac     840

tggagaggta caccaaaatt agataaagta acaattcaag ttttgaaccc taactcagct     900

tctcaagcca ttaagagtca taagtatgat attgccggtg taatcaatgt tcaatggaag     960

aacgttgcca atactaataa tgttaactgg atcgctaaga ttcctttatc ttatcgttac    1020

ctaggtttta aggtaggaaa gtgggatgca gctaaaggcg aaaacgtaat ggataagaat    1080

gcaaaaatga ataataaagc attacgtcaa gctattgctt atggtatgaa tatctccgct    1140

gtaaacaaac gttataccaa cggtttgact ttccatattc caactttaat tccagcacaa    1200

tttggtaagt actttgataa aaatgttaag ggctacgatt acaatatcaa aaagggtaat    1260

gaactattag ataaagctgg atataagaag aagggtaagt accgtgttca acccaacggt    1320

aagcctttga ctatccattt tgcagctatg agtggtgact ctactcaaga accaattatt    1380

caaaactata tccaacaatg gaagaaatta ggtttgaatg ttaagttaac tggtggtcgt    1440

ttgatggaaa tgaactcctt ctatgacaag gtccaaaatg attctaaaga cgtagatatg    1500

ttcatggttg catggtcatt atcttcagaa ccatcaccaa atgacttata caatgcaaaa    1560

gcaccataca actttagtcg ttttgtaact gctaagaaca ctaagttact tcaagaaatg    1620

gattcacaaa aggcatttaa tactaattac cgtgttaaga aattccatga atggcaaaaa    1680

tatatggatg atgaagccta cgttgttcca gtatctaaca gttatagtat tgatgcagtt    1740

aacagcaaga ttacaggtta ctcatctaaa ccatcagcag caaacagctt atggtacaat    1800

gtaggaattg caaaataa                                                    1818

<210>  9
<211>  2280
<212>  DNA
<213>  Lactobacillus johnsonii

<400>  9
aaaaatatat ggatgatgaa gcctacgttg ttccagtatc taacagttat agtattgatg      60

cagttaacag caagattaca ggttactcat ctaaaccatc agcagcaaac agcttatggt     120

acaatgtagg aattgcaaaa taatattaga taaatttctt cataaagatc gtcttaagtg     180

acggcctttt ttttatttaa gttgttttaa tcgttttcag tgctaaaatt aaatatatca     240

acttgtccaa tgttattgga ggaaaaatta atgaagaaag gcaaattaat tggtacatta     300

actttgcttt caggtgtagc tttaacttta gcagcctgtg gtaatagtaa tagcaataat     360

actagtcatc ctaatttcaa ggagtcaact cctaagaaga caattaagga tggtggaaat     420

gtcagcgttg ctgtagttac tgatacaccg tttactggta tttttaatga tgaattgtct     480

actaacaata cagactcaga agtaatgcaa tatggtgatg aatcattatt tgctacaaat     540

aatacctata aatacgttaa gggtggagca gcagacatta aaattaataa agatgccaaa     600

actgcaacca ttactattaa tcctaaagtt aagtggtctg atggccaacc tttagttgca     660

aaagattatg aatacgccta tgaaattatt gcaaataagg caacacattc tcagcgttat     720

acttcaagtt tagctaatct tgtaggatta gaagaatacc atgatggaaa gtccaatact     780

atttcaggta ttgaaatgcc agacggcgaa aatggtcgta cagtagtact ccactttaaa     840

gaaatgaagc cgggtatgac acaaagtggt aatggatata tttgggaagc agcagctcca     900

tatcattact taaaagatgt tccatttgat aaacttattt caagcaataa gatccgtaag     960

aatccattat tctatggtcc atataaagta agtaaagtgg ttcgtggaca atcagtttca    1020

tgggttccaa atgaacatta ctataagggt aaaccacact taaagaagat aactgcttcc    1080

gtaattactc cagcttcagt tgctcaatca attaagagta ataaatttga tgtaacgcaa    1140

gtaagtaatt ctcaatggcc aaatattaag ggtgctaaag gagtaaactt catcgctaat    1200

attccacttg catatagtta cttaggcttt aaggttggta aatgggatgc agccaaaggc    1260

gaaaatgtga tgaataagga cgcaaagatg aataatcgtt ctcttcgtca agctattgcc    1320

tacggtatga atgttgacca agtttataag agatactcat caggtctttc atttagaatt    1380

ccaactttaa ttccaaagca attcggtgat tacttcgata agaatgtaaa gggttacact    1440

tataacatta aaaagggtaa tgaactatta gataaagctg gttacaagaa gaaggggact    1500

taccgtgttc aaccaaatgg taagccttta acaattcgtt tagctgccat gagtggtagt    1560

aaagttcaag aaccaatcat tcaaaattac attcaacaat ggaagaaaat gggcttgaac    1620

gttaagttaa ctggtggccg tttaatggaa atgaattcat tctacgacaa ggttcaaaat 1680

gattctaaag atgtagatat gttcattggt gcatggtcac tctcttctga gccatcacct 1740

caagacttat acggcgcaaa ggcaccattt aactacagta gatttgtcac taaagaaaac 1800

actgacttgt tgaacgatat tgattctcaa aaagccttta ataatagcta cagagttaag 1860

aaattccatc aatggcaagc atacatggat aaggaagctt atgttgtgcc agttgctaat 1920

agctacagca tttacgcaat taacaataag ttaactggat actcattaga accatctaag 1980

agtatgggtg gtggattccc taactggtac tacgttggct atgcaaaata aataaaaatt 2040

atttaaaagt gcgacttata ttaggtcaca ctttttcttt atcttttttt aataggttaa 2100

aataggtata attaaagggt taaagaaagg aagcaactat ggtttcagta gcttttaaac 2160

aaaatttaaa gaaaaaatac ggaataaaat ttaataacga aaaattgctt gaagatgcct 2220

tcacacattc ttcttatgct aatgagcatc ccgggagaaa ggactatgag aaattagagt 2280

<210> 10
<211> 1782
<212> DNA
<213> Lactobacillus johnsonii

<400> 10
atgttattgg aggaaaaatt aatgaagaaa ggcaaattaa ttggtacatt aactttgctt 60

tcaggtgtag ctttaacttt agcagcctgt ggtaatagta atagcaataa tactagtcat 120

cctaatttca aggagtcaac tcctaagaag acaattaagg atggtggaaa tgtcagcgtt 180

gctgtagtta ctgatacacc gtttactggt atttttaatg atgaattgtc tactaacaat 240

acagactcag aagtaatgca atatggtgat gaatcattat ttgctacaaa taatacctat 300

aaatacgtta agggtggagc agcagacatt aaaattaata aagatgccaa aactgcaacc 360

attactatta atcctaaagt taagtggtct gatggccaac ctttagttgc aaaagattat 420

gaatacgcct atgaaattat tgcaaataag gcaacacatt ctcagcgtta tacttcaagt 480

ttagctaatc ttgtaggatt agaagaatac catgatggaa agtccaatac tatttcaggt 540

attgaaatgc cagacggcga aaatggtcgt acagtagtac tccactttaa agaaatgaag 600

ccgggtatga cacaaagtgg taatggatat atttgggaag cagcagctcc atatcattac 660

ttaaaagatg ttccatttga taaacttatt tcaagcaata agatccgtaa gaatccatta 720

ttctatggtc catataaagt aagtaaagtg gttcgtggac aatcagtttc atgggttcca 780

aatgaacatt actataaggg taaaccacac ttaaagaaga taactgcttc cgtaattact 840

ccagcttcag ttgctcaatc aattaagagt aataaatttg atgtaacgca agtaagtaat 900

tctcaatggc caaatattaa gggtgctaaa ggagtaaact tcatcgctaa tattccactt 960

gcatatagtt acttaggctt taaggttggt aaatgggatg cagccaaagg cgaaaatgtg 1020

atgaataagg acgcaaagat gaataatcgt tctcttcgtc aagctattgc ctacggtatg 1080

aatgttgacc aagtttataa gagatactca tcaggtcttt catttagaat tccaacttta 1140

attccaaagc aattcggtga ttacttcgat aagaatgtaa agggttacac ttataacatt 1200

aaaaagggta atgaactatt agataaagct ggttacaaga agaaggggac ttaccgtgtt 1260

caaccaaatg gtaagccttt aacaattcgt ttagctgcca tgagtggtag taaagttcaa 1320

gaaccaatca ttcaaaatta cattcaacaa tggaagaaaa tgggcttgaa cgttaagtta 1380

actggtggcc gtttaatgga aatgaattca ttctacgaca aggttcaaaa tgattctaaa 1440

gatgtagata tgttcattgg tgcatggtca ctctcttctg agccatcacc tcaagactta 1500

tacggcgcaa aggcaccatt taactacagt agatttgtca ctaaagaaaa cactgacttg 1560

ttgaacgata ttgattctca aaaagccttt aataatagct acagagttaa gaaattccat 1620

caatggcaag catacatgga taaggaagct tatgttgtgc cagttgctaa tagctacagc 1680

atttacgcaa ttaacaataa gttaactgga tactcattag aaccatctaa gagtatgggt 1740

ggtggattcc ctaactggta ctacgttggc tatgcaaaat aa 1782

<210> 11
<211> 1330
<212> DNA
<213> Lactobacillus johnsonii

<400> 11
caatcatgat taccaaaaat aaacaaccaa ttaacatttc cttctatagt cttcacaatg 60

aacatggtaa atatattggc tgcattgaag taacccaagc agtaagtaac ttacaagtta 120

aaggcagtaa attgcgcaat attttaaatg tccttaaaaa gcactaaagt taagctctta 180

gaatactttt ttcagtcaaa aatagagtaa aataaaataa gatagataca tttattgggg 240

gaatatattt ttatgaagaa aaaattcagc ttcctaattt tattgggagt aattttagtt 300

tctttaacta cagcttgttc aaataaaagc agtaaacaag caaagagtac tggaccttct 360

gcaagttcat taatcaatgc aaaatttaat tctagctttg ctaacggtca cttcactcaa 420

actactaatt ctaatgaaat gaaccaaaag tcaaaatcag aaggtttatt caaagacaaa 480

ggtgatgtaa ctaaccttac ctatacctta actcaaaaga agaaatctca aactgaacaa 540

atgtggctta ccaagaagga tatgtactta cttttagagc aaaacaaggg ccattggatt 600

aaaaactcaa ttgatgcaga tagctttgat tcagaccaag taaaggaaag attcgatcct 660

gcaactttta ataaaattaa caaatcttta gctcaaaaag ccactgttca aagaaaaaat 720

ggaaattatg aaattagttt tgatggtacc gacgctaatc tatggaatgc agtcaatcct 780

ttaattatcg atgccatgaa cactcctggt tctcaaaata tgcaggtagc ccgcttagta 840

aaatcagctc aagttcaaaa tcttaagatc acttatttaa ttgatccaac taccaaaaat 900

gtcgaatcaa tgacctttaa ggcccaatat actgctggcg gaaaatataa cttcacttgg 960

aacctaactt acgaccaatt aggtcaacac agtgatttag ccgttccttc tgacattcaa 1020

aagaatgcaa ttagtgctga agacatcaag aaagcacaag aagaacaaaa taatcaataa 1080

tcatttagaa agatacactg atggaaaaaa ataaaatcaa agataaaaag attatttttt 1140

ctaacaatca aggcggcaaa attgccgctt taattttagg tattattatc ctaatcgtag 1200

tagtttgggg cgcaattatt gcatttaata agccaagcgt taaagatgca ttaactacgg 1260

ctgccaatac ccaaattgct actgcacgcg ttaatgtcaa aactaccaat gcaaagtcaa 1320

aaatgtattc 1330

<210> 12
<211> 828
<212> DNA
<213> Lactobacillus johnsonii

<400> 12
atgaagaaaa aattcagctt cctaatttta ttgggagtaa ttttagtttc tttaactaca 60

gcttgttcaa ataaaagcag taaacaagca aagagtactg gaccttctgc aagttcatta 120

atcaatgcaa aatttaattc tagctttgct aacggtcact tcactcaaac tactaattct 180

aatgaaatga accaaaagtc aaaatcagaa ggtttattca aagacaaagg tgatgtaact 240

aaccttacct ataccttaac tcaaaagaag aaatctcaaa ctgaacaaat gtggcttacc 300

aagaaggata tgtacttact tttagagcaa aacaagggcc attggattaa aaactcaatt 360

gatgcagata gctttgattc agaccaagta aaggaaagat tcgatcctgc aacttttaat 420

aaaattaaca aatctttagc tcaaaaagcc actgttcaaa gaaaaaatgg aaattatgaa 480

attagttttg atggtaccga cgctaatcta tggaatgcag tcaatccttt aattatcgat 540

gccatgaaca ctcctggttc tcaaaatatg caggtagccc gcttagtaaa atcagctcaa 600

gttcaaaatc ttaagatcac ttatttaatt gatccaacta ccaaaaatgt cgaatcaatg 660

acctttaagg cccaatatac tgctggcgga aaatataact tcacttggaa cctaacttac 720

gaccaattag gtcaacacag tgatttagcc gttccttctg acattcaaaa gaatgcaatt 780

agtgctgaag acatcaagaa agcacaagaa gaacaaaata atcaataa 828

<210> 13
<211> 1400
<212> DNA
<213> Lactobacillus johnsonii

<400> 13
ctcttacttc atgtccttct ttttgaaaat cacgtacaac actaattgcc ttatatgcag 60

caatactacc cgtgatatat attgtagctt tcatttttta tcaaccttaa ttcataattt 120

ctttctacta taacacaagc ttactaagtc gaattactaa aaataaatta gcaaattaaa 180

aaatattagt aaattctgct atgatttata ttgaatgcag atctaaacaa cataattcga 240

aaggataaat atatagaatg agaaaacatt atcaaaagat ttttgctttc ttttttgcaa 300

ttagtagtct tgttttaatt cttactggct gctcaaaaaa agaaaattca tcaaataaaa 360

tttctatcgt aacaagtact aatgtttatg ccgatatcgc acaaaatgtc ttaggaaaat 420

atggtaaagc aactgctatt attactaaca gtgctactga tccacatgac tttgaaccca 480

caactgcaga cgcaaaaaaa gttcaaaatg caaaaattgt tgttgctaac ggtttgggct 540

atgattcttg gcttcctaag cttgctaaat caactaataa gtctgctgtt ttagttggcg 600

aagatttaat gaatctaaaa aatggcgcaa atccgcatat ttggtttgat ttaaacatgc 660

ctaaaaaata tgttaactat ttagttgagc gactctctaa aatagacaaa aaacatgcag 720

cttactacaa agaaaatggg aaggaatatc ttgcaaaaat caataagata caaaaaattg 780

cagattctat tgatggtgca aaacaaaagc cagtatatgt cagcgagcca gtgtttgact 840

atgccttaaa tgctactcac tttaagattg gtgataaagc ttttgaagaa gcaattgaaa 900

atgaaacaga tccaagtgcc aaaattattc atcaaatgaa ccaaacaatt aataatcgtg 960

gaatttcttt ctttgttaaa aattctcaag taagcagttc aactgttaat aattttgtaa 1020

aaagagctaa gtcaaaaaat attccaatct tgcaagttcg tgaaaccatc cccaacaata 1080

caagttacat aaaatggatg acagaaaatt atcagaattt agcaaatatt aataaaaaac 1140

tagactaagt tagtttatta aaaagagggc ctctaatacg agaccctctt ttttacttta 1200

aaatatcttt taactttata ttaaattgct gtggaatttc cgccataatt acatgtcccg 1260

tcggtgacat aataaagcta tttattcggc tattcttttg ttcaagcttc ttttcatatc 1320

cctctcgata ataaggactt tcctttgcaa caaaaaaagt agttggaatt gttgtttttt 1380

ctaaaactct tcgccaatct 1400

<210> 14
<211> 891
<212> DNA
<213> Lactobacillus johnsonii

<400> 14
atgagaaaac attatcaaaa gatttttgct ttcttttttg caattagtag tcttgtttta 60

attcttactg gctgctcaaa aaaagaaaat tcatcaaata aaatttctat cgtaacaagt 120

actaatgttt atgccgatat cgcacaaaat gtcttaggaa aatatggtaa agcaactgct 180

attattacta acagtgctac tgatccacat gactttgaac ccacaactgc agacgcaaaa 240

aaagttcaaa atgcaaaaat tgttgttgct aacggtttgg gctatgattc ttggcttcct 300

aagcttgcta aatcaactaa taagtctgct gttttagttg gcgaagattt aatgaatcta 360

aaaaatggcg caaatccgca tatttggttt gatttaaaca tgcctaaaaa atatgttaac 420

tatttagttg agcgactctc taaaatagac aaaaaacatg cagcttacta caaagaaaat 480

gggaaggaat atcttgcaaa aatcaataag atacaaaaaa ttgcagattc tattgatggt 540

gcaaaacaaa agccagtata tgtcagcgag ccagtgtttg actatgcctt aaatgctact 600

cactttaaga ttggtgataa agcttttgaa gaagcaattg aaaatgaaac agatccaagt 660

gccaaaatta ttcatcaaat gaaccaaaca attaataatc gtggaatttc tttctttgtt 720

aaaaattctc aagtaagcag ttcaactgtt aataattttg taaaaagagc taagtcaaaa 780

aatattccaa tcttgcaagt tcgtgaaacc atccccaaca atacaagtta cataaaatgg 840

atgacagaaa attatcagaa tttagcaaat attaataaaa aactagacta a 891

<210> 15
<211> 1820
<212> DNA
<213> Lactobacillus johnsonii

<400> 15
ctccgtttat tgaaaatatt gaaaaacgtg gaattgaatt gataactcta cgttgttcag 60

gacatgcgga tgtagctgaa ttaaaagaaa ttattgcggg ttttgagcca aaaattttgg 120

ctcctgtgca cacattgcat ccagaattag aagaaaaccc atttggagaa aggattttac 180

ctaagcgcgg agaggtattt acgcttagtt aaaattaaaa ttttattttg ttgtttgttt 240

ttattgggag gaacatatga agttcactaa aaaatttgca gcagttgcag ttgctgcttt 300

agcccttgta ggcactactg cttgctcaaa ttctaagagt aatgatagct caagtagtaa 360

gtcagctgat attccaacta agattaccaa gaaaactact gtagttttct ggcatggtat 420

gactggaact caagaaaaga ctttacaaag cttgactaag gattttgaaa agaagaatcc 480

taacattact gttaagcttg aaaaccaagg taagtatact gatttacaag caaagattaa 540

ctcaactttg caatcaccaa ataacttgcc tacaattact caagcttatc ctggctggct 600

tttaaatgcc gctaatagca aacgcttggt tgatatgacc ccttacatta acaatagtaa 660

gattggttgg ggctcagcta agaagagtga catccgtgaa gaattattaa agggtgcaca 720

aattaagggt actcaatatg gtattccatt taataaatct gttgaaatgt taacttataa 780

caaagattta ttagataaat atggtgttaa agtaccaaag acaatggctg aattaaaggc 840

agcatctaag aagatttacg aaaagagtaa ccacaaggtt gttggtgctg gattcgactc 900

actttcaaac tactacgttt taggtatgaa ggatgaaggc gtaaacttct ctaagaacat 960

caactttact ggttctaagt ctaagaaagt aatcaactac tatgctgatg gtattaaagc 1020

tggttacttc aagactgctg gttcagctgg ttacctttct ggtgacttct ctaaccaaaa 1080

ggttgcaatg tttgtaggta ctagtgctgg tgaaggattt gttaagatgg gtgttggtaa 1140

taagttccaa tacggtgttg cacctcgtcc ttctaagtac aacatgcaac aaggtactga 1200

tatctacatg ttcaacaagg gtactgcaga acaaaaggca gctgccttca tgtacattaa 1260

gtacttgtta ggtaagaacc aacaattgac ttgggctgat caaactggat acatccctgt 1320

aactaacaat gtgattaact ctagtgctta caagaactca actaagtcta aagttcctgc 1380

tcaattagac aaggcaatga agaacttata cagtgttcct gtagttaaga acagcaacac 1440

tgcatacact caattgaatt caattatgca aaacatcttt gcacaagcaa agaagggtca 1500

aaactggaat tcacaaattg atgccggtaa gactaagttc gaaagtgctt ggaatcaata 1560

aaaacagaat attaagtaaa aaatgaggac tgtcattatg acaatcctca tttttttata 1620

gatgttttag aagaaaagta gaatgaaggc aattaatgca ggtagcattt gaacaacaaa 1680

aatcttttta cttgctgtaa agcccccaaa aatagcgaca actacaacaa aggcaagcaa 1740

aagttgccat acagtgatta aagttggcat tctgaagaag aaaaatacaa ttaagattaa 1800

aagacctagc attccattat 1820

<210> 16
<211> 1305
<212> DNA
<213> Lactobacillus johnsonii

<400> 16
atgaagttca ctaaaaaatt tgcagcagtt gcagttgctg ctttagccct tgtaggcact 60

actgcttgct caaattctaa gagtaatgat agctcaagta gtaagtcagc tgatattcca 120

actaagatta ccaagaaaac tactgtagtt ttctggcatg gtatgactgg aactcaagaa 180

aagactttac aaagcttgac taaggatttt gaaaagaaga atcctaacat tactgttaag 240

cttgaaaacc aaggtaagta tactgattta caagcaaaga ttaactcaac tttgcaatca 300

ccaaataact tgcctacaat tactcaagct tatcctggct ggcttttaaa tgccgctaat 360

agcaaacgct tggttgatat gaccccttac attaacaata gtaagattgg ttggggctca 420

gctaagaaga gtgacatccg tgaagaatta ttaaagggtg cacaaattaa gggtactcaa 480

tatggtattc catttaataa atctgttgaa atgttaactt ataacaaaga tttattagat 540

aaatatggtg ttaaagtacc aaagacaatg gctgaattaa aggcagcatc taagaagatt 600

tacgaaaaga gtaaccacaa ggttgttggt gctggattcg actcactttc aaactactac 660

gttttaggta tgaaggatga aggcgtaaac ttctctaaga acatcaactt tactggttct 720

aagtctaaga aagtaatcaa ctactatgct gatggtatta aagctggtta cttcaagact 780

gctggttcag ctggttacct ttctggtgac ttctctaacc aaaaggttgc aatgtttgta 840

ggtactagtg ctggtgaagg atttgttaag atgggtgttg gtaataagtt ccaatacggt 900

gttgcacctc gtccttctaa gtacaacatg caacaaggta ctgatatcta catgttcaac 960

aagggtactg cagaacaaaa ggcagctgcc ttcatgtaca ttaagtactt gttaggtaag 1020

aaccaacaat tgacttgggc tgatcaaact ggatacatcc ctgtaactaa caatgtgatt 1080

aactctagtg cttacaagaa ctcaactaag tctaaagttc ctgctcaatt agacaaggca 1140

atgaagaact tatacagtgt tcctgtagtt aagaacagca acactgcata cactcaattg 1200

aattcaatta tgcaaaacat ctttgcacaa gcaaagaagg gtcaaaactg gaattcacaa 1260

attgatgccg gtaagactaa gttcgaaagt gcttggaatc aataa 1305

<210> 17
<211> 1400
<212> DNA
<213> Lactobacillus johnsonii

<400> 17
gctacaataa attcataagt tggaggtgtt actatgcaag aattaagaaa caatactctc 60

atttctctga ttgggggggg tagaaagcca ttctatcaac gtttctaagg gattttctac 120

tttaaatcct tattttcttg tacagtacat aaaaagccaa gttaaagatg actttaatgg 180

ggtcattctt tagcttggct tttttgcgtc ttcaattatt ttaattctaa tagctcgagt 240

gtaggaggaa atagagtgaa aagtaagtct tataagtatg ggattgctgc tttagtaggt 300

gtaagtgcct tagcattagc tggttgtcaa agtaatgaaa accaatcaag tacatctaag 360

actaatgcca agtcgaaatc ttcaatagtt aagattgaaa agaaaagtaa atctaagagt 420

agcaagtcgg aaaagcaaga aggtgaagtg aaaaaacaga gcagtgagaa ggttaataag 480

gcaagtagtt cgaagaaaga tgtagaaaaa gtaccaacag ttactgaatt gatgaataat 540

gtttcggcct ttaatgaagg taattttgat attcggctta attatgcaac tgttaaaggt 600

tatgacacgg gaataattca agaaaatcca cgagtagtac attataagtt caacggtggg 660

atgaactcag aggtttgggc aactgatcaa tttgtctaca gtttatcggg tgataaatgg 720

actaaaattg ggcaaactta taagattgga agatttgatc cgatgattag ccttccatct 780

ccccttatta atcaaatgaa agtctcaaaa actagtgatg gttatcaagc aaaatatgag 840

ggaaactctg ctgaagctaa agaattaatg gaacattata ttgtttgggt ttcaagtgaa 900

aataaatgga atccatttgg agaccaagac tttacatcag ctactctaac ttatagcttt 960

aataaacaaa aacagttaac tgagattcat tatgattgga ccgctggttc aaataaagga 1020

acagcttctt acactaatat caatcaacaa aaaattgcag ttccaaatga tgtaattcaa 1080

gcagcagcta agaattctga ctctattgat agtaacaatg atgataatag tgacgatgaa 1140

tagggtcgaa aatagatgga taaaataaaa gaaaataaat ttgttattaa agctaagaaa 1200

atttggtcta agcataagat cattgtttcg gttctaggag gcttcttggt tctgatatgt 1260

ttatttggaa ttagagaggc tagtcttgtt gatccattag atggtgttca acaagtggga 1320

tggaattcgt atacgggttc ttggtttgtt ccggataaag accgagatcg gattgcaagt 1380

aaaattagag catccatatt 1400

<210> 18
<211> 888
<212> DNA
<213> Lactobacillus johnsonii

<400> 18
gtgaaaagta agtcttataa gtatgggatt gctgctttag taggtgtaag tgccttagca 60

ttagctggtt gtcaaagtaa tgaaaaccaa tcaagtacat ctaagactaa tgccaagtcg 120

aaatcttcaa tagttaagat tgaaaagaaa agtaaatcta agagtagcaa gtcggaaaag 180

caagaaggtg aagtgaaaaa acagagcagt gagaaggtta ataaggcaag tagttcgaag 240

aaagatgtag aaaaagtacc aacagttact gaattgatga ataatgtttc ggcctttaat 300

gaaggtaatt ttgatattcg gcttaattat gcaactgtta aaggttatga cacgggaata 360

attcaagaaa atccacgagt agtacattat aagttcaacg gtgggatgaa ctcagaggtt 420

tgggcaactg atcaatttgt ctacagttta tcgggtgata aatggactaa aattgggcaa 480

acttataaga ttggaagatt tgatccgatg attagccttc catctcccct tattaatcaa 540

atgaaagtct caaaaactag tgatggttat caagcaaaat atgagggaaa ctctgctgaa 600

gctaaagaat taatggaaca ttatattgtt tgggtttcaa gtgaaaataa atggaatcca 660

tttggagacc aagactttac atcagctact ctaacttata gctttaataa acaaaaacag 720

ttaactgaga ttcattatga ttggaccgct ggttcaaata aaggaacagc ttcttacact 780

aatatcaatc aacaaaaaat tgcagttcca aatgatgtaa ttcaagcagc agctaagaat 840

tctgactcta ttgatagtaa caatgatgat aatagtgacg atgaatag 888

<210> 19
<211> 1650
<212> DNA
<213> Lactobacillus johnsonii

<400> 19
cagataattt ctttaaggaa aagacagtag ttctaactgg aaaattatct gcctttactc 60

gaagtgaatt tactaagaag cttcaggatc ttggtgcaaa agtcactggg tcagtttcta 120

agaagacaga ttatttgatc tacggagcag atgcgggctc taaaaaagat aaggctgaaa 180

aacttcaagt tccaatgtta actgaacaag aagcaattgc aaaaattgaa aaataaagaa 240

taagaaggaa aaataaattg aaaagatttt tgcaaatagc actactacta gcaactgggt 300

taagtttaag tgcatgtggc aatttaaaaa attctgatct tgccaataac cctactactt 360

ctacaacaaa gaaaaaaagt tatcaaacta ccagtactag taaaaatggg tataatgttc 420

ttttaaaaga tggagagtat gtaactagtc caatagaagg aacaactgaa aatactagtg 480

ataataatgt agatagtcgt gctttagaat ctgggttaat tgatttatct cacaatactt 540

tttcaagtag taagtatgtt tttcaagaag gacaaaaaat ttcggttgct gatgctactg 600

attggctagg aagaaagtct aaaagtaatc cagaaggtct aaatgcggaa aaaagtaata 660

aaaagaattc gtataatcct attattttag atcaaatttt agaacaagat tttttgacta 720

aatctaattc tagttacaaa atggatggta tgagtattgg gctagctctt aactcggttg 780

attactacca aaaagttaaa gatggtcccc aatatcataa agatatttct cgtgcagaac 840

aggagacttt tggtaaagaa gcagctaata agttagtttc gtatttacgt aaaaagaagg 900

gattaaaaga tatcccaatt ttggttggac tttttagtaa aaccagtaaa gattctcttg 960

taggtggaaa ttactttgca tatggaattg ctaatgcaaa tagtagtaaa ataaatgatt 1020

ggaaaacagt caataatcgt agtcaagtgc taccgactgt tggaagtgaa aaagcaatca 1080

attctactga tgcagcttca tttagtgatt ttaaagcagc tattcaaggt tacttcccta 1140

atattagtgg ggtaacagcg actgttcatt atcaaaataa aactttgaca caaatgaaca 1200

ttactgtaac tacccaattc tttggttatg cgcagattga gagtttttca agacttgtat 1260

tgtcagctgc taagaagtac ttacctaagg atgcaccaat agaaattaag attaattctg 1320

ttaatgatac gcaggcatta atcgctaaga attccgcaga cgatagttat tatgtccacg 1380

tctttggcgg tgaataggag gcaaacggtg aaaattacaa aagatgaaat caatcacgtt 1440

gcagcactat ctcgacttga gtttggtgaa gacgaaattg ataagtttac tgaacaaatg 1500

ggtgacatta tcaatatggc acatcaatta gccgaagttg atactgaagg agttcctgaa 1560

actgttcaag ttgttgatcg agagactgac tttagagaag acaagcctga acattgggaa 1620

agtcgcgcag aattaatgaa gaatgttcct 1650

<210> 20
<211> 1140
<212> DNA
<213> Lactobacillus johnsonii

<400> 20
ttgaaaagat ttttgcaaat agcactacta ctagcaactg ggttaagttt aagtgcatgt 60

ggcaatttaa aaaattctga tcttgccaat aaccctacta cttctacaac aaagaaaaaa 120

agttatcaaa ctaccagtac tagtaaaaat gggtataatg ttcttttaaa agatggagag 180

tatgtaacta gtccaataga aggaacaact gaaaatacta gtgataataa tgtagatagt 240

cgtgctttag aatctgggtt aattgattta tctcacaata ctttttcaag tagtaagtat 300

gtttttcaag aaggacaaaa aatttcggtt gctgatgcta ctgattggct aggaagaaag 360

tctaaaagta atccagaagg tctaaatgcg gaaaaaagta ataaaaagaa ttcgtataat 420

cctattattt tagatcaaat tttagaacaa gatttttgta ctaaatctaa ttctagttac 480

aaaatggatg gtatgagtat tgggctagct cttaactcgg ttgattacta ccaaaaagtt 540

aaagatggtc cccaatatca taaagatatt tctcgtgcag aacaggagac ttttggtaaa 600

gaagcagcta ataagttagt ttcgtattta cgtaaaaaga agggattaaa agatatccca 660

attttggttg gactttttag taaaaccagt aaagattctc ttgtaggtgg aaattacttt 720

gcatatggaa ttgctaatgc aaatagtagt aaaataaatg attggaaaac agtcaataat 780

cgtagtcaag tgctaccgac tgttggaagt gaaaaagcaa tcaattctac tgatgcagct 840

tcatttagtg attttaaagc agctattcaa ggttacttcc ctaatattag tggggtaaca 900

gcgactgttc attatcaaaa taaaactttg acacaaatga acattactgt aactacccaa 960

ttctttggtt atgcgcagat tgagagtttt tcaagacttg tattgtcagc tgctaagaag 1020

tacttaccta aggatgcacc aatagaaatt aagattaatt ctgttaatga tacgcaggca 1080

ttaatcgcta agaattccgc agacgatagt tattatgtcc acgtctttgg cggtgaatag 1140

<210> 21
<211> 2139
<212> PRT
<213> Lactobacillus johnsonii

<400> 21
   Met Leu Ser Lys Asn Asn Tyr Gln Glu Arg Leu Arg Lys Met Asp Asp
   1 5 10 15
   Lys Gln Glu Arg Phe Ser Ile Arg Lys Phe Ser Val Gly Ala Ala Ser
   20 25 30
   Val Leu Val Gly Thr Ala Val Leu Thr Met Gln Asn Val Gln Thr Ala
   35 40 45
   Arg Ala Asp Thr Thr Ser Asn Thr Thr Thr Lys Thr Thr Asp Leu Ser
   50 55 60
   Ser Glu Ala Glu Glu Gln Asn Lys Gln Asn Ala Tyr Asp Lys Thr Leu
   65 70 75 80
   Asn Glu Ala Gln Pro Thr Thr Lys Pro Ala Asp Ala Pro Val Glu Ser
   85 90 95
   Gln Asp Ser Lys Val Ala Ser Phe Ser Val Pro Lys Asn Glu Gly Thr
   100 105 110
   Phe Val Glu Lys Thr Leu Thr Pro Asn Ile Thr Glu Glu Thr Glu Ala
   115 120 125
   Met Gln Asp Lys Ala Glu Leu Ala Gln Val Asn Ser Glu Asp Lys Ser
       130                 135                 140
   Ala Glu Asn Asn Lys Thr Asn Val Lys Glu Glu Ser Gln Thr Val Glu
   145                 150                 155                 160
   Glu Asn Gln Met Asp Lys Arg Ser Ala Glu Ala Asn Ile Ser Asp Thr
                   165                 170                 175
   Glu Asn Thr Leu Val Ser Asn Thr Thr Leu Asn Val Pro Lys Thr Thr
               180                 185                 190
   Pro Ala Thr Thr Arg Phe Ser Ala Thr Ala Leu Ser Glu Ser Lys Phe
           195                 200                 205
   Gln Val Asn Ala Pro Arg Val Gly Gln Asp Thr Pro Asp Thr Gln Val
       210                 215                 220
   Lys Lys Asn Asn Tyr Lys Leu Val Thr Asn Ala Thr Ala Leu Gln Gln
   225                 230                 235                 240
   Ala Ile Asn Thr Gly Val Ala Gly Val Asn Ile Asp Arg Ser Ile Asp
                   245                 250                 255
   Ala Ser Asn Val Asp Leu Ala Ile Arg Asn Thr Phe Thr Ile Val Gly
               260                 265                 270
   Leu Asn Asp Ala Ala Ala Leu Asn Leu Gly Gln Lys Ser Leu Asn Asn
           275                 280                 285
   Ser Gly Asn Leu Thr Leu Gln Asp Ile Lys Ile Asn Gly Ser Ile Ser
       290                 295                 300
   Gly Ser Gly Thr Val Asn Ile Lys Gly Asn Val Thr Ser Asn Val Asn
   305                 310                 315                 320
   Ser Thr Asn Ser Ser Val Pro Thr Gln Asp Gln Tyr Asn Ala Gln Asn
                   325                 330                 335
   Tyr Thr Gly Ser Arg Thr Asn Phe Lys Asn Ser Asn Ile Ala Ala Arg
               340                 345                 350
   Asn Val Asn Ile Glu Asn Gly Ala Ser Leu Thr Ile Asn Ser Ser Glu
           355                 360                 365
   Ile Asn Asp Gly Ile Asn Leu Ala Asp Gly Gly Thr Val Lys Val Gly
       370                 375                 380
   Asp Asn Ala Thr Leu Asn Val Asn Leu Thr Asn Thr Ser Thr Thr Ala
   385                 390                 395                 400
   Thr Arg Tyr His Val Ala Gly Val Phe Ala Lys Asn Gly Gly Asn Phe
                   405                 410                 415
   Ile Ser Gly Tyr Lys Ser Asn Val Asn Phe Asn Thr Gly Leu Gly Gln
               420                 425                 430
   Ala Ile Ala Ile Gly Ala Thr Arg Pro Thr Ser Thr Asp Ala Asp Arg
           435                 440                 445
   Tyr Gly Gly Tyr Gly Thr Arg Ser Arg Asn Asp Gly Pro Thr Leu Val
       450                 455                 460
   Gln Leu Gly Asp Ser Ser Thr Phe Asn Phe Thr Gly Arg Asp Gly Ile
   465                 470                 475                 480
   Ile Leu Gly Asn Asn Ala Asn Phe Ile Ser Gly Glu Asn Ser Asn Val
                   485                 490                 495
   His Phe Glu Asn Lys Gly Arg Gly Val Ala Leu Asp Leu Ala Asn Asn
               500                 505                 510
   Ser Asn Ile Glu Ile Ser Lys His Ser Ile Thr Ser Phe His Ser Val
           515                 520                 525
   Gly Lys Thr Gly Thr Ser Gly Ser Phe Asp Gly Tyr Asn Tyr Ile Gly
       530                 535                 540
   Val Asn Glu Gly Gly Asn Ile Thr Val Asp Glu Tyr Ala Thr Phe Arg
   545                 550                 555                 560
   Val Ile Leu Glu Gly Arg Gly Asp Asn Pro Trp Asp Asp Val Ile Ser
                   565                 570                 575
   Leu Asp Ser Arg Asn Ala Asn Thr Asn Ala Ala Phe Thr Ser Lys Thr
               580                 585                 590
   Gly Ala Ile Val Asp Ile Arg Asp Asp Asn Thr Asn Phe Tyr Ala Glu
           595                 600                 605
   Leu Ile Ser Phe Pro Leu Gly Ser Ala Asn Ser Arg Ile Asp Ile Gln
       610                 615                 620
   Asp Pro Leu Met Leu Asn Leu Gln Arg Tyr Ser Asn Gly Gly Ala Thr
   625                 630                 635                 640
   Thr Gly Trp Met Ala Thr Gly Gly Asp Lys Ile Asn Thr Thr Ser Ala
                   645                 650                 655
   Gln Tyr Thr Ala Asn Leu Ile Tyr Met Ser Gly Asn Lys Gly Val Phe
               660                 665                 670
   Ser Val Ser Gly Gly Asp Tyr Asp Pro Ser Asn Pro Asn Ser Ser Gly
           675                 680                 685
   Phe Val Val Tyr Gln Gln Ile Lys Ser Asp Gly Ser Lys Gln Ile Trp
       690                 695                 700
   Leu Asn Val Asn Ser Val Asp Ile Pro Met Asn Gly Phe Gln Thr Lys
   705                 710                 715                 720
   Asp Ile Trp Asn Asn Gln Ala Asn Pro Asp Val Ser Ile Ala Gly Asn
                   725                 730                 735
   Gly Leu Thr Ala Gly Ile Lys Ala Asn Gln Val His Asn Phe Asp Gly
               740                 745                 750
   Ser Pro Leu Thr Gly Pro Asn Ala Pro Tyr Tyr Gly Ile Ser Thr Gln
           755                 760                 765
   Arg Ala Ser His Gln Ile Trp Ile Pro His Arg Thr Ser Leu Glu Val
       770                 775                 780
   Thr Gly Glu His Lys Ser Thr Ile Lys Tyr Val Asp Glu Asn Gly Lys
   785                 790                 795                 800
   Glu Ile Phe Pro Glu Asn Thr Gln Ser Leu Asp Met Lys Arg Ser Leu
                   805                 810                 815
   Ile Leu Asp Ile Thr Gln Asp Lys Ile Gln Asp Ile Gln Asn Tyr Ala
               820                 825                 830
   Leu Ser His Thr Ala Asp Glu Thr Leu Glu Tyr Ile Lys Asn Ser Gln
           835                 840                 845
   Ala Val Val Gln Asp Ser Gly Trp Asp Phe Thr Asp Ala Asn Gly Lys
       850                 855                 860
   Ala Val Ile Asp Pro Tyr Ser Thr Ile Asp Ser Pro Lys Leu Ala Gly
   865                 870                 875                 880
   Tyr Ala Ala Thr Ile Gln Ser Thr Asn Val Ser Thr Leu Lys Val Gly
   885                 890                 895
   Ala Asp Ala Ser Ala Val Thr Ala Lys Phe Met Val Glu Pro Ser Gln
               900                 905                 910
   Asp Met Val Gln Asn Gly Glu Leu Ser Thr Ala Tyr Lys Gln Asn Gly
           915                 920                 925
   Thr Thr Gly Ile Pro Ala Asp Tyr Val Thr Val Val Val Tyr Asn Lys
       930                 935                 940
   Gln Ala Thr Tyr Gln Ala Gly Lys Thr Asp Ser Lys Ser Val Val Arg
   945                 950                 955                 960
   Asp Ile Asn Tyr Leu Asp Gly Lys Thr Gly Lys Lys Ile Pro Ala Asn
                   965                 970                 975
   Leu Ile Lys Asp Asn Pro Val Ser Gln Thr Val Thr Met His Arg Thr
               980                 985                 990
   Glu Ile Leu Asp Asn Ser Gly Lys  Val Ile Gly Tyr Gly  Thr Ile Ser
           995                 1000                1005
   Glu Asp  Gly Lys Ser Tyr Thr  Leu Asn Asn Asp Trp  Ile Ile Asp
       1010                 1015                 1020
   Gly Asn  Trp Glu Ala Val Lys  Ser Pro Asp Leu Thr  Thr Asn Gly
       1025                 1030                 1035
   Tyr Lys  Thr Pro Arg Phe Glu  Asn Gly Asp Ile Ala  Lys Val Val
       1040                 1045                 1050
   Pro Ala  Glu Thr Val Asn Glu  Asn Thr Pro Asp Thr  Thr Val Asn
       1055                 1060                 1065
   Val Tyr  Tyr Asp His Asn Glu  Ile Pro Val Gly Pro  Asp Thr Pro
       1070                 1075                 1080
   Asp Lys  His Gly Val Asp Leu  Asn Glu Leu Thr Lys  Asp Val Asn
       1085                 1090                 1095
   Glu Thr  Val His Tyr Val Tyr  Ser Asp Gly Thr Lys  Ala Ala Pro
       1100                 1105                 1110
   Asp Val  Val Gln Thr Ser Lys  Trp Thr Arg Thr Leu  Thr Ile Asp
       1115                 1120                 1125
   Ala Val Thr Asn Glu Val Ile Ser Asp Gly Gln Tyr Thr Thr Asp
   1130 1135 1140
   Trp Ala Ile Pro Lys Gly Glu Lys Thr Val Tyr Asp Gln Val Asp
   1145 1150 1155
   Thr Pro Val Ile Lys Gly Tyr His Ala Asp Lys Arg Gln Ile Asn
   1160 1165 1170
   Ala Thr Val Val Thr Gln Asn Asn Ile Glu Glu Thr Val Thr Tyr
   1175 1180 1185
   Gln Pro Asn Gly Lys Ile Ile Pro Val Asp Pro Asn Gly Asp Pro
   1190 1195 1200
   Ile Pro Asn Val Pro Thr Pro Thr Tyr Pro Thr Asp Pro Thr Asp
   1205 1210 1215
   Pro Thr Lys Val Val Pro Asp Glu Pro Val Pro Glu Ile Pro Gly
   1220 1225 1230
   Leu Ile Pro Ser Val Pro Thr Val Thr Pro Glu Asn Pro Gly Lys
   1235 1240 1245
   Asp Thr Pro Val Pro Tyr Thr Pro Glu Val Pro Ala Lys Asp Gln
   1250 1255 1260
   Val Ala Val Val Asn Tyr Ile Asp Ala Asp Asp Asn Asn Ala Ile
   1265 1270 1275
   Ile Thr Ser Ser Gly Asn Leu Thr Gly Lys Ala Gly Glu Lys Ile
   1280 1285 1290
   Asp Tyr Ser Thr Ala Ser Thr Ile Glu Glu Leu Glu Asn Lys Gly
   1295 1300 1305
   Tyr Val Leu Val Ser Asp Gly Phe Pro Ala Gly Ala Thr Phe Asp
   1310 1315 1320
   Asn Asp Asp Asn Thr Thr Gln Ile Tyr Thr Val Val Leu Lys His
   1325 1330 1335
   Gly Thr Val Pro Val Thr Pro Glu Asn Pro Gly Lys Pro Gly Glu
   1340 1345 1350
   Pro Ile Asn Pro Asn Asp Pro Asp Gly Pro Lys Trp Pro Glu Gly
   1355 1360 1365
   Thr Gly Glu Asn Gly Val Lys Arg Thr Gly Thr Gln Thr Ile His
   1370 1375 1380
   Tyr Glu Gly Ala Gly Asp Lys Thr Pro Ser Asp Asp Val Gln Thr
   1385 1390 1395
   Phe Asp Phe Thr Lys Lys Met Leu Val Asp Lys Val Thr Gly Lys
   1400 1405 1410
   Ile Ile Asp Gly Gly Glu Trp Asn Val Thr Ser His Thr Phe Gly
   1415 1420 1425
   Tyr Lys Asp Thr Pro Val Ile Glu Gly Tyr His Ala Asp Lys Arg
   1430 1435 1440
   Asn Ala Gly Gly Ser Val Val Thr Pro Asp Asp Leu Asn Lys Thr
   1445 1450 1455
   Ile Thr Val Ser Tyr Gln Pro Asn Gly Lys Ile Ile Pro Val Asp
   1460 1465 1470
   Pro Asn Gly Asp Pro Ile Pro Asn Val Pro Thr Pro Thr Tyr Pro
   1475 1480 1485
   Thr Asp Pro Thr Asp Pro Thr Lys Val Val Pro Asp Glu Pro Val
   1490 1495 1500
   Pro Glu Ile Pro Gly Leu Ile Pro Ser Val Pro Thr Val Thr Pro
   1505 1510 1515
   Glu Asn Pro Gly Lys Asp Thr Pro Val Pro Tyr Thr Pro Glu Val
   1520 1525 1530
   Pro Ala Lys Asp Gln Val Ala Val Val Asn Tyr Ile Asp Ala Asp
   1535 1540 1545
   Asp Asn Asn Ala Ile Ile Thr Ser Ser Asp Asn Leu Thr Gly Lys
   1550 1555 1560
   Ala Gly Glu Lys Ile Asp Tyr Ser Thr Ala Ser Thr Ile Glu Glu
   1565 1570 1575
   Leu Glu Asn Lys Gly Tyr Val Leu Val Ser Asp Gly Phe Pro Ala
   1580 1585 1590
   Gly Ala Thr Phe Asp Asn Asp Asp Asn Thr Thr Gln Ile Tyr Thr
   1595 1600 1605
   Val Val Leu Lys His Gly Thr Val Pro Val Thr Pro Glu Asn Pro
   1610 1615 1620
   Gly Lys Pro Gly Glu Pro Ile Asn Pro Asn Asp Pro Asp Gly Pro
   1625 1630 1635
   Lys Trp Pro Glu Gly Thr Gly Glu Asn Gly Val Lys Arg Thr Gly
   1640 1645 1650
   Thr Gln Thr Ile His Tyr Glu Gly Ala Gly Asp Lys Thr Pro Ser
   1655 1660 1665
   Asp Asp Val Gln Thr Phe Asp Phe Thr Lys Lys Met Leu Val Asp
   1670 1675 1680
   Lys Val Thr Gly Lys Ile Ile Asp Gly Gly Glu Trp Asn Val Thr
   1685 1690 1695
   Ser His Thr Phe Gly Tyr Lys Asp Thr Pro Val Ile Glu Gly Tyr
   1700 1705 1710
   His Ala Asp Lys Arg Asn Ala Gly Gly Ser Val Val Thr Pro Asp
   1715 1720 1725
   Asp Leu Asn Lys Thr Ile Thr Val Ser Tyr Gln Pro Asn Gly Lys
   1730 1735 1740
   Ile Ile Pro Val Asp Pro Asn Gly Asp Pro Ile Pro Asn Val Pro
   1745 1750 1755
   Thr Pro Thr Tyr Pro Thr Asp Pro Thr Asp Pro Thr Lys Val Val
   1760 1765 1770
   Pro Asp Glu Pro Val Pro Glu Ile Pro Gly Leu Ile Pro Ser Val
   1775 1780 1785
   Pro Thr Val Thr Pro Glu Asn Pro Gly Lys Asp Thr Ser Val Pro
   1790 1795 1800
   Tyr Asn Pro Val Lys Asp Pro Asp Lys Val Thr Thr Val Glu Gly
   1805 1810 1815
   Lys Gln Ile Ile His Phe Val Asp Gly Asp Asn Asn Lys Pro Leu
   1820 1825 1830
   Lys Asp Pro Asn Ile Gln Thr Tyr Glu Phe Lys Ile Thr Asn Gly
   1835                 1840                 1845
   Val Pro Asn Glu Ser Ser His Thr Phe Thr Leu Val Asn Val Pro
       1850                 1855                 1860
   Val Ile Pro Gly Tyr Val Ala Glu Ile Lys Ser Ala Gly Gly Lys
       1865                 1870                 1875
   Thr Val Thr Pro Asp Lys Pro Leu Ala Glu Val Thr Val Val Tyr
       1880                 1885                 1890
   His Lys Ile Gly Lys Ile Val Pro Val Asp Pro Asn Gly Asn Pro
       1895                 1900                 1905
   Ile Pro Asn Val Pro Thr Pro Ser Tyr Thr Asn Asp Pro Thr Asp
       1910                 1915                 1920
   Pro Thr Lys Val Val Pro Asn Glu Pro Val Pro Ala Ile Pro Gly
       1925                 1930                 1935
   Lys Thr Pro Asp Lys Thr Thr Val Thr Pro Val Asp Pro Thr Lys
       1940                 1945                 1950
   Asp Thr Pro Val Val Tyr Lys Asp Asp Lys Val Pro Pro Ala Pro
       1955                 1960                 1965
   Asn Thr Gln Lys Ala Val Val Asn Phe Ile Asp Val Asn Thr Gly
       1970                 1975                 1980
   Lys Ile Ile Ser Thr Ser Gly Ile Leu Ser Gly Arg Pro Gly Glu
       1985                 1990                 1995
   Asp Ile Asn Lys Leu Phe Ser Ser Ala Glu Val Ile Lys Gln Leu
       2000                 2005                 2010
   Glu Ala Ala Gly Tyr Glu Val Val Tyr Asn Ala Phe Asp Gly Asp
       2015                 2020                 2025
   Gly Val Thr Lys Tyr Phe Asp Asn Asp Gly Asn Thr Val Gln Gln
       2030                 2035                 2040
   Phe Thr Ile Ala Leu Lys Leu Lys Glu Lys Ala Lys Thr Pro Asp
       2045                 2050                 2055
   Pro Val Leu Pro Asp Pro Glu Val Pro Val Glu Lys Glu Thr Arg
       2060                 2065                 2070
   Ser Glu Gln Pro Val Lys Gln Asn Val Ala Val Pro Thr Pro Glu
       2075                2080                2085
   Lys Pro Val Glu Lys Lys Thr Thr Asp Asn Lys Glu Val Leu Pro
       2090                2095                2100
   Gln Thr Gly Ala Asp Ser Asn Glu Ala Ala Thr Ile Leu Gly Ala
       2105                2110                2115
   Val Ala Thr Ala Ile Gly Met Thr Ser Leu Ile Gly Ala Lys Arg
       2120                2125                2130
   Arg Lys Lys Asp Asp Lys
       2135

<210> 22
<211> 754
<212> PRT
<213> Lactobacillus johnsonii

<400> 22

Met Ala Asn Thr Asn Ala Ala Phe Thr Ser Lys Thr Gly Ala Ile Val
1               5                   10                  15

Asp Ile Arg Asp Asp Asn Thr Asn Phe Tyr Ala Glu Leu Ile Ser Phe
            20                  25                  30

Pro Leu Gly Ser Ala Asn Ser Arg Ile Asp Ile Gln Asp Pro Leu Met
        35                  40                  45

Leu Asn Leu Gln Arg Tyr Ser Asn Gly Gly Ala Thr Thr Gly Trp Met
    50                  55                  60

Ala Thr Gly Gly Asp Lys Ile Asn Thr Thr Ser Ala Gln Tyr Thr Ala
65                  70                  75                  80

Asn Leu Ile Tyr Met Ser Gly Asn Lys Gly Val Phe Ser Val Ser Gly
                85                  90                  95

Gly Asp Tyr Asp Pro Ser Asn Pro Asn Ser Ser Gly Phe Val Val Tyr
            100                 105                 110

Gln Gln Ile Lys Ser Asp Gly Ser Lys Gln Ile Trp Leu Asn Val Asn
        115                 120                 125

Ser Val Asp Ile Pro Met Asn Gly Phe Gln Thr Lys Asp Ile Trp Asn
    130                 135                 140

Asn Gln Ala Asn Pro Asp Val Ser Ile Ala Gly Asn Gly Leu Thr Ala
145                 150                 155                 160

Gly Ile Lys Ala Asn Gln Val His Asn Phe Asp Gly Ser Pro Leu Thr
                165                 170                 175

Gly Pro Asn Ala Pro Tyr Tyr Gly Ile Ser Thr Gln Arg Ala Ser His
            180                 185                 190

Gln Ile Trp Ile Pro His Arg Thr Ser Leu Glu Val Thr Gly Glu His
        195                 200                 205

Lys Ser Thr Ile Lys Tyr Val Asp Glu Asn Gly Lys Glu Ile Phe Pro
    210                 215                 220

Glu Asn Thr Gln Ser Leu Asp Met Lys Arg Ser Leu Ile Leu Asp Ile
225                 230                 235                 240

Thr Gln Asp Lys Ile Gln Asp Ile Gln Asn Tyr Ala Leu Ser His Thr
                245                 250                 255

Ala Asp Glu Thr Leu Glu Tyr Ile Lys Asn Ser Gln Ala Val Val Gln
            260                 265                 270

Asp Ser Gly Trp Asp Phe Thr Asp Ala Asn Gly Lys Ala Val Ile Asp
        275                 280                 285

Pro Tyr Ser Thr Ile Asp Ser Pro Lys Leu Ala Gly Tyr Ala Ala Thr
    290                 295                 300

Ile Gln Ser Thr Asn Val Ser Thr Leu Lys Val Gly Ala Asp Ala Ser
305                 310                 315                 320

Ala Val Thr Ala Lys Phe Met Val Glu Pro Ser Gln Asp Met Val Gln
                325                 330                 335

Asn Gly Glu Leu Ser Thr Ala Tyr Lys Gln Asn Gly Thr Thr Gly Ile
            340                 345                 350

Pro Ala Asp Tyr Val Thr Val Val Val Tyr Asn Lys Gln Ala Thr Tyr
        355                 360                 365

Gln Ala Gly Lys Thr Asp Ser Lys Ser Val Val Arg Asp Ile Asn Tyr
    370                 375                 380

Leu Asp Gly Lys Thr Gly Lys Lys Ile Pro Ala Asn Leu Ile Lys Asp
385                 390                 395                 400

Asn Pro Val Ser Gln Thr Val Thr Met His Arg Thr Glu Ile Leu Asp
                405                 410                 415

Asn Ser Gly Lys Val Ile Gly Tyr Gly Thr Ile Ser Glu Asp Gly Lys
            420                 425                 430

Ser Tyr Thr Leu Asn Asn Asp Trp Ile Ile Asp Gly Asn Trp Glu Ala
        435                 440                 445

Val Lys Ser Pro Asp Leu Thr Thr Asn Gly Tyr Lys Thr Pro Arg Phe
    450                 455                 460

Glu Asn Gly Asp Ile Ala Lys Val Val Pro Ala Glu Thr Val Asn Glu
465                 470                 475                 480

Asn Thr Pro Asp Thr Thr Val Asn Val Tyr Tyr Asp His Asn Glu Ile
                485                 490                 495

Pro Val Gly Pro Asp Thr Pro Asp Lys His Gly Val Asp Leu Asn Glu
            500                 505                 510

Leu Thr Lys Asp Val Asn Glu Thr Val His Tyr Val Tyr Ser Asp Gly
        515                 520                 525

Thr Lys Ala Ala Pro Asp Val Val Gln Thr Ser Lys Trp Thr Arg Thr
    530                 535                 540

Leu Thr Ile Asp Ala Val Thr Asn Glu Val Ile Ser Asp Gly Gln Tyr
545                 550                 555                 560

Thr Thr Asp Trp Ala Ile Pro Lys Gly Glu Lys Thr Val Tyr Asp Gln
                565                 570                 575

Val Asp Thr Pro Val Ile Lys Gly Tyr His Ala Asp Lys Arg Gln Ile
            580                 585                 590

Asn Ala Thr Val Val Thr Gln Asn Asn Ile Glu Glu Thr Val Thr Tyr
        595                 600                 605

Gln Pro Asn Gly Lys Ile Ile Pro Val Asp Pro Asn Gly Asp Pro Ile
    610                 615                 620

Pro Asn Val Pro Thr Pro Thr Tyr Pro Thr Asp Pro Thr Asp Pro Thr
625                 630                 635                 640

Lys Val Val Pro Asp Glu Pro Val Pro Glu Ile Pro Gly Leu Ile Pro
                645                 650                 655

Ser Val Pro Thr Val Thr Pro Glu Asn Pro Gly Lys Asp Thr Pro Val
            660                 665                 670

Pro Tyr Thr Pro Glu Val Pro Ala Lys Asp Gln Val Ala Val Val Asn
        675                 680                 685

Tyr Ile Asp Ala Asp Asp Asn Asn Ala Ile Ile Thr Ser Ser Gly Asn
    690                 695                 700

Leu Thr Gly Lys Ala Gly Glu Lys Ile Asp Tyr Ser Thr Ala Ser Thr
705                 710                 715                 720

Ile Glu Glu Leu Glu Asn Lys Gly Tyr Val Leu Val Arg Asp Pro Asn
                725                 730                 735

Ser Ser Ser Val Asp Lys Leu Ala Ala Ala Leu Glu His His His His
            740                 745                 750

His His

<210>  23
<211>  2209
<212>  PRT
<213>  Lactobacillus johnsonii

<400>  23

Met Lys Lys Lys Arg Glu Lys Tyr Thr Trp Leu Leu Leu Ser Thr Ala
1               5                   10                  15

Leu Ile Thr Ser Gly Gln Ile Leu Gly Gly Gly Glu Gln Ile Ile Lys
            20                  25                  30

Ala Ser Val Glu Ser Gln Thr Asn Ser Val Lys Lys Ser Lys Thr Ala
        35                  40                  45

Val Glu His Ser Thr Thr Ala Leu Ser Arg Gln Ala Val Glu Ala Gln
    50                  55                  60

Leu Ala Ala Gln Gly Val Asn Phe Glu Arg Leu Thr Pro Glu Glu Gln
65                  70                  75                  80

Gln Glu Val Tyr Val Asp Val Ile Val Gln Leu Glu Ala Leu Pro Ala
                85                  90                  95

Ser Glu Asn Gly Ser Ile Asp Ser Gln Thr Ala Ser Arg Ala Glu Ile
            100                 105                 110

Glu Gln Ala Ser Asn Lys Val Ile Ala Ala Gln Ser Gly Ile Lys Asp
        115                 120                 125

Glu Val Gln Lys Ile Thr Asn Gln Ala Ile Asp Lys Ser Tyr Gly Tyr
    130                 135                 140

Val Val Asn Gly Phe Ala Thr Lys Ala Lys Val Gly Asp Ile Lys Lys
145                 150                 155                 160

Leu Arg Glu Ile Lys Gly Val Lys Ser Val Thr Leu Ala Lys Val Tyr
                165                 170                 175

Phe Ala Ala Asp Thr Ser Ala Asn Asn Met Ala Asn Val Ser Thr Val
            180                 185                 190

Trp Ser Asn Tyr Gln Tyr Lys Gly Glu Gly Thr Val Val Ser Ile Ile
        195                 200                 205

Asp Thr Gly Ile Asp Pro Asn His Lys Asp Leu Arg Leu Ser Asp Glu
    210                 215                 220

Ser Lys Val Lys Leu Thr Ala Lys Asp Ile Asp Gly Phe Thr Glu Asn
225                 230                 235                 240

Ser Gly Tyr Gly Arg Tyr Phe Thr Ser Lys Val Pro Phe Gly His Asn
                245                 250                 255

Tyr Ser Asp Asn Asn Asp Ile Ile Thr Asp Asp Asp Pro Lys Glu Gln
            260                 265                 270

His Gly Met His Val Ala Gly Ile Val Ala Ala Asn Gly Thr Gly Lys
        275                 280                 285

Asn Ser Ala Ser Ser Val Val Gly Val Ala Pro Glu Ala Gln Leu Leu
    290                 295                 300

Ala Met Lys Ala Phe Ser Asn Ser Asp Ser Ser Ser Thr Thr Asp Ser
305                 310                 315                 320

Thr Ser Val Ile Gly Ala Val Asp Asp Ser Ala Lys Leu Gly Ala Asp
                325                 330                 335

Val Leu Asn Met Ser Leu Gly Ser Val Ser Gly Glu Gln Thr Glu Asp
            340                 345                 350

Asp Pro Glu Ile Ala Ala Val Glu Lys Ala Val Lys His Gly Thr Ala

        355                 360                 365

Ala Val Ile Ser Ala Gly Asn Ser Gly Thr Ser Thr Ser Asn Gln Glu
    370                 375                 380

Gly Val Asn Lys Asp Phe Tyr Gly Asn Pro Asp Met Glu Thr Ile Gly
385                 390                 395                 400

Ser Pro Gly Thr Ser Arg Ser Ala Thr Thr Val Ala Ser Ala Glu Asn
                405                 410                 415

Thr Lys Val Thr Thr Asp Gly Met Thr Val Ser Thr Ala Asp Gly Lys
            420                 425                 430

Lys Ile Phe Gly Pro Ser Val Thr Gln Leu Ser Pro Asn Thr Ser His
        435                 440                 445

Asp Ala Phe Asp Ser Lys Lys Phe Tyr Ile Val Lys Asp Ala Ser Gly
    450                 455                 460

Lys Leu Gly Met Gly Thr Pro Ser Gln Tyr Thr Ala Asp Val Lys Gly
465                 470                 475                 480

Lys Val Ala Val Val Ser Arg Gly Glu Ile Thr Phe Thr Asp Lys Gln
                485                 490                 495

Lys Tyr Ala Gln Ala Ala Gly Ala Ala Gly Leu Ile Ile Val Asn Asn
            500                 505                 510

Ala Gly Gly Asn Thr Pro Leu Thr Ser Val Leu Tyr Asn Glu Gly Phe
        515                 520                 525

Pro Thr Ala Gly Leu Ser Thr Asp Asp Gly Asn Lys Leu Val Ala Tyr
    530                 535                 540

Val Glu Ala His Pro Asp Glu Leu Leu Arg Val Asn Val Glu Val Gln
545                 550                 555                 560

Pro Leu Asn Asn Val Ile Arg Glu Glu Asp Leu Met Ser Ser Phe Thr
                565                 570                 575

Ser Tyr Gly Pro Val Ser Asp Leu Ser Phe Lys Pro Asp Ile Thr Ala
            580                 585                 590

Pro Gly Gly Asn Ile Trp Ser Leu Gln Asn Asn Asn Gly Tyr Ile Asn
        595                 600                 605

Met Ser Gly Thr Ser Met Ala Ser Pro Phe Ile Ala Gly Ser Gln Ala
    610                 615                 620

Leu Leu Val Gln Ala Met Asn Asp Lys Thr Gly Lys Phe Tyr Glu Thr
625                 630                 635                 640

Tyr Gln Lys Met Ser Gly Ser Glu Arg Ala Ala Leu Ile Lys Asn Ile
                645                 650                 655

Gln Met Asn Thr Ala Asn Ile Glu Val Asp Val Asp His Gly Ser Val
            660                 665                 670

Ile Glu Ser Pro Arg Arg Gln Gly Ala Gly Leu Val Asn Val Glu Ala
        675                 680                 685

Ala Ile Asn Ala Ile Leu His Asn Pro Ser Thr Val Ser Gly Ser Asn
    690                 695                 700

Gly Tyr Pro Gly Val Glu Leu Lys Asp Phe Gln Asp Arg Gln His Gln
705                 710                 715                 720

Phe Thr Ile Lys Phe Thr Asn Arg Thr Asn Lys Asp Ile Glu Tyr Gly
                725                 730                 735

Leu Asn Glu Asn Gly Lys Phe Ser Asp Val Tyr Thr Ser Glu Thr Asp
            740                 745                 750

Pro Lys Thr Gly Val Leu Phe Glu Lys Lys Ile Asp Gly Ala Ser Leu
        755                 760                 765

Thr Pro Ser Glu Lys Ile Val Val Pro Ala Asn Ser Thr Lys Glu Val
    770                 775                 780

Thr Ile Asn Leu Ser Leu Pro Asp Asn Phe Lys Glu Asn Gln Tyr Val
785                 790                 795                 800

Glu Gly Phe Met Ala Phe Thr Gly Ser Asp Asn Ser His Leu Lys Ile
                805                 810                 815

Pro Tyr Met Gly Phe Phe Gly Asp Trp Ala Ala Pro Ala Ile Phe Asp
            820                 825                 830

Gly Leu Asn Gly Leu Ala Phe Asn Pro Gly Asn Asn Asn Leu Gly Thr
        835                 840                 845

Ile Val Thr Ala Gly Asn Lys Asn Gly Ala Val Gly Tyr Ala Gly Leu
    850                 855                 860

Asn Gln Asp Glu Asp Gly Asn Tyr Arg Val Asp Pro Asp Ala Ile Ala
865                 870                 875                 880

Leu Ser Thr Ala Asp Gly Ala Ser Val Ser Trp Val Arg Pro Gln Tyr
                885                 890                 895

Phe Leu Phe Arg Asn Ala Asn Asp Val Lys Ala Glu Ile Leu Asn Gln
            900                 905                 910

Asp Gly Glu Val Ile Asn Thr Leu Val Ser Leu Ala His Val Thr Lys
        915                 920                 925

Ser Tyr Trp Ala Ala Ser Ser Gln Arg Tyr Ala Lys Phe Asn Tyr Ala
    930                 935                 940

Pro Ala Trp Asp Gly Thr Tyr Phe Asn Gln Gln Thr Asn Lys Thr Glu
945                 950                 955                 960

Lys Val Pro Asp Gly Thr Tyr Thr Tyr Arg Val Thr Gly Thr Val Asp
                965                 970                 975

Gly Thr Lys Lys Gln Gln His Tyr Asp Ile Lys Val Lys Val Asp Ser
            980                 985                 990

Val Lys Pro Glu Val Lys Asn Leu  Lys Leu Gly Ser His  Lys Asp Gln
        995                 1000                 1005

Thr Gly  Lys Val Ser Tyr Val  Leu Lys Ala Glu Ala  Lys Asp Asn
    1010                 1015                 1020

Phe Ser  Gly Leu Asn Gly Gln  Ala Asn Thr Tyr Val  Asn Gly Glu
    1025                 1030                 1035

Leu Asn  Arg Ser Val Ala Tyr  Asp Ile Val Gly Ser  Ser Ser Asp
    1040                 1045                 1050

Gly Tyr  Gln Lys Ile Glu Val  Pro Leu Ser Asp Glu  Gln Val Lys
    1055                 1060                 1065

Thr Leu  Arg Ala Gly Lys Asn  Asp Leu Ala Ile Ala  Val Phe Asp
    1070                 1075                 1080

Asn Ala  Thr Asn Ala Gly Thr  Asn Ser Gly Thr Ser  Asn Lys Pro
    1085                 1090                 1095

Gly Glu  Ile Asn Phe Gly Leu  Ile Ile Asp Asn Asn  Leu Pro Gln
    1100                 1105                 1110

Lys Ile Thr Thr Val Ser Asp Gly Tyr Asp Met Thr Asp Asp Ser
    1115                1120                1125

Tyr Thr Ile Ser Gly Thr Tyr Pro Glu Lys Val Tyr Gly Thr Tyr
    1130                1135                1140

Thr Asp Lys Asp Gly Lys Glu His Asp Leu Asn Ile Ser Tyr Asp
    1145                1150                1155

Glu Ala Ser Glu Arg Phe Val Thr Lys Leu Pro Leu Ser Val Ser
    1160                1165                1170

Asp Tyr Asp Thr Asn Val Lys Phe Tyr Ala Asp Glu Glu His Glu
    1175                1180                1185

Thr Leu Ile Thr Gln Lys Arg Ile Asn Val Ser Leu Val Pro Pro
    1190                1195                1200

Lys Leu Glu Ser Leu Lys Val Asp Asp Gln Glu Thr Tyr Thr Gly
    1205                1210                1215

Asn Glu Glu Ala Lys Leu Ser Gln Thr Ser Glu Asp Thr Val Glu
    1220                1225                1230

Val Ser Gly Lys Val Ser Asp Asp Thr Asp Lys Val Ala Val Lys
    1235                1240                1245

Val Ala Gly Lys Thr Tyr Ser Ala Lys Pro Thr Lys Glu His Thr
    1250                1255                1260

Phe Lys Val Lys Val Pro Val Ser Tyr Gly Glu Asn Thr Met Asn
    1265                1270                1275

Ile Val Leu Thr Asp Lys Asp Gly Asn Ser Ser Ser Val Lys Gln
    1280                1285                1290

Ile Val Lys Ser Ser Asp Arg Gly Lys Thr Val Val Ser Ala Lys
    1295                1300                1305

Asp Val Thr Phe Asp Asn Gly Ile Lys Phe Gly Thr Thr Ser Val
    1310                1315                1320

Asn Thr Glu Thr Glu Asn Tyr Asp Pro Lys Thr Gly Lys Leu Thr
    1325                1330                1335

Leu Thr Gly Lys Val Asn Arg Pro Thr Thr Thr Val Arg Ile Gly

1340                 1345                 1350

Asp His Thr Val Lys Val Lys Ala Asp Gly Thr Phe Lys Leu Val
    1355                 1360                 1365

Leu Asp Leu Gly Lys His Gly Ala Lys Val Phe Pro Val Leu Ile
    1370                 1375                 1380

Gly Asp Thr Thr Val Asn Asp Thr Val Gln Glu Arg Leu Thr Phe
    1385                 1390                 1395

Tyr Val Asp Ser Asn Asn Pro Glu Leu Thr Leu Asn Gln Glu Lys
    1400                 1405                 1410

Asp Gln Ser Gly Tyr Val Pro Val Tyr Thr Asn Lys Glu Glu Phe
    1415                 1420                 1425

Lys Leu Gln Gly Thr Ile Ser Asp Asp Tyr Pro Tyr Tyr Ser Leu
    1430                 1435                 1440

Leu Ile Asn Asp Asn Asn Val Asp Ala Asn Trp Asp Asp Val Asp
    1445                 1450                 1455

Tyr Asn Gly Asn Lys Asn Leu Lys Lys Ser Phe Ser His Ser Val
    1460                 1465                 1470

Lys Leu Lys Glu Gly Lys Asn Thr Phe Asn Val Val Val Val Asp
    1475                 1480                 1485

Asn Asn Asp Asn Arg Ser Glu Val Gln Thr Leu Val Val Tyr Tyr
    1490                 1495                 1500

Lys Lys Ala Gln Lys Leu Ala Ser Pro Gln Ile Thr Ala Thr Thr
    1505                 1510                 1515

Ala Ser Asp Lys Lys Ser Val Thr Val Thr Gly Lys Ala Lys Asp
    1520                 1525                 1530

Gly Asn Val Leu Tyr Ser Thr Asp Asn Gly Asn Lys Tyr Asn Val
    1535                 1540                 1545

Leu Pro Glu Asp Gly Val Thr Val Lys Asn Asn Gly Lys Leu Leu
    1550                 1555                 1560

Phe Lys Thr Val Asp Lys Tyr Gly Asn Glu Ser Glu Val Val Glu
    1565                 1570                 1575

Tyr Asp Val Lys Thr Ile Gly Lys Glu Glu Ser Thr Val Asp Lys
    1580                1585                1590

Ser Val Ala Gln Ala Arg Lys Asp Leu Arg Lys Lys Leu Asp Gln
    1595                1600                1605

Ala Arg Ala Leu Gly Asn Thr Gly Lys Tyr Thr His Glu Ser Ala
    1610                1615                1620

Lys Lys Leu Ala Gln Ala Arg Gln Glu Ala Ser Lys Ala Leu Lys
    1625                1630                1635

Asp Lys Asn Ala Thr Leu Gln Glu Leu Lys Gln Ala Ser Glu Gln
    1640                1645                1650

Leu Glu Glu Ala Ile Lys Asn Leu Val Glu Lys Pro Val Asp Gln
    1655                1660                1665

Asn Lys Asp Lys Asp Lys Val Glu Asp Lys Asp Ser Gln Val Asn
    1670                1675                1680

Ala Leu Lys Glu Lys Leu Glu Glu Thr Val Lys Ala Gly Glu Lys
    1685                1690                1695

Phe Asp Lys Asp Lys Tyr Thr Asp Asp Ser Val Glu Lys Val Thr
    1700                1705                1710

Lys Ala Leu Asp Glu Ala Lys Val Val Leu Ala Asn Lys Asp Ala
    1715                1720                1725

Asn Ser Thr Asp Val Gln Asp Ala Ile Asp Ser Ile Val Asn Ala
    1730                1735                1740

Thr Lys Ser Leu Lys Glu Lys Gln Val Ser Pro Glu Lys Thr Thr
    1745                1750                1755

Gln Gln Glu Asp Thr Pro Lys Glu Asn Lys Asp Thr Glu Glu Val
    1760                1765                1770

Leu Ala Ala Lys Asn Ala Leu Lys Glu Lys Ala Asp Lys Leu Ser
    1775                1780                1785

His Leu Asp Thr Thr Lys Tyr Thr Ser Glu Ser Ala Glu Asn Leu
    1790                1795                1800

Ser Asn Ala Leu Lys Lys Val Asn Gln Val Leu Thr Asn Lys Asp
    1805                1810                1815

Ala Asn Lys Ala Gln Val Gln Glu Ala Leu Asp Ser Leu Ser Gln
    1820                1825                1830

Ala Glu Lys Asp Leu Val Glu Lys Thr Glu Ser Asn Lys Asp Val
    1835                1840                1845

Glu Thr Ala Lys Glu Gln Leu Arg Glu Glu Leu Asn Lys His Lys
    1850                1855                1860

Asp Glu Asp Lys Ser Gln Tyr Thr Asp Asp Ser Ala Lys Val Lys
    1865                1870                1875

Asp Asn Ala Glu Lys Ile Ser Glu Gly Val Leu Asp Ser Lys Asp
    1880                1885                1890

Ala Gln Ala Asp Glu Val Asn Lys Ala Lys Asp Ser Leu Val Glu
    1895                1900                1905

Ala Glu Lys Gly Leu Val Lys Lys Glu Glu Asn Lys Pro Ala Glu
    1910                1915                1920

Ser Asp Thr Gln Glu Val Asp Lys Ala Arg Glu Ala Leu Glu Gln
    1925                1930                1935

Glu Val Asn Lys Asn Ala Asn Val Asn Leu Asp Gly Tyr Thr Pro
    1940                1945                1950

Glu Ser Gln Asp Lys Phe Lys Glu Ile Leu Asn Gly Val Arg Asp
    1955                1960                1965

Val Leu Asn Asp Lys Asn Ala Ser Ala Ser Ser Leu Glu Lys Ala
    1970                1975                1980

Glu Lys Val Leu Glu Thr Ala Thr Gly Val Leu Thr Gln Val Glu
    1985                1990                1995

His Gln Val Glu Leu Pro Lys Val Glu Gln Pro Val Val Thr Pro
    2000                2005                2010

Glu Lys Lys Gln Thr Glu Gln Glu Glu Ala Lys Lys Ser Glu Ser
    2015                2020                2025

Ser Ser Val Ser Thr Ser Lys Asp Glu Val Lys Glu Pro Glu Glu
    2030                2035                2040

Lys Lys Gln Asp Ala His Ser Val Ser Asp Lys Gly Thr Gly Thr
    2045                2050                2055

Ser Val Glu Lys Lys Gly Gln Thr Pro Ala Asp Ala Leu Ser Gln
    2060                2065                2070

Val Glu His Gln Ala Glu Leu Ser Lys Asn Glu Gln Glu Glu Ala
    2075                2080                2085

Lys Lys Ser Glu Ser His Ser Ala Ser Thr Ser Lys Asp Glu Val
    2090                2095                2100

Lys Glu Pro Glu Glu Lys Lys Gln Asp Ala Glu Ser Val Ser Asp
    2105                2110                2115

Lys Glu Ser Ser Thr Leu Val Gly Lys Lys His Gln Val Ala Arg
    2120                2125                2130

Ser Ala Gln Glu Ser Ser Lys Lys Asn Ala Asp Asn Lys Val Pro
    2135                2140                2145

Thr His Lys Asn Ser Asn Lys Ala Gln Asn Thr Thr Asn Ser Ser
    2150                2155                2160

Thr Arg Ser Thr Lys Ser Ser Asn Asn Lys Glu Leu Pro Lys Thr
    2165                2170                2175

Gly Glu Arg Glu Thr Phe Phe Gly Leu Leu Val Thr Gly Ile Thr
    2180                2185                2190

Ala Leu Phe Ala Ser Leu Gly Thr Val Leu Arg Ile Lys Asn Lys
    2195                2200                2205

Lys

<210> 24
<211> 1182
<212> PRT
<213> Lactobacillus johnsonii

<400> 24

Met Ala Tyr Arg Val Thr Gly Thr Val Asp Gly Thr Lys Lys Gln Gln
1               5                   10                  15

His Tyr Asp Ile Lys Val Lys Val Asp Ser Val Lys Pro Glu Val Lys
            20                  25                  30

Asn Leu Lys Leu Gly Ser His Lys Asp Gln Thr Gly Lys Val Ser Tyr
        35                  40                  45

Val Leu Lys Ala Glu Ala Lys Asp Asn Phe Ser Gly Leu Asn Gly Gln
    50                  55                  60

Ala Asn Thr Tyr Val Asn Gly Glu Leu Asn Arg Ser Val Ala Tyr Asp
65                  70                  75                  80

Ile Val Gly Ser Ser Ser Asp Gly Tyr Gln Lys Ile Glu Val Pro Leu
                85                  90                  95

Ser Asp Glu Gln Val Lys Thr Leu Arg Ala Gly Lys Asn Asp Leu Ala
            100                 105                 110

Ile Ala Val Phe Asp Asn Ala Thr Asn Ala Gly Thr Asn Ser Gly Thr
        115                 120                 125

Ser Asn Lys Pro Gly Glu Ile Asn Phe Gly Leu Ile Ile Asp Asn Asn
    130                 135                 140

Leu Pro Gln Lys Ile Thr Thr Val Ser Asp Gly Tyr Asp Met Thr Asp
145                 150                 155                 160

Asp Ser Tyr Thr Ile Ser Gly Thr Tyr Pro Glu Lys Val Tyr Gly Thr
                165                 170                 175

Tyr Thr Asp Lys Asp Gly Lys Glu His Asp Leu Asn Ile Ser Tyr Asp
            180                 185                 190

Glu Ala Ser Glu Arg Phe Val Thr Lys Leu Pro Leu Ser Val Ser Asp
        195                 200                 205

Tyr Asp Thr Asn Val Lys Phe Tyr Ala Asp Glu Glu His Glu Thr Leu
    210                 215                 220

Ile Thr Gln Lys Arg Ile Asn Val Ser Leu Val Pro Pro Lys Leu Glu
225                 230                 235                 240

Ser Leu Lys Val Asp Asp Gln Glu Thr Tyr Thr Gly Asn Glu Glu Ala
                245                 250                 255

Lys Leu Ser Gln Thr Ser Glu Asp Thr Val Glu Val Ser Gly Lys Val
            260                 265                 270

Ser Asp Asp Thr Asp Lys Val Ala Val Lys Val Ala Gly Lys Thr Tyr
        275                 280                 285

Ser Ala Lys Pro Thr Lys Glu His Thr Phe Lys Val Lys Val Pro Val

290                 295                 300

Ser Tyr Gly Glu Asn Thr Met Asn Ile Val Leu Thr Asp Lys Asp Gly
305                 310                 315                 320

Asn Ser Ser Ser Val Lys Gln Ile Val Lys Ser Ser Asp Arg Gly Lys
                325                 330                 335

Thr Val Val Ser Ala Lys Asp Val Thr Phe Asp Asn Gly Ile Lys Phe
            340                 345                 350

Gly Thr Thr Ser Val Asn Thr Glu Thr Glu Asn Tyr Asp Pro Lys Thr
        355                 360                 365

Gly Lys Leu Thr Leu Thr Gly Lys Val Asn Arg Pro Thr Thr Thr Val
    370                 375                 380

Arg Ile Gly Asp His Thr Val Lys Val Lys Ala Asp Gly Thr Phe Lys
385                 390                 395                 400

Leu Val Leu Asp Leu Gly Lys His Gly Ala Lys Val Phe Pro Val Leu
                405                 410                 415

Ile Gly Asp Thr Thr Val Asn Asp Thr Val Gln Glu Arg Leu Thr Phe
            420                 425                 430

Tyr Val Asp Ser Asn Asn Pro Glu Leu Thr Leu Asn Gln Glu Lys Asp
        435                 440                 445

Gln Ser Gly Tyr Val Pro Val Tyr Thr Asn Lys Glu Glu Phe Lys Leu
    450                 455                 460

Gln Gly Thr Ile Ser Asp Asp Tyr Pro Tyr Tyr Ser Leu Leu Ile Asn
465                 470                 475                 480

Asp Asn Asn Val Asp Ala Asn Trp Asp Asp Val Asp Tyr Asn Gly Asn
                485                 490                 495

Lys Asn Leu Lys Lys Ser Phe Ser His Ser Val Lys Leu Lys Glu Gly
            500                 505                 510

Lys Asn Thr Phe Asn Val Val Val Val Asp Asn Asn Asp Asn Arg Ser
        515                 520                 525

Glu Val Gln Thr Leu Val Val Tyr Tyr Lys Lys Ala Gln Lys Leu Ala
    530                 535                 540

Ser Pro Gln Ile Thr Ala Thr Thr Ala Ser Asp Lys Lys Ser Val Thr
545                 550                 555                 560

Val Thr Gly Lys Ala Lys Asp Gly Asn Val Leu Tyr Ser Thr Asp Asn
                565                 570                 575

Gly Asn Lys Tyr Asn Val Leu Pro Glu Asp Gly Val Thr Val Lys Asn
            580                 585                 590

Asn Gly Lys Leu Leu Phe Lys Thr Val Asp Lys Tyr Gly Asn Glu Ser
        595                 600                 605

Glu Val Val Glu Tyr Asp Val Lys Thr Ile Gly Lys Glu Glu Ser Thr
    610                 615                 620

Val Asp Lys Ser Val Ala Gln Ala Arg Lys Asp Leu Arg Lys Lys Leu
625                 630                 635                 640

Asp Gln Ala Arg Ala Leu Gly Asn Thr Gly Lys Tyr Thr His Glu Ser
                645                 650                 655

Ala Lys Lys Leu Ala Gln Ala Arg Gln Glu Ala Ser Lys Ala Leu Lys
            660                 665                 670

Asp Lys Asn Ala Thr Leu Gln Glu Leu Lys Gln Ala Ser Glu Gln Leu
        675                 680                 685

Glu Glu Ala Ile Lys Asn Leu Val Glu Lys Pro Val Asp Gln Asn Lys
    690                 695                 700

Asp Lys Asp Lys Val Glu Asp Lys Asp Ser Gln Val Asn Ala Leu Lys
705                 710                 715                 720

Glu Lys Leu Glu Glu Thr Val Lys Ala Gly Glu Lys Phe Asp Lys Asp
                725                 730                 735

Lys Tyr Thr Asp Asp Ser Val Glu Lys Val Thr Lys Ala Leu Asp Glu
            740                 745                 750

Ala Lys Val Val Leu Ala Asn Lys Asp Ala Asn Ser Thr Asp Val Gln
        755                 760                 765

Asp Ala Ile Asp Ser Ile Val Asn Ala Thr Lys Ser Leu Lys Glu Lys
    770                 775                 780

Gln Val Ser Pro Glu Lys Thr Thr Gln Gln Glu Asp Thr Pro Lys Glu
785                 790                 795                 800

Asn Lys Asp Thr Glu Glu Val Leu Ala Ala Lys Asn Ala Leu Lys Glu
                805                 810                 815

Lys Ala Asp Lys Leu Ser His Leu Asp Thr Thr Lys Tyr Thr Ser Glu
            820                 825                 830

Ser Ala Glu Asn Leu Ser Asn Ala Leu Lys Lys Val Asn Gln Val Leu
        835                 840                 845

Thr Asn Lys Asp Ala Asn Lys Ala Gln Val Gln Glu Ala Leu Asp Ser
    850                 855                 860

Leu Ser Gln Ala Glu Lys Asp Leu Val Glu Lys Thr Glu Ser Asn Lys
865                 870                 875                 880

Asp Val Glu Thr Ala Lys Glu Gln Leu Arg Glu Glu Leu Asn Lys His
                885                 890                 895

Lys Asp Glu Asp Lys Ser Gln Tyr Thr Asp Asp Ser Ala Lys Val Lys
            900                 905                 910

Asp Asn Ala Glu Lys Ile Ser Glu Gly Val Leu Asp Ser Lys Asp Ala
        915                 920                 925

Gln Ala Asp Glu Val Asn Lys Ala Lys Asp Ser Leu Val Glu Ala Glu
    930                 935                 940

Lys Gly Leu Val Lys Lys Glu Glu Asn Lys Pro Ala Glu Ser Asp Thr
945                 950                 955                 960

Gln Glu Val Asp Lys Ala Arg Glu Ala Leu Glu Gln Glu Val Asn Lys
                965                 970                 975

Asn Ala Asn Val Asn Leu Asp Gly Tyr Thr Pro Glu Ser Gln Asp Lys
            980                 985                 990

Phe Lys Glu Ile Leu Asn Gly Val  Arg Asp Val Leu Asn  Asp Lys Asn
        995                 1000                1005

Ala Ser  Ala Ser Ser Leu Glu  Lys Ala Glu Lys Val  Leu Glu Thr
    1010                 1015                1020

Ala Thr  Gly Val Leu Thr Gln  Val Glu His Gln Val  Glu Leu Pro
    1025                 1030                1035

Lys Val  Glu Gln Pro Val Val  Thr Pro Glu Lys Lys  Gln Thr Glu
    1040                 1045                1050

Gln Glu Glu Ala Lys Lys Ser Glu Ser Ser Ser Val Ser Thr Ser
    1055                1060                1065

Lys Asp Glu Val Lys Glu Pro Glu Glu Lys Lys Gln Asp Ala His
    1070                1075                1080

Ser Val Ser Asp Lys Gly Thr Gly Thr Ser Val Glu Lys Lys Gly
    1085                1090                1095

Gln Thr Pro Ala Asp Ala Leu Ser Gln Val Glu His Gln Ala Glu
    1100                1105                1110

Leu Ser Lys Asn Glu Gln Glu Glu Ala Lys Lys Ser Glu Ser His
    1115                1120                1125

Ser Ala Ser Thr Ser Lys Asp Glu Val Lys Glu Pro Glu Glu Lys
    1130                1135                1140

Lys Gln Asp Ala Glu Ser Val Ser Asp Lys Glu Ser Ser Thr Leu
    1145                1150                1155

Val Gly Lys Asp Pro Asn Ser Ser Ser Val Asp Lys Leu Ala Ala
    1160                1165                1170

Ala Leu Glu His His His His His His
    1175                1180

<210>  25
<211>  4734
<212>  PRT
<213>  Lactobacillus johnsonii

<400>  25

Met Val Ser Lys Asn Asn Ile Glu Met Lys Tyr Lys Lys Met Arg Asp
1               5                   10                  15

Glu Lys Gln Arg Phe Ser Ile Arg Lys Phe Ser Val Gly Ala Ala Ser
            20                  25                  30

Val Leu Ile Gly Leu Ser Phe Ser Leu Tyr Asn Gly Gln Gln Ala Phe
        35                  40                  45

Ala Asp Thr Val Asp Asn Gly Asn Lys Ser Val Val Ala Asn Ala Gln
    50                  55                  60

Asp Lys Glu Gln Thr Asp Asn Lys Gln Asn Thr Asp Thr Asn Pro Gln
65                  70                  75                  80

Asn Asn Glu Asp Lys Thr Asn Asn Asn Gln Thr Gln Asn Thr Val Thr
                85                  90                  95

Ala Asp Leu Asp His Ser Thr Val Gly Ala Ser Tyr Lys Ala Asp Thr
            100                 105                 110

Thr Asn Ala Ser Glu Ala Asn Lys Ser Ser Asp Lys Ser Asp Glu Lys
        115                 120                 125

Gln Ala Ala Thr Thr Glu Thr Ala Lys Thr Glu Glu Ala Ala Thr Lys
    130                 135                 140

Glu Val Glu Ala Lys Asn Thr Ala Asn Asp Asp Val Ala Lys Lys Thr
145                 150                 155                 160

Glu Glu Ala Ala Ala Lys Thr Asp Thr Lys Val Asp Val Asn Lys Thr
                165                 170                 175

Val Val Glu Asn Val Lys Thr Glu Ala Ala Thr Pro Asn Val Glu Thr
            180                 185                 190

Arg Asn Ile Glu Thr Gln Thr Ala Lys Thr Asp Glu Glu Lys Val Glu
        195                 200                 205

Thr Ala Val Asp Asn Thr Ser Ser Asp Leu Lys Asn Ala Val Glu Asn
    210                 215                 220

Ala Asn Thr Glu Val Ser Thr Asn Thr Phe Asn Val Asn Lys Ala Val
225                 230                 235                 240

Arg Asn Ala Leu Phe Leu Ser Ala Asn Asn Leu Ala Glu Arg Lys Val
                245                 250                 255

Ala Ala Ile Ala Leu Pro Ala Glu Ala Glu Asp Pro Asn Ala Val Thr
            260                 265                 270

Val Ser Asp Ala Lys Gly Phe Ile Asp Ala Ile Gln Asn Gly Thr Ala
        275                 280                 285

Thr Thr Ile Asn Val Ala Ser Asp Leu Asn Leu Ala Asp Gln Thr Asp
    290                 295                 300

Lys Lys Tyr Thr Glu Ile Asn Ile Lys Asn Lys Arg Asn Ile Val Ile
305                 310                 315                 320

Gln Ser Asp Asn Pro Glu Glu Lys Arg Thr Ile Asp Phe Ser Gly Tyr

325                 330                 335

Ser Phe Asp Met Asn Thr Glu Asn Ser Val Thr Phe Lys Asp Leu Asn
            340                 345                 350

Ile Tyr Ala Arg Ser Tyr Trp Gly Leu Val Tyr Asn Ala Gly Gly Tyr
        355                 360                 365

Thr Phe Asn Asn Val Asn Phe Thr Gly Ser Gln Leu Ile Tyr Thr Lys
    370                 375                 380

Pro Ser Ile Asn Ser Thr Leu Thr Phe Lys Asn Asn Ile Thr Ala Asn
385                 390                 395                 400

Ser Val Ser Ser Tyr Val Gly Pro Leu Asp Gly Lys Thr Arg Asp Thr
                405                 410                 415

Gln Gln Asn Gly Gly Gln Gln Ile Leu Gln Phe Glu Gly Gly Thr Asn
            420                 425                 430

Gln Ile Ile Phe Asp Glu Asn Ser Asn Val Thr Leu Thr Thr Glu Asp
        435                 440                 445

Ala Asn Ala Leu Glu Ile Asp Gly Gly Thr Thr Thr Ile Asp Val Lys
    450                 455                 460

Asp Gly Ala Asn Val Ala Ile Asn Pro His Ser Lys Gly Asn Pro Glu
465                 470                 475                 480

Ser Arg Asn Gly Ile Gly Thr Gly Tyr Val Ala Arg Ala Ile Ala Ala
                485                 490                 495

Asn Ala Lys Met Thr Ile Asn Ile Asp Pro Asn Ser Thr Leu Thr Ile
            500                 505                 510

Asn Thr Glu Lys Val Asp Asp Asp Lys Asp Val Ala Gly Ala Leu Tyr
        515                 520                 525

Leu Asn Ser Asp Ala Ala Leu Asn Val Asn Gly Lys Leu Val Ile Asn
    530                 535                 540

Ser Asn Gly Thr Pro Ser Ala Ser Lys Ser Asn Gly Val Pro Val Tyr
545                 550                 555                 560

Ile Asn Gly Ser Ala Asn Ile Asn Val Gly Asn Gly Gly Ser Phe Val
                565                 570                 575

Leu Asp Ala Thr Asn Leu Gly Asp Tyr Ser Ser Ser Leu Ile Ser Val
            580                 585                 590

Asn Gly Lys Gly Thr Val Lys Leu Asp Pro His Ser Thr Phe Lys Ile
        595                 600                 605

Ser Gly Asp Gly Thr Gly Ala Val Thr Ala Ile Asn Leu Ser Ser Gly
    610                 615                 620

Ser Thr Phe Thr Ser Asp Gln Pro Asp Ser Phe Thr Ile Asp Leu Ser
625                 630                 635                 640

Ala Asn Thr Ser Asn Gly Lys Ser Leu Ile Lys Asn Gly Thr Ile Asn
                645                 650                 655

Phe Ser Arg Val Lys Thr Val Thr Asp Gly Asn Glu Ser Gln Pro Leu
            660                 665                 670

Gly Lys Ile Asp Val Thr Tyr Asp Arg Asn Gly Asn Ala Thr Ser Tyr
        675                 680                 685

Thr Ile Thr Ala Gln Asp Lys Asn Thr Val Asn Gln Val Ala Glu Gly
    690                 695                 700

Leu Ala Asn Lys Ser Leu Ile Ser Leu Val Lys Ala Gly Glu Asp Val
705                 710                 715                 720

Thr Leu Ser Asn Leu His Leu Ser Lys Asn Asn Val Leu Thr Gly Thr
                725                 730                 735

Val Ala Ser Ser Gly Ser Asp Asn Pro Ile Tyr Val Thr Val Thr Val
            740                 745                 750

Gly Gly Val Ser Thr Asn Val Pro Val Val Gly His Tyr Thr Val Tyr
        755                 760                 765

Thr Lys Thr Asn Lys Thr Val Thr Ser Asn Asn Val Asp Tyr Ala Ala
    770                 775                 780

Gln Thr Ala Ser Thr Gly Gly Asn Phe Ser Ile Asp Leu Ser Lys Leu
785                 790                 795                 800

Ala Ser Ser Leu Thr Asn Asp Ala Gln Val Thr Val Thr Ala Thr Lys
                805                 810                 815

Asp Phe Val Glu Ser Ala Gln Thr Lys Ser Val Ala Ala Leu Arg Ala
            820                 825                 830

Leu Asn Thr Thr Thr Leu Lys Glu Leu Val Asp Ala Ala Pro Ala Glu
        835                 840                 845

Glu Ala Lys Pro Ser Tyr Tyr Asn Ala Thr Glu Glu Ala Gln Lys Ala
    850                 855                 860

Tyr Thr Asp Ala Ile Ser Lys Gly Asn Asp Ile Leu Ala Asp Pro Asn
865                 870                 875                 880

Asn Tyr Asp Gln Val Asp Val Asp Asn Ala Val Thr Ala Ile Gln Asn
                885                 890                 895

Ala Gln Lys Ala Leu Thr Gly Lys Glu Thr Asp Lys Lys Ala Leu Gln
            900                 905                 910

Lys Ala Val Asp Asp Ala Ser Lys Val Glu Ser Ser Asp Asn Tyr Thr
        915                 920                 925

Asn Ala Asp Ala Arg Leu Gln Asn Asp Tyr Gln Ala Ala Ile Lys Ala
    930                 935                 940

Gly Gln Gly Ile Leu Ser Lys Glu Asn Ala Thr Gln Thr Glu Val Asp
945                 950                 955                 960

Asn Ala Leu Thr Thr Ile Asn Asn Ala Lys Asp Ala Leu Asn Gly Asp
                965                 970                 975

Ala Lys Lys Ala Ala Ser Lys Glu Ala Leu Gln Lys Ala Val Asp Glu
            980                 985                 990

Ala Pro Thr Val Lys Ser Asp Asp Ala Ala Tyr Tyr Asn Gly Ser Ala
        995                 1000                1005

Glu Ala Lys Thr Ala Tyr Asp Asp Ala Ile Ser Ala Gly Gln Ile
    1010                1015                1020

Val Leu Asp Asn Pro Asp Ala Thr Ala Thr Gln Ile Thr Asp Ala
    1025                1030                1035

Leu Asn Ala Ile Asn Thr Ala Lys Gly Asn Leu Lys Gly Glu Ala
    1040                1045                1050

Thr Asp Lys Ala Ala Leu Gln Thr Ala Val Asp Asn Ser Ala Thr
    1055                1060                1065

Val Lys Glu Ser Asn Asn Tyr Thr Asn Ala Asp Gln Thr Gln Lys
    1070                1075                1080

Thr Ala Tyr Asp Lys Ala Val Thr Ala Ala Gln Thr Val Leu Asp
    1085                1090                1095

Lys Thr Asn Ala Thr Gln Ala Glu Val Asn Gln Ala Leu Gln Asp
    1100                1105                1110

Leu Glu Thr Ala Asn Arg Asn Leu Asn Gly Asp Ala Lys Thr Glu
    1115                1120                1125

Ala Ala Asn Lys Ala Ala Leu Glu Ala Ala Val Lys Asp Ala Pro
    1130                1135                1140

Asn Val Arg Asn Thr Pro Ala Tyr Tyr Asn Gly Ser Glu Glu Thr
    1145                1150                1155

Gln Thr Ala Tyr Asn Asn Ala Ile Thr Ala Gly Gln Thr Val Leu
    1160                1165                1170

Asn Glu Ala Asn Pro Ser Ala Ser Glu Val Lys Asn Ala Leu Asp
    1175                1180                1185

Ala Ile Asn Ala Ala Lys Asp Asn Leu Lys Gly Lys Ala Thr Asn
    1190                1195                1200

Thr Glu Ala Leu Glu Thr Ala Leu Thr Asn Ala Asn Asn Ala Lys
    1205                1210                1215

Glu Thr Gly Asn Tyr Thr Asn Ala Asp Gln Ala Asn Gln Glu Ala
    1220                1225                1230

Leu Asn Asn Ala Ile Ile Ala Gly Gln Glu Ile Leu Lys Asn Thr
    1235                1240                1245

Ser Ala Thr Gln Ala Gln Val Asp Ser Ala Ala Lys Ala Ile Thr
    1250                1255                1260

Asp Ala Ile Ser Gly Leu Asn Gly Asp Thr Asn Leu Ala Asn Ala
    1265                1270                1275

Lys Thr Ala Ala Thr Glu Asp Ile Gln Lys Ala Leu Asp Thr Lys
    1280                1285                1290

Thr Thr Glu Ile Thr Asp Ala Thr Asn Ile Asp Gln Ala Thr Lys
    1295                1300                1305

Asp Gln Leu Ile Ala Asp Ala Lys Lys Ala Ala Glu Asp Ala Asn

1310                 1315                 1320

Thr Ala Ile Asn Gln Ser Thr Asp Thr Asp Thr Val Asn Thr Ala
    1325                 1330                 1335

Lys Thr Glu Gly Ile Ala Asn Ile Asn Lys Val Thr Val Pro Ser
    1340                 1345                 1350

Leu Ala Asp Ala Lys Thr Lys Ala Ala Lys Glu Ile Asp Gln Ala
    1355                 1360                 1365

Leu Thr Asp Lys Thr Lys Glu Ile Asn Asn Ala Glu Asn Ile Asp
    1370                 1375                 1380

Gln Thr Thr Lys Asp Gln Leu Ile Lys Glu Ala Thr Asp Ala Ala
    1385                 1390                 1395

Asn Thr Ala Lys Asp Ala Ile Glu Lys Ser Thr Thr Asn Asp Glu
    1400                 1405                 1410

Ala Thr Lys Ala Gly Gln Ala Gly Val Asp Ala Ile Thr Asn Val
    1415                 1420                 1425

Lys Val Pro Ser Val Thr Asp Ser Gln Asn Ala Ala Lys Glu Val
    1430                 1435                 1440

Ile Asp Asp Ala Leu Asn Ala Lys Thr Lys Glu Ile Asn Asp Ala
    1445                 1450                 1455

Asn Asn Ile Asp Gln Thr Thr Lys Asp Gln Leu Ile Lys Glu Ala
    1460                 1465                 1470

Thr Asp Ala Ala Asn Lys Ala Lys Glu Ala Ile Asp Lys Ala Thr
    1475                 1480                 1485

Thr Ala Asp Ala Ile Lys Thr Ala Gln Asp Glu Gly Thr Thr Asn
    1490                 1495                 1500

Ile Asn Asn Val Thr Val Pro Ser Leu Glu Asp Ala Lys Lys Ala
    1505                 1510                 1515

Ala Asn Lys Ala Val Asp Asp Ala Leu Thr Ala Gln Thr Glu Val
    1520                 1525                 1530

Ile Asn Lys Ala Asn Asn Leu Ser Asp Ala Glu Lys Lys Asp Leu
    1535                 1540                 1545

Ile Asp Gln Val Thr Ala Glu Ala Asn Lys Ala Lys Glu Asn Ile
    1550                1555                1560

Glu Thr Ala Thr Thr Asn Asn Glu Ala Ala Gln Ala Gly Gln Ala
    1565                1570                1575

Gly Val Asp Ala Ile Lys Lys Ile Val Pro Thr Ser Leu Asp Thr
    1580                1585                1590

Val Lys Ser Asp Ala Asn Lys Ala Ile Asp Asp Ala Leu Thr Lys
    1595                1600                1605

Lys Leu Glu Glu Ile Asn Ser Ala Asn Asp Leu Thr Thr Asp Glu
    1610                1615                1620

Lys Thr Ala Leu Thr Gln Glu Ala Asn Thr Ala Ala Asp Lys Ala
    1625                1630                1635

Lys Glu Glu Ile Thr Asn Ala Thr Thr Asn Asp Ala Val Ile Glu
    1640                1645                1650

Ala Gln Asn Asn Gly Val Thr Ala Ile Asp Gly Ile Lys Val Pro
    1655                1660                1665

Thr Glu Ser Ala Val Lys Glu Ala Ala Lys Gln Ala Val Ala Asp
    1670                1675                1680

Ala Ala Thr Ala Lys Asn Gln Ala Ile Asp Ala Ser Asn Leu Thr
    1685                1690                1695

Asp Glu Glu Lys Ala Ala Leu Lys Gln Lys Val Thr Asp Ala Gln
    1700                1705                1710

Asn Ala Ala Asp Gln Ala Ile Asp Asn Ala Thr Thr Asn Ala Ala
    1715                1720                1725

Val Thr Glu Ala Gln Thr Asn Gly Ile Lys Ala Ile Asn Gly Ile
    1730                1735                1740

Glu Val Thr Thr Ser Thr Val Lys Glu Ala Ala Lys Lys Ala Val
    1745                1750                1755

Ala Asp Ala Ala Thr Ala Lys Asn Asn Ala Ile Asp Ala Ser Asn
    1760                1765                1770

Leu Thr Asp Glu Glu Lys Ala Ala Leu Lys Gln Lys Val Thr Asp
    1775                1780                1785

Ala Gln Asn Ala Ala Asp Gln Ala Ile Asp Asn Ala Thr Thr Asn
    1790                1795                1800

Val Ala Val Thr Glu Ala Gln Thr Asn Gly Val Asn Ala Ile Asn
    1805                1810                1815

Gly Ile Glu Val Pro Thr Thr Ser Ala Thr Lys Glu His Ala Ile
    1820                1825                1830

Thr Asp Leu Asn Ala Ala Val Asp Asp Ala Lys Lys Ala Ile Asp
    1835                1840                1845

Gln Asp Ser Asn Leu Thr Asp Glu Glu Lys Gln Ala Ala Lys Asp
    1850                1855                1860

Gln Ile Asp Arg Asp Ala Thr Lys Ala Gln Glu Ala Ile Asn Asn
    1865                1870                1875

Ala Lys Thr Asn Asp Asp Val Lys Lys Ala Gly Asp Ala Gly Thr
    1880                1885                1890

Leu Ala Ile Asp Lys Asp Val Ala Asn Ala Ala Ile Asp Asn Ala
    1895                1900                1905

Val Ala Gly Lys Lys Ala Glu Ile Ser Lys Thr Pro Leu Thr Asp
    1910                1915                1920

Glu Glu Lys Thr Ala Leu Asn Asn Glu Val Asp Gln Lys Ala Gln
    1925                1930                1935

Glu Ala Lys Glu Ala Ile Asn Asn Ala Thr Thr Pro Glu Ala Val
    1940                1945                1950

Thr Thr Ala Gln Asp Ser Gly Val Asn Asn Ile Asn Glu Thr Ser
    1955                1960                1965

Val Pro Ser Glu Ser Ala Ala Lys Gln Ala Ala Lys Glu Ala Val
    1970                1975                1980

Ala Lys Ala Val Asp Glu Lys Asn Ala Ala Ile Asp Ser Ser Asn
    1985                1990                1995

Leu Thr Glu Glu Glu Lys Glu Ala Leu Lys Gln Lys Val Thr Glu
    2000                2005                2010

Ala Gln Thr Ala Ala Asp Gln Ala Ile Asp Asn Ala Thr Thr Asn
    2015                2020                2025

Ala Gly Val Thr Glu Ala Gln Thr Asn Gly Val Asn Ala Ile Asn
    2030                2035                2040

Gly Ile Glu Val Pro Asn Lys Ser Asp Ala Lys Glu Gln Ala Ile
    2045                2050                2055

Thr Asp Leu Asn Thr Ala Val Asp Asn Ala Lys Lys Ala Ile Asp
    2060                2065                2070

Gln Asp Ser Asn Leu Thr Asp Glu Glu Lys Gln Ala Ala Lys Asp
    2075                2080                2085

Gln Ile Asp Ser Asp Ala Lys Asn Ala Gln Asp Ala Ile Asn Asn
    2090                2095                2100

Ala Lys Thr Asn Asp Asp Val Lys Lys Ala Ala Tyr Asp Gly Thr
    2105                2110                2115

Leu Ala Ile Asp Lys Asp Val Ala Asn Ala Ala Ile Asp Asn Ala
    2120                2125                2130

Val Ala Gly Lys Lys Ala Glu Ile Ser Asn Ser Ser Leu Thr Asp
    2135                2140                2145

Glu Glu Lys Thr Ala Leu Asn Asn Glu Val Asp Gln Lys Ala Asn
    2150                2155                2160

Ser Ala Lys Asp Ala Ile Asn Asn Ala Thr Thr Pro Glu Ala Val
    2165                2170                2175

Thr Thr Ala Gln Gly Asn Gly Ile Lys Asn Ile Asn Ala Thr Ser
    2180                2185                2190

Val Pro Thr Thr Ser Thr Ala Lys Glu Ala Ala Lys Lys Ala Val
    2195                2200                2205

Ala Glu Ala Ala Glu Ala Lys Asn Ser Ala Ile Asp Ser Ser Asn
    2210                2215                2220

Leu Thr Asp Glu Glu Lys Ala Ala Leu Lys Gln Lys Val Thr Glu
    2225                2230                2235

Ala Gln Asn Gly Ala Asp His Ala Ile Asp Asn Ala Thr Thr Asn
    2240                2245                2250

Ala Ala Val Thr Glu Ala Lys Asp Asn Gly Ile Ser Ala Ile Asp

2255                    2260                    2265

Gly Ile Glu Val Pro Ile Thr Ser Ala Thr Lys Glu Gln Ala Ile
    2270                    2275                    2280

Thr Asp Leu Asn Thr Ala Val Asp Asp Ala Lys Lys Thr Ile Asp
    2285                    2290                    2295

Gln Asp Asn Asn Leu Thr Asp Glu Gln Lys Gln Ala Ala Lys Asp
    2300                    2305                    2310

Gln Ile Asp Ser Asp Ala Lys Thr Ala Gln Asp Ala Ile Asn Asn
    2315                    2320                    2325

Ala Lys Thr Asp Asn Asp Val Asn Asn Ala Val Asn Ser Gly Lys
    2330                    2335                    2340

Val Ala Ile Asn Lys Asp Val Ala Asn Ala Ala Ile Asp Asn Ala
    2345                    2350                    2355

Val Ala Gly Lys Leu Lys Glu Ile Gln Asp Pro Leu Thr Thr Glu
    2360                    2365                    2370

Glu Lys Gln Ala Tyr Thr Asp Leu Ile Asn Ser Glu Ala Thr Asn
    2375                    2380                    2385

Ala Lys Gln Asn Ile Ala Asn Ala Thr Thr Val Glu Ala Val Thr
    2390                    2395                    2400

Thr Ala Gln Thr Asn Gly Val Asn Glu Ile Thr Asn Thr Glu Ile
    2405                    2410                    2415

Pro Thr Thr Ser Ser Ala Lys Glu Lys Ala Ile Ala Ala Ile Asn
    2420                    2425                    2430

Asp Ala Leu Gln Thr Lys Thr Asp Glu Ile Asn Asn Ala Ser Asn
    2435                    2440                    2445

Ile Asn Thr Gln Glu Lys Thr Asp Leu Ile Asn Gln Ala Thr Glu
    2450                    2455                    2460

Ala Ala Asn Ala Ala Lys Asn Asn Ile Asn Asn Ala Thr Thr Asn
    2465                    2470                    2475

Ala Asp Val Asp Thr Ala Gln Ile Lys Gly Glu Lys Ala Ile Ala
    2480                    2485                    2490

Asp Val Thr Val Pro Asn Leu Ser Asp Val Lys Lys Glu Ser Ile
    2495                2500                2505

Asp Leu Ile Asn Lys Ala Leu Asp Ala Lys Thr Asn Glu Ile Asn
    2510                2515                2520

Asn Ala Ser Asn Leu Ser Gln Asp Glu Lys Gln Gly Leu Ile Asn
    2525                2530                2535

Asp Ala Thr Asn Ala Ala Thr Glu Ala Ile Asn Asn Val Asn Gln
    2540                2545                2550

Ala Gln Thr Asn Asp Asp Ala Lys Ala Ala Ala Thr Thr Gly Val
    2555                2560                2565

Gln Asn Ile Glu Asn Ile Thr Ile Pro Thr Leu Asp Glu Ala Lys
    2570                2575                2580

Lys Asn Ala Asn Gln Ala Ile Asp Asp Ala Leu Asn Ser Lys Val
    2585                2590                2595

Asn Glu Ile Asn Asn Ala Ser Asn Leu Asn Glu Thr Glu Lys Gln
    2600                2605                2610

Lys Leu Val Asp Gln Ala Asn Glu Ala Ala Thr Thr Ala Lys Asn
    2615                2620                2625

Asn Val Glu Lys Ala Thr Thr Asn Asp Asp Ala Arg Asp Ala Ala
    2630                2635                2640

Asn Ala Gly Ile Asp Asn Ile Lys Gly Ile Thr Phe Thr Ser Leu
    2645                2650                2655

Glu Asp Ala Lys Asn Ala Ala Asn Thr Ala Ile Asp Asn Ala Leu
    2660                2665                2670

Gln Val Lys Thr Asp Glu Ile Asn Asn Ala Ser Asn Leu Ser Thr
    2675                2680                2685

Glu Glu Lys Gln Asp Leu Ile Asn Gln Ala Ser Glu Val Ala Lys
    2690                2695                2700

Asn Ala Lys Asp Asn Ile Asn Asn Ala Thr Thr Asn Asp Ala Val
    2705                2710                2715

Thr Glu Ala Gln Asn Lys Gly Ile Ala Asp Ile Ala Asn Val Thr
    2720                2725                2730

Val Pro Ser Leu Asp Gln Val Lys Gln Asp Ala Ile Asn Ala Ile
    2735                2740                2745

Lys Gln Val Gln Asp Ala Lys Asn Lys Gln Ile Ser Ala Ala Ser
    2750                2755                2760

Asn Leu Ser Thr Glu Glu Gln Lys Glu Leu Ser Asp Gln Val Asp
    2765                2770                2775

Lys Ile Ala Asn Asp Ala Ile Ala Gln Ile Asn Asp Ser Ser Thr
    2780                2785                2790

Thr Thr Asn Asp Ala Val Thr Ala Thr Arg Asp Asp Ala Ile Lys
    2795                2800                2805

Gln Ile Thr Asp Leu Phe Ile Pro Thr Leu Asp Gly Ala Gln Thr
    2810                2815                2820

Asp Ala Leu Asn Ala Ile Glu Ser Ala Lys Asn Ala Lys Leu Asn
    2825                2830                2835

Asp Ile Asn Asn Ala Thr His Leu Thr Asp Gln Glu Lys Gln Ala
    2840                2845                2850

Leu Val Asp Gln Thr Asn Lys Val Ala Asp Asp Ala Thr Lys Glu
    2855                2860                2865

Ile Lys Ala Ala Gln Thr Asn Asp Ala Val Lys Ser Ala Glu Thr
    2870                2875                2880

Ala Gly Leu Asp Asn Ile Asn Asn Ile Ala Ile Pro Thr Leu Val
    2885                2890                2895

Gln Lys Gln Gln Glu Ala Ile Glu Glu Leu Asn Val Ala Arg Asp
    2900                2905                2910

Ala Lys Ser Ser Ala Ile Asp Asn Ala Thr Asp Leu Thr Thr Asp
    2915                2920                2925

Glu Lys Asn Ser Leu Lys Asp Lys Val Gln Ala Glu Tyr Ser Asn
    2930                2935                2940

Ala Val Ser Asn Ile Thr Ser Ala Thr Thr Asp Glu Ala Val Thr
    2945                2950                2955

Thr Ala Lys Glu Asn Gly Ile Ala Ala Ile Lys Asp Ile Gln Ile
    2960                2965                2970

Pro Thr Lys Ser Pro Ala Lys Glu Gln Ala Thr Ser Asp Leu Asn
    2975                2980                2985

Thr Ala Val Asp Glu Ala Lys Asn Ala Ile Asp Gln Asp Asn Asn
    2990                2995                3000

Leu Thr Asp Glu Glu Lys Gln Ala Ala Lys Asp Gln Ile Asp Ser
    3005                3010                3015

Asp Ala Lys Lys Ala Gln Glu Ala Ile Asp Asn Ala Thr Thr Asp
    3020                3025                3030

Asp Glu Val Asn Ser Ala Val Asp Asn Gly Lys Leu Ala Ile Asp
    3035                3040                3045

Lys Asp Ile Ala Asn Ala Ala Ile Asp Asn Ala Val Ala Gly Lys
    3050                3055                3060

Lys Ala Glu Ile Ser Lys Ser Ser Leu Thr Ser Glu Glu Lys Ala
    3065                3070                3075

Asp Leu Asn Asn Glu Val Asp Gln Lys Ala Lys Asp Ala Lys Glu
    3080                3085                3090

Ala Ile Lys Ala Ala Thr Thr Pro Glu Thr Val Thr Thr Ala Gln
    3095                3100                3105

Glu Asn Gly Ile Lys Asn Ile Asn Asp Thr Lys Val Pro Thr Glu
    3110                3115                3120

Ser Ala Ala Lys Glu Ala Ala Lys Lys Ala Val Ala Glu Ala Ala
    3125                3130                3135

Glu Ala Lys Asn Asn Ala Ile Asp Ser Ser Asp Leu Thr Asn Glu
    3140                3145                3150

Glu Lys Ala Ala Leu Lys Gln Glu Val Ala Asp Ala Gln Asn Ala
    3155                3160                3165

Ala Asp Arg Ala Ile Asp Ala Ala Thr Thr Asn Thr Ala Val Thr
    3170                3175                3180

Glu Ala Gln Asp Asn Gly Ile Lys Ala Ile Glu Asn Val Thr Val
    3185                3190                3195

Pro Thr Glu Ser Ala Ala Lys Glu Thr Ala Lys Lys Ala Val Ala

3200                3205                3210

Glu Ala Ala Glu Ala Lys Asn Asn Ala Ile Asp Ser Ser Asn Leu
    3215                3220                3225

Thr Ser Glu Glu Lys Ala Ala Leu Lys Gln Glu Val Ala Glu Ala
    3230                3235                3240

Gln Asn Ala Ala Asn Thr Ala Ile Asp Asn Ala Thr Thr Asn Ala
    3245                3250                3255

Ala Val Thr Glu Ala Glu Asp Asn Gly Ile Lys Ala Ile Asn Ser
    3260                3265                3270

Ile Glu Val Pro Thr Lys Ser Asp Ala Lys Glu Gln Ala Thr Ser
    3275                3280                3285

Asp Leu Asn Ser Ala Val Asp Glu Ala Lys Lys Ala Ile Asp Gln
    3290                3295                3300

Asp Ser Asn Leu Thr Asp Glu Glu Lys Gln Val Ala Lys Asp Gln
    3305                3310                3315

Ile Asp Ser Asp Ala Lys Lys Ala Gln Glu Ala Ile Asp Thr Ala
    3320                3325                3330

Lys Thr Asn Asp Asp Val Lys Lys Ala Ile Asp Asp Gly Thr Leu
    3335                3340                3345

Ala Ile Asp Lys Asp Val Ala Asn Ala Ala Ile Asp Asn Ala Val
    3350                3355                3360

Ala Gly Lys Lys Ala Glu Ile Ser Lys Ser Pro Leu Thr Asp Glu
    3365                3370                3375

Glu Lys Thr Ala Leu Asn Asn Glu Val Asp Glu Lys Ala Asn Ser
    3380                3385                3390

Ala Lys Asp Ala Ile Asn Lys Ala Thr Thr Pro Glu Gly Val Thr
    3395                3400                3405

Glu Ala Gln Asn Ser Gly Ile Lys Ser Ile Asp Asp Val Asn Val
    3410                3415                3420

Pro Thr Glu Ser Val Ala Lys Glu Ala Ala Lys Lys Ala Val Ala
    3425                3430                3435

Glu Ala Ala Glu Ala Lys Asn Asn Ala Ile Asp Ser Ser Asn Leu
    3440                3445                3450

Thr Asp Glu Glu Lys Ala Ala Leu Lys Gln Glu Val Ser Asp Ala
    3455                3460                3465

Gln Thr Ala Ala Asn Thr Ala Ile Asp Asn Ala Ile Thr Asn Ala
    3470                3475                3480

Glu Val Thr Glu Ala Glu Asp Asn Gly Val Lys Thr Ile Asn Gly
    3485                3490                3495

Ile Glu Val Pro Thr Lys Ser Thr Thr Lys Glu Gln Ala Thr Asn
    3500                3505                3510

Asp Leu Asn Asn Glu Val Glu Asn Ala Lys Lys Ala Ile Asp Gln
    3515                3520                3525

Asp Asn Asn Leu Thr Asp Glu Gln Lys Gln Ala Ala Lys Asp Gln
    3530                3535                3540

Ile Asp Ser Asp Ala Lys Lys Ala Gln Asp Ala Ile Asn Asn Ala
    3545                3550                3555

Lys Asn Asn Asp Asp Val Lys Lys Ala Val Asp Asp Gly Lys Leu
    3560                3565                3570

Val Ile Asp Lys Asp Val Ala Asn Ala Ala Ile Asp Asn Ala Val
    3575                3580                3585

Ala Gly Lys Lys Asp Glu Ile Ser Lys Ser Pro Leu Thr Asp Glu
    3590                3595                3600

Glu Lys Ala Ala Leu Asn Asn Glu Val Asp Gln Lys Ala Glu Glu
    3605                3610                3615

Ala Lys Glu Ala Ile Lys Ala Ala Thr Thr Pro Gly Ala Val Thr
    3620                3625                3630

Thr Ala Gln Glu Asn Gly Ile Lys Asn Ile Asn Glu Thr Glu Val
    3635                3640                3645

Pro Thr Glu Ser Val Ala Lys Glu Ala Ala Lys Arg Ala Val Ala
    3650                3655                3660

Glu Ala Ala Asp Ala Lys Asn Lys Ala Ile Asp Ser Ser Asn Leu
    3665                3670                3675

Thr Asp Glu Glu Lys Ala Ala Leu Lys Gln Glu Val Ser Asp Ala
    3680                3685                3690

Gln Thr Ala Ala Asn Thr Ala Ile Asp Asn Ala Thr Thr Asn Ala
    3695                3700                3705

Ala Val Thr Glu Ala Glu Asp Asn Gly Ile Lys Ala Ile Asn Ser
    3710                3715                3720

Ile Glu Val Pro Thr Lys Ser Asp Ala Lys Glu Gln Ala Thr Ser
    3725                3730                3735

Asp Leu Asn Ser Ala Val Asp Glu Ala Lys Lys Ala Ile Asp Gln
    3740                3745                3750

Asp Ser Asn Leu Thr Asp Glu Glu Lys Gln Val Ala Lys Asp Gln
    3755                3760                3765

Ile Asp Ser Asp Ala Lys Lys Ala Gln Glu Ala Ile Asp Thr Ala
    3770                3775                3780

Lys Thr Asn Asp Asp Val Lys Lys Ala Ile Asp Asp Gly Thr Leu
    3785                3790                3795

Ala Ile Asp Lys Asp Val Ala Asn Ala Ala Ile Asp Asn Ala Val
    3800                3805                3810

Ala Gly Lys Lys Ala Glu Ile Ser Lys Ser Pro Leu Thr Asp Glu
    3815                3820                3825

Glu Lys Thr Ala Leu Asn Asn Glu Val Asp Glu Lys Ala Asn Ser
    3830                3835                3840

Ala Lys Asp Ala Ile Asn Lys Ala Thr Thr Pro Glu Gly Val Thr
    3845                3850                3855

Glu Ala Gln Asn Ser Gly Ile Lys Ser Ile Asp Asp Val Asn Val
    3860                3865                3870

Pro Thr Glu Ser Val Ala Lys Glu Ala Ala Lys Lys Ala Val Ala
    3875                3880                3885

Glu Ala Ala Glu Ala Lys Asn Asn Ala Ile Asp Ser Ser Asn Leu
    3890                3895                3900

Thr Asp Glu Glu Lys Ala Ala Leu Lys Gln Glu Val Ser Asp Ala
    3905                3910                3915

Gln Thr Ala Ala Asn Thr Ala Ile Asp Asn Ala Ile Thr Asn Ala
    3920                3925                3930

Glu Val Thr Glu Ala Glu Asp Asn Gly Val Lys Thr Ile Asn Gly
    3935                3940                3945

Ile Glu Val Pro Thr Lys Ser Thr Thr Lys Glu Gln Ala Thr Asn
    3950                3955                3960

Asp Leu Asn Asn Glu Val Glu Asn Ala Lys Lys Ala Ile Asp Gln
    3965                3970                3975

Asp Ser Asn Leu Thr Asp Glu Glu Lys Gln Val Ala Lys Asp Gln
    3980                3985                3990

Ile Asp Ser Asp Ala Lys Lys Ala Gln Asp Ala Ile Asn Asn Ala
    3995                4000                4005

Lys Thr Asn Asp Asp Val Lys Lys Ala Ile Asp Asp Gly Thr Leu
    4010                4015                4020

Ala Ile Asp Lys Asp Val Ala Asn Ala Ala Ile Asp Asn Ala Val
    4025                4030                4035

Ala Gly Lys Leu Lys Glu Ile Asn Asp Ser Leu Thr Asn Glu Glu
    4040                4045                4050

Lys Gln Ala Tyr Thr Asp Leu Ile Asn Asn Glu Ala Asp Asn Ala
    4055                4060                4065

Lys Gln Lys Ile Ala Glu Ala Thr Thr Pro Glu Glu Val Thr Arg
    4070                4075                4080

Ala Gln Glu Glu Gly Val Lys Asn Ile Asn Asn Ile Asn Val Pro
    4085                4090                4095

Thr Thr Ser Pro Ala Lys Asp Ala Ala Asn Ala Ala Ile Asp Gln
    4100                4105                4110

Ala Leu Lys Asn Lys Lys Asp Glu Ile Asn Asn Ala Thr Asn Ile
    4115                4120                4125

Ser Ser Glu Glu Lys Thr Asp Leu Ile Lys Gln Ala Thr Glu Ala
    4130                4135                4140

Ala Asn Ile Ala Lys Asp Asn Ile Asn Asn Ala Thr Thr Asn Ser

4145                4150                4155

Glu Val Glu Thr Ala Gln Val Asp Gly Glu Lys Ala Ile Ala Asp
    4160                4165                4170

Val Thr Gly Pro Gly Leu Ser Asp Ile Lys Lys Glu Ser Ile Asp
    4175                4180                4185

Leu Ile Asn Lys Ala Leu Asn Glu Lys Gln Asp Glu Ile Asn Asn
    4190                4195                4200

Ala Ser Asn Leu Ser Gln Asp Glu Ser Thr Glu Leu Ile Asp Gln
    4205                4210                4215

Ala Lys Lys Ile Ala Thr Glu Ala Ile Asn Glu Ile Asn Asn Ala
    4220                4225                4230

Gln Thr Asn Asp Glu Ala Lys Glu Ala Ala Asp Thr Gly Val Lys
    4235                4240                4245

Asn Ile Glu Asn Val Ser Ile Pro Ser Ile Glu Asp Ala Lys Lys
    4250                4255                4260

Asn Ala Thr Gln Ala Ile Asp Asp Ala Leu Asn Ser Lys Lys Asn
    4265                4270                4275

Glu Ile Asn Asn Ala Ser Asn Leu Thr Asp Ser Glu Lys Thr Asp
    4280                4285                4290

Leu Ile Asn Gln Ala Thr Glu Ile Ala Asn Ala Ala Lys Asp Ala
    4295                4300                4305

Ile Asn Ser Ala Thr Thr Asn Thr Ala Val Glu Ala Ala Glu Tyr
    4310                4315                4320

Lys Gly Val Ala Asp Ile Asn Asn Ile His Phe Thr Asn Leu Asp
    4325                4330                4335

Asp Ser Lys Lys Ala Ala Asn Ser Ala Ile Glu Asp Ala Leu Thr
    4340                4345                4350

Thr Lys Lys Asp Glu Ile Asn Asn Ala Ser Asn Leu Ser Asp Ser
    4355                4360                4365

Glu Lys Ala Lys Leu Ile Asn Gln Ala Thr Glu Ile Ala Asn Ala
    4370                4375                4380

Ala Lys Ala Ala Ile Asn Asn Ala Thr Thr Asn Ser Ala Val Thr
    4385                4390                4395

Ala Ala Glu Asn Lys Gly Ile Glu Asp Ile Ala Asn Ile Asn Val
    4400                4405                4410

Pro Ser Leu Ala Glu Thr Lys Gln Ala Ala Ile Asp Ala Ile Gln
    4415                4420                4425

Gln Val Gln Lys Ala Lys Asn Ser Gln Ile Glu Glu Ala Lys Asn
    4430                4435                4440

Leu Ser Ala Asp Glu Gln Lys Asn Leu Ile Asp Gln Val Asn Lys
    4445                4450                4455

Ile Ala Gln Asp Ala Ile Asn Lys Leu Asn Asp Pro Ala Thr Thr
    4460                4465                4470

Thr Asn Glu Val Ile Thr Asp Thr Arg Asp Lys Ala Ile Asp Gln
    4475                4480                4485

Ile Thr Asn Leu Phe Ile Pro Thr Leu Asp Ser Val Gln Lys Asp
    4490                4495                4500

Ala Gln Glu Ala Ile Asn Ser Ala Gln Glu Thr Lys Ile Asp Glu
    4505                4510                4515

Ile Asn Lys Ala Asp Asn Leu Thr Asp Gln Met Lys Gln Asn Leu
    4520                4525                4530

Ile Asp Gln Val Asp Gln Val Ala Asp Lys Ala Thr Lys Ala Ile
    4535                4540                4545

Asn Asn Ala Gln Thr Asn Asp Asp Val Lys Glu Ala Glu Ile Glu
    4550                4555                4560

Gly Leu Glu Asp Ile Asp Ser Ile Lys Val Pro Ser Leu Val Glu
    4565                4570                4575

Ser Lys Asp Asp Ala Ile Lys Glu Ile Asn Asp Ala Leu Lys Lys
    4580                4585                4590

Lys Thr Asp Glu Ile Asn Ala Ala Asp Leu Asp Gln Lys Gln Lys
    4595                4600                4605

Asp Glu Leu Ile Ser Gln Ile Thr Asp Ile Ala Thr Glu Thr Lys
    4610                4615                4620

Thr Lys Val Phe Asn Ala Thr Thr Asn Ala Glu Val Asp Ala Glu
    4625                4630                4635

Ala Glu Ala Gly Ile Lys Ala Ile Glu Ala Val Lys Ile Pro Ala
    4640                4645                4650

Arg Thr Ala Asp Asn Ser Asn Thr Glu Ser Glu Ser Lys Glu Gln
    4655                4660                4665

Thr Val Ile Thr Asn Ser Val Gln Pro Lys Arg Asn Ala Val His
    4670                4675                4680

His Lys Asn Gly Thr Pro Val Asn Lys Lys Ala Thr Leu Pro Gln
    4685                4690                4695

Thr Gly Lys Lys Asp Asn Leu Thr Leu Thr Leu Ala Gly Ala Ala
    4700                4705                4710

Leu Leu Gly Leu Ala Gly Val Phe Ser Leu Phe Gly Leu Gly Asp
    4715                4720                4725

Lys Arg Lys Lys Asn Lys
    4730

<210> 26
<211> 1033
<212> PRT
<213> Lactobacillus johnsonii

<400> 26

Met Val Ala Arg Ala Ile Ala Ala Asn Ala Lys Met Thr Ile Asn Ile
1               5                   10                  15

Asp Pro Asn Ser Thr Leu Thr Ile Asn Thr Glu Lys Val Asp Asp Asp
            20                  25                  30

Lys Asp Val Ala Gly Ala Leu Tyr Leu Asn Ser Asp Ala Ala Leu Asn
        35                  40                  45

Val Asn Gly Lys Leu Val Ile Asn Ser Asn Gly Thr Pro Ser Ala Ser
    50                  55                  60

Lys Ser Asn Gly Val Pro Val Tyr Ile Asn Gly Ser Ala Asn Ile Asn
65                  70                  75                  80

Val Gly Asn Gly Gly Ser Phe Val Leu Asp Ala Thr Asn Leu Gly Asp
                85                  90                  95

Tyr Ser Ser Ser Leu Ile Ser Val Asn Gly Lys Gly Thr Val Lys Leu
            100                 105                 110

Asp Pro His Ser Thr Phe Lys Ile Ser Gly Asp Gly Thr Gly Ala Val
        115                 120                 125

Thr Ala Ile Asn Leu Ser Ser Gly Ser Thr Phe Thr Ser Asp Gln Pro
    130                 135                 140

Asp Ser Phe Thr Ile Asp Leu Ser Ala Asn Thr Ser Asn Gly Lys Ser
145                 150                 155                 160

Leu Ile Lys Asn Gly Thr Ile Asn Phe Ser Arg Val Lys Thr Val Thr
                165                 170                 175

Asp Gly Asn Glu Ser Gln Pro Leu Gly Lys Ile Asp Val Thr Tyr Asp
            180                 185                 190

Arg Asn Gly Asn Ala Thr Ser Tyr Thr Ile Thr Ala Gln Asp Lys Asn
        195                 200                 205

Thr Val Asn Gln Val Ala Glu Gly Leu Ala Asn Lys Ser Leu Ile Ser
    210                 215                 220

Leu Val Lys Ala Gly Glu Asp Val Thr Leu Ser Asn Leu His Leu Ser
225                 230                 235                 240

Lys Asn Asn Val Leu Thr Gly Thr Val Ala Ser Ser Gly Ser Asp Asn
                245                 250                 255

Pro Ile Tyr Val Thr Val Thr Val Gly Gly Val Ser Thr Asn Val Pro
            260                 265                 270

Val Val Gly His Tyr Thr Val Tyr Thr Lys Thr Asn Lys Thr Val Thr
        275                 280                 285

Ser Asn Asn Val Asp Tyr Ala Ala Gln Thr Ala Ser Thr Gly Gly Asn
    290                 295                 300

Phe Ser Ile Asp Leu Ser Lys Leu Ala Ser Ser Leu Thr Asn Asp Ala
305                 310                 315                 320

Gln Val Thr Val Thr Ala Thr Lys Asp Phe Val Glu Ser Ala Gln Thr
                325                 330                 335

Lys Ser Val Ala Ala Leu Arg Ala Leu Asn Thr Thr Thr Leu Lys Glu
            340                 345                 350

Leu Val Asp Ala Ala Pro Ala Glu Glu Ala Lys Pro Ser Tyr Tyr Asn
        355                 360                 365

Ala Thr Glu Glu Ala Gln Lys Ala Tyr Thr Asp Ala Ile Ser Lys Gly
    370                 375                 380

Asn Asp Ile Leu Ala Asp Pro Asn Asn Tyr Asp Gln Val Asp Val Asp
385                 390                 395                 400

Asn Ala Val Thr Ala Ile Gln Asn Ala Gln Lys Ala Leu Thr Gly Lys
                405                 410                 415

Glu Thr Asp Lys Lys Ala Leu Gln Lys Ala Val Asp Asp Ala Ser Lys
            420                 425                 430

Val Glu Ser Ser Asp Asn Tyr Thr Asn Ala Asp Ala Arg Leu Gln Asn
        435                 440                 445

Asp Tyr Gln Ala Ala Ile Lys Ala Gly Gln Gly Ile Leu Ser Lys Glu
    450                 455                 460

Asn Ala Thr Gln Thr Glu Val Asp Asn Ala Leu Thr Thr Ile Asn Asn
465                 470                 475                 480

Ala Lys Asp Ala Leu Asn Gly Asp Ala Lys Lys Ala Ala Ser Lys Glu
                485                 490                 495

Ala Leu Gln Lys Ala Val Asp Glu Ala Pro Thr Val Lys Ser Asp Asp
            500                 505                 510

Ala Ala Tyr Tyr Asn Gly Ser Ala Glu Ala Lys Thr Ala Tyr Asp Asp
        515                 520                 525

Ala Ile Ser Ala Gly Gln Ile Val Leu Asp Asn Pro Asp Ala Thr Ala
    530                 535                 540

Thr Gln Ile Thr Asp Ala Leu Asn Ala Ile Asn Thr Ala Lys Gly Asn
545                 550                 555                 560

Leu Lys Gly Glu Ala Thr Asp Lys Ala Ala Leu Gln Thr Ala Val Asp
                565                 570                 575

Asn Ser Ala Thr Val Lys Glu Ser Asn Asn Tyr Thr Asn Ala Asp Gln
            580                 585                 590

Thr Gln Lys Thr Ala Tyr Asp Lys Ala Val Thr Ala Ala Gln Thr Val

595                 600                 605

Leu Asp Lys Thr Asn Ala Thr Gln Ala Glu Val Asn Gln Ala Leu Gln
    610                 615                 620

Asp Leu Glu Thr Ala Asn Arg Asn Leu Asn Gly Asp Ala Lys Thr Glu
625                 630                 635                 640

Ala Ala Asn Lys Ala Ala Leu Glu Ala Ala Val Lys Asp Ala Pro Asn
                645                 650                 655

Val Arg Asn Thr Pro Ala Tyr Tyr Asn Gly Ser Glu Glu Thr Gln Thr
            660                 665                 670

Ala Tyr Asn Asn Ala Ile Thr Ala Gly Gln Thr Val Leu Asn Glu Ala
        675                 680                 685

Asn Pro Ser Ala Ser Glu Val Lys Asn Ala Leu Asp Ala Ile Asn Ala
    690                 695                 700

Ala Lys Asp Asn Leu Lys Gly Lys Ala Thr Asn Thr Glu Ala Leu Glu
705                 710                 715                 720

Thr Ala Leu Thr Asn Ala Asn Asn Ala Lys Glu Thr Gly Asn Tyr Thr
                725                 730                 735

Asn Ala Asp Gln Ala Asn Gln Glu Ala Leu Asn Asn Ala Ile Ile Ala
            740                 745                 750

Gly Gln Glu Ile Leu Lys Asn Thr Ser Ala Thr Gln Ala Gln Val Asp
        755                 760                 765

Ser Ala Ala Lys Ala Ile Thr Asp Ala Ile Ser Gly Leu Asn Gly Asp
    770                 775                 780

Thr Asn Leu Ala Asn Ala Lys Thr Ala Ala Thr Glu Asp Ile Gln Lys
785                 790                 795                 800

Ala Leu Asp Thr Lys Thr Thr Glu Ile Thr Asp Ala Thr Asn Ile Asp
                805                 810                 815

Gln Ala Thr Lys Asp Gln Leu Ile Ala Asp Ala Lys Lys Ala Ala Glu
            820                 825                 830

Asp Ala Asn Thr Ala Ile Asn Gln Ser Thr Asp Thr Asp Thr Val Asn
        835                 840                 845

Thr Ala Lys Thr Glu Gly Ile Ala Asn Ile Asn Lys Val Thr Val Pro
    850                 855                 860

Ser Leu Ala Asp Ala Lys Thr Lys Ala Ala Lys Glu Ile Asp Gln Ala
865                 870                 875                 880

Leu Thr Asp Lys Thr Lys Glu Ile Asn Asn Ala Glu Asn Ile Asp Gln
                885                 890                 895

Thr Thr Lys Asp Gln Leu Ile Lys Glu Ala Thr Asp Ala Ala Asn Thr
            900                 905                 910

Ala Lys Asp Ala Ile Glu Lys Ser Thr Thr Asn Asp Glu Ala Thr Lys
        915                 920                 925

Ala Gly Gln Ala Gly Val Asp Ala Ile Thr Asn Val Lys Val Pro Ser
    930                 935                 940

Val Thr Asp Ser Gln Asn Ala Ala Lys Glu Val Ile Asp Asp Ala Leu
945                 950                 955                 960

Asn Ala Lys Thr Lys Glu Ile Asn Asp Ala Asn Asn Ile Asp Gln Thr
                965                 970                 975

Thr Lys Asp Gln Leu Ile Lys Glu Ala Thr Asp Ala Ala Asn Lys Ala
            980                 985                 990

Lys Glu Ala Ile Asp Lys Ala Thr  Thr Ala Asp Ala Ile  Lys Thr Ala
        995                 1000                 1005

Gln Asp  Glu Gly Asp Pro Asn  Ser Ser Ser Val Asp  Lys Leu Ala
    1010                 1015                 1020

Ala Ala  Leu Glu His His His  His His His
    1025                 1030

<210>  27
<211>  605
<212>  PRT
<213>  Lactobacillus johnsonii

<400>  27

Met Ser Leu Met Met Ile Ile Leu Asn His Gln Leu Val Pro Asn Arg
1               5                   10                  15

Trp Arg Asn Ile Thr Met Lys Lys Ala Lys Leu Phe Gly Ser Leu Thr
            20                  25                  30

Leu Leu Ser Gly Val Ala Leu Thr Leu Ala Ala Cys Gly Asn Asn Ser
        35                  40                  45

Asn Ser Lys Val Asp Asn Pro Thr Lys Asn Phe Lys Ser Ala Thr Pro
    50                  55                  60

Lys Lys Ala Val Lys Lys Gly Gly Thr Val Ser Val Ala Leu Glu Ser
65                  70                  75                  80

Asp Thr Pro Phe Thr Gly Ile Phe Leu Asn Glu Leu Ser Asp Thr Gln
                85                  90                  95

Asn Asp Ser Asp Ala Met Ala Pro Ala Asn Glu Gly Leu Phe Asp Thr
            100                 105                 110

Asp Asp Thr Tyr Gln Ile Asn Asp Lys Gly Pro Ala Thr Leu Lys Leu
        115                 120                 125

Asp Asn Asp Asn Lys Thr Ala Thr Ile Lys Ile Lys Asn Gly Val Lys
    130                 135                 140

Trp Ser Asp Gly Lys Gln Val Thr Ala Lys Asp Val Glu Tyr Ser Tyr
145                 150                 155                 160

Glu Ile Ile Ala Asn Lys Ala Thr Lys Ser Ser Arg Tyr Thr Asp Ser
                165                 170                 175

Leu Gln Asn Ile Val Gly Met Ser Glu Tyr His Asp Gly Lys Ser Asn
            180                 185                 190

Thr Ile Ser Gly Ile Glu Met Pro Asp Gly Glu Asn Gly Arg Thr Val
        195                 200                 205

Val Ile His Phe Lys Glu Met Lys Pro Gly Met Lys Phe Ser Gly Asn
    210                 215                 220

Gly Tyr Phe Trp Glu Ala Ala Ala Pro Tyr His Tyr Leu Lys Asp Ile
225                 230                 235                 240

Pro Phe Asp Lys Leu Gln Ser Ser Asp Gln Val Arg Lys Lys Pro Leu
                245                 250                 255

Phe Phe Gly Pro Tyr Lys Met Ser Lys Val Val Arg Gly Gln Ala Val
            260                 265                 270

Thr Phe Val Pro Asn Lys Tyr Tyr Trp Arg Gly Thr Pro Lys Leu Asp
        275                 280                 285

Lys Val Thr Ile Gln Val Leu Asn Pro Asn Ser Ala Ser Gln Ala Ile
    290                 295                 300

Lys Ser His Lys Tyr Asp Ile Ala Gly Val Ile Asn Val Gln Trp Lys
305                 310                 315                 320

Asn Val Ala Asn Thr Asn Asn Val Asn Trp Ile Ala Lys Ile Pro Leu
                325                 330                 335

Ser Tyr Arg Tyr Leu Gly Phe Lys Val Gly Lys Trp Asp Ala Ala Lys
            340                 345                 350

Gly Glu Asn Val Met Asp Lys Asn Ala Lys Met Asn Asn Lys Ala Leu
        355                 360                 365

Arg Gln Ala Ile Ala Tyr Gly Met Asn Ile Ser Ala Val Asn Lys Arg
    370                 375                 380

Tyr Thr Asn Gly Leu Thr Phe His Ile Pro Thr Leu Ile Pro Ala Gln
385                 390                 395                 400

Phe Gly Lys Tyr Phe Asp Lys Asn Val Lys Gly Tyr Asp Tyr Asn Ile
                405                 410                 415

Lys Lys Gly Asn Glu Leu Leu Asp Lys Ala Gly Tyr Lys Lys Lys Gly
            420                 425                 430

Lys Tyr Arg Val Gln Pro Asn Gly Lys Pro Leu Thr Ile His Phe Ala
        435                 440                 445

Ala Met Ser Gly Asp Ser Thr Gln Glu Pro Ile Ile Gln Asn Tyr Ile
    450                 455                 460

Gln Gln Trp Lys Lys Leu Gly Leu Asn Val Lys Leu Thr Gly Gly Arg
465                 470                 475                 480

Leu Met Glu Met Asn Ser Phe Tyr Asp Lys Val Gln Asn Asp Ser Lys
                485                 490                 495

Asp Val Asp Met Phe Met Val Ala Trp Ser Leu Ser Ser Glu Pro Ser
            500                 505                 510

Pro Asn Asp Leu Tyr Asn Ala Lys Ala Pro Tyr Asn Phe Ser Arg Phe
        515                 520                 525

Val Thr Ala Lys Asn Thr Lys Leu Leu Gln Glu Met Asp Ser Gln Lys
    530                 535                 540

Ala Phe Asn Thr Asn Tyr Arg Val Lys Lys Phe His Glu Trp Gln Lys
545                 550                 555                 560

Tyr Met Asp Asp Glu Ala Tyr Val Val Pro Val Ser Asn Ser Tyr Ser
                565                 570                 575

Ile Asp Ala Val Asn Ser Lys Ile Thr Gly Tyr Ser Ser Lys Pro Ser
            580                 585                 590

Ala Ala Asn Ser Leu Trp Tyr Asn Val Gly Ile Ala Lys
        595                 600                 605

<210>  28
<211>  585
<212>  PRT
<213>  Lactobacillus johnsonii

<400>  28

Met Ala Cys Gly Asn Asn Ser Asn Ser Lys Val Asp Asn Pro Thr Lys
1               5                   10                  15

Asn Phe Lys Ser Ala Thr Pro Lys Lys Ala Val Lys Lys Gly Gly Thr
            20                  25                  30

Val Ser Val Ala Leu Glu Ser Asp Thr Pro Phe Thr Gly Ile Phe Leu
        35                  40                  45

Asn Glu Leu Ser Asp Thr Gln Asn Asp Ser Asp Ala Met Ala Pro Ala
    50                  55                  60

Asn Glu Gly Leu Phe Asp Thr Asp Asp Thr Tyr Gln Ile Asn Asp Lys
65                  70                  75                  80

Gly Pro Ala Thr Leu Lys Leu Asp Asn Asp Asn Lys Thr Ala Thr Ile
                85                  90                  95

Lys Ile Lys Asn Gly Val Lys Trp Ser Asp Gly Lys Gln Val Thr Ala
            100                 105                 110

Lys Asp Val Glu Tyr Ser Tyr Glu Ile Ile Ala Asn Lys Ala Thr Lys
        115                 120                 125

Ser Ser Arg Tyr Thr Asp Ser Leu Gln Asn Ile Val Gly Met Ser Glu
    130                 135                 140

Tyr His Asp Gly Lys Ser Asn Thr Ile Ser Gly Ile Glu Met Pro Asp
145                 150                 155                 160

Gly Glu Asn Gly Arg Thr Val Val Ile His Phe Lys Glu Met Lys Pro
                165                 170                 175

Gly Met Lys Phe Ser Gly Asn Gly Tyr Phe Trp Glu Ala Ala Ala Pro
            180                 185                 190

Tyr His Tyr Leu Lys Asp Ile Pro Phe Asp Lys Leu Gln Ser Ser Asp
        195                 200                 205

Gln Val Arg Lys Lys Pro Leu Phe Phe Gly Pro Tyr Lys Met Ser Lys
    210                 215                 220

Val Val Arg Gly Gln Ala Val Thr Phe Val Pro Asn Lys Tyr Tyr Trp
225                 230                 235                 240

Arg Gly Thr Pro Lys Leu Asp Lys Val Thr Ile Gln Val Leu Asn Pro
                245                 250                 255

Asn Ser Ala Ser Gln Ala Ile Lys Ser His Lys Tyr Asp Ile Ala Gly
            260                 265                 270

Val Ile Asn Val Gln Trp Lys Asn Val Ala Asn Thr Asn Asn Val Asn
        275                 280                 285

Trp Ile Ala Lys Ile Pro Leu Ser Tyr Arg Tyr Leu Gly Phe Lys Val
    290                 295                 300

Gly Lys Trp Asp Ala Ala Lys Gly Glu Asn Val Met Asp Lys Asn Ala
305                 310                 315                 320

Lys Met Asn Asn Lys Ala Leu Arg Gln Ala Ile Ala Tyr Gly Met Asn
                325                 330                 335

Ile Ser Ala Val Asn Lys Arg Tyr Thr Asn Gly Leu Thr Phe His Ile
            340                 345                 350

Pro Thr Leu Ile Pro Ala Gln Phe Gly Lys Tyr Phe Asp Lys Asn Val
        355                 360                 365

Lys Gly Tyr Asp Tyr Asn Ile Lys Lys Gly Asn Glu Leu Leu Asp Lys
    370                 375                 380

Ala Gly Tyr Lys Lys Lys Gly Lys Tyr Arg Val Gln Pro Asn Gly Lys
385                 390                 395                 400

Pro Leu Thr Ile His Phe Ala Ala Met Ser Gly Asp Ser Thr Gln Glu

405                 410                 415

Pro Ile Ile Gln Asn Tyr Ile Gln Gln Trp Lys Lys Leu Gly Leu Asn
            420                 425                 430

Val Lys Leu Thr Gly Gly Arg Leu Met Glu Met Asn Ser Phe Tyr Asp
        435                 440                 445

Lys Val Gln Asn Asp Ser Lys Asp Val Asp Met Phe Met Val Ala Trp
    450                 455                 460

Ser Leu Ser Ser Glu Pro Ser Pro Asn Asp Leu Tyr Asn Ala Lys Ala
465                 470                 475                 480

Pro Tyr Asn Phe Ser Arg Phe Val Thr Ala Lys Asn Thr Lys Leu Leu
                485                 490                 495

Gln Glu Met Asp Ser Gln Lys Ala Phe Asn Thr Asn Tyr Arg Val Lys
            500                 505                 510

Lys Phe His Glu Trp Gln Lys Tyr Met Asp Asp Glu Ala Tyr Val Val
        515                 520                 525

Pro Val Ser Asn Ser Tyr Ser Ile Asp Ala Val Asn Ser Lys Ile Thr
    530                 535                 540

Gly Tyr Ser Ser Lys Pro Ser Ala Ala Asn Ser Leu Trp Tyr Asn Val
545                 550                 555                 560

Gly Ile Ala Lys Asp Pro Asn Ser Ser Ser Val Asp Lys Leu Ala Ala
                565                 570                 575

Ala Leu Glu His His His His His His
            580                 585

<210>  29
<211>  593
<212>  PRT
<213>  Lactobacillus johnsonii

<400>  29

Met Leu Leu Glu Glu Lys Leu Met Lys Lys Gly Lys Leu Ile Gly Thr
1               5                   10                  15

Leu Thr Leu Leu Ser Gly Val Ala Leu Thr Leu Ala Ala Cys Gly Asn
            20                  25                  30

Ser Asn Ser Asn Asn Thr Ser His Pro Asn Phe Lys Glu Ser Thr Pro

35                  40                  45

Lys Lys Thr Ile Lys Asp Gly Gly Asn Val Ser Val Ala Val Val Thr
    50                  55                  60

Asp Thr Pro Phe Thr Gly Ile Phe Asn Asp Glu Leu Ser Thr Asn Asn
65                  70                  75                  80

Thr Asp Ser Glu Val Met Gln Tyr Gly Asp Glu Ser Leu Phe Ala Thr
                85                  90                  95

Asn Asn Thr Tyr Lys Tyr Val Lys Gly Gly Ala Ala Asp Ile Lys Ile
            100                 105                 110

Asn Lys Asp Ala Lys Thr Ala Thr Ile Thr Ile Asn Pro Lys Val Lys
        115                 120                 125

Trp Ser Asp Gly Gln Pro Leu Val Ala Lys Asp Tyr Glu Tyr Ala Tyr
    130                 135                 140

Glu Ile Ile Ala Asn Lys Ala Thr His Ser Gln Arg Tyr Thr Ser Ser
145                 150                 155                 160

Leu Ala Asn Leu Val Gly Leu Glu Glu Tyr His Asp Gly Lys Ser Asn
                165                 170                 175

Thr Ile Ser Gly Ile Glu Met Pro Asp Gly Glu Asn Gly Arg Thr Val
            180                 185                 190

Val Leu His Phe Lys Glu Met Lys Pro Gly Met Thr Gln Ser Gly Asn
        195                 200                 205

Gly Tyr Ile Trp Glu Ala Ala Ala Pro Tyr His Tyr Leu Lys Asp Val
    210                 215                 220

Pro Phe Asp Lys Leu Ile Ser Ser Asn Lys Ile Arg Lys Asn Pro Leu
225                 230                 235                 240

Phe Tyr Gly Pro Tyr Lys Val Ser Lys Val Val Arg Gly Gln Ser Val
                245                 250                 255

Ser Trp Val Pro Asn Glu His Tyr Tyr Lys Gly Lys Pro His Leu Lys
            260                 265                 270

Lys Ile Thr Ala Ser Val Ile Thr Pro Ala Ser Val Ala Gln Ser Ile
        275                 280                 285

Lys Ser Asn Lys Phe Asp Val Thr Gln Val Ser Asn Ser Gln Trp Pro
    290                 295                 300

Asn Ile Lys Gly Ala Lys Gly Val Asn Phe Ile Ala Asn Ile Pro Leu
305                 310                 315                 320

Ala Tyr Ser Tyr Leu Gly Phe Lys Val Gly Lys Trp Asp Ala Ala Lys
                325                 330                 335

Gly Glu Asn Val Met Asn Lys Asp Ala Lys Met Asn Asn Arg Ser Leu
            340                 345                 350

Arg Gln Ala Ile Ala Tyr Gly Met Asn Val Asp Gln Val Tyr Lys Arg
        355                 360                 365

Tyr Ser Ser Gly Leu Ser Phe Arg Ile Pro Thr Leu Ile Pro Lys Gln
    370                 375                 380

Phe Gly Asp Tyr Phe Asp Lys Asn Val Lys Gly Tyr Thr Tyr Asn Ile
385                 390                 395                 400

Lys Lys Gly Asn Glu Leu Leu Asp Lys Ala Gly Tyr Lys Lys Lys Gly
                405                 410                 415

Thr Tyr Arg Val Gln Pro Asn Gly Lys Pro Leu Thr Ile Arg Leu Ala
            420                 425                 430

Ala Met Ser Gly Ser Lys Val Gln Glu Pro Ile Ile Gln Asn Tyr Ile
        435                 440                 445

Gln Gln Trp Lys Lys Met Gly Leu Asn Val Lys Leu Thr Gly Gly Arg
    450                 455                 460

Leu Met Glu Met Asn Ser Phe Tyr Asp Lys Val Gln Asn Asp Ser Lys
465                 470                 475                 480

Asp Val Asp Met Phe Ile Gly Ala Trp Ser Leu Ser Ser Glu Pro Ser
                485                 490                 495

Pro Gln Asp Leu Tyr Gly Ala Lys Ala Pro Phe Asn Tyr Ser Arg Phe
            500                 505                 510

Val Thr Lys Glu Asn Thr Asp Leu Leu Asn Asp Ile Asp Ser Gln Lys
        515                 520                 525

Ala Phe Asn Asn Ser Tyr Arg Val Lys Lys Phe His Gln Trp Gln Ala
    530                 535                 540

Tyr Met Asp Lys Glu Ala Tyr Val Val Pro Val Ala Asn Ser Tyr Ser
545                 550                 555                 560

Ile Tyr Ala Ile Asn Asn Lys Leu Thr Gly Tyr Ser Leu Glu Pro Ser
                565                 570                 575

Lys Ser Met Gly Gly Gly Phe Pro Asn Trp Tyr Tyr Val Gly Tyr Ala
            580                 585                 590

Lys

<210>  30
<211>  587
<212>  PRT
<213>  Lactobacillus johnsonii

<400>  30

Met Ala Cys Gly Asn Ser Asn Ser Asn Asn Thr Ser His Pro Asn Phe
1               5                   10                  15

Lys Glu Ser Thr Pro Lys Lys Thr Ile Lys Asp Gly Gly Asn Val Ser
            20                  25                  30

Val Ala Val Val Thr Asp Thr Pro Phe Thr Gly Ile Phe Asn Asp Glu
        35                  40                  45

Leu Ser Thr Asn Asn Thr Asp Ser Glu Val Met Gln Tyr Gly Asp Glu
    50                  55                  60

Ser Leu Phe Ala Thr Asn Asn Thr Tyr Lys Tyr Val Lys Gly Gly Ala
65                  70                  75                  80

Ala Asp Ile Lys Ile Asn Lys Asp Ala Lys Thr Ala Thr Ile Thr Ile
                85                  90                  95

Asn Pro Lys Val Lys Trp Ser Asp Gly Gln Pro Leu Val Ala Lys Asp
            100                 105                 110

Tyr Glu Tyr Ala Tyr Glu Ile Ile Ala Asn Lys Ala Thr His Ser Gln
        115                 120                 125

Arg Tyr Thr Ser Ser Leu Ala Asn Leu Val Gly Leu Glu Glu Tyr His
    130                 135                 140

Asp Gly Lys Ser Asn Thr Ile Ser Gly Ile Glu Met Pro Asp Gly Glu
145                 150                 155                 160

Asn Gly Arg Thr Val Val Leu His Phe Lys Glu Met Lys Pro Gly Met
                165                 170                 175

Thr Gln Ser Gly Asn Gly Tyr Ile Trp Glu Ala Ala Ala Pro Tyr His
            180                 185                 190

Tyr Leu Lys Asp Val Pro Phe Asp Lys Leu Ile Ser Ser Asn Lys Ile
        195                 200                 205

Arg Lys Asn Pro Leu Phe Tyr Gly Pro Tyr Lys Val Ser Lys Val Val
    210                 215                 220

Arg Gly Gln Ser Val Ser Trp Val Pro Asn Glu His Tyr Tyr Lys Gly
225                 230                 235                 240

Lys Pro His Leu Lys Lys Ile Thr Ala Ser Val Ile Thr Pro Ala Ser
                245                 250                 255

Val Ala Gln Ser Ile Lys Ser Asn Lys Phe Asp Val Thr Gln Val Ser
            260                 265                 270

Asn Ser Gln Trp Pro Asn Ile Lys Gly Ala Lys Gly Val Asn Phe Ile
        275                 280                 285

Ala Asn Ile Pro Leu Ala Tyr Ser Tyr Leu Gly Phe Lys Val Gly Lys
    290                 295                 300

Trp Asp Ala Ala Lys Gly Glu Asn Val Met Asn Lys Asp Ala Lys Met
305                 310                 315                 320

Asn Asn Arg Ser Leu Arg Gln Ala Ile Ala Tyr Gly Met Asn Val Asp
                325                 330                 335

Gln Val Tyr Lys Arg Tyr Ser Ser Gly Leu Ser Phe Arg Ile Pro Thr
            340                 345                 350

Leu Ile Pro Lys Gln Phe Gly Asp Tyr Phe Asp Lys Asn Val Lys Gly
        355                 360                 365

Tyr Thr Tyr Asn Ile Lys Lys Gly Asn Glu Leu Leu Asp Lys Ala Gly
    370                 375                 380

Tyr Lys Lys Lys Gly Thr Tyr Arg Val Gln Pro Asn Gly Lys Pro Leu
385                 390                 395                 400

Thr Ile Arg Leu Ala Ala Met Ser Gly Ser Lys Val Gln Glu Pro Ile
                405                 410                 415

Ile Gln Asn Tyr Ile Gln Gln Trp Lys Lys Met Gly Leu Asn Val Lys
            420                 425                 430

Leu Thr Gly Gly Arg Leu Met Glu Met Asn Ser Phe Tyr Asp Lys Val
        435                 440                 445

Gln Asn Asp Ser Lys Asp Val Asp Met Phe Ile Gly Ala Trp Ser Leu
    450                 455                 460

Ser Ser Glu Pro Ser Pro Gln Asp Leu Tyr Gly Ala Lys Ala Pro Phe
465                 470                 475                 480

Asn Tyr Ser Arg Phe Val Thr Lys Glu Asn Thr Asp Leu Leu Asn Asp
                485                 490                 495

Ile Asp Ser Gln Lys Ala Phe Asn Asn Ser Tyr Arg Val Lys Lys Phe
            500                 505                 510

His Gln Trp Gln Ala Tyr Met Asp Lys Glu Ala Tyr Val Val Pro Val
        515                 520                 525

Ala Asn Ser Tyr Ser Ile Tyr Ala Ile Asn Asn Lys Leu Thr Gly Tyr
    530                 535                 540

Ser Leu Glu Pro Ser Lys Ser Met Gly Gly Gly Phe Pro Asn Trp Tyr
545                 550                 555                 560

Tyr Val Gly Tyr Ala Lys Asp Pro Asn Ser Ser Ser Val Asp Lys Leu
                565                 570                 575

Ala Ala Ala Leu Glu His His His His His His
            580                 585

<210> 31
<211> 275
<212> PRT
<213> Lactobacillus johnsonii

<400> 31

Met Lys Lys Lys Phe Ser Phe Leu Ile Leu Leu Gly Val Ile Leu Val
1               5                   10                  15

Ser Leu Thr Thr Ala Cys Ser Asn Lys Ser Ser Lys Gln Ala Lys Ser
            20                  25                  30

Thr Gly Pro Ser Ala Ser Ser Leu Ile Asn Ala Lys Phe Asn Ser Ser
        35                  40                  45

Phe Ala Asn Gly His Phe Thr Gln Thr Thr Asn Ser Asn Glu Met Asn
    50                  55                  60

Gln Lys Ser Lys Ser Glu Gly Leu Phe Lys Asp Lys Gly Asp Val Thr
65                  70                  75                  80

Asn Leu Thr Tyr Thr Leu Thr Gln Lys Lys Lys Ser Gln Thr Glu Gln
                85                  90                  95

Met Trp Leu Thr Lys Lys Asp Met Tyr Leu Leu Leu Glu Gln Asn Lys
            100                 105                 110

Gly His Trp Ile Lys Asn Ser Ile Asp Ala Asp Ser Phe Asp Ser Asp
        115                 120                 125

Gln Val Lys Glu Arg Phe Asp Pro Ala Thr Phe Asn Lys Ile Asn Lys
    130                 135                 140

Ser Leu Ala Gln Lys Ala Thr Val Gln Arg Lys Asn Gly Asn Tyr Glu
145                 150                 155                 160

Ile Ser Phe Asp Gly Thr Asp Ala Asn Leu Trp Asn Ala Val Asn Pro
                165                 170                 175

Leu Ile Ile Asp Ala Met Asn Thr Pro Gly Ser Gln Asn Met Gln Val
            180                 185                 190

Ala Arg Leu Val Lys Ser Ala Gln Val Gln Asn Leu Lys Ile Thr Tyr
        195                 200                 205

Leu Ile Asp Pro Thr Thr Lys Asn Val Glu Ser Met Thr Phe Lys Ala
    210                 215                 220

Gln Tyr Thr Ala Gly Gly Lys Tyr Asn Phe Thr Trp Asn Leu Thr Tyr
225                 230                 235                 240

Asp Gln Leu Gly Gln His Ser Asp Leu Ala Val Pro Ser Asp Ile Gln
                245                 250                 255

Lys Asn Ala Ile Ser Ala Glu Asp Ile Lys Lys Ala Gln Glu Glu Gln
            260                 265                 270

Asn Asn Gln
        275

<210>  32

<211>  277
<212>  PRT
<213>  Lactobacillus johnsonii

<400>  32

Met Ala Cys Ser Asn Lys Ser Ser Lys Gln Ala Lys Ser Thr Gly Pro
1               5                   10                  15

Ser Ala Ser Ser Leu Ile Asn Ala Lys Phe Asn Ser Ser Phe Ala Asn
            20                  25                  30

Gly His Phe Thr Gln Thr Thr Asn Ser Asn Glu Met Asn Gln Lys Ser
        35                  40                  45

Lys Ser Glu Gly Leu Phe Lys Asp Lys Gly Asp Val Thr Asn Leu Thr
    50                  55                  60

Tyr Thr Leu Thr Gln Lys Lys Lys Ser Gln Thr Glu Gln Met Trp Leu
65                  70                  75                  80

Thr Lys Lys Asp Met Tyr Leu Leu Leu Glu Gln Asn Lys Gly His Trp
                85                  90                  95

Ile Lys Asn Ser Ile Asp Ala Asp Ser Phe Asp Ser Asp Gln Val Lys
            100                 105                 110

Glu Arg Phe Asp Pro Ala Thr Phe Asn Lys Ile Asn Lys Ser Leu Ala
        115                 120                 125

Gln Lys Ala Thr Val Gln Arg Lys Asn Gly Asn Tyr Glu Ile Ser Phe
    130                 135                 140

Asp Gly Thr Asp Ala Asn Leu Trp Asn Ala Val Asn Pro Leu Ile Ile
145                 150                 155                 160

Asp Ala Met Asn Thr Pro Gly Ser Gln Asn Met Gln Val Ala Arg Leu
                165                 170                 175

Val Lys Ser Ala Gln Val Gln Asn Leu Lys Ile Thr Tyr Leu Ile Asp
            180                 185                 190

Pro Thr Thr Lys Asn Val Glu Ser Met Thr Phe Lys Ala Gln Tyr Thr
        195                 200                 205

Ala Gly Gly Lys Tyr Asn Phe Thr Trp Asn Leu Thr Tyr Asp Gln Leu
    210                 215                 220

Gly Gln His Ser Asp Leu Ala Val Pro Ser Asp Ile Gln Lys Asn Ala

225                 230                 235                 240

Ile Ser Ala Glu Asp Ile Lys Lys Ala Gln Glu Glu Gln Asn Asn Gln
                245                 250                 255

Asp Pro Asn Ser Ser Ser Val Asp Lys Leu Ala Ala Ala Leu Glu His
            260                 265                 270

His His His His His
        275

<210>  33
<211>  296
<212>  PRT
<213>  Lactobacillus johnsonii

<400>  33

Met Arg Lys His Tyr Gln Lys Ile Phe Ala Phe Phe Phe Ala Ile Ser
1               5                   10                  15

Ser Leu Val Leu Ile Leu Thr Gly Cys Ser Lys Lys Glu Asn Ser Ser
            20                  25                  30

Asn Lys Ile Ser Ile Val Thr Ser Thr Asn Val Tyr Ala Asp Ile Ala
        35                  40                  45

Gln Asn Val Leu Gly Lys Tyr Gly Lys Ala Thr Ala Ile Ile Thr Asn
    50                  55                  60

Ser Ala Thr Asp Pro His Asp Phe Glu Pro Thr Thr Ala Asp Ala Lys
65                  70                  75                  80

Lys Val Gln Asn Ala Lys Ile Val Val Ala Asn Gly Leu Gly Tyr Asp
                85                  90                  95

Ser Trp Leu Pro Lys Leu Ala Lys Ser Thr Asn Lys Ser Ala Val Leu
            100                 105                 110

Val Gly Glu Asp Leu Met Asn Leu Lys Asn Gly Ala Asn Pro His Ile
        115                 120                 125

Trp Phe Asp Leu Asn Met Pro Lys Lys Tyr Val Asn Tyr Leu Val Glu
    130                 135                 140

Arg Leu Ser Lys Ile Asp Lys Lys His Ala Ala Tyr Tyr Lys Glu Asn
145                 150                 155                 160

Gly Lys Glu Tyr Leu Ala Lys Ile Asn Lys Ile Gln Lys Ile Ala Asp

                165                 170                 175

Ser Ile Asp Gly Ala Lys Gln Lys Pro Val Tyr Val Ser Glu Pro Val
            180                 185                 190

Phe Asp Tyr Ala Leu Asn Ala Thr His Phe Lys Ile Gly Asp Lys Ala
        195                 200                 205

Phe Glu Glu Ala Ile Glu Asn Glu Thr Asp Pro Ser Ala Lys Ile Ile
    210                 215                 220

His Gln Met Asn Gln Thr Ile Asn Asn Arg Gly Ile Ser Phe Phe Val
225                 230                 235                 240

Lys Asn Ser Gln Val Ser Ser Ser Thr Val Asn Asn Phe Val Lys Arg
                245                 250                 255

Ala Lys Ser Lys Asn Ile Pro Ile Leu Gln Val Arg Glu Thr Ile Pro
            260                 265                 270

Asn Asn Thr Ser Tyr Ile Lys Trp Met Thr Glu Asn Tyr Gln Asn Leu
        275                 280                 285

Ala Asn Ile Asn Lys Lys Leu Asp
    290                 295

<210> 34
<211> 295
<212> PRT
<213> Lactobacillus johnsonii

<400> 34

Met Ala Cys Ser Lys Lys Glu Asn Ser Ser Asn Lys Ile Ser Ile Val
1               5                   10                  15

Thr Ser Thr Asn Val Tyr Ala Asp Ile Ala Gln Asn Val Leu Gly Lys
            20                  25                  30

Tyr Gly Lys Ala Thr Ala Ile Ile Thr Asn Ser Ala Thr Asp Pro His
        35                  40                  45

Asp Phe Glu Pro Thr Thr Ala Asp Ala Lys Lys Val Gln Asn Ala Lys
    50                  55                  60

Ile Val Val Ala Asn Gly Leu Gly Tyr Asp Ser Trp Leu Pro Lys Leu
65                  70                  75                  80

Ala Lys Ser Thr Asn Lys Ser Ala Val Leu Val Gly Glu Asp Leu Met

85                  90                  95

Asn Leu Lys Asn Gly Ala Asn Pro His Ile Trp Phe Asp Leu Asn Met
            100                 105                 110

Pro Lys Lys Tyr Val Asn Tyr Leu Val Glu Arg Leu Ser Lys Ile Asp
        115                 120                 125

Lys Lys His Ala Ala Tyr Tyr Lys Glu Asn Gly Lys Glu Tyr Leu Ala
    130                 135                 140

Lys Ile Asn Lys Ile Gln Lys Ile Ala Asp Ser Ile Asp Gly Ala Lys
145                 150                 155                 160

Gln Lys Pro Val Tyr Val Ser Glu Pro Val Phe Asp Tyr Ala Leu Asn
                165                 170                 175

Ala Thr His Phe Lys Ile Gly Asp Lys Ala Phe Glu Glu Ala Ile Glu
            180                 185                 190

Asn Glu Thr Asp Pro Ser Ala Lys Ile Ile His Gln Met Asn Gln Thr
        195                 200                 205

Ile Asn Asn Arg Gly Ile Ser Phe Phe Val Lys Asn Ser Gln Val Ser
    210                 215                 220

Ser Ser Thr Val Asn Asn Phe Val Lys Arg Ala Lys Ser Lys Asn Ile
225                 230                 235                 240

Pro Ile Leu Gln Val Arg Glu Thr Ile Pro Asn Asn Thr Ser Tyr Ile
                245                 250                 255

Lys Trp Met Thr Glu Asn Tyr Gln Asn Leu Ala Asn Ile Asn Lys Lys
            260                 265                 270

Leu Glu Asp Pro Asn Ser Ser Ser Val Asp Lys Leu Ala Ala Ala Leu
        275                 280                 285

Glu His His His His His His
    290                 295

<210>  35
<211>  434
<212>  PRT
<213>  Lactobacillus johnsonii

<400>  35

Met Lys Phe Thr Lys Lys Phe Ala Ala Val Ala Val Ala Ala Leu Ala

1                   5                   10                  15

Leu Val Gly Thr Thr Ala Cys Ser Asn Ser Lys Ser Asn Asp Ser Ser
                20                  25                  30

Ser Ser Lys Ser Ala Asp Ile Pro Thr Lys Ile Thr Lys Lys Thr Thr
            35                  40                  45

Val Val Phe Trp His Gly Met Thr Gly Thr Gln Glu Lys Thr Leu Gln
    50                  55                  60

Ser Leu Thr Lys Asp Phe Glu Lys Lys Asn Pro Asn Ile Thr Val Lys
65                  70                  75                  80

Leu Glu Asn Gln Gly Lys Tyr Thr Asp Leu Gln Ala Lys Ile Asn Ser
                85                  90                  95

Thr Leu Gln Ser Pro Asn Asn Leu Pro Thr Ile Thr Gln Ala Tyr Pro
                100                 105                 110

Gly Trp Leu Leu Asn Ala Ala Asn Ser Lys Arg Leu Val Asp Met Thr
            115                 120                 125

Pro Tyr Ile Asn Asn Ser Lys Ile Gly Trp Gly Ser Ala Lys Lys Ser
    130                 135                 140

Asp Ile Arg Glu Glu Leu Leu Lys Gly Ala Gln Ile Lys Gly Thr Gln
145                 150                 155                 160

Tyr Gly Ile Pro Phe Asn Lys Ser Val Glu Met Leu Thr Tyr Asn Lys
                165                 170                 175

Asp Leu Leu Asp Lys Tyr Gly Val Lys Val Pro Lys Thr Met Ala Glu
                180                 185                 190

Leu Lys Ala Ala Ser Lys Lys Ile Tyr Glu Lys Ser Asn His Lys Val
            195                 200                 205

Val Gly Ala Gly Phe Asp Ser Leu Ser Asn Tyr Tyr Val Leu Gly Met
    210                 215                 220

Lys Asp Glu Gly Val Asn Phe Ser Lys Asn Ile Asn Phe Thr Gly Ser
225                 230                 235                 240

Lys Ser Lys Lys Val Ile Asn Tyr Tyr Ala Asp Gly Ile Lys Ala Gly
                245                 250                 255

Tyr Phe Lys Thr Ala Gly Ser Ala Gly Tyr Leu Ser Gly Asp Phe Ser
            260                 265                 270

Asn Gln Lys Val Ala Met Phe Val Gly Thr Ser Ala Gly Glu Gly Phe
        275                 280                 285

Val Lys Met Gly Val Gly Asn Lys Phe Gln Tyr Gly Val Ala Pro Arg
    290                 295                 300

Pro Ser Lys Tyr Asn Met Gln Gln Gly Thr Asp Ile Tyr Met Phe Asn
305                 310                 315                 320

Lys Gly Thr Ala Glu Gln Lys Ala Ala Ala Phe Met Tyr Ile Lys Tyr
                325                 330                 335

Leu Leu Gly Lys Asn Gln Gln Leu Thr Trp Ala Asp Gln Thr Gly Tyr
            340                 345                 350

Ile Pro Val Thr Asn Asn Val Ile Asn Ser Ser Ala Tyr Lys Asn Ser
        355                 360                 365

Thr Lys Ser Lys Val Pro Ala Gln Leu Asp Lys Ala Met Lys Asn Leu
    370                 375                 380

Tyr Ser Val Pro Val Val Lys Asn Ser Asn Thr Ala Tyr Thr Gln Leu
385                 390                 395                 400

Asn Ser Ile Met Gln Asn Ile Phe Ala Gln Ala Lys Lys Gly Gln Asn
                405                 410                 415

Trp Asn Ser Gln Ile Asp Ala Gly Lys Thr Lys Phe Glu Ser Ala Trp
            420                 425                 430

Asn Gln

<210>  36
<211>  435
<212>  PRT
<213>  Lactobacillus johnsonii

<400>  36

Met Ala Cys Ser Asn Ser Lys Ser Asn Asp Ser Ser Ser Ser Lys Ser
1               5                   10                  15

Ala Asp Ile Pro Thr Lys Ile Thr Lys Lys Thr Thr Val Val Phe Trp
            20                  25                  30

His Gly Met Thr Gly Thr Gln Glu Lys Thr Leu Gln Ser Leu Thr Lys
        35                  40                  45

Asp Phe Glu Lys Lys Asn Pro Asn Ile Thr Val Lys Leu Glu Asn Gln
    50                  55                  60

Gly Lys Tyr Thr Asp Leu Gln Ala Lys Ile Asn Ser Thr Leu Gln Ser
65                  70                  75                  80

Pro Asn Asn Leu Pro Thr Ile Thr Gln Ala Tyr Pro Gly Trp Leu Leu
                85                  90                  95

Asn Ala Ala Asn Ser Lys Arg Leu Val Asp Met Thr Pro Tyr Ile Asn
            100                 105                 110

Asn Ser Lys Ile Gly Trp Gly Ser Ala Lys Lys Ser Asp Ile Arg Glu
        115                 120                 125

Glu Leu Leu Lys Gly Ala Gln Ile Lys Gly Thr Gln Tyr Gly Ile Pro
    130                 135                 140

Phe Asn Lys Ser Val Glu Met Leu Thr Tyr Asn Lys Asp Leu Leu Asp
145                 150                 155                 160

Lys Tyr Gly Val Lys Val Pro Lys Thr Met Ala Glu Leu Lys Ala Ala
                165                 170                 175

Ser Lys Lys Ile Tyr Glu Lys Ser Asn His Lys Val Val Gly Ala Gly
            180                 185                 190

Phe Asp Ser Leu Ser Asn Tyr Tyr Val Leu Gly Met Lys Asp Glu Gly
        195                 200                 205

Val Asn Phe Ser Lys Asn Ile Asn Phe Thr Gly Ser Lys Ser Lys Lys
    210                 215                 220

Val Ile Asn Tyr Tyr Ala Asp Gly Ile Lys Ala Gly Tyr Phe Lys Thr
225                 230                 235                 240

Ala Gly Ser Ala Gly Tyr Leu Ser Gly Asp Phe Ser Asn Gln Lys Val
                245                 250                 255

Ala Met Phe Val Gly Thr Ser Ala Gly Glu Gly Phe Val Lys Met Gly
            260                 265                 270

Val Gly Asn Lys Phe Gln Tyr Gly Val Ala Pro Arg Pro Ser Lys Tyr
        275                 280                 285

Asn Met Gln Gln Gly Thr Asp Ile Tyr Met Phe Asn Lys Gly Thr Ala
    290                 295                 300

Glu Gln Lys Ala Ala Ala Phe Met Tyr Ile Lys Tyr Leu Leu Gly Lys
305                 310                 315                 320

Asn Gln Gln Leu Thr Trp Ala Asp Gln Thr Gly Tyr Ile Pro Val Thr
                325                 330                 335

Asn Asn Val Ile Asn Ser Ser Ala Tyr Lys Asn Ser Thr Lys Ser Lys
            340                 345                 350

Val Pro Ala Gln Leu Asp Lys Ala Met Lys Asn Leu Tyr Ser Val Pro
        355                 360                 365

Val Val Lys Asn Ser Asn Thr Ala Tyr Thr Gln Leu Asn Ser Ile Met
    370                 375                 380

Gln Asn Ile Phe Ala Gln Ala Lys Lys Gly Gln Asn Trp Asn Ser Gln
385                 390                 395                 400

Ile Asp Ala Gly Lys Thr Lys Phe Glu Ser Ala Trp Asn Gln Asp Pro
                405                 410                 415

Asn Ser Ser Ser Val Asp Lys Leu Ala Ala Ala Leu Glu His His His
            420                 425                 430

His His His
        435

<210>  37
<211>  295
<212>  PRT
<213>  Lactobacillus johnsonii

<400>  37

Val Lys Ser Lys Ser Tyr Lys Tyr Gly Ile Ala Ala Leu Val Gly Val
1               5                   10                  15

Ser Ala Leu Ala Leu Ala Gly Cys Gln Ser Asn Glu Asn Gln Ser Ser
            20                  25                  30

Thr Ser Lys Thr Asn Ala Lys Ser Lys Ser Ser Ile Val Lys Ile Glu
        35                  40                  45

Lys Lys Ser Lys Ser Lys Ser Ser Lys Ser Glu Lys Gln Glu Gly Glu
    50                  55                  60

Val Lys Lys Gln Ser Ser Glu Lys Val Asn Lys Ala Ser Ser Ser Lys
65                  70                  75                  80

Lys Asp Val Glu Lys Val Pro Thr Val Thr Glu Leu Met Asn Asn Val
                85                  90                  95

Ser Ala Phe Asn Glu Gly Asn Phe Asp Ile Arg Leu Asn Tyr Ala Thr
            100                 105                 110

Val Lys Gly Tyr Asp Thr Gly Ile Ile Gln Glu Asn Pro Arg Val Val
        115                 120                 125

His Tyr Lys Phe Asn Gly Gly Met Asn Ser Glu Val Trp Ala Thr Asp
    130                 135                 140

Gln Phe Val Tyr Ser Leu Ser Gly Asp Lys Trp Thr Lys Ile Gly Gln
145                 150                 155                 160

Thr Tyr Lys Ile Gly Arg Phe Asp Pro Met Ile Ser Leu Pro Ser Pro
                165                 170                 175

Leu Ile Asn Gln Met Lys Val Ser Lys Thr Ser Asp Gly Tyr Gln Ala
            180                 185                 190

Lys Tyr Glu Gly Asn Ser Ala Glu Ala Lys Glu Leu Met Glu His Tyr
        195                 200                 205

Ile Val Trp Val Ser Ser Glu Asn Lys Trp Asn Pro Phe Gly Asp Gln
    210                 215                 220

Asp Phe Thr Ser Ala Thr Leu Thr Tyr Ser Phe Asn Lys Gln Lys Gln
225                 230                 235                 240

Leu Thr Glu Ile His Tyr Asp Trp Thr Ala Gly Ser Asn Lys Gly Thr
                245                 250                 255

Ala Ser Tyr Thr Asn Ile Asn Gln Gln Lys Ile Ala Val Pro Asn Asp
            260                 265                 270

Val Ile Gln Ala Ala Ala Lys Asn Ser Asp Ser Ile Asp Ser Asn Asn
        275                 280                 285

Asp Asp Asn Ser Asp Asp Glu
    290                 295

<210>  38
<211>  295

<212>  PRT
<213>  Lactobacillus johnsonii

<400>  38

Met Gly Cys Gln Ser Asn Glu Asn Gln Ser Ser Thr Ser Lys Thr Asn
1               5                   10                  15

Ala Lys Ser Lys Ser Ser Ile Val Lys Ile Glu Lys Lys Ser Lys Ser
            20                  25                  30

Lys Ser Ser Lys Ser Glu Lys Gln Glu Gly Glu Val Lys Lys Gln Ser
        35                  40                  45

Ser Glu Lys Val Asn Lys Ala Ser Ser Ser Lys Lys Asp Val Glu Lys
    50                  55                  60

Val Pro Thr Val Thr Glu Leu Met Asn Asn Val Ser Ala Phe Asn Glu
65                  70                  75                  80

Gly Asn Phe Asp Ile Arg Leu Asn Tyr Ala Thr Val Lys Gly Tyr Asp
                85                  90                  95

Thr Gly Ile Ile Gln Glu Asn Pro Arg Val Val His Tyr Lys Phe Asn
            100                 105                 110

Gly Gly Met Asn Ser Glu Val Trp Ala Thr Asp Gln Phe Val Tyr Ser
        115                 120                 125

Leu Ser Gly Asp Lys Trp Thr Lys Ile Gly Gln Thr Tyr Lys Ile Gly
    130                 135                 140

Arg Phe Asp Pro Met Ile Ser Leu Pro Ser Pro Leu Ile Asn Gln Met
145                 150                 155                 160

Lys Val Ser Lys Thr Ser Asp Gly Tyr Gln Ala Lys Tyr Glu Gly Asn
                165                 170                 175

Ser Ala Glu Ala Lys Glu Leu Met Glu His Tyr Ile Val Trp Val Ser
            180                 185                 190

Ser Glu Asn Lys Trp Asn Pro Phe Gly Asp Gln Asp Phe Thr Ser Ala
        195                 200                 205

Thr Leu Thr Tyr Ser Phe Asn Lys Gln Lys Gln Leu Thr Glu Ile His
    210                 215                 220

Tyr Asp Trp Thr Ala Gly Ser Asn Lys Gly Thr Ala Ser Tyr Thr Asn
225                 230                 235                 240

Ile Asn Gln Gln Lys Ile Ala Val Pro Asn Asp Val Ile Gln Ala Ala
                245                 250                 255

Ala Lys Asn Ser Asp Ser Ile Asp Ser Asn Asn Asp Asp Asn Ser Asp
            260                 265                 270

Asp Glu Asp Pro Asn Ser Ser Ser Val Asp Lys Leu Ala Ala Ala Leu
        275                 280                 285

Glu His His His His His His
    290                 295

<210>  39
<211>  379
<212>  PRT
<213>  Lactobacillus johnsonii

<400>  39

Leu Lys Arg Phe Leu Gln Ile Ala Leu Leu Leu Ala Thr Gly Leu Ser
1               5                   10                  15

Leu Ser Ala Cys Gly Asn Leu Lys Asn Ser Asp Leu Ala Asn Asn Pro
            20                  25                  30

Thr Thr Ser Thr Thr Lys Lys Lys Ser Tyr Gln Thr Thr Ser Thr Ser
        35                  40                  45

Lys Asn Gly Tyr Asn Val Leu Leu Lys Asp Gly Glu Tyr Val Thr Ser
    50                  55                  60

Pro Ile Glu Gly Thr Thr Glu Asn Thr Ser Asp Asn Asn Val Asp Ser
65                  70                  75                  80

Arg Ala Leu Glu Ser Gly Leu Ile Asp Leu Ser His Asn Thr Phe Ser
                85                  90                  95

Ser Ser Lys Tyr Val Phe Gln Glu Gly Gln Lys Ile Ser Val Ala Asp
            100                 105                 110

Ala Thr Asp Trp Leu Gly Arg Lys Ser Lys Ser Asn Pro Glu Gly Leu
        115                 120                 125

Asn Ala Glu Lys Ser Asn Lys Lys Asn Ser Tyr Asn Pro Ile Ile Leu
    130                 135                 140

Asp Gln Ile Leu Glu Gln Asp Phe Leu Thr Lys Ser Asn Ser Ser Tyr
145                 150                 155                 160

Lys Met Asp Gly Met Ser Ile Gly Leu Ala Leu Asn Ser Val Asp Tyr
                165                 170                 175

Tyr Gln Lys Val Lys Asp Gly Pro Gln Tyr His Lys Asp Ile Ser Arg
            180                 185                 190

Ala Glu Gln Glu Thr Phe Gly Lys Glu Ala Ala Asn Lys Leu Val Ser
        195                 200                 205

Tyr Leu Arg Lys Lys Lys Gly Leu Lys Asp Ile Pro Ile Leu Val Gly
    210                 215                 220

Leu Phe Ser Lys Thr Ser Lys Asp Ser Leu Val Gly Gly Asn Tyr Phe
225                 230                 235                 240

Ala Tyr Gly Ile Ala Asn Ala Asn Ser Ser Lys Ile Asn Asp Trp Lys
                245                 250                 255

Thr Val Asn Asn Arg Ser Gln Val Leu Pro Thr Val Gly Ser Glu Lys
            260                 265                 270

Ala Ile Asn Ser Thr Asp Ala Ala Ser Phe Ser Asp Phe Lys Ala Ala
        275                 280                 285

Ile Gln Gly Tyr Phe Pro Asn Ile Ser Gly Val Thr Ala Thr Val His
    290                 295                 300

Tyr Gln Asn Lys Thr Leu Thr Gln Met Asn Ile Thr Val Thr Thr Gln
305                 310                 315                 320

Phe Phe Gly Tyr Ala Gln Ile Glu Ser Phe Ser Arg Leu Val Leu Ser
                325                 330                 335

Ala Ala Lys Lys Tyr Leu Pro Lys Asp Ala Pro Ile Glu Ile Lys Ile
            340                 345                 350

Asn Ser Val Asn Asp Thr Gln Ala Leu Ile Ala Lys Asn Ser Ala Asp
        355                 360                 365

Asp Ser Tyr Tyr Val His Val Phe Gly Gly Glu
    370                 375

<210>  40
<211>  383
<212>  PRT
<213>  Lactobacillus johnsonii

<400> 40

Met Ala Cys Gly Asn Leu Lys Asn Ser Asp Leu Ala Asn Asn Pro Thr
1               5                   10                  15

Thr Ser Thr Thr Lys Lys Lys Ser Tyr Gln Thr Thr Ser Thr Ser Lys
            20                  25                  30

Asn Gly Tyr Asn Val Leu Leu Lys Asp Gly Glu Tyr Val Thr Ser Pro
        35                  40                  45

Ile Glu Gly Thr Thr Glu Asn Thr Ser Asp Asn Asn Val Asp Ser Arg
    50                  55                  60

Ala Leu Glu Ser Gly Leu Ile Asp Leu Ser His Asn Thr Phe Ser Ser
65                  70                  75                  80

Ser Lys Tyr Val Phe Gln Glu Gly Gln Lys Ile Ser Val Ala Asp Ala
                85                  90                  95

Thr Asp Trp Leu Gly Arg Lys Ser Lys Ser Asn Pro Glu Gly Leu Asn
            100                 105                 110

Ala Glu Lys Ser Asn Lys Lys Asn Ser Tyr Asn Pro Ile Ile Leu Asp
        115                 120                 125

Gln Ile Leu Glu Gln Asp Phe Leu Thr Lys Ser Asn Ser Ser Tyr Lys
    130                 135                 140

Met Asp Gly Met Ser Ile Gly Leu Ala Leu Asn Ser Val Asp Tyr Tyr
145                 150                 155                 160

Gln Lys Val Lys Asp Gly Pro Gln Tyr His Lys Asp Ile Ser Arg Ala
                165                 170                 175

Glu Gln Glu Thr Phe Gly Lys Glu Ala Ala Asn Lys Leu Val Ser Tyr
            180                 185                 190

Leu Arg Lys Lys Lys Gly Leu Lys Asp Ile Pro Ile Leu Val Gly Leu
        195                 200                 205

Phe Ser Lys Thr Ser Lys Asp Ser Leu Val Gly Gly Asn Tyr Phe Ala
    210                 215                 220

Tyr Gly Ile Ala Asn Ala Asn Ser Ser Lys Ile Asn Asp Trp Lys Thr
225                 230                 235                 240

Val Asn Asn Arg Ser Gln Val Leu Pro Thr Val Gly Ser Glu Lys Ala

245                 250                 255

Ile Asn Ser Thr Asp Ala Ala Ser Phe Ser Asp Phe Lys Ala Ala Ile
            260                 265                 270

Gln Gly Tyr Phe Pro Asn Ile Ser Gly Val Thr Ala Thr Val His Tyr
        275                 280                 285

Gln Asn Lys Thr Leu Thr Gln Met Asn Ile Thr Val Thr Thr Gln Phe
    290                 295                 300

Phe Gly Tyr Ala Gln Ile Glu Ser Phe Ser Arg Leu Val Leu Ser Ala
305                 310                 315                 320

Ala Lys Lys Tyr Leu Pro Lys Asp Ala Pro Ile Glu Ile Lys Ile Asn
                325                 330                 335

Ser Val Asn Asp Thr Gln Ala Leu Ile Ala Lys Asn Ser Ala Asp Asp
            340                 345                 350

Ser Tyr Tyr Val His Val Phe Gly Gly Glu Asp Pro Asn Ser Ser Ser
        355                 360                 365

Val Asp Lys Leu Ala Ala Ala Leu Glu His His His His His His
    370                 375                 380

<210>  41
<211>  33
<212>  DNA
<213>  Artificial Sequence

<220>
<223>  oligonucleotide primer

<400>  41
atatatccat ggcttatcgc gtgactggta cgg                                33

<210>  42
<211>  31
<212>  DNA
<213>  Artificial Sequence

<220>
<223>  oligonucleotide primer

<400>  42
atatatggat ccttccctac taatgtgctg c                                  31

<210>  43
<211>  28
<212>  DNA
<213>  Artificial Sequence

<220>
<223>  oligonucleotide primer

<400>  43
atatatccat ggctaacaca aatgctgc                                          28


<210>  44
<211>  30
<212>  DNA
<213>  Artificial Sequence

<220>
<223>  oligonucleotide primer

<400>  44
atatatggat cccttacaag tacgtaaccc                                        30


<210>  45
<211>  32
<212>  DNA
<213>  Artificial Sequence

<220>
<223>  oligonucleotide primer

<400>  45
atatatccat ggttgctcgt gctattgctg cc                                     32


<210>  46
<211>  30
<212>  DNA
<213>  Artificial Sequence

<220>
<223>  oligonucleotide primer

<400>  46
atatatggat ccccttcatc ttgtgcagtc                                        30


<210>  47
<211>  49
<212>  DNA
<213>  Artificial Sequence

<220>
<223>  oligonucleotide primer

<400>  47
taacatccat ggcatgtggt aacaattcaa attcaaaagt agataaccc                   49


<210>  48
<211>  34
<212>  DNA
<213>  Artificial Sequence

<220>
<223>  oligonucleotide primer

<400>  48
atatatggat cctttgcaat tcctacattg tacc                                    34


<210>  49
<211>  45
<212>  DNA
<213>  Artificial Sequence

<220>
<223>  oligonucleotide primer

<400>  49
taacttccat ggcctgtggt aatagtaata gcaataatac tagtc                        45


<210>  50
<211>  36
<212>  DNA
<213>  Artificial Sequence

<220>
<223>  oligonucleotide primer

<400>  50
atatatggat cctttgcata gccaacgtag taccag                                  36


<210>  51
<211>  40
<212>  DNA
<213>  Artificial Sequence

<220>
<223>  oligonucleotide primer

<400>  51
atatatccat ggcttgttca aataaaagca gtaaacaagc                              40


<210>  52
<211>  35
<212>  DNA
<213>  Artificial Sequence

<220>
<223>  oligonucleotide primer

<400>  52
atatatggat cctgattatt ttgttcttct tgtgc                                   35


<210>  53
<211>  37
<212>  DNA
<213>  Artificial Sequence

<220>
<223>  oligonucleotide primer

<400>  53
atatatccat ggcatgctca aaaaaagaaa attcatc                                 37

<210> 54
<211> 35
<212> DNA
<213> Artificial Sequence

<220>
<223> oligonucleotide primer

<400> 54
atatatggat cctctagttt tttattaata tttgc 35


<210> 55
<211> 43
<212> DNA
<213> Artificial Sequence

<220>
<223> oligonucleotide primer

<400> 55
atatatccat ggcttgctca aattctaaga gtaatgatag ctc 43


<210> 56
<211> 39
<212> DNA
<213> Artificial Sequence

<220>
<223> oligonucleotide primer

<400> 56
atatatggat cctgattcca agcactttcg aacttagtc 39


<210> 57
<211> 33
<212> DNA
<213> Artificial Sequence

<220>
<223> oligonucleotide primer

<400> 57
atatatccat gggttgtcaa agtaatgaaa acc 33


<210> 58
<211> 32
<212> DNA
<213> Artificial Sequence

<220>
<223> oligonucleotide primer

<400> 58
atatatggat cctcatcgtc actattatca tc 32


<210> 59

<211>  36
<212>  DNA
<213>  Artificial Sequence

<220>
<223>  oligonucleotide primer

<400>  59
atatatccat ggcatgtggc aatttaaaaa attctg                                36

SEQ.ID. 1: CSP2 gene +/- 250 bp

CATTTTTAATGATTTTAGTGTATAAAATAATATACAAATTTTTGAATATTATCATATAAT
AAACACATTATTTATATATAAAAACAATGAAATACAGTAAAAATCAGCAATTAAGCAAG
AAAAATACTGATTAAATAACTTCTAGACCTATTCTTGTAATGAAAAATATTTGTTATAAT
AACACTAAAGATCCGATAGTATTACTAAAATATATTAATTATATAACAGTTAAATGGAGG
AAAATAAGGAATGCTATCTAAGAATAACTACCAAGAACGCTTAAGAAAAATGGATGACAA
ACAAGAACGTTTTTCAATCCGTAAATTTTCTGTCGGTGCAGCTTCTGTTTTAGTCGGAAC
AGCTGTATTAACTATGCAAAATGTTCAAACAGCACGCGCCGATACTACTTCAAATACTAC
AACTAAGACAACAGATTTATCTTCAGAAGCTGAAGAACAAAACAAGCAAAATGCTTATGA
TAAAACTCTTAATGAGGCACAACCTACAACTAAGCCTGCAGATGCTCCTGTGGAGAGTCA
AGATAGTAAGGTGGCCTCTTTTAGTGTGCCTAAAAATGAAGGAACATTTGTAGAAAAAAC
ACTAACGCCTAATATTACTGAAGAGACAGAGGCAATGCAGGATAAGGCTGAGTTAGCTCA
AGTAAATAGTGAGGATAAGTCAGCTGAAAACAATAAAACTAATGTAAAGGAAGAAAGTCA
GACTGTTGAAGAGAATCAAATGGATAAGCGATCAGCTGAAGCGAATATATCGGATACAGA
AAACACATTAGTAAGTAACACAACTTTAAATGTCCCTAAGACAACTCCAGCTACTACACG
CTTTTCAGCAACAGCACTAAGTGAGAGCAAATTTCAAGTCAATGCTCCACGCGTGGGCCA
AGATACTCCTGATACACAAGTTAAGAAAAATAATTATAAACTTGTAACCAATGCTACTGC
ACTTCAACAAGCAATTAATACTGGTGTTGCAGGTGTTAATATTGATAGAAGTATTGATGC
TTCTAATGTTGATTTGGCTATTAGAAATACTTTTACAATTGTTGGTCTTAACGATGCAGC
AGCACTTAATTTAGGTCAAAAATCTCTTAATAATTCCGGTAATTTAACTCTCCAAGATAT
TAAGATTAATGGATCAATCTCTGGTAGCGGTACGGTAAATATTAAGGGAAATGTTACTTC
TAATGTAAATAGTACTAATTCTTCTGTTCCAACTCAGGATCAATATAATGCTCAAAATTA
TACTGGTAGTAGAACTAACTTTAAGAACTCTAACATTGCTGCTAGGAACGTAAATATTGA
AAATGGTGCTTCCTTAACTATTAACAGTTCTGAAATTAATGATGGAATTAACTTAGCTGA
TGGCGGTACTGTTAAAGTTGGCGATAATGCTACTTTAAATGTTAACCTGACTAATACATC
AACTACTGCTACCCGTTACCATGTTGCTGGTGTGTTTGCCAAGAATGGTGGTAACTTTAT
TAGTGGTTACAAGTCAAATGTTAATTTTAATACAGGATTAGGCCAAGCTATTGCGATCGG
AGCAACCCGACCAACAAGTACTGACGCCGATAGATACGGTGGTTATGGTACACGCTCAAG
AAATGATGGTCCAACATTAGTTCAATTAGGTGACTCATCGACTTTTAACTTTACTGGACG
TGACGGTATTATCTTAGGTAATAATGCTAACTTTATTTCTGGAGAGAATTCAAATGTTCA
TTTTGAAAACAAGGGACGAGGAGTTGCTTTAGATTTAGCTAATAACTCAAATATCGAAAT
TTCTAAACATTCCATTACTTCTTTCCATTCTGTTGGCAAGACAGGAACGTCTGGTAGCTT

TGATGGCTATAACTATATTGGTGTTAACGAAGGTGGTAATATTACTGTTGACGAATATGC
TACCTTCCGTGTAATTCTTGAAGGTCGTGGCGATAATCCATGGGATGACGTAATTTCCTT
AGATTCTAGAAATGCTAACACAAATGCTGCTTTCACTTCTAAGACAGGAGCAATTGTTGA
CATTCGTGATGATAATACTAACTTTTACGCAGAACTTATTTCATTCCCACTAGGGAGTGC
AAACTCACGCATTGATATTCAAGATCCCCTAATGCTTAACCTGCAACGCTACTCTAATGG
TGGAGCAACGACTGGTTGGATGGCTACTGGTGGTGACAAAATCAACACAACTTCGGCACA
ATATACTGCTAACTTAATTTATATGAGTGGTAATAAGGGAGTATTTAGTGTTAGTGGTGG
GGATTACGATCCATCTAATCCTAATAGTTCCGGCTTTGTTGTTTACCAACAAATTAAGTC
AGATGGCTCAAAACAAATCTGGCTCAATGTAAATAGCGTTGATATTCCAATGAATGGATT
CCAAACTAAAGATATTTGGAATAATCAAGCAAACCCAGACGTATCAATTGCAGGTAATGG
TTTAACTGCAGGTATTAAAGCGAACCAAGTTCATAACTTTGATGGGTCTCCACTAACTGG
TCCGAATGCACCATACTATGGAATTTCAACCCAACGTGCTAGTCATCAAATTTGGATTCC
TCATAGAACATCATTGGAAGTTACTGGTGAACACAAGAGTACTATTAAGTACGTTGATGA
AAATGGAAAAGAAATTTTTCCAGAAAATACTCAATCATTAGATATGAAACGTAGTCTGAT
ACTTGATATTACACAAGATAAGATTCAAGATATTCAGAATTATGCTTTAAGTCATACTGC
AGATGAAACTTTAGAGTACATTAAAAACAGTCAAGCAGTAGTTCAAGATTCAGGTTGGGA
TTTCACTGATGCTAATGGTAAAGCTGTTATTGATCCATATAGTACAATTGACTCACCTAA
ATTGGCTGGCTATGCTGCAACGATCCAATCAACAAATGTTTCAACTTTAAAGGTTGGAGC
AGATGCTTCTGCAGTAACTGCTAAATTTATGGTTGAACCATCTCAAGATATGGTTCAAAA
TGGCGAATTATCAACTGCTTACAAGCAAAATGGCACTACTGGAATTCCTGCAGATTATGT
AACAGTAGTCGTTTATAATAAACAAGCAACTTATCAGGCAGGTAAAACTGATAGTAAGAG
TGTTGTTCGTGATATTAACTACTTAGATGGTAAGACAGGCAAAAAGATTCCAGCTAACCT
AATTAAAGATAATCCAGTTTCTCAAACTGTTACTATGCACCGTACTGAAATTCTTGATAA
TTCAGGCAAGGTAATAGGTTATGGACTATTAGTGAAGATGGTAAGTCGTACACACTTAA
CAACGATTGGATTATTGATGGTAACTGGAAGCTGTAAAATCACCAGACTTAACCACTAA
TGGTTACAAGACACCACGTTTTGAAAATGGTGATATAGCCAAAGTAGTTCCAGCAGAAAC
TGTAAATGAAAATACCCCTGATACTACTGTAAATGTTTACTACGACCACAATGAAATCCC
AGTTGGTCCAGATACTCCAGATAAGCATGGGGTAGATTTAAACGAATTAACTAAAGATGT
TAACGAAACTGTTCACTACGTATACAGTGATGGAACTAAGGCAGCTCCTGATGTTGTTCA
AACTTCTAAGTGGACTAGAACTTTAACTATTGATGCAGTAACTAATGAAGTAATTTCCGA
TGGTCAATATACCACTGATTGGGCAATTCCTAAGGGTGAAAAGACTGTTTACGATCAAGT
AGATACTCCAGTAATCAAAGGCTATCACGCAGATAAGCGTCAAATCAATGCTACAGTGGT
TACTCAAAATAATATTGAAGAGACTGTTACTTACCAACCAAATGGTAAGATCATTCCAGT
TGATCCAAATGGAGATCCAATTCCAAATGTACCAACCCCAACTTACCCAACAGATCCAAC

AGACCCAACTAAGGTAGTACCGGATGAACCAGTACCAGAAATTCCAGGATTGATTCCAAG
TGTTCCAACAGTAACACCAGAAAATCCAGGTAAGGATACGCCAGTACCATATACCCCAGA
AGTTCCAGCTAAGGATCAAGTAGCAGTTGTTAACTACATTGATGCAGATGACAACAACGC
AATCATCACTAGCTCAGGCAACTTAACTGGTAAAGCTGGCGAAAAGATTGACTACAGTAC
TGCTTCAACGATTGAAGAACTAGAAAACAAGGGTTACGTACTTGTAAGTGATGGCTTCCC
AGCAGGAGCAACGTTTGATAATGATGACAATACAACTCAAATCTACACAGTTGTATTGAA
GCACGGTACAGTTCCAGTAACACCAGAAAACCCAGGCAAACCAGGTGAACCAATTAATCC
TAACGATCCAGATGGTCCTAAGTGGCCAGAAGGCACTGGTGAAAATGGTGTAAAGAGAAC
AGGAACTCAAACTATTCACTACGAAGGAGCAGGCGACAAGACTCCTAGTGATGATGTACA
AACATTCGACTTCACTAAGAAGATGCTGGTAGATAAGGTAACTGGTAAGATCATTGATGG
AGGCGAATGGAATGTAACTAGTCACACATTTGGCTACAAGGATACTCCAGTAATTGAAGG
CTACCACGCAGATAAGCGTAATGCCGGAGGATCTGTGGTAACACCAGATGACTTGAACAA
GACTATCACAGTAAGTTACCAACCAAATGGTAAGATCATTCCAGTTGATCCAAATGGAGA
TCCAATTCCAAATGTACCAACCCCAACTTACCCAACAGATCCAACAGACCCAACTAAGGT
AGTACCGGATGAACCAGTACCAGAAATTCCAGGATTGATTCCAAGTGTTCCAACAGTAAC
ACCAGAAAATCCAGGTAAGGATACGCCAGTACCATATACCCCAGAAGTTCCAGCTAAGGA
TCAAGTAGCAGTTGTTAACTACATTGATGCAGATGACAACAACGCAATCATCACTAGCTC
AGACAACTTAACTGGTAAAGCTGGCGAAAAGATTGACTACAGTACTGCTTCAACGATTGA
AGAACTAGAAAACAAGGGTTACGTACTTGTAAGTGATGGCTTCCCAGCAGGAGCAACGTT
TGATAATGATGACAATACAACTCAAATCTACACAGTTGTATTGAAGCACGGTACAGTTCC
AGTAACACCAGAAAACCCAGGCAAACCAGGTGAACCAATTAATCCTAACGATCCAGATGG
TCCTAAGTGGCCAGAAGGCACTGGTGAAAATGGTGTAAAGAGAACAGGAACTCAAACTAT
TCACTACGAAGGAGCAGGCGACAAGACTCCTAGTGATGATGTACAAACATTCGACTTCAC
TAAGAAGATGCTGGTAGATAAGGTAACTGGTAAGATCATTGATGGAGGCGAATGGAATGT
AACTAGTCACACATTTGGCTACAAGGATACTCCAGTAATTGAAGGCTACCACGCAGATAA
GCGTAATGCCGGAGGATCTGTGGTAACACCAGATGACTTGAACAAGACTATCACAGTAAG
TTACCAACCAAATGGTAAGATCATTCCAGTTGATCCAAATGGAGATCCAATTCCAAATGT
ACCAACCCCAACTTACCCAACAGATCCAACAGACCCAACTAAGGTAGTACCGGATGAACC
AGTACCAGAAATTCCAGGATTGATTCCAAGTGTTCCAACAGTAACACCAGAAAATCCAGG
TAAGGATACATCAGTACCATATAACCCAGTTAAGGATCCAGATAAGGTAACTACAGTAGA
AGGTAAACAAATTATTCACTTTGTAGATGGCGATAATAACAAGCCACTTAAGGATCCAAA
TATTCAAACTTATGAATTCAAGATTACTAATGGTGTGCCAAACGAATCCAGTCATACCTT
TACTCTAGTTAATGTACCGGTGATTCCAGGATATGTTGCAGAAATTAAGTCTGCTGGTGG
AAAGACGGTAACTCCAGATAAGCCTCTTGCAGAAGTAACAGTTGTTTACCATAAGATCGG

TAAGATCGTTCCAGTTGATCCAAATGGAAATCCAATTCCAAATGTACCAACTCCAAGCTA
CACTAACGATCCGACAGATCCAACTAAGGTAGTACCAAATGAACCGGTGCCAGCTATTCC
TGGAAAGACACCAGATAAGACGACAGTAACTCCAGTTGATCCAACTAAGGATACACCTGT
TGTTTACAAGGATGATAAAGTACCACCTGCTCCAAATACACAAAAAGCTGTAGTTAACTT
TATTGATGTTAATACTGGAAAAATTATCAGTACTTCTGGTATTTTAAGTGGTCGCCCAGG
AGAAGATATTAATAAGTTATTCTCGTCTGCTGAAGTAATTAAGCAATTAGAGGCTGCAGG
TTACGAAGTTGTTTATAATGCATTTGACGGCGATGGTGTGACTAAGTACTTTGACAATGA
TGGCAATACGGTACAACAATTTACTATTGCTTTGAAACTGAAAGAAAAAGCTAAGACGCC
AGATCCCGTTCTTCCTGATCCAGAAGTTCCTGTAGAAAAGGAAACTCGATCTGAACAACC
AGTTAAGCAAAATGTTGCTGTTCCTACTCCAGAAAAACCTGTAGAAAAGAAGACTACTGA
CAATAAGGAAGTCTTACCGCAAACAGGTGCTGATAGTAATGAAGCGGCTACTATTTTAGG
TGCCGTAGCAACAGCAATTGGCATGACAAGCTTGATTGGCGCAAAGAGACGCAAGAAAGA
TGATAAATAATTTTTTAAATTAAATATCTTTTTTCAAAATTAGCTAGTAAAAGGCGTTG
AGACTTGATTCTCAGCGCCTTTTTTTAATACATTCTATTTATAATAGTGGATCCATATAA
GAAAAGACATACAATATATAAAGTAATTCACGTTAACAAGGGGGCATGAAATATATGGAT
AATTTAAGTGAGAAAGTTGCATCAGTAGCTGTAAAATATACGGATTTAGATAATAATCTG
GCTGAACTTTTTACTTCTGA

SEQ.ID. 2:  CSP2 coding sequence only

ATGCTATCTAAGAATAACTACCAAGAACGCTTAAGAAAAATGGATGACAAACAAGAACGT
TTTTCAATCCGTAAATTTTCTGTCGGTGCAGCTTCTGTTTTAGTCGGAACAGCTGTATTA
ACTATGCAAAATGTTCAAACAGCACGCGCCGATACTACTTCAAATACTACAACTAAGACA
ACAGATTTATCTTCAGAAGCTGAAGAACAAAACAAGCAAAATGCTTATGATAAAACTCTT
AATGAGGCACAACCTACAACTAAGCCTGCAGATGCTCCTGTGGAGAGTCAAGATAGTAAG
GTGGCCTCTTTTAGTGTGCCTAAAAATGAAGGAACATTTGTAGAAAAACACTAACGCCT
AATATTACTGAAGAGACAGAGGCAATGCAGGATAAGGCTGAGTTAGCTCAAGTAAATAGT
GAGGATAAGTCAGCTGAAAACAATAAAACTAATGTAAAGGAAGAAAGTCAGACTGTTGAA
GAGAATCAAATGGATAAGCGATCAGCTGAAGCGAATATATCGGATACAGAAAACACATTA
GTAAGTAACACAACTTTAAATGTCCCTAAGACAACTCCAGCTACTACACGCTTTTCAGCA
ACAGCACTAAGTGAGAGCAAATTTCAAGTCAATGCTCCACGCGTGGCCAAGATACTCCT
GATACACAAGTTAAGAAAAATAATTATAAACTTGTAACCAATGCTACTGCACTTCAACAA
GCAATTAATACTGGTGTTGCAGGTGTTAATATTGATAGAAGTATTGATGCTTCTAATGTT
GATTTGGCTATTAGAAATACTTTTACAATTGTTGGTCTTAACGATGCAGCAGCACTTAAT

TTAGGTCAAAAATCTCTTAATAATTCCGGTAATTTAACTCTCCAAGATATTAAGATTAAT
GGATCAATCTCTGGTAGCGGTACGGTAAATATTAAGGGAAATGTTACTTCTAATGTAAAT
AGTACTAATTCTTCTGTTCCAACTCAGGATCAATATAATGCTCAAAATTATACTGGTAGT
AGAACTAACTTTAAGAACTCTAACATTGCTGCTAGGAACGTAAATATTGAAAATGGTGCT
TCCTTAACTATTAACAGTTCTGAAATTAATGATGGAATTAACTTAGCTGATGGCGGTACT
GTTAAAGTTGGCGATAATGCTACTTTAAATGTTAACCTGACTAATACATCAACTACTGCT
ACCCGTTACCATGTTGCTGGTGTGTTTGCCAAGAATGGTGGTAACTTTATTAGTGGTTAC
AAGTCAAATGTTAATTTTAATACAGGATTAGGCCAAGCTATTGCGATCGGAGCAACCCGA
CCAACAAGTACTGACGCCGATAGATACGGTGGTTATGGTACACGCTCAAGAAATGATGGT
CCAACATTAGTTCAATTAGGTGACTCATCGACTTTTAACTTTACTGGACGTGACGGTATT
ATCTTAGGTAATAATGCTAACTTTATTTCTGGAGAGAATTCAAATGTTCATTTTGAAAAC
AAGGGACGAGGAGTTGCTTTAGATTTAGCTAATAACTCAAATATCGAAATTTCTAAACAT
TCCATTACTTCTTTCCATTCTGTTGGCAAGACAGGAACGTCTGGTAGCTTTGATGGCTAT
AACTATATTGGTGTTAACGAAGGTGGTAATATTACTGTTGACGAATATGCTACCTTCCGT
GTAATTCTTGAAGGTCGTGGCGATAATCCATGGGATGACGTAATTTCCTTAGATTCTAGA
AATGCTAACACAAATGCTGCTTTCACTTCTAAGACAGGAGCAATTGTTGACATTCGTGAT
GATAATACTAACTTTTACGCAGAACTTATTTCATTCCCACTAGGGAGTGCAAACTCACGC
ATTGATATTCAAGATCCCCTAATGCTTAACCTGCAACGCTACTCTAATGGTGGAGCAACG
ACTGGTTGGATGGCTACTGGTGGTGACAAAATCAACACAACTTCGGCACAATATACTGCT
AACTTAATTTATATGAGTGGTAATAAGGGAGTATTTAGTGTTAGTGGTGGGGATTACGAT
CCATCTAATCCTAATAGTTCCGGCTTTGTTGTTTACCAACAAATTAAGTCAGATGGCTCA
AAACAAATCTGGCTCAATGTAAATAGCGTTGATATTCCAATGAATGGATTCCAAACTAAA
GATATTTGGAATAATCAAGCAAACCCAGACGTATCAATTGCAGGTAATGGTTTAACTGCA
GGTATTAAAGCGAACCAAGTTCATAACTTTGATGGGTCTCCACTAACTGGTCCGAATGCA
CCATACTATGGAATTTCAACCCAACGTGCTAGTCATCAAATTTGGATTCCTCATAGAACA
TCATTGGAAGTTACTGGTGAACACAAGAGTACTATTAAGTACGTTGATGAAAATGGAAAA
GAAATTTTTCCAGAAAATACTCAATCATTAGATATGAAACGTAGTCTGATACTTGATATT
ACACAAGATAAGATTCAAGATATTCAGAATTATGCTTTAAGTCATACTGCAGATGAAACT
TTAGAGTACATTAAAAACAGTCAAGCAGTAGTTCAAGATTCAGGTTGGGATTTCACTGAT
GCTAATGGTAAAGCTGTTATTGATCCATATAGTACAATTGACTCACCTAAATTGGCTGGC
TATGCTGCAACGATCCAATCAACAAATGTTTCAACTTTAAAGGTTGGAGCAGATGCTTCT
GCAGTAACTGCTAAATTTATGGTTGAACCATCTCAAGATATGGTTCAAAATGGCGAATTA
TCAACTGCTTACAAGCAAAATGGCACTACTGGAATTCCTGCAGATTATGTAACAGTAGTC
GTTTATAATAAACAAGCAACTTATCAGGCAGGTAAAACTGATAGTAAGAGTGTTGTTCGT

GATATTAACTACTTAGATGGTAAGACAGGCAAAAAGATTCCAGCTAACCTAATTAAAGAT
AATCCAGTTTCTCAAACTGTTACTATGCACCGTACTGAAATTCTTGATAATTCAGGCAAG
GTAATAGGTTATGGACTATTAGTGAAGATGGTAAGTCGTACACACTTAACAACGATTGG
ATTATTGATGGTAACTGGGAAGCTGTAAAATCACCAGACTTAACCACTAATGGTTACAAG
ACACCACGTTTTGAAAATGGTGATATAGCCAAAGTAGTTCCAGCAGAAACTGTAAATGAA
AATACCCCTGATACTACTGTAAATGTTTACTACGACCACAATGAAATCCCAGTTGGTCCA
GATACTCCAGATAAGCATGGGGTAGATTTAAACGAATTAACTAAAGATGTTAACGAAACT
GTTCACTACGTATACAGTGATGGAACTAAGGCAGCTCCTGATGTTGTTCAAACTTCTAAG
TGGACTAGAACTTTAACTATTGATGCAGTAACTAATGAAGTAATTTCCGATGGTCAATAT
ACCACTGATTGGGCAATTCCTAAGGGTGAAAAGACTGTTTACGATCAAGTAGATACTCCA
GTAATCAAAGGCTATCACGCAGATAAGCGTCAAATCAATGCTACAGTGGTTACTCAAAAT
AATATTGAAGAGACTGTTACTTACCAACCAAATGGTAAGATCATTCCAGTTGATCCAAAT
GGAGATCCAATTCCAAATGTACCAACCCCAACTTACCCAACAGATCCAACAGACCCAACT
AAGGTAGTACCGGATGAACCAGTACCAGAAATTCCAGGATTGATTCCAAGTGTTCCAACA
GTAACACCAGAAAATCCAGGTAAGGATACGCCAGTACCATATACCCCAGAAGTTCCAGCT
AAGGATCAAGTAGCAGTTGTTAACTACATTGATGCAGATGACAACAACGCAATCATCACT
AGCTCAGGCAACTTAACTGGTAAAGCTGGCGAAAAGATTGACTACAGTACTGCTTCAACG
ATTGAAGAACTAGAAAACAAGGGTTACGTACTTGTAAGTGATGGCTTCCCAGCAGGAGCA
ACGTTTGATAATGATGACAATACAACTCAAATCTACACAGTTGTATTGAAGCACGGTACA
GTTCCAGTAACACCAGAAAACCCAGGCAAACCAGGTGAACCAATTAATCCTAACGATCCA
GATGGTCCTAAGTGGCCAGAAGGCACTGGTGAAAATGGTGTAAAGAGAACAGGAACTCAA
ACTATTCACTACGAAGGAGCAGGCGACAAGACTCCTAGTGATGATGTACAAACATTCGAC
TTCACTAAGAAGATGCTGGTAGATAAGGTAACTGGTAAGATCATTGATGGAGGCGAATGG
AATGTAACTAGTCACACATTTGGCTACAAGGATACTCCAGTAATTGAAGGCTACCACGCA
GATAAGCGTAATGCCGGAGGATCTGTGGTAACACCAGATGACTTGAACAAGACTATCACA
GTAAGTTACCAACCAAATGGTAAGATCATTCCAGTTGATCCAAATGGAGATCCAATTCCA
AATGTACCAACCCCAACTTACCCAACAGATCCAACAGACCCAACTAAGGTAGTACCGGAT
GAACCAGTACCAGAAATTCCAGGATTGATTCCAAGTGTTCCAACAGTAACACCAGAAAAT
CCAGGTAAGGATACGCCAGTACCATATACCCCAGAAGTTCCAGCTAAGGATCAAGTAGCA
GTTGTTAACTACATTGATGCAGATGACAACAACGCAATCATCACTAGCTCAGACAACTTA
ACTGGTAAAGCTGGCGAAAAGATTGACTACAGTACTGCTTCAACGATTGAAGAACTAGAA
AACAAGGGTTACGTACTTGTAAGTGATGGCTTCCCAGCAGGAGCAACGTTTGATAATGAT
GACAATACAACTCAAATCTACACAGTTGTATTGAAGCACGGTACAGTTCCAGTAACACCA
GAAAACCCAGGCAAACCAGGTGAACCAATTAATCCTAACGATCCAGATGGTCCTAAGTGG

CCAGAAGGCACTGGTGAAAATGGTGTAAAGAGAACAGGAACTCAAACTATTCACTACGAA
GGAGCAGGCGACAAGACTCCTAGTGATGATGTACAAACATTCGACTTCACTAAGAAGATG
CTGGTAGATAAGGTAACTGGTAAGATCATTGATGGAGGCGAATGGAATGTAACTAGTCAC
ACATTTGGCTACAAGGATACTCCAGTAATTGAAGGCTACCACGCAGATAAGCGTAATGCC
GGAGGATCTGTGGTAACACCAGATGACTTGAACAAGACTATCACAGTAAGTTACCAACCA
AATGGTAAGATCATTCCAGTTGATCCAAATGGAGATCCAATTCCAAATGTACCAACCCCA
ACTTACCCAACAGATCCAACAGACCCAACTAAGGTAGTACCGGATGAACCAGTACCAGAA
ATTCCAGGATTGATTCCAAGTGTTCCAACAGTAACACCAGAAAATCCAGGTAAGGATACA
TCAGTACCATATAACCCAGTTAAGGATCCAGATAAGGTAACTACAGTAGAAGGTAAACAA
ATTATTCACTTTGTAGATGGCGATAATAACAAGCCACTTAAGGATCCAAATATTCAAACT
TATGAATTCAAGATTACTAATGGTGTGCCAAACGAATCCAGTCATACCTTTACTCTAGTT
AATGTACCGGTGATTCCAGGATATGTTGCAGAAATTAAGTCTGCTGGTGGAAAGACGGTA
ACTCCAGATAAGCCTCTTGCAGAAGTAACAGTTGTTTACCATAAGATCGGTAAGATCGTT
CCAGTTGATCCAAATGGAAATCCAATTCCAAATGTACCAACTCCAAGCTACACTAACGAT
CCGACAGATCCAACTAAGGTAGTACCAAATGAACCGGTGCCAGCTATTCCTGGAAAGACA
CCAGATAAGACGACAGTAACTCCAGTTGATCCAACTAAGGATACACCTGTTGTTTACAAG
GATGATAAAGTACCACCTGCTCCAAATACACAAAAAGCTGTAGTTAACTTTATTGATGTT
AATACTGGAAAAATTATCAGTACTTCTGGTATTTTAAGTGGTCGCCCAGGAGAAGATATT
AATAAGTTATTCTCGTCTGCTGAAGTAATTAAGCAATTAGAGGCTGCAGGTTACGAAGTT
GTTTATAATGCATTTGACGGCGATGGTGTGACTAAGTACTTTGACAATGATGGCAATACG
GTACAACAATTTACTATTGCTTTGAAACTGAAAGAAAAAGCTAAGACGCCAGATCCCGTT
CTTCCTGATCCAGAAGTTCCTGTAGAAAAGGAAACTCGATCTGAACAACCAGTTAAGCAA
AATGTTGCTGTTCCTACTCCAGAAAAACCTGTAGAAAAGAAGACTACTGACAATAAGGAA
GTCTTACCGCAAACAGGTGCTGATAGTAATGAAGCGGCTACTATTTTAGGTGCCGTAGCA
ACAGCAATTGGCATGACAAGCTTGATTGGCGCAAAGAGACGCAAGAAAGATGATAAATAA

SEQ.ID. 3: CSP8 gene +/- 250 bp

ATTTATTTAATAATTTAAACAGTTATTTATTTGTAAATCAATGTTAATTTTTTGCTACAT
TATTAGTACGATTTGGAAGAAAGCAGTAAATACTGTACATTTGATAGTTATTTTTTTGTA
TAAAAAAGCATTGAACTTTAATATAAATTGGATATAATATTAAAATAAAAATATATCAAA
TTAAATATATTCTTATTTTATTATAAGTTAGTTACAAGGGTCAGTTAATCAATTCTTTAA
GGAGGATTTAAATGAAAAAGAAAAGAGAAAAATATACTTGGTTATTACTCAGTACAGCTC
TTATAACTAGTGGTCAAATACTAGGTGGAGGAGAACAAATTATAAAGGCAAGTGTAGAAA

GCCAAACTAACAGCGTTAAAAAATCTAAGACAGCTGTAGAACATTCTACGACAGCTCTCT
CGAGACAAGCTGTTGAGGCCCAACTTGCCGCTCAAGGTGTTAATTTTGAACGATTAACAC
CCGAGGAACAGCAAGAGGTCTATGTAGATGTGATTGTCCAACTCGAGGCTCTACCAGCTT
CTGAAAATGGATCAATTGATTCACAAACAGCAAGTAGAGCAGAAATTGAGCAAGCATCGA
ATAAAGTAATTGCTGCTCAATCTGGAATTAAGGATGAAGTTCAAAAAATTACGAATCAAG
CCATTGATAAAAGTTATGGGTATGTAGTTAATGGTTTTGCAACAAAAGCAAAAGTTGGAG
ATATTAAAAAATTAAGAGAGATTAAAGGTGTAAAATCAGTTACTTTAGCTAAGGTATATT
TTGCAGCCGATACCTCAGCAAATAACATGGCTAATGTTTCGACGGTTTGGTCAAACTATC
AATATAAGGGTGAAGGAACAGTTGTTTCAATTATTGATACGGGAATTGATCCAAACCATA
AAGATTTACGATTAAGTGATGAATCTAAGGTTAAATTGACTGCTAAGGATATTGATGGAT
TTACTGAAAATAGTGGATATGGACGCTACTTTACCAGTAAAGTTCCATTTGGACATAATT
ATTCAGATAATAACGATATTATTACGGATGATGATCCAAAGGAACAACATGGGATGCACG
TTGCGGGAATTGTTGCTGCTAATGGTACTGGAAGAATTCAGCAAGCTCAGTAGTTGGAG
TTGCGCCTGAAGCACAATTACTTGCGATGAAGGCTTTTTCTAATTCAGATAGCTCCTCCA
CAACCGATTCAACAAGCGTAATCGGAGCAGTTGATGACTCAGCTAAATTGGGAGCAGACG
TCCTTAATATGTCATTAGGATCTGTATCTGGTGAACAAACTGAGGATGATCCAGAAATCG
CGGCTGTAGAAAAAGCTGTTAAGCATGGAACAGCAGCCGTAATTTCAGCCGGAAACTCTG
GAACTTCAACCTCTAATCAAGAGGGAGTTAATAAAGACTTTTATGGTAATCCTGATATGG
AAACTATCGGAAGTCCTGGTACTAGTAGAAGTGCTACAACTGTTGCATCTGCTGAAAATA
CTAAGGTTACTACCGATGGGATGACTGTTTCTACCGCTGATGGCAAAAAGATATTTGGAC
CAAGTGTAACGCAACTTTCTCCAAATACATCTCACGATGCTTTCGATAGTAAAAAATTCT
ATATTGTAAAAGATGCATCTGGAAAACTAGGAATGGGAACTCCTAGTCAATATACAGCTG
ATGTAAAAGGAAAAGTTGCAGTTGTTTCGCGAGGTGAAATTACATTCACTGATAAACAAA
AATATGCCCAAGCAGCTGGCGCCGCAGGACTTATTATTGTAAATAATGCTGGAGGAAATA
CGCCATTAACGTCAGTTTTATACAATGAAGGTTTTCCTACAGCTGGTTTATCAACTGACG
ATGGTAATAAGCTTGTTGCTTATGTTGAAGCTCATCCAGATGAATTATTACGAGTAAACG
TTGAAGTTCAACCATTAAACAATGTTATTCGTGAAGAAGATTTAATGTCTAGCTTTACGT
CTTATGGACCAGTATCTGATCTATCATTTAAGCCTGATATTACAGCTCCCGGAGGAAATA
TTTGGTCTCTCCAAAATAATAATGGTTATATCAATATGTCAGGTACATCAATGGCTTCAC
CATTTATTGCTGGATCACAAGCTTTACTTGTACAAGCTATGAATGATAAGACAGGTAAGT
TTTATGAAACATACCAAAAGATGTCTGGAAGTGAACGTGCAGCTTTAATAAAAAATATTC
AGATGAATACTGCTAATATTGAAGTTGACGTTGATCATGGCTCAGTTATTGAATCACCTC
GTAGACAGGGAGCCGGCTTAGTCAATGTAGAGGCTGCAATTAATGCTATTTTGCATAATC
CTTCAACTGTTAGTGGAAGTAATGGTTATCCTGGTGTTGAATTGAAAGATTTCCAGGATC

GCCAACATCAATTTACTATTAAATTTACAAATCGGACCAATAAAGACATTGAATATGGTT
TAAATGAGAACGGTAAGTTTTCTGATGTATATACTTCAGAAACCGATCCAAAAACAGGTG
TCTTATTCGAAAAGAAGATTGATGGTGCTTCACTTACACCAAGTGAAAAGATAGTTGTTC
CAGCAAACTCTACTAAAGAAGTAACTATTAACTTATCATTACCTGATAACTTTAAAGAAA
ATCAATATGTAGAAGGATTTATGGCATTTACTGGGTCCGATAACTCACATTTAAAGATTC
CATACATGGGATTCTTTGGTGATTGGGCTGCACCTGCTATTTTTGATGGTTTAAACGGAT
TAGCATTTAACCCAGGAAATAATAACCTCGGAACTATTGTGACTGCAGGAAATAAAAATG
GAGCAGTTGGTTATGCCGGCTTAAACCAAGATGAAGATGGAAATTATAGAGTTGATCCAG
ATGCAATTGCTTTATCTACAGCAGATGGAGCATCTGTATCGTGGGTGAGACCACAATACT
TCTTGTTTAGAAATGCTAATGATGTTAAGGCTGAAATCTTAAATCAAGATGGTGAAGTAA
TTAATACGCTAGTTTCATTGGCACATGTTACTAAGTCATATTGGGCAGCAAGTAGTCAAA
GATATGCTAAGTTTAATTATGCACCTGCTTGGGATGGTACATATTTCAATCAACAAACAA
ATAAGACCGAAAAAGTACCAGATGGAACTTATACTTATCGCGTGACTGGTACGGTTGATG
GTACTAAGAAGCAGCAACATTATGATATTAAAGTTAAAGTTGATAGTGTGAAACCAGAAG
TTAAAAACTTGAAATTAGGATCACATAAGGATCAAACTGGAAAAGTATCATATGTTCTTA
AAGCAGAAGCGAAAGATAACTTCAGTGGATTAAATGGACAAGCAAATACTTATGTAAATG
GAGAACTTAATAGAAGTGTCGCATATGATATTGTTGGTTCATCTAGTGATGGTTATCAAA
AGATTGAAGTTCCACTTAGCGATGAGCAAGTTAAAACTCTTAGGGCTGGAAAAAATGATT
TAGCTATTGCTGTATTTGATAATGCTACTAACGCTGGTACTAATTCAGGGACATCAAATA
AACCAGGTGAAATTAACTTTGGACTAATTATTGATAATAACTTGCCGCAAAAGATCACAA
CGGTAAGCGATGGTTATGATATGACTGATGATTCATATACTATTTCAGGTACATATCCCG
AGAAAGTATATGGTACATATACAGATAAAGATGGAAAAGAACACGATCTTAACATAAGTT
ATGATGAAGCTAGTGAACGGTTTGTTACTAAGTTGCCATTATCAGTAAGTGACTATGACA
CCAATGTTAAATTCTATGCTGATGAAGAACATGAAACACTAATTACTCAAAAACGTATTA
ATGTTAGTCTTGTTCCACCAAAACTTGAAAGTCTTAAAGTAGATGATCAAGAAACATATA
CTGGAAATGAAGAAGCTAAACTAAGTCAAACTTCTGAAGACACAGTAGAAGTATCAGGAA
AAGTTAGTGATGATACTGATAAAGTTGCTGTAAAAGTTGCAGGTAAGACTTATAGTGCTA
AACCAACTAAGGAACACACCTTTAAAGTTAAAGTTCCAGTAAGCTACGGCGAAAATACAA
TGAATATTGTTCTTACTGACAAAGATGGAATAGTTCATCTGTTAAGCAAATTGTTAAGT
CAAGTGATCGTGGTAAGACTGTTGTTTCCGCAAAGGATGTTACCTTTGATAATGGAATTA
AGTTTGGAACAACTAGCGTTAATACCGAGACCGAAAACTACGATCCTAAGACTGGTAAGT
TAACTTTAACTGGAAAAGTAAATCGCCCAACTACTACTGTTAGAATTGGTGATCATACAG
TTAAAGTTAAAGCAGATGGAACATTTAAACTAGTACTTGATCTTGGTAAGCATGGAGCTA
AAGTTTTCCCAGTTTTAATTGGTGATACTACTGTTAATGATACTGTTCAAGAACGCTTAA

CATTCTACGTAGATTCAAATAATCCAGAATTAACCCTTAACCAAGAGAAGGATCAATCAG
GTTATGTACCTGTTTATACAAATAAAGAAGAATTTAAGCTTCAAGGTACAATTAGTGATG
ATTATCCATATTATAGTTTGCTCATAAATGATAATAATGTTGATGCAAATTGGGATGATG
TTGATTACAACGGTAATAAGAATCTGAAGAAGAGCTTTAGCCATTCTGTTAAACTTAAAG
AAGGCAAAAATACTTTTAATGTTGTAGTAGTCGACAACAATGATAACCGCAGTGAAGTCC
AAACTTTAGTTGTGTACTATAAGAAAGCTCAGAAATTGGCCTCACCGCAAATTACTGCTA
CAACAGCGTCAGATAAAAAATCAGTAACTGTAACTGGAAAGGCTAAAGATGGCAACGTTT
TATACAGTACTGATAATGGAAATAAATACAATGTTCTTCCAGAAGATGGTGTAACTGTCA
AAAATAATGGCAAGCTTCTATTTAAGACTGTAGATAAATACGGTAATGAATCCGAAGTAG
TTGAGTATGATGTCAAAACTATTGGAAAAGAAGAGTACTGTAGATAAAAGCGTTGCAC
AAGCAAGAAAAGATTTAAGAAAGAAGCTTGATCAAGCACGTGCTTTAGGAAATACAGGTA
AGTACACACATGAATCAGCTAAAAAATTAGCTCAAGCTCGCCAAGAAGCAAGTAAAGCGT
TGAAAGATAAGAATGCTACTCTCCAAGAATTGAAGCAGGCAAGCGAACAACTAGAAGAAG
CAATTAAGAATTTGGTTGAAAAGCCTGTAGATCAAAATAAAGATAAAGACAAAGTAGAAG
ATAAAGACTCACAAGTAAACGCACTTAAAGAAAAATTGGAAGAGACAGTTAAGGCTGGTG
AAAAATTCGATAAGGATAAATATACTGATGATTCAGTAGAAAAAGTAACTAAAGCTCTTG
ATGAAGCTAAAGTAGTTTTAGCTAATAAAGATGCAAATTCAACTGATGTTCAAGATGCGA
TAGATTCTATCGTTAATGCAACGAAATCTCTTAAGGAAAAGCAAGTATCTCCAGAAAAAA
CAACTCAGCAAGAAGATACACCAAAGGAAAATAAAGATACCGAGGAAGTCCTTGCTGCAA
AGAATGCTTTAAAAGAAAAGGCAGACAAACTTAGTCATTTGGATACCACGAAGTATACTT
CTGAGAGTGCAGAAAACTTGAGTAATGCACTGAAGAAAGTAAATCAGGTTTTGACTAATA
AAGATGCTAATAAAGCTCAAGTTCAAGAAGCACTTGATAGTTTATCTCAAGCTGAGAAAG
ATTTAGTTGAAAAAACAGAATCAAATAAAGATGTTGAAACAGCTAAAGAACAATTAAGAG
AAGAACTTAATAAGCATAAAGATGAAGATAAATCTCAGTATACTGATGATTCTGCTAAAG
TGAAGGATAATGCTGAAAAGATTTCAGAAGGAGTACTCGATTCAAAAGATGCACAAGCAG
ACGAGGTTAATAAAGCAAAAGATAGTCTTGTTGAAGCAGAAAAAGGCTTAGTTAAGAAAG
AAGAAAATAAGCCTGCTGAATCTGATACTCAAGAAGTTGATAAAGCTAGAGAGGCATTAG
AACAAGAGGTTAATAAGAATGCCAATGTAAACTTAGACGGATATACGCCAGAATCGCAAG
ATAAATTCAAAGAAATATTGAATGGTGTCCGGGATGTTTTAAATGATAAAAATGCATCTG
CAAGCAGCTTAGAAAAAGCAGAAAAAGTACTTGAAACTGCTACAGGTGTTTTAACACAGG
TAGAACATCAAGTGGAATTGCCTAAGGTTGAACAACCAGTTGTAACTCCTGAGAAGAAGC
AGACTGAACAAGAAGAAGCTAAAAAATCTGAATCTAGTTCTGTATCAACGTCTAAAGATG
AAGTGAAAGAACCAGAAGAAAGAAACAAGATGCTCACTCTGTATCTGATAAAGGAACTG
GAACTTCAGTAGAGAAGAAGGGGCAAACACCTGCAGATGCTCTATCACAAGTAGAACATC

AGGCAGAATTATCTAAGAATGAACAAGAAGAAGCTAAAAAATCTGAATCTCATTCTGCAT
CAACGTCTAAAGATGAAGTGAAAGAACCAGAAGAAAAGAAACAAGATGCTGAATCTGTAT
CTGATAAAGAGAGCAGCACATTAGTAGGAAAAAGCATCAAGTTGCAAGAAGTGCTCAAG
AATCTTCAAAGAAGAATGCTGATAATAAGGTGCCAACTCATAAAAATAGCAATAAAGCTC
AAAATACTACAAATAGTTCAACTCGAAGTACTAAGAGTAGCAATAATAAAGAATTACCTA
AGACTGGAGAAAGAGAAACATTCTTTGGATTACTAGTTACAGGAATAACTGCCTTATTTG
CTAGTTTAGGAACAGTACTGAGAATAAAGAATAAAAAGTAGTTATTTAAGGAATATGTTT
TTAATTAAAATCCATTTTCTATGAGCAAAGAAAAATGCGTTAAGATATTTAATCTTAGCG
CATTTTTTATTCGCTTAAAGTTAAGCTATTCTTTCTTCTTCCGATCACCGATTAATCCAA
GTAAGAAGCTAATACCAGCTACTGCTAATCCGATTAGTTGAGCTTGGTTTCTTTTCTTC
CAGTTTGTGGAAGCTCTTCCTTCTTCTTGTTACTTTCAACGTTATTTGCA

SEQ.ID. 4: CSP8 coding sequence only

ATGAAAAAGAAAAGAGAAAAATATACTTGGTTATTACTCAGTACAGCTCTTATAACTAGT
GGTCAAATACTAGGTGGAGGAGAACAAATTATAAAGGCAAGTGTAGAAAGCCAAACTAAC
AGCGTTAAAAAATCTAAGACAGCTGTAGAACATTCTACGACAGCTCTCTCGAGACAAGCT
GTTGAGGCCCAACTTGCCGCTCAAGGTGTTAATTTTGAACGATTAACACCCGAGGAACAG
CAAGAGGTCTATGTAGATGTGATTGTCCAACTCGAGGCTCTACCAGCTTCTGAAAATGGA
TCAATTGATTCACAAACAGCAAGTAGAGCAGAAATTGAGCAAGCATCGAATAAAGTAATT
GCTGCTCAATCTGGAATTAAGGATGAAGTTCAAAAAATTACGAATCAAGCCATTGATAAA
AGTTATGGGTATGTAGTTAATGGTTTTGCAACAAAAGCAAAAGTTGGAGATATTAAAAAA
TTAAGAGAGATTAAAGGTGTAAAATCAGTTACTTTAGCTAAGGTATATTTTGCAGCCGAT
ACCTCAGCAAATAACATGGCTAATGTTTCGACGGTTTGGTCAAACTATCAATATAAGGGT
GAAGGAACAGTTGTTTCAATTATTGATACGGAATTGATCCAAACCATAAAGATTTACGA
TTAAGTGATGAATCTAAGGTTAAATTGACTGCTAAGGATATTGATGGATTTACTGAAAAT
AGTGGATATGGACGCTACTTTACCAGTAAAGTTCCATTTGGACATAATTATTCAGATAAT
AACGATATTATTACGGATGATGATCCAAAGGAACAACATGGGATGCACGTTGCGGGAATT
GTTGCTGCTAATGGTACTGGGAAGAATTCAGCAAGCTCAGTAGTTGGAGTTGCGCCTGAA
GCACAATTACTTGCGATGAAGGCTTTTTCTAATTCAGATAGCTCCTCCACAACCGATTCA
ACAAGCGTAATCGGAGCAGTTGATGACTCAGCTAAATTGGGAGCAGACGTCCTTAATATG
TCATTAGGATCTGTATCTGGTGAACAAACTGAGGATGATCCAGAAATCGCGGCTGTAGAA
AAAGCTGTTAAGCATGGAACAGCAGCCGTAATTTCAGCCGGAAACTCTGGAACTTCAACC
TCTAATCAAGAGGGAGTTAATAAAGACTTTTATGGTAATCCTGATATGGAAACTATCGGA

AGTCCTGGTACTAGTAGAAGTGCTACAACTGTTGCATCTGCTGAAAATACTAAGGTTACT
ACCGATGGGATGACTGTTTCTACCGCTGATGGCAAAAAGATATTTGGACCAAGTGTAACG
CAACTTTCTCCAAATACATCTCACGATGCTTTCGATAGTAAAAAATTCTATATTGTAAAA
GATGCATCTGGAAAACTAGGAATGGGAACTCCTAGTCAATATACAGCTGATGTAAAAGGA
AAAGTTGCAGTTGTTTCGCGAGGTGAAATTACATTCACTGATAAACAAAAATATGCCCAA
GCAGCTGGCGCCGCAGGACTTATTATTGTAAATAATGCTGGAGGAAATACGCCATTAACG
TCAGTTTTATACAATGAAGGTTTTCCTACAGCTGGTTTATCAACTGACGATGGTAATAAG
CTTGTTGCTTATGTTGAAGCTCATCCAGATGAATTATTACGAGTAAACGTTGAAGTTCAA
CCATTAAACAATGTTATTCGTGAAGAAGATTTAATGTCTAGCTTTACGTCTTATGGACCA
GTATCTGATCTATCATTTAAGCCTGATATTACAGCTCCCGGAGGAAATATTTGGTCTCTC
CAAAATAATAATGGTTATATCAATATGTCAGGTACATCAATGGCTTCACCATTTATTGCT
GGATCACAAGCTTTACTTGTACAAGCTATGAATGATAAGACAGGTAAGTTTTATGAAACA
TACCAAAAGATGTCTGGAAGTGAACGTGCAGCTTTAATAAAAAATATTCAGATGAATACT
GCTAATATTGAAGTTGACGTTGATCATGGCTCAGTTATTGAATCACCTCGTAGACAGGGA
GCCGGCTTAGTCAATGTAGAGGCTGCAATTAATGCTATTTTGCATAATCCTTCAACTGTT
AGTGGAAGTAATGGTTATCCTGGTGTTGAATTGAAAGATTTCCAGGATCGCCAACATCAA
TTTACTATTAAATTTACAAATCGGACCAATAAAGACATTGAATATGGTTTAAATGAGAAC
GGTAAGTTTTCTGATGTATATACTTCAGAAACCGATCCAAAAACAGGTGTCTTATTCGAA
AAGAAGATTGATGGTGCTTCACTTACACCAAGTGAAAAGATAGTTGTTCCAGCAAACTCT
ACTAAAGAAGTAACTATTAACTTATCATTACCTGATAACTTTAAAGAAAATCAATATGTA
GAAGGATTTATGGCATTTACTGGGTCCGATAACTCACATTTAAAGATTCCATACATGGGA
TTCTTTGGTGATTGGGCTGCACCTGCTATTTTTGATGGTTTAAACGGATTAGCATTTAAC
CCAGGAAATAATAACCTCGGAACTATTGTGACTGCAGGAAATAAAAATGGAGCAGTTGGT
TATGCCGGCTTAAACCAAGATGAAGATGGAAATTATAGAGTTGATCCAGATGCAATTGCT
TTATCTACAGCAGATGGAGCATCTGTATCGTGGGTGAGACCACAATACTTCTTGTTTAGA
AATGCTAATGATGTTAAGGCTGAAATCTTAAATCAAGATGGTGAAGTAATTAATACGCTA
GTTTCATTGGCACATGTTACTAAGTCATATTGGGCAGCAAGTAGTCAAAGATATGCTAAG
TTTAATTATGCACCTGCTTGGGATGGTACATATTTCAATCAACAAACAAATAAGACCGAA
AAAGTACCAGATGGAACTTATACTTATCGCGTGACTGGTACGGTTGATGGTACTAAGAAG
CAGCAACATTATGATATTAAAGTTAAAGTTGATAGTGTGAAACCAGAAGTTAAAAACTTG
AAATTAGGATCACATAAGGATCAAACTGGAAAAGTATCATATGTTCTTAAAGCAGAAGCG
AAAGATAACTTCAGTGGATTAAATGGACAAGCAAATACTTATGTAAATGGAGAACTTAAT
AGAAGTGTCGCATATGATATTGTTGGTTCATCTAGTGATGGTTATCAAAAGATTGAAGTT
CCACTTAGCGATGAGCAAGTTAAAACTCTTAGGGCTGGAAAAAATGATTTAGCTATTGCT

GTATTTGATAATGCTACTAACGCTGGTACTAATTCAGGGACATCAAATAAACCAGGTGAA
ATTAACTTTGGACTAATTATTGATAATAACTTGCCGCAAAAGATCACAACGGTAAGCGAT
GGTTATGATATGACTGATGATTCATATACTATTTCAGGTACATATCCCGAGAAAGTATAT
GGTACATATACAGATAAAGATGGAAAAGAACACGATCTTAACATAAGTTATGATGAAGCT
AGTGAACGGTTTGTTACTAAGTTGCCATTATCAGTAAGTGACTATGACACCAATGTTAAA
TTCTATGCTGATGAAGAACATGAAACACTAATTACTCAAAAACGTATTAATGTTAGTCTT
GTTCCACCAAAACTTGAAAGTCTTAAAGTAGATGATCAAGAAACATATACTGGAAATGAA
GAAGCTAAACTAAGTCAAACTTCTGAAGACACAGTAGAAGTATCAGGAAAAGTTAGTGAT
GATACTGATAAAGTTGCTGTAAAAGTTGCAGGTAAGACTTATAGTGCTAAACCAACTAAG
GAACACACCTTTAAAGTTAAAGTTCCAGTAAGCTACGGCGAAAATACAATGAATATTGTT
CTTACTGACAAAGATGGGAATAGTTCATCTGTTAAGCAAATTGTTAAGTCAAGTGATCGT
GGTAAGACTGTTGTTTCCGCAAAGGATGTTACCTTTGATAATGGAATTAAGTTTGGAACA
ACTAGCGTTAATACCGAGACCGAAAACTACGATCCTAAGACTGGTAAGTTAACTTTAACT
GGAAAAGTAAATCGCCCAACTACTACTGTTAGAATTGGTGATCATACAGTTAAAGTTAAA
GCAGATGGAACATTTAAACTAGTACTTGATCTTGGTAAGCATGGAGCTAAAGTTTTCCCA
GTTTTAATTGGTGATACTACTGTTAATGATACTGTTCAAGAACGCTTAACATTCTACGTA
GATTCAAATAATCCAGAATTAACCCTTAACCAAGAGAAGGATCAATCAGGTTATGTACCT
GTTTATACAAATAAAGAAGAATTTAAGCTTCAAGGTACAATTAGTGATGATTATCCATAT
TATAGTTTGCTCATAAATGATAATAATGTTGATGCAAATTGGGATGATGTTGATTACAAC
GGTAATAAGAATCTGAAGAAGAGCTTTAGCCATTCTGTTAAACTTAAAGAAGGCAAAAAT
ACTTTTAATGTTGTAGTAGTCGACAACAATGATAACCGCAGTGAAGTCCAAACTTTAGTT
GTGTACTATAAGAAAGCTCAGAAATTGGCCTCACCGCAAATTACTGCTACAACAGCGTCA
GATAAAAAATCAGTAACTGTAACTGGAAAGGCTAAAGATGGCAACGTTTTATACAGTACT
GATAATGGAAATAAATACAATGTTCTTCCAGAAGATGGTGTAACTGTCAAAAATAATGGC
AAGCTTCTATTTAAGACTGTAGATAAATACGGTAATGAATCCGAAGTAGTTGAGTATGAT
GTCAAAACTATTGGAAAAGAAGAGAGTACTGTAGATAAAAGCGTTGCACAAGCAAGAAAA
GATTTAAGAAAGAAGCTTGATCAAGCACGTGCTTTAGGAAATACAGGTAAGTACACACAT
GAATCAGCTAAAAAATTAGCTCAAGCTCGCCAAGAAGCAAGTAAAGCGTTGAAAGATAAG
AATGCTACTCTCCAAGAATTGAAGCAGGCAAGCGAACAACTAGAAGAAGCAATTAAGAAT
TTGGTTGAAAAGCCTGTAGATCAAAATAAAGATAAAGACAAAGTAGAAGATAAAGACTCA
CAAGTAAACGCACTTAAAGAAAAATTGGAAGAGACAGTTAAGGCTGGTGAAAAATTCGAT
AAGGATAAATATACTGATGATTCAGTAGAAAAAGTAACTAAAGCTCTTGATGAAGCTAAA
GTAGTTTTAGCTAATAAAGATGCAAATTCAACTGATGTTCAAGATGCGATAGATTCTATC
GTTAATGCAACGAAATCTCTTAAGGAAAAGCAAGTATCTCCAGAAAAAACAACTCAGCAA

GAAGATACACCAAAGGAAAATAAAGATACCGAGGAAGTCCTTGCTGCAAAGAATGCTTTA
AAAGAAAAGGCAGACAAACTTAGTCATTTGGATACCACGAAGTATACTTCTGAGAGTGCA
GAAAACTTGAGTAATGCACTGAAGAAAGTAAATCAGGTTTTGACTAATAAAGATGCTAAT
AAAGCTCAAGTTCAAGAAGCACTTGATAGTTTATCTCAAGCTGAGAAAGATTTAGTTGAA
AAAACAGAATCAAATAAAGATGTTGAAACAGCTAAAGAACAATTAAGAGAAGAACTTAAT
AAGCATAAAGATGAAGATAAATCTCAGTATACTGATGATTCTGCTAAAGTGAAGGATAAT
GCTGAAAAGATTTCAGAAGGAGTACTCGATTCAAAAGATGCACAAGCAGACGAGGTTAAT
AAAGCAAAAGATAGTCTTGTTGAAGCAGAAAAAGGCTTAGTTAAGAAAGAAGAAAATAAG
CCTGCTGAATCTGATACTCAAGAAGTTGATAAAGCTAGAGAGGCATTAGAACAAGAGGTT
AATAAGAATGCCAATGTAAACTTAGACGGATATACGCCAGAATCGCAAGATAAATTCAAA
GAAATATTGAATGGTGTCCGGGATGTTTTAAATGATAAAAATGCATCTGCAAGCAGCTTA
GAAAAAGCAGAAAAAGTACTTGAAACTGCTACAGGTGTTTTAACACAGGTAGAACATCAA
GTGGAATTGCCTAAGGTTGAACAACCAGTTGTAACTCCTGAGAAGAAGCAGACTGAACAA
GAAGAAGCTAAAAAATCTGAATCTAGTTCTGTATCAACGTCTAAAGATGAAGTGAAAGAA
CCAGAAGAAAAGAAACAAGATGCTCACTCTGTATCTGATAAAGGAACTGGAACTTCAGTA
GAGAAGAAGGGGCAAACACCTGCAGATGCTCTATCACAAGTAGAACATCAGGCAGAATTA
TCTAAGAATGAACAAGAAGAAGCTAAAAAATCTGAATCTCATTCTGCATCAACGTCTAAA
GATGAAGTGAAAGAACCAGAAGAAAAGAAACAAGATGCTGAATCTGTATCTGATAAAGAG
AGCAGCACATTAGTAGGGAAAAAGCATCAAGTTGCAAGAAGTGCTCAAGAATCTTCAAAG
AAGAATGCTGATAATAAGGTGCCAACTCATAAAAATAGCAATAAAGCTCAAAATACTACA
AATAGTTCAACTCGAAGTACTAAGAGTAGCAATAATAAAGAATTACCTAAGACTGGAGAA
AGAGAAACATTCTTTGGATTACTAGTTACAGGAATAACTGCCTTATTTGCTAGTTTAGGA
ACAGTACTGAGAATAAAGAATAAAAAGTAG

SEQ.ID. 5: CSP10 gene +/- 250 bp

ATACTATTTATAGGAAGATTTAATTCTAAAGAAAGTCATATAAATTATCTAAGAGTATAG
AATAAGAAAAGATTACATGTTACTATATCACTTGTAATTAATTTTAGATAATAGTACATT
AACAGTGATGACGAGCATTTATGTTAAGTAGTAAAAGGGATGATAAAAGTAAGGTTAAAT
AGATTTAAAATTTAAGCTAATTTAAATAAATAACTTGTCTAATTAAACAAACTTAAATTT
TGAAGTTGGAGGAAAATATGGTTTCAAAAAACAATATTGAAATGAAATATAAAAAAATGA
GAGACGAAAAACAACGTTTCTCAATTCGTAAATTTAGTGTGGGAGCTGCATCCGTTTTAA
TTGGGCTAAGTTTTAGTTTATATAACGGACAACAAGCATTTGCTGATACTGTTGATAATG
GCAATAAGTCTGTTGTAGCAAATGCACAAGATAAAGAACAAACAGACAATAAACAAAATA

CTGATACTAATCCTCAAAATAACGAAGATAAAACAAATAATAATCAAACTCAAAATACAG
TAACTGCTGATCTTGATCACAGTACTGTTGGGGCTAGTTACAAGGCTGATACTACTAATG
CTAGCGAAGCTAACAAGAGTAGTGATAAGTCAGATGAAAAGCAAGCTGCAACTACTGAAA
CTGCTAAAACTGAAGAAGCTGCTACTAAGGAAGTAGAAGCAAAGAATACTGCAAATGATG
ATGTAGCTAAGAAAACTGAAGAGGCAGCTGCTAAGACAGATACTAAGGTCGATGTTAATA
AAACAGTAGTAGAAAATGTAAAGACTGAAGCTGCTACTCCTAATGTAGAGACTAGGAACA
TTGAAACTCAAACTGCAAAGACTGATGAAGAAAAAGTAGAAACAGCAGTTGACAATACTT
CTTCTGATTTAAAAAACGCAGTAGAGAATGCAAATACTGAAGTTTCAACTAATACATTTA
ACGTAAATAAAGCAGTTCGAAATGCATTATTCCTTAGTGCAAATAATCTTGCTGAACGTA
AGGTAGCAGCTATAGCACTTCCAGCTGAAGCAGAAGATCCTAATGCCGTAACAGTTAGTG
ATGCGAAGGGATTTATTGATGCAATTCAAAATGGACTGCTACTACTATTAATGTTGCAA
GTGATCTTAATTTAGCAGACCAAACAGATAAGAAGTATACAGAAATTAATATCAAGAATA
AGCGTAATATTGTAATTCAATCAGATAATCCAGAAGAAAAACGTACAATTGATTTCTCTG
GATATTCATTTGATATGAATACAGAGAATTCCGTAACCTTTAAAGATTTGAATATTTATG
CTCGTAGTTACTGGGGCCTTGTTTATAATGCTGGTGGCTACACATTTAATAATGTTAACT
TTACTGGTTCACAACTTATCTATACTAAACCAAGTATTAATTCAACCTTAACATTTAAGA
ATAATATTACTGCAAATTCTGTTTCCTCTTATGTAGGACCACTTGATGGTAAGACCCGCG
ATACTCAACAAAACGGTGGCAGCAAATTCTTCAATTTGAAGGTGGAACAAATCAAATTA
TTTTTGATGAAAACAGTAATGTGACCTTAACAACAGAAGATGCTAATGCTCTTGAAATTG
ATGGTGGGACCACAACAATTGATGTAAAAGACGGTGCAAATGTAGCAATTAATCCACATT
CTAAGGGTAATCCTGAAAGTCGTAATGGAATTGGAACTGGGTATGTTGCTCGTGCTATTG
CTGCCAATGCAAAGATGACAATTAATATTGATCCAAATTCAACTTTAACTATTAATACTG
AAAAGGTAGATGACGATAAAGATGTTGCAGGAGCATTATATTTAAATTCTGATGCTGCTC
TTAATGTAAATGGTAAATTAGTTATCAATTCAAATGGCACACCAAGTGCAAGCAAAAGCA
ATGGTGTTCCAGTATACATCAATGGAAGTGCAAATATTAATGTTGGAAATGGTGGTAGTT
TCGTATTAGATGCTACAAATCTTGGAGACTATAGTTCAAGTCTAATTTCTGTTAATGGTA
AGGGGACGGTTAAGTTAGATCCTCACTCTACTTTCAAGATTAGCGGTGACGGTACTGGAG
CTGTAACTGCAATTAATCTTTCAAGCGGTAGTACTTTCACTTCTGACCAACCAGATTCAT
TTACTATTGATCTCTCCGCAAATACCAGTAATGGTAAGTCACTAATTAAAAATGGTACTA
TTAATTTCAGTCGTGTTAAGACGGTTACTGATGGAAACGAATCACAGCCTTTGGCAAAA
TTGATGTAACTTATGATAGAAATGGTAATGCTACTTCTTACACAATTACTGCACAAGATA
AAAATACGGTTAATCAAGTAGCAGAGGGACTGGCTAACAAGAGCTTGATTAGCTTAGTTA
AAGCTGGTGAGGACGTTACCCTATCTAACCTTCATTTAAGTAAGAATAATGTATTGACTG
GTACTGTAGCTTCAAGTGGTAGTGACAATCCAATTTATGTAACTGTTACCGTTGGTGGGG

TAAGCACTAATGTGCCAGTAGTAGGACATTACACTGTGTACACCAAAACTAATAAAACAG
TAACTTCAAACAATGTTGATTACGCTGCACAAACTGCTTCAACTGGTGGTAATTTCTCTA
TTGATTTATCAAAACTTGCAAGTAGCTTGACTAATGATGCACAAGTTACAGTTACTGCTA
CAAAAGATTTCGTTGAATCCGCTCAAACTAAAAGCGTAGCAGCATTACGTGCACTTAATA
CTACTACTCTTAAAGAATTAGTAGATGCTGCTCCAGCAGAAGAAGCAAAGCCAAGTTACT
ACAATGCTACTGAAGAAGCTCAAAAGGCCTATACTGATGCAATTTCAAAAGGTAATGACA
TTCTTGCCGATCCAAATAATTACGATCAAGTTGACGTTGATAATGCCGTAACTGCCATTC
AAAATGCTCAGAAGGCTTTAACTGGTAAAGAAACTGATAAGAAAGCCCTTCAAAAAGCAG
TTGATGACGCATCAAAAGTAGAATCAAGCGATAACTACACTAATGCGGATGCTAGGCTTC
AAAATGATTACCAAGCTGCAATTAAAGCAGGTCAAGGTATTTTAAGTAAAGAAAATGCTA
CTCAAACTGAGGTAGATAATGCATTAACTACCATTAATAATGCAAAAGATGCTTTAAATG
GGGATGCTAAGAAAGCTGCAAGTAAGGAAGCTCTTCAAAAGGCAGTTGATGAAGCACCTA
CTGTAAAATCAGATGATGCAGCATACTACAATGGTTCTGCTGAAGCTAAAACTGCTTATG
ACGATGCAATCAGTGCAGGTCAAATAGTTTTAGATAATCCAGATGCAACTGCTACTCAAA
TTACTGATGCTTTAAATGCCATTAATACTGCTAAGGGTAATCTTAAGGGTGAAGCAACTG
ATAAAGCAGCGCTTCAAACAGCAGTTGACAATTCTGCAACTGTAAAAGAAAGCAATAACT
ATACTAATGCAGACCAAACTCAAAAGACTGCATATGATAAAGCAGTTACAGCTGCACAAA
CAGTTTTAGATAAAACTAATGCAACTCAAGCAGAAGTGAACCAAGCTCTTCAAGATCTTG
AAACTGCAAATCGTAACCTAAATGGTGATGCTAAGACTGAAGCTGCAAACAAGGCTGCCT
TAGAAGCTGCTGTTAAGGATGCGCCAAATGTAAGAAATACACCTGCATACTACAACGGTA
GTGAAGAAACCCAAACAGCATATAATAATGCAATTACTGCTGGTCAAACAGTTTTGAATG
AAGCAAATCCATCTGCAAGTGAAGTTAAGAATGCTTTAGATGCAATTAATGCAGCAAAAG
ATAACCTTAAAGGTAAGGCTACTAACACTGAAGCTTTGGAAACGGCTTTAACCAATGCTA
ATAATGCTAAGGAAACTGGTAATTACACTAATGCCGACCAAGCAAATCAAGAAGCCCTTA
ATAATGCGATTATTGCTGGTCAAGAGATTTTAAAGAATACTAGTGCAACTCAGGCGCAAG
TAGACAGTGCAGCAAAGGCTATTACTGATGCAATTAGTGGTTTGAATGGGGATACTAACC
TTGCAAATGCTAAGACTGCTGCTACTGAAGATATTCAAAAGGCTTTAGATACTAAGACTA
CCGAAATCACTGACGCTACTAATATTGATCAAGCTACTAAGGATCAATTAATTGCCGATG
CTAAGAAGGCAGCAGAGGATGCTAATACTGCGATTAATCAATCAACCGATACTGATACAG
TTAATACTGCTAAAACTGAAGGGATTGCTAACATCAATAAGGTAACAGTTCCAAGTTTAG
CCGATGCTAAAACTAAGGCAGCCAAAGAAATTGATCAAGCATTAACTGATAAGACCAAGG
AAATCAATAACGCTGAAAATATTGATCAAACAACAAAAGATCAGTTAATTAAAGAAGCAA
CTGATGCAGCAAATACAGCAAAGGATGCTATTGAAAAATCTACAACTAATGACGAAGCAA
CTAAAGCAGGCCAAGCTGGTGTAGATGCAATTACCAATGTAAAGGTACCAAGCGTTACTG

ATAGTCAAAATGCTGCAAAAGAAGTAATTGATGATGCTCTTAACGCTAAGACTAAGGAAA
TCAACGATGCTAACAATATTGATCAAACAACTAAGGATCAATTAATCAAAGAGGCAACCG
ATGCAGCTAATAAGGCTAAAGAAGCCATTGATAAAGCAACTACAGCTGACGCAATTAAGA
CTGCACAAGATGAAGGAACAACTAATATTAATAATGTTACTGTTCCAAGCTTGGAGGATG
CCAAGAAGGCAGCTAATAAAGCAGTAGATGATGCTCTTACTGCTCAAACTGAGGTAATTA
ATAAAGCTAATAATCTTAGTGATGCAGAAAAGAAAGATTTAATTGATCAAGTAACTGCTG
AAGCCAATAAGGCTAAAGAAAATATTGAAACTGCTACAACAAACAATGAGGCAGCTCAAG
CTGGTCAAGCTGGTGTGGATGCAATTAAGAAGATTGTTCCAACAAGCTTAGATACTGTGA
AATCTGATGCAAATAAAGCAATTGACGATGCTCTAACTAAGAAATTAGAAGAGATCAATT
CCGCTAATGATTTAACTACTGATGAAAAGACTGCATTGACACAAGAAGCCAATACAGCTG
CAGATAAGGCAAAAGAAGAAATTACCAATGCAACGACTAATGATGCTGTAATTGAAGCAC
AAAATAATGGTGTTACTGCAATTGATGGCATTAAAGTTCCAACTGAATCAGCAGTCAAAG
AAGCAGCTAAGCAGGCAGTAGCCGACGCAGCAACTGCAAAGAATCAAGCAATTGATGCTT
CAAACTTAACAGATGAAGAAAAAGCTGCCTTGAAGCAAAAGGTTACTGATGCCCAAAATG
CTGCAGATCAAGCAATTGACAATGCAACAACAAATGCGGCAGTAACTGAAGCACAAACCA
ATGGTATTAAGGCAATTAATGGTATTGAAGTAACTACCTCAACTGTTAAAGAAGCAGCTA
AGAAGGCAGTAGCCGACGCAGCAACTGCAAAGAACAATGCAATTGATGCTTCAAACTTAA
CAGATGAAGAAAAAGCTGCCTTAAAGCAAAAAGTTACAGATGCCCAAAATGCTGCAGATC
AAGCAATTGACAACGCAACAACAAATGTAGCAGTGACTGAAGCACAAACCAATGGTGTCA
ATGCAATTAATGGTATTGAAGTACCAACTACATCAGCTACTAAGGAACACGCCATAACTG
ACCTTAATGCGGCTGTTGATGATGCTAAGAAAGCAATTGATCAAGATAGTAACTTGACTG
ATGAAGAGAAGCAAGCAGCTAAGGATCAAATTGATAGGGATGCTACTAAAGCACAAGAAG
CAATCAATAATGCCAAAACTAACGATGATGTTAAGAAGGCGGGTGACGCTGGTACATTAG
CAATTGATAAAGACGTTGCTAATGCAGCCATTGATAACGCAGTTGCTGGAAGAAGGCCG
AAATTTCCAAGACCCCATTAACAGATGAAGAAAAGACTGCTTTGAACAATGAAGTAGATC
AAAAAGCTCAAGAGGCTAAAGAAGCAATCAATAATGCCACTACTCCAGAAGCAGTAACAA
CTGCTCAAGACAGTGGTGTTAATAACATTAATGAAACTTCAGTGCCATCTGAATCAGCAG
CAAAACAAGCAGCTAAGGAAGCTGTGGCTAAGGCAGTTGATGAAAAGAATGCAGCAATTG
ATTCTTCAAACTTAACCGAAGAAGAAAAAGAAGCTTTGAAGCAAAAGGTTACTGAAGCAC
AAACAGCTGCAGATCAAGCAATTGATAATGCAACTACCAACGCTGGAGTAACTGAAGCAC
AAACCAATGGTGTGAATGCAATTAATGGTATTGAAGTACCGAATAAATCAGATGCTAAAG
AACAAGCCATAACTGATCTTAATACTGCTGTTGATAATGCTAAGAAAGCAATTGATCAAG
ATAGCAACTTAACAGATGAAGAAAAACAAGCAGCTAAAGATCAAATTGATTCTGATGCTA
AGAATGCTCAAGATGCCATCAACAATGCAAAGACTAATGATGATGTTAAGAAGGCAGCTT

ATGACGGTACATTAGCAATTGATAAAGACGTTGCTAATGCAGCCATTGATAACGCAGTCG
CTGGTAAGAAAGCAGAAATTTCTAACTCATCATTAACAGACGAAGAAAAGACTGCTTTAA
ACAATGAAGTCGATCAAAAGGCAAATAGTGCTAAAGATGCAATTAACAATGCAACTACTC
CAGAAGCAGTAACTACTGCACAAGGAAATGGCATTAAGAATATCAATGCAACTTCCGTAC
CAACTACATCAACAGCTAAAGAAGCAGCTAAGAAGGCAGTAGCAGAAGCTGCAGAAGCCA
AGAACAGTGCAATCGACTCGTCAAACTTAACAGATGAAGAAAAAGCTGCATTGAAACAAA
AAGTTACTGAAGCACAAAATGGCGCAGATCACGCTATCGATAATGCAACTACCAACGCAG
CCGTAACTGAAGCTAAAGACAATGGTATTAGTGCAATTGACGGCATTGAAGTTCCAATTA
CATCAGCTACTAAGGAACAAGCCATAACTGATCTTAATACGGCTGTTGATGACGCTAAGA
AGACAATTGATCAAGATAACAACTTAACTGATGAACAAAAACAAGCAGCTAAAGATCAAA
TTGATTCTGATGCTAAGACTGCCCAAGATGCCATCAACAATGCTAAGACTGATAATGACG
TAAACAATGCAGTTAACAGTGGTAAGGTTGCCATTAACAAAGATGTTGCTAATGCAGCTA
TCGACAATGCAGTTGCTGGTAAGCTTAAGGAGATCCAAGATCCATTAACTACTGAAGAAA
AACAAGCTTACACTGATTTAATTAACTCTGAAGCAACTAATGCAAAACAAAATATTGCTA
ATGCAACTACTGTTGAAGCAGTAACTACTGCACAAACAAATGGTGTAAATGAAATTACTA
ATACCGAAATTCCAACTACTTCTTCCGCCAAAGAAAAGGCAATTGCTGCCATCAATGATG
CTCTTCAAACGAAGACCGACGAAATCAACAACGCATCTAATATCAACACTCAAGAAAAGA
CTGATTTAATTAATCAAGCAACTGAAGCAGCCAATGCAGCTAAGAACAATATCAACAACG
CCACTACTAATGCTGATGTTGATACTGCACAAATAAAGGGTGAAAAAGCAATTGCTGATG
TAACAGTTCCAAACTTGTCTGATGTTAAGAAAGAAAGTATTGATCTAATTAACAAAGCTT
TAGATGCCAAGACTAATGAAATCAATAATGCTTCTAACCTTTCTCAAGATGAAAAGCAAG
GTTTGATCAATGATGCAACTAATGCCGCAACTGAGGCAATTAACAACGTCAACCAAGCTC
AAACAAATGACGATGCTAAAGCTGCTGCAACTACTGGCGTTCAAAACATCGAAAACATTA
CTATTCCAACTCTTGATGAAGCGAAGAAGAACGCTAACCAAGCTATTGATGATGCATTGA
ACTCTAAAGTAAATGAGATTAACAATGCTTCTAACTTGAATGAAACTGAAAAGCAAAAAT
TAGTTGATCAGGCAAATGAAGCAGCAACTACTGCTAAGAATAATGTTGAAAAGGCCACTA
CCAATGATGATGCTCGCGATGCAGCTAATGCTGGAATTGATAACATTAAAGGTATTACCT
TTACTTCACTAGAAGATGCTAAAAATGCAGCAAACACTGCAATTGACAATGCTTTGCAAG
TTAAGACAGATGAAATTAATAATGCATCTAACTTAAGTACTGAAGAAAAGCAAGACTTAA
TCAATCAAGCAAGTGAAGTAGCTAAAAATGCAAAAGACAATATCAACAATGCAACTACCA
ATGATGCTGTAACTGAAGCACAAAATAAGGGTATTGCAGATATTGCCAATGTGACTGTTC
CAAGCTTAGATCAAGTTAAACAAGATGCAATCAATGCAATTAAGCAAGTTCAAGATGCTA
AGAATAAGCAAATCTCTGCTGCATCTAACTTAAGCACTGAAGAGCAAAAAGAATTAAGTG
ATCAAGTAGATAAAATTGCTAACGATGCAATTGCTCAGATTAATGATTCATCAACTACTA

CAAATGATGCAGTTACTGCAACCCGTGATGATGCAATCAAGCAAATTACTGATCTATTTA
TTCCAACTCTAGACGGTGCACAAACAGATGCTCTTAATGCAATTGAAAGTGCTAAGAATG
CTAAACTAAATGACATTAACAACGCTACTCACTTAACTGATCAAGAAAAGCAAGCTTTAG
TTGATCAAACCAATAAAGTGGCTGATGATGCAACTAAGGAAATTAAGGCTGCTCAAACAA
ATGACGCAGTCAAGAGTGCAGAAACAGCTGGCTTAGATAACATTAATAACATTGCTATTC
CAACTTTAGTTCAGAAGCAACAAGAAGCTATTGAAGAATTGAATGTAGCACGTGATGCTA
AGAGTAGTGCAATTGATAATGCTACTGATTTAACTACTGATGAAAAGAACAGCTTGAAAG
ATAAAGTTCAAGCAGAATACAGTAACGCGGTATCAAATATCACCAGTGCAACAACTGATG
AAGCAGTAACAACTGCTAAAGAAAACGGCATCGCAGCAATTAAAGACATTCAAATTCCAA
CTAAGTCACCTGCTAAGGAACAAGCAACAAGTGACCTTAATACTGCAGTTGATGAAGCTA
AGAACGCAATCGATCAAGACAACAACTTGACTGATGAAGAGAAGCAAGCAGCTAAGGATC
AAATTGATTCTGATGCTAAGAAGGCGCAAGAAGCAATTGATAATGCTACGACTGATGATG
AGGTTAATAGTGCTGTTGATAATGGTAAGCTTGCCATTGATAAAGATATTGCTAATGCAG
CAATCGACAATGCAGTTGCTGGTAAGAAAGCAGAAATTTCTAAATCATCCTTAACATCTG
AAGAAAAAGCTGATTTAAACAATGAAGTCGATCAAAAGGCTAAAGATGCTAAAGAAGCAA
TTAAGGCTGCCACTACTCCAGAAACTGTAACTACTGCCCAAGAAAATGGTATTAAGAATA
TCAATGATACTAAAGTACCAACTGAGTCAGCAGCTAAAGAAGCTGCTAAGAAGGCAGTGG
CAGAAGCTGCAGAAGCCAAGAACAATGCAATCGATTCTTCAGACTTAACAAATGAAGAAA
AGGCTGCATTGAAGCAAGAAGTTGCTGATGCACAAAACGCAGCAGATAGAGCAATAGATG
CTGCAACTACAAACACTGCAGTAACTGAAGCGCAAGACAATGGTATTAAAGCAATTGAGA
ATGTTACTGTACCTACCGAATCAGCAGCCAAAGAAACTGCTAAGAAGGCAGTAGCAGAGG
CTGCAGAAGCTAAAAACAATGCAATTGATTCTTCAAACTTAACATCTGAAGAAAAAGCTG
CATTGAAACAAGAAGTAGCAGAAGCACAAAATGCTGCCAATACCGCAATTGATAACGCAA
CTACCAATGCCGCAGTAACCGAAGCAGAAGATAATGGTATTAAAGCAATTAATTCTATTG
AAGTACCAACTAAGTCAGATGCTAAGGAACAAGCAACAAGCGATCTTAATAGTGCTGTTG
ATGAAGCGAAGAAGGCAATCGATCAAGATAGCAACTTGACTGATGAAGAGAAACAAGTAG
CTAAAGATCAAATTGACTCTGATGCTAAGAAAGCGCAAGAAGCAATTGATACTGCTAAGA
CTAACGATGACGTTAAGAAGGCAATCGATGACGGTACATTAGCAATTGATAAAGACGTTG
CTAATGCAGCAATCGATAACGCAGTTGCAGGCAAGAAGGCTGAAATTTCTAAGTCACCAT
TAACTGATGAAGAAAAGACTGCTTTGAACAATGAAGTAGATGAAAAGGCAAATAGTGCTA
AAGATGCTATCAATAAGGCAACTACTCCAGAGGGAGTAACTGAAGCACAAAATAGTGGTA
TTAAATCCATTGATGATGTCAATGTACCAACTGAGTCAGTAGCTAAGGAAGCAGCTAAGA
AGGCAGTAGCCGAAGCTGCAGAAGCTAAGAACAATGCAATTGATTCTTCAAACTTAACAG
ACGAAGAAAAAGCTGCATTGAAGCAAGAAGTTAGTGATGCACAAACTGCTGCTAATACTG

CCATTGATAATGCAATTACCAACGCAGAAGTAACTGAAGCTGAAGACAATGGTGTTAAGA
CAATTAATGGTATTGAAGTACCAACTAAATCAACTACTAAAGAACAAGCAACAAATGATC
TTAACAACGAAGTTGAAAATGCTAAGAAGGCAATTGATCAAGATAACAACTTAACTGATG
AGCAAAAACAAGCAGCCAAGGATCAAATTGATTCGGACGCTAAGAAAGCTCAAGATGCCA
TCAACAATGCTAAGAATAATGATGATGTTAAGAAGGCAGTTGATGATGGAAAACTAGTAA
TTGACAAAGATGTTGCCAACGCTGCCATCGATAACGCAGTTGCTGGCAAGAAGGATGAAA
TTTCTAAATCACCATTAACTGATGAAGAAAAAGCTGCTTTAAACAATGAAGTTGATCAAA
AGGCAGAAGAAGCTAAGGAAGCAATTAAGGCTGCCACTACTCCAGGAGCTGTAACTACTG
CACAAGAAAATGGTATTAAGAATATCAATGAGACTGAAGTACCAACTGAGTCCGTAGCTA
AGGAAGCAGCTAAGAGGGCAGTAGCTGAAGCTGCAGACGCAAAGAATAAAGCAATTGATT
CTTCAAACTTAACAGACGAAGAAAAAGCTGCATTGAAGCAAGAAGTTAGTGATGCACAAA
CTGCTGCTAATACTGCCATTGATAATGCAACTACCAATGCCGCAGTAACCGAAGCAGAAG
ATAATGGTATTAAAGCAATTAATTCTATTGAAGTACCAACTAAGTCAGATGCTAAGGAAC
AAGCAACAAGCGATCTTAATAGTGCTGTTGATGAAGCGAAGAAGGCAATCGATCAAGATA
GCAACTTGACTGATGAAGAGAAACAAGTAGCTAAAGATCAAATTGACTCTGATGCTAAGA
AAGCGCAAGAAGCAATTGATACTGCTAAGACTAACGATGACGTTAAGAAGGCAATCGATG
ACGGTACATTAGCAATTGATAAAGACGTTGCTAATGCAGCAATCGATAACGCAGTTGCAG
GCAAGAAGGCTGAAATTTCTAAGTCACCATTAACTGATGAAGAAAAGACTGCTTTGAACA
ATGAAGTAGATGAAAAGGCAAATAGTGCTAAAGATGCTATCAATAAGGCAACTACTCCAG
AGGGAGTAACTGAAGCACAAAATAGTGGTATTAAATCCATTGATGATGTCAATGTACCAA
CTGAATCAGTAGCTAAAGAAGCAGCTAAGAAGGCAGTAGCCGAAGCTGCAGAAGCTAAGA
ACAATGCAATTGATTCTTCAAACTTAACAGACGAAGAAAAAGCTGCATTGAAGCAAGAAG
TTAGTGATGCACAAACTGCTGCTAATACTGCCATTGATAATGCAATTACCAACGCAGAAG
TAACTGAAGCTGAAGACAATGGTGTTAAGACAATTAATGGTATTGAAGTACCAACTAAAT
CAACTACTAAAGAACAAGCAACAAATGATCTTAACAACGAAGTTGAAAATGCTAAGAAGG
CAATTGATCAAGATAGCAACTTGACTGATGAAGAGAAACAAGTAGCTAAAGATCAAATTG
ACTCTGATGCTAAGAAAGCTCAAGATGCCATCAACAATGCTAAGACTAACGATGACGTTA
AGAAGGCAATCGATGATGGTACATTAGCAATTGATAAAGACGTTGCTAATGCAGCTATCG
ACAATGCAGTTGCTGGTAAGCTTAAGGAAATTAATGATTCATTAACAAATGAAGAAAAAC
AAGCTTACACTGATCTAATCAATAATGAAGCAGATAATGCAAAACAAAAGATTGCAGAAG
CAACTACTCCTGAAGAAGTAACTCGTGCTCAAGAAGAAGGAGTTAAGAACATTAATAATA
TTAACGTTCCAACTACTTCTCCAGCTAAGGACGCAGCGAATGCTGCAATTGATCAGGCTT
TGAAGAACAAGAAGGATGAAATCAATAATGCAACTAACATTTCTTCTGAAGAAAAGACTG
ATTTAATTAAACAAGCAACTGAAGCTGCAAATATTGCTAAAGACAACATCAACAATGCAA

CTACTAATTCAGAAGTTGAAACTGCTCAAGTTGATGGTGAAAAAGCTATTGCGGACGTTA
CTGGTCCAGGTTTATCTGACATCAAGAAGGAAAGTATTGATCTTATCAACAAAGCCTTAA
ATGAAAAGCAAGATGAAATCAATAACGCTTCTAACTTGAGTCAAGATGAAAGCACAGAGC
TTATTGATCAAGCAAAGAAGATTGCTACTGAAGCAATTAATGAAATCAATAATGCTCAAA
CTAATGATGAAGCTAAGGAAGCTGCAGATACTGGAGTTAAGAACATTGAAAATGTTTCAA
TTCCAAGTATTGAAGATGCTAAGAAGAATGCAACTCAAGCAATTGATGATGCTTTGAATT
CTAAGAAGAATGAAATTAACAACGCATCTAACTTAACTGATTCAGAAAAGACTGATTTAA
TCAATCAAGCAACTGAAATTGCCAATGCTGCTAAGGATGCTATCAACAGTGCAACGACTA
ATACTGCAGTTGAAGCTGCCGAATATAAAGGTGTTGCTGATATTAACAACATTCACTTCA
CTAACTTAGACGATTCTAAGAAGGCAGCAAATAGTGCGATTGAAGATGCTTTGACCACTA
AGAAGGATGAAATTAATAATGCATCTAACTTGAGTGATTCAGAAAAGGCTAAGTTAATCA
ATCAAGCAACTGAAATTGCCAATGCTGCTAAGGCTGCAATTAACAATGCAACTACTAATT
CGGCTGTTACTGCGGCAGAAAACAAGGGAATTGAAGATATTGCAAATATTAATGTTCCAA
GTTTAGCTGAAACTAAACAGGCAGCAATTGACGCAATTCAGCAAGTTCAAAAAGCTAAGA
ACAGTCAAATTGAAGAAGCAAAGAATCTAAGTGCAGACGAGCAAAAGAACTTAATTGATC
AAGTAAATAAGATTGCTCAGGACGCAATTAATAAGCTTAACGATCCAGCAACAACTACAA
ATGAAGTTATTACTGACACTCGTGATAAAGCAATTGATCAAATTACTAACTTGTTCATTC
CAACCTTAGATAGTGTCCAAAAAGATGCTCAGGAAGCAATTAACTCTGCTCAGGAAACTA
AGATTGACGAAATTAATAAGGCTGATAATTTAACTGATCAAATGAAGCAAAACTTAATTG
ACCAAGTAGATCAAGTTGCTGATAAGGCTACTAAGGCCATTAATAATGCTCAAACAAACG
ATGATGTTAAAGAAGCAGAAATCGAGGGCTTAGAAGATATTGATAGCATTAAAGTTCCAA
GCTTAGTTGAAAGTAAGGATGATGCTATTAAGGAAATCAATGATGCTTTGAAGAAGAAGA
CTGATGAAATCAATGCAGCTGATCTTGATCAAAAGCAAAAAGACGAATTAATCAGTCAAA
TTACTGATATTGCTACTGAGACTAAGACCAAGGTATTCAATGCTACAACTAATGCAGAAG
TTGATGCTGAAGCCGAAGCTGGTATTAAAGCCATCGAAGCTGTGAAGATTCCAGCTCGTA
CTGCTGACAACAGCAATACTGAGAGTGAAAGTAAGGAACAAACTGTAATTACAAATTCAG
TTCAACCTAAGAGAAATGCTGTTCATCATAAGAATGGCACTCCAGTAAACAAGAAGGCTA
CTTTACCACAAACTGGTAAGAAGGATAATCTAACTTTAACGTTAGCAGGAGCTGCACTTC
TTGGTTTAGCAGGTGTATTCTCATTGTTTGGATTAGGAGACAAGAGAAAAAAGAATAAAT
AGTCAACATCGTTGATATGAAAAAGAGCGATACTCCCTAAAGTGGAATATCGCCTTTTTT
GTTTGGAAATGTAATAAAGGCAGAAGCACAATTAAATGCTTCTGCCTTTATTACATGATT
TTATTCCGCTGTGACTGGGAATAAAAATGAAATTACTGGTATAACTACATAAAGAATCAT
GGCAAGTCTAGGAAGAACAAACGATAGCCCAATGAGAACAATATTTAAAAATAAGGTGAC
AGCAACCTTT

SEQ.ID. 6: CSP10 coding sequence only

ATGGTTTCAAAAAACAATATTGAAATGAAATATAAAAAAATGAGAGACGAAAAACAACGT
TTCTCAATTCGTAAATTTAGTGTGGGAGCTGCATCCGTTTTAATTGGGCTAAGTTTTAGT
TTATATAACGGACAACAAGCATTTGCTGATACTGTTGATAATGGCAATAAGTCTGTTGTA
GCAAATGCACAAGATAAAGAACAAACAGACAATAAACAAAATACTGATACTAATCCTCAA
AATAACGAAGATAAAACAAATAATAATCAAACTCAAAATACAGTAACTGCTGATCTTGAT
CACAGTACTGTTGGGGCTAGTTACAAGGCTGATACTACTAATGCTAGCGAAGCTAACAAG
AGTAGTGATAAGTCAGATGAAAAGCAAGCTGCAACTACTGAAACTGCTAAAACTGAAGAA
GCTGCTACTAAGGAAGTAGAAGCAAAGAATACTGCAAATGATGATGTAGCTAAGAAAACT
GAAGAGGCAGCTGCTAAGACAGATACTAAGGTCGATGTTAATAAAACAGTAGTAGAAAAT
GTAAAGACTGAAGCTGCTACTCCTAATGTAGAGACTAGGAACATTGAAACTCAAACTGCA
AAGACTGATGAAGAAAAAGTAGAAACAGCAGTTGACAATACTTCTTCTGATTTAAAAAAC
GCAGTAGAGAATGCAAATACTGAAGTTTCAACTAATACATTTAACGTAAATAAAGCAGTT
CGAAATGCATTATTCCTTAGTGCAAATAATCTTGCTGAACGTAAGGTAGCAGCTATAGCA
CTTCCAGCTGAAGCAGAAGATCCTAATGCCGTAACAGTTAGTGATGCGAAGGGATTTATT
GATGCAATTCAAAATGGGACTGCTACTACTATTAATGTTGCAAGTGATCTTAATTTAGCA
GACCAAACAGATAAGAAGTATACAGAAATTAATATCAAGAATAAGCGTAATATTGTAATT
CAATCAGATAATCCAGAAGAAAAACGTACAATTGATTTCTCTGGATATTCATTTGATATG
AATACAGAGAATTCCGTAACCTTTAAAGATTTGAATATTTATGCTCGTAGTTACTGGGGC
CTTGTTTATAATGCTGGTGGCTACACATTTAATAATGTTAACTTTACTGGTTCACAACTT
ATCTATACTAAACCAAGTATTAATTCAACCTTAACATTTAAGAATAATATTACTGCAAAT
TCTGTTTCCTCTTATGTAGGACCACTTGATGGTAAGACCCGCGATACTCAACAAAACGGT
GGGCAGCAAATTCTTCAATTTGAAGGTGGAACAAATCAAATTATTTTTGATGAAAACAGT
AATGTGACCTTAACAACAGAAGATGCTAATGCTCTTGAAATTGATGGTGGGACCACAACA
ATTGATGTAAAAGACGGTGCAAATGTAGCAATTAATCCACATTCTAAGGGTAATCCTGAA
AGTCGTAATGGAATTGGAACTGGGTATGTTGCTCGTGCTATTGCTGCCAATGCAAAGATG
ACAATTAATATTGATCCAAATTCAACTTTAACTATTAATACTGAAAAGGTAGATGACGAT
AAAGATGTTGCAGGAGCATTATATTTAAATTCTGATGCTGCTCTTAATGTAAATGGTAAA
TTAGTTATCAATTCAAATGGCACACCAAGTGCAAGCAAAAGCAATGGTGTTCCAGTATAC
ATCAATGGAAGTGCAAATATTAATGTTGGAAATGGTGGTAGTTTCGTATTAGATGCTACA
AATCTTGGAGACTATAGTTCAAGTCTAATTTCTGTTAATGGTAAGGGGACGGTTAAGTTA
GATCCTCACTCTACTTTCAAGATTAGCGGTGACGGTACTGGAGCTGTAACTGCAATTAAT

CTTTCAAGCGGTAGTACTTTCACTTCTGACCAACCAGATTCATTTACTATTGATCTCTCC
GCAAATACCAGTAATGGTAAGTCACTAATTAAAAATGGTACTATTAATTTCAGTCGTGTT
AAGACGGTTACTGATGGAAACGAATCACAGCCTTTGGCAAAATTGATGTAACTTATGAT
AGAAATGGTAATGCTACTTCTTACACAATTACTGCACAAGATAAAAATACGGTTAATCAA
GTAGCAGAGGGACTGGCTAACAAGAGCTTGATTAGCTTAGTTAAAGCTGGTGAGGACGTT
ACCCTATCTAACCTTCATTTAAGTAAGAATAATGTATTGACTGGTACTGTAGCTTCAAGT
GGTAGTGACAATCCAATTTATGTAACTGTTACCGTTGGTGGGGTAAGCACTAATGTGCCA
GTAGTAGGACATTACACTGTGTACACCAAAACTAATAAAACAGTAACTTCAAACAATGTT
GATTACGCTGCACAAACTGCTTCAACTGGTGGTAATTTCTCTATTGATTTATCAAAACTT
GCAAGTAGCTTGACTAATGATGCACAAGTTACAGTTACTGCTACAAAAGATTTCGTTGAA
TCCGCTCAAACTAAAAGCGTAGCAGCATTACGTGCACTTAATACTACTACTCTTAAAGAA
TTAGTAGATGCTGCTCCAGCAGAAGAAGCAAAGCCAAGTTACTACAATGCTACTGAAGAA
GCTCAAAAGGCCTATACTGATGCAATTTCAAAAGGTAATGACATTCTTGCCGATCCAAAT
AATTACGATCAAGTTGACGTTGATAATGCCGTAACTGCCATTCAAAATGCTCAGAAGGCT
TTAACTGGTAAAGAAACTGATAAGAAAGCCCTTCAAAAAGCAGTTGATGACGCATCAAAA
GTAGAATCAAGCGATAACTACACTAATGCGGATGCTAGGCTTCAAAATGATTACCAAGCT
GCAATTAAAGCAGGTCAAGGTATTTTAAGTAAAGAAAATGCTACTCAAACTGAGGTAGAT
AATGCATTAACTACCATTAATAATGCAAAAGATGCTTTAAATGGGGATGCTAAGAAAGCT
GCAAGTAAGGAAGCTCTTCAAAAGGCAGTTGATGAAGCACCTACTGTAAAATCAGATGAT
GCAGCATACTACAATGGTTCTGCTGAAGCTAAAACTGCTTATGACGATGCAATCAGTGCA
GGTCAAATAGTTTTAGATAATCCAGATGCAACTGCTACTCAAATTACTGATGCTTTAAAT
GCCATTAATACTGCTAAGGGTAATCTTAAGGGTGAAGCAACTGATAAAGCAGCGCTTCAA
ACAGCAGTTGACAATTCTGCAACTGTAAAAGAAAGCAATAACTATACTAATGCAGACCAA
ACTCAAAAGACTGCATATGATAAAGCAGTTACAGCTGCACAAACAGTTTTAGATAAAACT
AATGCAACTCAAGCAGAAGTGAACCAAGCTCTTCAAGATCTTGAAACTGCAAATCGTAAC
CTAAATGGTGATGCTAAGACTGAAGCTGCAAACAAGGCTGCCTTAGAAGCTGCTGTTAAG
GATGCGCCAAATGTAAGAAATACACCTGCATACTACAACGGTAGTGAAGAAACCCAAACA
GCATATAATAATGCAATTACTGCTGGTCAAACAGTTTTGAATGAAGCAAATCCATCTGCA
AGTGAAGTTAAGAATGCTTTAGATGCAATTAATGCAGCAAAAGATAACCTTAAAGGTAAG
GCTACTAACACTGAAGCTTTGGAAACGGCTTTAACCAATGCTAATAATGCTAAGGAAACT
GGTAATTACACTAATGCCGACCAAGCAAATCAAGAAGCCCTTAATAATGCGATTATTGCT
GGTCAAGAGATTTTAAAGAATACTAGTGCAACTCAGGCGCAAGTAGACAGTGCAGCAAAG
GCTATTACTGATGCAATTAGTGGTTTGAATGGGGATACTAACCTTGCAAATGCTAAGACT
GCTGCTACTGAAGATATTCAAAAGGCTTTAGATACTAAGACTACCGAAATCACTGACGCT

ACTAATATTGATCAAGCTACTAAGGATCAATTAATTGCCGATGCTAAGAAGGCAGCAGAG
GATGCTAATACTGCGATTAATCAATCAACCGATACTGATACAGTTAATACTGCTAAAACT
GAAGGGATTGCTAACATCAATAAGGTAACAGTTCCAAGTTTAGCCGATGCTAAAACTAAG
GCAGCCAAAGAAATTGATCAAGCATTAACTGATAAGACCAAGGAAATCAATAACGCTGAA
AATATTGATCAAACAACAAAAGATCAGTTAATTAAAGAAGCAACTGATGCAGCAAATACA
GCAAAGGATGCTATTGAAAAATCTACAACTAATGACGAAGCAACTAAAGCAGGCCAAGCT
GGTGTAGATGCAATTACCAATGTAAAGGTACCAAGCGTTACTGATAGTCAAAATGCTGCA
AAAGAAGTAATTGATGATGCTCTTAACGCTAAGACTAAGGAAATCAACGATGCTAACAAT
ATTGATCAAACAACTAAGGATCAATTAATCAAAGAGGCAACCGATGCAGCTAATAAGGCT
AAAGAAGCCATTGATAAAGCAACTACAGCTGACGCAATTAAGACTGCACAAGATGAAGGA
ACAACTAATATTAATAATGTTACTGTTCCAAGCTTGGAGGATGCCAAGAAGGCAGCTAAT
AAAGCAGTAGATGATGCTCTTACTGCTCAAACTGAGGTAATTAATAAAGCTAATAATCTT
AGTGATGCAGAAAAGAAAGATTTAATTGATCAAGTAACTGCTGAAGCCAATAAGGCTAAA
GAAAATATTGAAACTGCTACAACAAACAATGAGGCAGCTCAAGCTGGTCAAGCTGGTGTG
GATGCAATTAAGAAGATTGTTCCAACAAGCTTAGATACTGTGAAATCTGATGCAAATAAA
GCAATTGACGATGCTCTAACTAAGAAATTAGAAGAGATCAATTCCGCTAATGATTTAACT
ACTGATGAAAAGACTGCATTGACACAAGAAGCCAATACAGCTGCAGATAAGGCAAAAGAA
GAAATTACCAATGCAACGACTAATGATGCTGTAATTGAAGCACAAAATAATGGTGTTACT
GCAATTGATGGCATTAAAGTTCCAACTGAATCAGCAGTCAAAGAAGCAGCTAAGCAGGCA
GTAGCCGACGCAGCAACTGCAAAGAATCAAGCAATTGATGCTTCAAACTTAACAGATGAA
GAAAAAGCTGCCTTGAAGCAAAAGGTTACTGATGCCCAAAATGCTGCAGATCAAGCAATT
GACAATGCAACAACAAATGCGGCAGTAACTGAAGCACAAACCAATGGTATTAAGGCAATT
AATGGTATTGAAGTAACTACCTCAACTGTTAAAGAAGCAGCTAAGAAGGCAGTAGCCGAC
GCAGCAACTGCAAAGAACAATGCAATTGATGCTTCAAACTTAACAGATGAAGAAAAAGCT
GCCTTAAAGCAAAAAGTTACAGATGCCCAAAATGCTGCAGATCAAGCAATTGACAACGCA
ACAACAAATGTAGCAGTGACTGAAGCACAAACCAATGGTGTCAATGCAATTAATGGTATT
GAAGTACCAACTACATCAGCTACTAAGGAACACGCCATAACTGACCTTAATGCGGCTGTT
GATGATGCTAAGAAAGCAATTGATCAAGATAGTAACTTGACTGATGAAGAGAAGCAAGCA
GCTAAGGATCAAATTGATAGGGATGCTACTAAAGCACAAGAAGCAATCAATAATGCCAAA
ACTAACGATGATGTTAAGAAGGCGGGTGACGCTGGTACATTAGCAATTGATAAAGACGTT
GCTAATGCAGCCATTGATAACGCAGTTGCTGGGAAGAAGGCCGAAATTTCCAAGACCCCA
TTAACAGATGAAGAAAAGACTGCTTTGAACAATGAAGTAGATCAAAAAGCTCAAGAGGCT
AAAGAAGCAATCAATAATGCCACTACTCCAGAAGCAGTAACAACTGCTCAAGACAGTGGT
GTTAATAACATTAATGAAACTTCAGTGCCATCTGAATCAGCAGCAAAACAAGCAGCTAAG

GAAGCTGTGGCTAAGGCAGTTGATGAAAAGAATGCAGCAATTGATTCTTCAAACTTAACC
GAAGAAGAAAAAGAAGCTTTGAAGCAAAAGGTTACTGAAGCACAAACAGCTGCAGATCAA
GCAATTGATAATGCAACTACCAACGCTGGAGTAACTGAAGCACAAACCAATGGTGTGAAT
GCAATTAATGGTATTGAAGTACCGAATAAATCAGATGCTAAAGAACAAGCCATAACTGAT
CTTAATACTGCTGTTGATAATGCTAAGAAAGCAATTGATCAAGATAGCAACTTAACAGAT
GAAGAAAAACAAGCAGCTAAAGATCAAATTGATTCTGATGCTAAGAATGCTCAAGATGCC
ATCAACAATGCAAAGACTAATGATGATGTTAAGAAGGCAGCTTATGACGGTACATTAGCA
ATTGATAAAGACGTTGCTAATGCAGCCATTGATAACGCAGTCGCTGGTAAGAAAGCAGAA
ATTTCTAACTCATCATTAACAGACGAAGAAAAGACTGCTTTAAACAATGAAGTCGATCAA
AAGGCAAATAGTGCTAAAGATGCAATTAACAATGCAACTACTCCAGAAGCAGTAACTACT
GCACAAGGAAATGGCATTAAGAATATCAATGCAACTTCCGTACCAACTACATCAACAGCT
AAAGAAGCAGCTAAGAAGGCAGTAGCAGAAGCTGCAGAAGCCAAGAACAGTGCAATCGAC
TCGTCAAACTTAACAGATGAAGAAAAAGCTGCATTGAAACAAAAAGTTACTGAAGCACAA
AATGGCGCAGATCACGCTATCGATAATGCAACTACCAACGCAGCCGTAACTGAAGCTAAA
GACAATGGTATTAGTGCAATTGACGGCATTGAAGTTCCAATTACATCAGCTACTAAGGAA
CAAGCCATAACTGATCTTAATACGGCTGTTGATGACGCTAAGAAGACAATTGATCAAGAT
AACAACTTAACTGATGAACAAAAACAAGCAGCTAAAGATCAAATTGATTCTGATGCTAAG
ACTGCCCAAGATGCCATCAACAATGCTAAGACTGATAATGACGTAAACAATGCAGTTAAC
AGTGGTAAGGTTGCCATTAACAAAGATGTTGCTAATGCAGCTATCGACAATGCAGTTGCT
GGTAAGCTTAAGGAGATCCAAGATCCATTAACTACTGAAGAAAAACAAGCTTACACTGAT
TTAATTAACTCTGAAGCAACTAATGCAAAACAAAATATTGCTAATGCAACTACTGTTGAA
GCAGTAACTACTGCACAAACAAATGGTGTAAATGAAATTACTAATACCGAAATTCCAACT
ACTTCTTCCGCCAAAGAAAAGGCAATTGCTGCCATCAATGATGCTCTTCAAACGAAGACC
GACGAAATCAACAACGCATCTAATATCAACACTCAAGAAAAGACTGATTTAATTAATCAA
GCAACTGAAGCAGCCAATGCAGCTAAGAACAATATCAACAACGCCACTACTAATGCTGAT
GTTGATACTGCACAAATAAAGGGTGAAAAAGCAATTGCTGATGTAACAGTTCCAAACTTG
TCTGATGTTAAGAAAGAAAGTATTGATCTAATTAACAAAGCTTTAGATGCCAAGACTAAT
GAAATCAATAATGCTTCTAACCTTTCTCAAGATGAAAAGCAAGGTTTGATCAATGATGCA
ACTAATGCCGCAACTGAGGCAATTAACAACGTCAACCAAGCTCAAACAAATGACGATGCT
AAAGCTGCTGCAACTACTGGCGTTCAAAACATCGAAAACATTACTATTCCAACTCTTGAT
GAAGCGAAGAAGAACGCTAACCAAGCTATTGATGATGCATTGAACTCTAAAGTAAATGAG
ATTAACAATGCTTCTAACTTGAATGAAACTGAAAAGCAAAAATTAGTTGATCAGGCAAAT
GAAGCAGCAACTACTGCTAAGAATAATGTTGAAAAGGCCACTACCAATGATGATGCTCGC
GATGCAGCTAATGCTGGAATTGATAACATTAAAGGTATTACCTTTACTTCACTAGAAGAT

GCTAAAAATGCAGCAAACACTGCAATTGACAATGCTTTGCAAGTTAAGACAGATGAAATT
AATAATGCATCTAACTTAAGTACTGAAGAAAAGCAAGACTTAATCAATCAAGCAAGTGAA
GTAGCTAAAAATGCAAAAGACAATATCAACAATGCAACTACCAATGATGCTGTAACTGAA
GCACAAAATAAGGGTATTGCAGATATTGCCAATGTGACTGTTCCAAGCTTAGATCAAGTT
AAACAAGATGCAATCAATGCAATTAAGCAAGTTCAAGATGCTAAGAATAAGCAAATCTCT
GCTGCATCTAACTTAAGCACTGAAGAGCAAAAAGAATTAAGTGATCAAGTAGATAAAATT
GCTAACGATGCAATTGCTCAGATTAATGATTCATCAACTACTACAAATGATGCAGTTACT
GCAACCCGTGATGATGCAATCAAGCAAATTACTGATCTATTTATTCCAACTCTAGACGGT
GCACAAACAGATGCTCTTAATGCAATTGAAAGTGCTAAGAATGCTAAACTAAATGACATT
AACAACGCTACTCACTTAACTGATCAAGAAAAGCAAGCTTTAGTTGATCAAACCAATAAA
GTGGCTGATGATGCAACTAAGGAAATTAAGGCTGCTCAAACAAATGACGCAGTCAAGAGT
GCAGAAACAGCTGGCTTAGATAACATTAATAACATTGCTATTCCAACTTTAGTTCAGAAG
CAACAAGAAGCTATTGAAGAATTGAATGTAGCACGTGATGCTAAGAGTAGTGCAATTGAT
AATGCTACTGATTTAACTACTGATGAAAAGAACAGCTTGAAAGATAAAGTTCAAGCAGAA
TACAGTAACGCGGTATCAAATATCACCAGTGCAACAACTGATGAAGCAGTAACAACTGCT
AAAGAAAACGGCATCGCAGCAATTAAAGACATTCAAATTCCAACTAAGTCACCTGCTAAG
GAACAAGCAACAAGTGACCTTAATACTGCAGTTGATGAAGCTAAGAACGCAATCGATCAA
GACAACAACTTGACTGATGAAGAGAAGCAAGCAGCTAAGGATCAAATTGATTCTGATGCT
AAGAAGGCGCAAGAAGCAATTGATAATGCTACGACTGATGATGAGGTTAATAGTGCTGTT
GATAATGGTAAGCTTGCCATTGATAAAGATATTGCTAATGCAGCAATCGACAATGCAGTT
GCTGGTAAGAAAGCAGAAATTTCTAAATCATCCTTAACATCTGAAGAAAAAGCTGATTTA
AACAATGAAGTCGATCAAAAGGCTAAAGATGCTAAAGAAGCAATTAAGGCTGCCACTACT
CCAGAAACTGTAACTACTGCCCAAGAAAATGGTATTAAGAATATCAATGATACTAAAGTA
CCAACTGAGTCAGCAGCTAAAGAAGCTGCTAAGAAGGCAGTGGCAGAAGCTGCAGAAGCC
AAGAACAATGCAATCGATTCTTCAGACTTAACAAATGAAGAAAAGGCTGCATTGAAGCAA
GAAGTTGCTGATGCACAAAACGCAGCAGATAGAGCAATAGATGCTGCAACTACAAACACT
GCAGTAACTGAAGCGCAAGACAATGGTATTAAAGCAATTGAGAATGTTACTGTACCTACC
GAATCAGCAGCCAAAGAAACTGCTAAGAAGGCAGTAGCAGAGGCTGCAGAAGCTAAAAAC
AATGCAATTGATTCTTCAAACTTAACATCTGAAGAAAAAGCTGCATTGAAACAAGAAGTA
GCAGAAGCACAAAATGCTGCCAATACCGCAATTGATAACGCAACTACCAATGCCGCAGTA
ACCGAAGCAGAAGATAATGGTATTAAAGCAATTAATTCTATTGAAGTACCAACTAAGTCA
GATGCTAAGGAACAAGCAACAAGCGATCTTAATAGTGCTGTTGATGAAGCGAAGAAGGCA
ATCGATCAAGATAGCAACTTGACTGATGAAGAGAAACAAGTAGCTAAAGATCAAATTGAC
TCTGATGCTAAGAAAGCGCAAGAAGCAATTGATACTGCTAAGACTAACGATGACGTTAAG

AAGGCAATCGATGACGGTACATTAGCAATTGATAAAGACGTTGCTAATGCAGCAATCGAT
AACGCAGTTGCAGGCAAGAAGGCTGAAATTTCTAAGTCACCATTAACTGATGAAGAAAAG
ACTGCTTTGAACAATGAAGTAGATGAAAAGGCAAATAGTGCTAAAGATGCTATCAATAAG
GCAACTACTCCAGAGGGAGTAACTGAAGCACAAAATAGTGGTATTAAATCCATTGATGAT
GTCAATGTACCAACTGAGTCAGTAGCTAAGGAAGCAGCTAAGAAGGCAGTAGCCGAAGCT
GCAGAAGCTAAGAACAATGCAATTGATTCTTCAAACTTAACAGACGAAGAAAAAGCTGCA
TTGAAGCAAGAAGTTAGTGATGCACAAACTGCTGCTAATACTGCCATTGATAATGCAATT
ACCAACGCAGAAGTAACTGAAGCTGAAGACAATGGTGTTAAGACAATTAATGGTATTGAA
GTACCAACTAAATCAACTACTAAAGAACAAGCAACAAATGATCTTAACAACGAAGTTGAA
AATGCTAAGAAGGCAATTGATCAAGATAACAACTTAACTGATGAGCAAAAACAAGCAGCC
AAGGATCAAATTGATTCGGACGCTAAGAAAGCTCAAGATGCCATCAACAATGCTAAGAAT
AATGATGATGTTAAGAAGGCAGTTGATGATGGAAAACTAGTAATTGACAAAGATGTTGCC
AACGCTGCCATCGATAACGCAGTTGCTGGCAAGAAGGATGAAATTTCTAAATCACCATTA
ACTGATGAAGAAAAAGCTGCTTTAAACAATGAAGTTGATCAAAAGGCAGAAGAAGCTAAG
GAAGCAATTAAGGCTGCCACTACTCCAGGAGCTGTAACTACTGCACAAGAAAATGGTATT
AAGAATATCAATGAGACTGAAGTACCAACTGAGTCCGTAGCTAAGGAAGCAGCTAAGAGG
GCAGTAGCTGAAGCTGCAGACGCAAAGAATAAAGCAATTGATTCTTCAAACTTAACAGAC
GAAGAAAAAGCTGCATTGAAGCAAGAAGTTAGTGATGCACAAACTGCTGCTAATACTGCC
ATTGATAATGCAACTACCAATGCCGCAGTAACCGAAGCAGAAGATAATGGTATTAAAGCA
ATTAATTCTATTGAAGTACCAACTAAGTCAGATGCTAAGGAACAAGCAACAAGCGATCTT
AATAGTGCTGTTGATGAAGCGAAGAAGGCAATCGATCAAGATAGCAACTTGACTGATGAA
GAGAAACAAGTAGCTAAAGATCAAATTGACTCTGATGCTAAGAAAGCGCAAGAAGCAATT
GATACTGCTAAGACTAACGATGACGTTAAGAAGGCAATCGATGACGGTACATTAGCAATT
GATAAAGACGTTGCTAATGCAGCAATCGATAACGCAGTTGCAGGCAAGAAGGCTGAAATT
TCTAAGTCACCATTAACTGATGAAGAAAAGACTGCTTTGAACAATGAAGTAGATGAAAAG
GCAAATAGTGCTAAAGATGCTATCAATAAGGCAACTACTCCAGAGGGAGTAACTGAAGCA
CAAAATAGTGGTATTAAATCCATTGATGATGTCAATGTACCAACTGAATCAGTAGCTAAA
GAAGCAGCTAAGAAGGCAGTAGCCGAAGCTGCAGAAGCTAAGAACAATGCAATTGATTCT
TCAAACTTAACAGACGAAGAAAAAGCTGCATTGAAGCAAGAAGTTAGTGATGCACAAACT
GCTGCTAATACTGCCATTGATAATGCAATTACCAACGCAGAAGTAACTGAAGCTGAAGAC
AATGGTGTTAAGACAATTAATGGTATTGAAGTACCAACTAAATCAACTACTAAAGAACAA
GCAACAAATGATCTTAACAACGAAGTTGAAAATGCTAAGAAGGCAATTGATCAAGATAGC
AACTTGACTGATGAAGAGAAACAAGTAGCTAAAGATCAAATTGACTCTGATGCTAAGAAA
GCTCAAGATGCCATCAACAATGCTAAGACTAACGATGACGTTAAGAAGGCAATCGATGAT

GGTACATTAGCAATTGATAAAGACGTTGCTAATGCAGCTATCGACAATGCAGTTGCTGGT
AAGCTTAAGGAAATTAATGATTCATTAACAAATGAAGAAAAACAAGCTTACACTGATCTA
ATCAATAATGAAGCAGATAATGCAAAACAAAAGATTGCAGAAGCAACTACTCCTGAAGAA
GTAACTCGTGCTCAAGAAGAAGGAGTTAAGAACATTAATAATATTAACGTTCCAACTACT
TCTCCAGCTAAGGACGCAGCGAATGCTGCAATTGATCAGGCTTTGAAGAACAAGAAGGAT
GAAATCAATAATGCAACTAACATTTCTTCTGAAGAAAAGACTGATTTAATTAAACAAGCA
ACTGAAGCTGCAAATATTGCTAAAGACAACATCAACAATGCAACTACTAATTCAGAAGTT
GAAACTGCTCAAGTTGATGGTGAAAAAGCTATTGCGGACGTTACTGGTCCAGGTTTATCT
GACATCAAGAAGGAAAGTATTGATCTTATCAACAAAGCCTTAAATGAAAAGCAAGATGAA
ATCAATAACGCTTCTAACTTGAGTCAAGATGAAAGCACAGAGCTTATTGATCAAGCAAAG
AAGATTGCTACTGAAGCAATTAATGAAATCAATAATGCTCAAACTAATGATGAAGCTAAG
GAAGCTGCAGATACTGGAGTTAAGAACATTGAAAATGTTTCAATTCCAAGTATTGAAGAT
GCTAAGAAGAATGCAACTCAAGCAATTGATGATGCTTTGAATTCTAAGAAGAATGAAATT
AACAACGCATCTAACTTAACTGATTCAGAAAAGACTGATTTAATCAATCAAGCAACTGAA
ATTGCCAATGCTGCTAAGGATGCTATCAACAGTGCAACGACTAATACTGCAGTTGAAGCT
GCCGAATATAAAGGTGTTGCTGATATTAACAACATTCACTTCACTAACTTAGACGATTCT
AAGAAGGCAGCAAATAGTGCGATTGAAGATGCTTTGACCACTAAGAAGGATGAAATTAAT
AATGCATCTAACTTGAGTGATTCAGAAAAGGCTAAGTTAATCAATCAAGCAACTGAAATT
GCCAATGCTGCTAAGGCTGCAATTAACAATGCAACTACTAATTCGGCTGTTACTGCGGCA
GAAAACAAGGGAATTGAAGATATTGCAAATATTAATGTTCCAAGTTTAGCTGAAACTAAA
CAGGCAGCAATTGACGCAATTCAGCAAGTTCAAAAAGCTAAGAACAGTCAAATTGAAGAA
GCAAAGAATCTAAGTGCAGACGAGCAAAAGAACTTAATTGATCAAGTAAATAAGATTGCT
CAGGACGCAATTAATAAGCTTAACGATCCAGCAACAACTACAAATGAAGTTATTACTGAC
ACTCGTGATAAAGCAATTGATCAAATTACTAACTTGTTCATTCCAACCTTAGATAGTGTC
CAAAAAGATGCTCAGGAAGCAATTAACTCTGCTCAGGAAACTAAGATTGACGAAATTAAT
AAGGCTGATAATTTAACTGATCAAATGAAGCAAAACTTAATTGACCAAGTAGATCAAGTT
GCTGATAAGGCTACTAAGGCCATTAATAATGCTCAAACAAACGATGATGTTAAGAAGCA
GAAATCGAGGGCTTAGAAGATATTGATAGCATTAAAGTTCCAAGCTTAGTTGAAAGTAAG
GATGATGCTATTAAGGAAATCAATGATGCTTTGAAGAAGAAGACTGATGAAATCAATGCA
GCTGATCTTGATCAAAAGCAAAAAGACGAATTAATCAGTCAAATTACTGATATTGCTACT
GAGACTAAGACCAAGGTATTCAATGCTACAACTAATGCAGAAGTTGATGCTGAAGCCGAA
GCTGGTATTAAAGCCATCGAAGCTGTGAAGATTCCAGCTCGTACTGCTGACAACAGCAAT
ACTGAGAGTGAAAGTAAGGAACAAACTGTAATTACAAATTCAGTTCAACCTAAGAGAAAT
GCTGTTCATCATAAGAATGGCACTCCAGTAAACAAGAAGGCTACTTTACCACAAACTGGT

AAGAAGGATAATCTAACTTTAACGTTAGCAGGAGCTGCACTTCTTGGTTTAGCAGGTGTA
TTCTCATTGTTTGGATTAGGAGACAAGAGAAAAAAGAATAAATAGT

SEQ.ID. 7: LP 1 gene +/- 250 bp

GGATGCTCGTCAACGTGAAAATTAAATTTGATTATTTAAATATTAAAAAGCAACTCATAA
TTGAGTTGCTTTTTTGCATGTCTTTGTATAAAAACTAAAGAATGTAGGAAATATTGATAT
TGTAAATGGGCATAAAATAAGTTTTTTATGGTAAACATAATAGTGAACAAACGTTAATAT
ATCAGCGCTTTATAGCAATTTAGTCGAATTAAAATTAAAAAATATTAAAAATAATTGAGT
GAAAACATTGTATTATGAGTTTGATGATGATAATATTAAATCATCAACTTGTCCCAAATA
GATGGAGGAATATAACTATGAAGAAAGCCAAGCTATTTGGAAGTTTAACTTTACTTTCTG
GTGTTGCTTTAACATTAGCAGCATGTGGTAACAATTCAAATTCAAAAGTAGATAACCCAA
CTAAGAACTTTAAGTCAGCTACTCCAAAGAAAGCTGTTAAAAAGGGTGGTACAGTAAGTG
TTGCACTTGAATCTGATACACCATTTACTGGTATCTTTTTAAATGAACTTTCTGATACTC
AGAATGACTCTGATGCTATGGCACCTGCTAATGAAGGGTTATTTGATACTGATGATACTT
ATCAAATTAATGACAAAGGACCAGCTACATTAAAATTAGATAATGATAACAAAACTGCTA
CTATTAAAATTAAGAATGGTGTTAAGTGGTCAGATGGTAAGCAAGTAACTGCTAAGGATG
TTGAATATTCTTACGAAATTATTGCTAACAAGGCAACTAAGTCATCACGTTATACTGACT
CACTTCAAAATATTGTTGGTATGAGTGAATACCACGATGGCAAATCTAACACTATTTCTG
GTATTGAAATGCCGGATGGTGAAAATGGAAGAACCGTTGTTATTCACTTCAAAGAAATGA
AGCCAGGTATGAAGTTCAGTGGTAATGGCTACTTCTGGGAAGCTGCAGCTCCATATCACT
ATTTGAAGGATATCCCATTTGATAAGCTACAATCATCTGATCAAGTAAGAAAGAAGCCAC
TATTCTTTGGTCCGTATAAGATGAGTAAGGTTGTACGTGGTCAAGCAGTAACATTTGTTC
CTAACAAGTACTACTGGAGAGGTACACCAAAATTAGATAAAGTAACAATTCAAGTTTTGA
ACCCTAACTCAGCTTCTCAAGCCATTAAGAGTCATAAGTATGATATTGCCGGTGTAATCA
ATGTTCAATGGAAGAACGTTGCCAATACTAATAATGTTAACTGGATCGCTAAGATTCCTT
TATCTTATCGTTACCTAGGTTTTAAGGTAGGAAAGTGGGATGCAGCTAAAGGCGAAAACG
TAATGGATAAGAATGCAAAAATGAATAATAAAGCATTACGTCAAGCTATTGCTTATGGTA
TGAATATCTCCGCTGTAAACAAACGTTATACCAACGGTTTGACTTTCCATATTCCAACTT
TAATTCCAGCACAATTTGGTAAGTACTTTGATAAAAATGTTAAGGGCTACGATTACAATA
TCAAAAAGGGTAATGAACTATTAGATAAAGCTGGATATAAGAAGAAGGGTAAGTACCGTG
TTCAACCCAACGGTAAGCCTTTGACTATCCATTTTGCAGCTATGAGTGGTGACTCTACTC
AAGAACCAATTATTCAAAACTATATCCAACAATGGAAGAAATTAGGTTTGAATGTTAAGT
TAACTGGTGGTCGTTTGATGGAAATGAACTCCTTCTATGACAAGGTCCAAAATGATTCTA

AAGACGTAGATATGTTCATGGTTGCATGGTCATTATCTTCAGAACCATCACCAAATGACT
TATACAATGCAAAAGCACCATACAACTTTAGTCGTTTTGTAACTGCTAAGAACACTAAGT
TACTTCAAGAAATGGATTCACAAAAGGCATTTAATACTAATTACCGTGTTAAGAAATTCC
ATGAATGGCAAAAATATATGGATGATGAAGCCTACGTTGTTCCAGTATCTAACAGTTATA
GTATTGATGCAGTTAACAGCAAGATTACAGGTTACTCATCTAAACCATCAGCAGCAAACA
GCTTATGGTACAATGTAGGAATTGCAAAATAATATTAGATAAATTTCTTCATAAAGATCG
TCTTAAGTGACGGCCTTTTTTTATTTAAGTTGTTTTAATCGTTTTCAGTGCTAAAATTA
AATATATCAACTTGTCCAATGTTATTGGAGGAAAAATTAATGAAGAAAGGCAAATTAATT
GGTACATTAACTTTGCTTTCAGGTGTAGCTTTAACTTTAGCAGCCTGTGGTAATAGTAAT
AGCAATAATACTAGTCATCCTAATTTCAAGGAGTCAACTC

SEQ.ID. 8: LP 1 coding sequence only

ATGAGTTTGATGATGATAATATTAAATCATCAACTTGTCCCAAATAGATGGAGGAATATA
ACTATGAAGAAAGCCAAGCTATTTGGAAGTTTAACTTTACTTTCTGGTGTTGCTTTAACA
TTAGCAGCATGTGGTAACAATTCAAATTCAAAAGTAGATAACCCAACTAAGAACTTTAAG
TCAGCTACTCCAAAGAAAGCTGTTAAAAAGGGTGGTACAGTAAGTGTTGCACTTGAATCT
GATACACCATTTACTGGTATCTTTTTAAATGAACTTTCTGATACTCAGAATGACTCTGAT
GCTATGGCACCTGCTAATGAAGGGTTATTTGATACTGATGATACTTATCAAATTAATGAC
AAAGGACCAGCTACATTAAAATTAGATAATGATAACAAAACTGCTACTATTAAAATTAAG
AATGGTGTTAAGTGGTCAGATGGTAAGCAAGTAACTGCTAAGGATGTTGAATATTCTTAC
GAAATTATTGCTAACAAGGCAACTAAGTCATCACGTTATACTGACTCACTTCAAAATATT
GTTGGTATGAGTGAATACCACGATGGCAAATCTAACACTATTTCTGGTATTGAAATGCCG
GATGGTGAAAATGGAAGAACCGTTGTTATTCACTTCAAAGAAATGAAGCCAGGTATGAAG
TTCAGTGGTAATGGCTACTTCTGGGAAGCTGCAGCTCCATATCACTATTTGAAGGATATC
CCATTTGATAAGCTACAATCATCTGATCAAGTAAGAAAGAAGCCACTATTCTTTGGTCCG
TATAAGATGAGTAAGGTTGTACGTGGTCAAGCAGTAACATTTGTTCCTAACAAGTACTAC
TGGAGAGGTACACCAAAATTAGATAAAGTAACAATTCAAGTTTTGAACCCTAACTCAGCT
TCTCAAGCCATTAAGAGTCATAAGTATGATATTGCCGGTGTAATCAATGTTCAATGGAAG
AACGTTGCCAATACTAATAATGTTAACTGGATCGCTAAGATTCCTTTATCTTATCGTTAC
CTAGGTTTTAAGGTAGGAAAGTGGGATGCAGCTAAAGGCGAAAACGTAATGGATAAGAAT
GCAAAAATGAATAATAAAGCATTACGTCAAGCTATTGCTTATGGTATGAATATCTCCGCT
GTAAACAAACGTTATACCAACGGTTTGACTTTCCATATTCCAACTTTAATTCCAGCACAA
TTTGGTAAGTACTTTGATAAAAATGTTAAGGGCTACGATTACAATATCAAAAAGGGTAAT

GAACTATTAGATAAAGCTGGATATAAGAAGAAGGGTAAGTACCGTGTTCAACCCAACGGT
AAGCCTTTGACTATCCATTTTGCAGCTATGAGTGGTGACTCTACTCAAGAACCAATTATT
CAAAACTATATCCAACAATGGAAGAAATTAGGTTTGAATGTTAAGTTAACTGGTGGTCGT
TTGATGGAAATGAACTCCTTCTATGACAAGGTCCAAAATGATTCTAAAGACGTAGATATG
TTCATGGTTGCATGGTCATTATCTTCAGAACCATCACCAAATGACTTATACAATGCAAAA
GCACCATACAACTTTAGTCGTTTTGTAACTGCTAAGAACACTAAGTTACTTCAAGAAATG
GATTCACAAAAGGCATTTAATACTAATTACCGTGTTAAGAAATTCCATGAATGGCAAAAA
TATATGGATGATGAAGCCTACGTTGTTCCAGTATCTAACAGTTATAGTATTGATGCAGTT
AACAGCAAGATTACAGGTTACTCATCTAAACCATCAGCAGCAAACAGCTTATGGTACAAT
GTAGGAATTGCAAAATAA

SEQ.ID. 9: LP 3 gene +/- 250 bp

AAAAATATATGGATGATGAAGCCTACGTTGTTCCAGTATCTAACAGTTATAGTATTGATG
CAGTTAACAGCAAGATTACAGGTTACTCATCTAAACCATCAGCAGCAAACAGCTTATGGT
ACAATGTAGGAATTGCAAAATAATATTAGATAAATTTCTTCATAAAGATCGTCTTAAGTG
ACGGCCTTTTTTTATTTAAGTTGTTTAATCGTTTTCAGTGCTAAAATTAAATATATCA
ACTTGTCCAATGTTATTGGAGGAAAAATTAATGAAGAAAGGCAAATTAATTGGTACATTA
ACTTTGCTTTCAGGTGTAGCTTTAACTTTAGCAGCCTGTGGTAATAGTAATAGCAATAAT
ACTAGTCATCCTAATTTCAAGGAGTCAACTCCTAAGAAGACAATTAAGGATGGTGGAAAT
GTCAGCGTTGCTGTAGTTACTGATACACCGTTTACTGGTATTTTTAATGATGAATTGTCT
ACTAACAATACAGACTCAGAAGTAATGCAATATGGTGATGAATCATTATTTGCTACAAAT
AATACCTATAAATACGTTAAGGGTGGAGCAGCAGACATTAAAATTAATAAAGATGCCAAA
ACTGCAACCATTACTATTAATCCTAAAGTTAAGTGGTCTGATGGCCAACCTTTAGTTGCA
AAAGATTATGAATACGCCTATGAAATTATTGCAAATAAGGCAACACATTCTCAGCGTTAT
ACTTCAAGTTTAGCTAATCTTGTAGGATTAGAAGAATACCATGATGGAAAGTCCAATACT
ATTTCAGGTATTGAAATGCCAGACGGCGAAAATGGTCGTACAGTAGTACTCCACTTTAAA
GAAATGAAGCCGGGTATGACACAAAGTGGTAATGGATATATTTGGAAGCAGCAGCTCCA
TATCATTACTTAAAAGATGTTCCATTTGATAAACTTATTTCAAGCAATAAGATCCGTAAG
AATCCATTATTCTATGGTCCATATAAAGTAAGTAAAGTGGTTCGTGGACAATCAGTTTCA
TGGGTTCCAAATGAACATTACTATAAGGGTAAACCACACTTAAAGAAGATAACTGCTTCC
GTAATTACTCCAGCTTCAGTTGCTCAATCAATTAAGAGTAATAAATTTGATGTAACGCAA
GTAAGTAATTCTCAATGGCCAAATATTAAGGGTGCTAAAGGAGTAAACTTCATCGCTAAT
ATTCCACTTGCATATAGTTACTTAGGCTTTAAGGTTGGTAAATGGGATGCAGCCAAAGGC

GAAAATGTGATGAATAAGGACGCAAAGATGAATAATCGTTCTCTTCGTCAAGCTATTGCC
TACGGTATGAATGTTGACCAAGTTTATAAGAGATACTCATCAGGTCTTTCATTTAGAATT
CCAACTTTAATTCCAAAGCAATTCGGTGATTACTTCGATAAGAATGTAAAGGGTTACACT
TATAACATTAAAAAGGGTAATGAACTATTAGATAAAGCTGGTTACAAGAAGAAGGGACT
TACCGTGTTCAACCAAATGGTAAGCCTTTAACAATTCGTTTAGCTGCCATGAGTGGTAGT
AAAGTTCAAGAACCAATCATTCAAAATTACATTCAACAATGGAAGAAAATGGGCTTGAAC
GTTAAGTTAACTGGTGGCCGTTTAATGGAAATGAATTCATTCTACGACAAGGTTCAAAAT
GATTCTAAAGATGTAGATATGTTCATTGGTGCATGGTCACTCTCTTCTGAGCCATCACCT
CAAGACTTATACGGCGCAAAGGCACCATTTAACTACAGTAGATTTGTCACTAAAGAAAAC
ACTGACTTGTTGAACGATATTGATTCTCAAAAAGCCTTTAATAATAGCTACAGAGTTAAG
AAATTCCATCAATGGCAAGCATACATGGATAAGGAAGCTTATGTTGTGCCAGTTGCTAAT
AGCTACAGCATTTACGCAATTAACAATAAGTTAACTGGATACTCATTAGAACCATCTAAG
AGTATGGGTGGTGGATTCCCTAACTGGTACTACGTTGGCTATGCAAAATAAATAAAAATT
ATTTAAAAGTGCGACTTATATTAGGTCACACTTTTTCTTTATCTTTTTTTAATAGGTTAA
AATAGGTATAATTAAAGGGTTAAAGAAAGGAAGCAACTATGGTTTCAGTAGCTTTTAAAC
AAAATTTAAAGAAAAAATACGGAATAAAATTTAATAACGAAAAATTGCTTGAAGATGCCT
TCACACATTCTTCTTATGCTAATGAGCATCCCGGAGAAAGGACTATGAGAAATTAGAGT

SEQ.ID. 10: LP 3 coding sequence only

ATGTTATTGGAGGAAAAATTAATGAAGAAAGGCAAATTAATTGGTACATTAACTTTGCTT
TCAGGTGTAGCTTTAACTTTAGCAGCCTGTGGTAATAGTAATAGCAATAATACTAGTCAT
CCTAATTTCAAGGAGTCAACTCCTAAGAAGACAATTAAGGATGGTGGAAATGTCAGCGTT
GCTGTAGTTACTGATACACCGTTTACTGGTATTTTTAATGATGAATTGTCTACTAACAAT
ACAGACTCAGAAGTAATGCAATATGGTGATGAATCATTATTTGCTACAAATAATACCTAT
AAATACGTTAAGGGTGGAGCAGCAGACATTAAAATTAATAAAGATGCCAAAACTGCAACC
ATTACTATTAATCCTAAAGTTAAGTGGTCTGATGGCCAACCTTTAGTTGCAAAAGATTAT
GAATACGCCTATGAAATTATTGCAAATAAGGCAACACATTCTCAGCGTTATACTTCAAGT
TTAGCTAATCTTGTAGGATTAGAAGAATACCATGATGGAAAGTCCAATACTATTTCAGGT
ATTGAAATGCCAGACGGCGAAAATGGTCGTACAGTAGTACTCCACTTTAAAGAAATGAAG
CCGGGTATGACACAAAGTGGTAATGGATATATTTGGAAGCAGCAGCTCCATATCATTAC
TTAAAAGATGTTCCATTTGATAAACTTATTTCAAGCAATAAGATCCGTAAGAATCCATTA
TTCTATGGTCCATATAAAGTAAGTAAAGTGGTTCGTGGACAATCAGTTTCATGGGTTCCA
AATGAACATTACTATAAGGGTAAACCACACTTAAAGAAGATAACTGCTTCCGTAATTACT

CCAGCTTCAGTTGCTCAATCAATTAAGAGTAATAAATTTGATGTAACGCAAGTAAGTAAT
TCTCAATGGCCAAATATTAAGGGTGCTAAAGGAGTAAACTTCATCGCTAATATTCCACTT
GCATATAGTTACTTAGGCTTTAAGGTTGGTAAATGGGATGCAGCCAAAGGCGAAAATGTG
ATGAATAAGGACGCAAAGATGAATAATCGTTCTCTTCGTCAAGCTATTGCCTACGGTATG
AATGTTGACCAAGTTTATAAGAGATACTCATCAGGTCTTTCATTTAGAATTCCAACTTTA
ATTCCAAAGCAATTCGGTGATTACTTCGATAAGAATGTAAAGGGTTACACTTATAACATT
AAAAAGGGTAATGAACTATTAGATAAAGCTGGTTACAAGAAGGGGACTTACCGTGTT
CAACCAAATGGTAAGCCTTTAACAATTCGTTTAGCTGCCATGAGTGGTAGTAAAGTTCAA
GAACCAATCATTCAAAATTACATTCAACAATGGAAGAAAATGGGCTTGAACGTTAAGTTA
ACTGGTGGCCGTTTAATGGAAATGAATTCATTCTACGACAAGGTTCAAAATGATTCTAAA
GATGTAGATATGTTCATTGGTGCATGGTCACTCTCTTCTGAGCCATCACCTCAAGACTTA
TACGGCGCAAAGGCACCATTTAACTACAGTAGATTTGTCACTAAAGAAAACACTGACTTG
TTGAACGATATTGATTCTCAAAAAGCCTTTAATAATAGCTACAGAGTTAAGAAATTCCAT
CAATGGCAAGCATACATGGATAAGGAAGCTTATGTTGTGCCAGTTGCTAATAGCTACAGC
ATTTACGCAATTAACAATAAGTTAACTGGATACTCATTAGAACCATCTAAGAGTATGGGT
GGTGGATTCCCTAACTGGTACTACGTTGGCTATGCAAAATAA

SEQ.ID. 11: LP 7 gene +/- 250 bp

CAATCATGATTACCAAAAATAAACAACCAATTAACATTTCCTTCTATAGTCTTCACAATG
AACATGGTAAATATATTGGCTGCATTGAAGTAACCCAAGCAGTAAGTAACTTACAAGTTA
AAGGCAGTAAATTGCGCAATATTTTAAATGTCCTTAAAAAGCACTAAAGTTAAGCTCTTA
GAATACTTTTTTCAGTCAAAAATAGAGTAAAATAAAATAAGATAGATACATTTATTGGGG
GAATATATTTTTATGAAGAAAAAATTCAGCTTCCTAATTTTATTGGGAGTAATTTTAGTT
TCTTTAACTACAGCTTGTTCAAATAAAAGCAGTAAACAAGCAAAGAGTACTGGACCTTCT
GCAAGTTCATTAATCAATGCAAAATTTAATTCTAGCTTTGCTAACGGTCACTTCACTCAA
ACTACTAATTCTAATGAAATGAACCAAAAGTCAAAATCAGAAGGTTTATTCAAAGACAAA
GGTGATGTAACTAACCTTACCTATACCTTAACTCAAAAGAAGAAATCTCAAACTGAACAA
ATGTGGCTTACCAAGAAGGATATGTACTTACTTTTAGAGCAAAACAAGGGCCATTGGATT
AAAAACTCAATTGATGCAGATAGCTTTGATTCAGACCAAGTAAAGGAAAGATTCGATCCT
GCAACTTTTAATAAAATTAACAAATCTTTAGCTCAAAAAGCCACTGTTCAAAGAAAAAAT
GGAAATTATGAAATTAGTTTTGATGGTACCGACGCTAATCTATGGAATGCAGTCAATCCT
TTAATTATCGATGCCATGAACACTCCTGGTTCTCAAAATATGCAGGTAGCCCGCTTAGTA
AAATCAGCTCAAGTTCAAAATCTTAAGATCACTTATTTAATTGATCCAACTACCAAAAAT

GTCGAATCAATGACCTTTAAGGCCCAATATACTGCTGGCGGAAAATATAACTTCACTTGG
AACCTAACTTACGACCAATTAGGTCAACACAGTGATTTAGCCGTTCCTTCTGACATTCAA
AAGAATGCAATTAGTGCTGAAGACATCAAGAAAGCACAAGAAGAACAAAATAATCAATAA
TCATTTAGAAAGATACACTGATGGAAAAAAATAAAATCAAAGATAAAAAGATTATTTTTT
CTAACAATCAAGGCGGCAAAATTGCCGCTTTAATTTTAGGTATTATTATCCTAATCGTAG
TAGTTTGGGGCGCAATTATTGCATTTAATAAGCCAAGCGTTAAAGATGCATTAACTACGG
CTGCCAATACCCAAATTGCTACTGCACGCGTTAATGTCAAAACTACCAATGCAAAGTCAA
AAATGTATTC

SEQ.ID. 12: LP 7 coding sequence only

ATGAAGAAAAAATTCAGCTTCCTAATTTTATTGGGAGTAATTTTAGTTTCTTTAACTACAGC
TTGTTCAAATAAAAGCAGTAAACAAGCAAAGAGTACTGGACCTTCTGCAAGTTCATTAATCA
ATGCAAAATTTAATTCTAGCTTTGCTAACGGTCACTTCACTCAAACTACTAATTCTAATGAA
ATGAACCAAAAGTCAAAATCAGAAGGTTTATTCAAAGACAAAGGTGATGTAACTAACCTTAC
CTATACCTTAACTCAAAAGAAGAAATCTCAAACTGAACAAATGTGGCTTACCAAGAAGGATA
TGTACTTACTTTTAGAGCAAAACAAGGGCCATTGGATTAAAAACTCAATTGATGCAGATAGC
TTTGATTCAGACCAAGTAAAGGAAAGATTCGATCCTGCAACTTTTAATAAAATTAACAAATC
TTTAGCTCAAAAAGCCACTGTTCAAAGAAAAAATGGAAATTATGAAATTAGTTTTGATGGTA
CCGACGCTAATCTATGGAATGCAGTCAATCCTTTAATTATCGATGCCATGAACACTCCTGGT
TCTCAAAATATGCAGGTAGCCCGCTTAGTAAAATCAGCTCAAGTTCAAAATCTTAAGATCAC
TTATTTAATTGATCCAACTACCAAAAATGTCGAATCAATGACCTTTAAGGCCCAATATACTG
CTGGCGGAAAATATAACTTCACTTGGAACCTAACTTACGACCAATTAGGTCAACACAGTGAT
TTAGCCGTTCCTTCTGACATTCAAAAGAATGCAATTAGTGCTGAAGACATCAAGAAAGCACA
AGAAGAACAAAATAATCAATAA

SEQ.ID. 13: LP 8 gene +/- 250 bp

CTCTTACTTCATGTCCTTCTTTTTGAAAATCACGTACAACACTAATTGCCTTATATGCAG
CAATACTACCCGTGATATATATTGTAGCTTTCATTTTTTATCAACCTTAATTCATAATTT
CTTTCTACTATAACACAAGCTTACTAAGTCGAATTACTAAAAATAAATTAGCAAATTAAA
AAATATTAGTAAATTCTGCTATGATTTATATTGAATGCAGATCTAAACAACATAATTCGA
AAGGATAAATATATATAGAATGAGAAAACATTATCAAAAGATTTTTGCTTTCTTTTTTGCAA
TTAGTAGTCTTGTTTTAATTCTTACTGGCTGCTCAAAAAAAGAAAATTCATCAAATAAAA

TTTCTATCGTAACAAGTACTAATGTTTATGCCGATATCGCACAAAATGTCTTAGGAAAAT
ATGGTAAAGCAACTGCTATTATTACTAACAGTGCTACTGATCCACATGACTTTGAACCCA
CAACTGCAGACGCAAAAAAAGTTCAAAATGCAAAAATTGTTGTTGCTAACGGTTTGGGCT
ATGATTCTTGGCTTCCTAAGCTTGCTAAATCAACTAATAAGTCTGCTGTTTTAGTTGGCG
AAGATTTAATGAATCTAAAAAATGGCGCAAATCCGCATATTTGGTTTGATTTAAACATGC
CTAAAAAATATGTTAACTATTTAGTTGAGCGACTCTCTAAAATAGACAAAAAACATGCAG
CTTACTACAAAGAAAATGGAAGGAATATCTTGCAAAAATCAATAAGATACAAAAAATTG
CAGATTCTATTGATGGTGCAAAACAAAAGCCAGTATATGTCAGCGAGCCAGTGTTTGACT
ATGCCTTAAATGCTACTCACTTTAAGATTGGTGATAAAGCTTTTGAAGAAGCAATTGAAA
ATGAAACAGATCCAAGTGCCAAAATTATTCATCAAATGAACCAAACAATTAATAATCGTG
GAATTTCTTTCTTTGTTAAAAATTCTCAAGTAAGCAGTTCAACTGTTAATAATTTTGTAA
AAAGAGCTAAGTCAAAAAATATTCCAATCTTGCAAGTTCGTGAAACCATCCCCAACAATA
CAAGTTACATAAAATGGATGACAGAAAATTATCAGAATTTAGCAAATATTAATAAAAAAC
TAGACTAAGTTAGTTTATTAAAAAGAGGGCCTCTAATACGAGACCCTCTTTTTTACTTTA
AAATATCTTTTAACTTTATATTAAATTGCTGTGGAATTTCCGCCATAATTACATGTCCCG
TCGGTGACATAATAAAGCTATTTATTCGGCTATTCTTTTGTTCAAGCTTCTTTTCATATC
CCTCTCGATAATAAGGACTTTCCTTTGCAACAAAAAAAGTAGTTGGAATTGTTGTTTTTT
CTAAAACTCTTCGCCAATCT

SEQ.ID. 14: LP 8 coding sequence only

ATGAGAAAACATTATCAAAAGATTTTTGCTTTCTTTTTTGCAATTAGTAGTCTTGTTTTA
ATTCTTACTGGCTGCTCAAAAAAAGAAAATTCATCAAATAAAATTTCTATCGTAACAAGT
ACTAATGTTTATGCCGATATCGCACAAAATGTCTTAGGAAAATATGGTAAAGCAACTGCT
ATTATTACTAACAGTGCTACTGATCCACATGACTTTGAACCCACAACTGCAGACGCAAAA
AAAGTTCAAAATGCAAAAATTGTTGTTGCTAACGGTTTGGGCTATGATTCTTGGCTTCCT
AAGCTTGCTAAATCAACTAATAAGTCTGCTGTTTTAGTTGGCGAAGATTTAATGAATCTA
AAAAATGGCGCAAATCCGCATATTTGGTTTGATTTAAACATGCCTAAAAAATATGTTAAC
TATTTAGTTGAGCGACTCTCTAAAATAGACAAAAAACATGCAGCTTACTACAAAGAAAAT
GGGAAGGAATATCTTGCAAAAATCAATAAGATACAAAAAATTGCAGATTCTATTGATGGT
GCAAAACAAAAGCCAGTATATGTCAGCGAGCCAGTGTTTGACTATGCCTTAAATGCTACT
CACTTTAAGATTGGTGATAAAGCTTTTGAAGAAGCAATTGAAAATGAAACAGATCCAAGT
GCCAAAATTATTCATCAAATGAACCAAACAATTAATAATCGTGGAATTTCTTTCTTTGTT
AAAAATTCTCAAGTAAGCAGTTCAACTGTTAATAATTTTGTAAAAAGAGCTAAGTCAAAA

AATATTCCAATCTTGCAAGTTCGTGAAACCATCCCCAACAATACAAGTTACATAAAATGG
ATGACAGAAAATTATCAGAATTTAGCAAATATTAATAAAAAACTAGACTAA

SEQ.ID. 15: LP 11 gene +/- 250 bp

CTCCGTTTATTGAAAATATTGAAAAACGTGGAATTGAATTGATAACTCTACGTTGTTCAG
GACATGCGGATGTAGCTGAATTAAAAGAAATTATTGCGGGTTTTGAGCCAAAAATTTTGG
CTCCTGTGCACACATTGCATCCAGAATTAGAAGAAAACCCATTTGGAGAAAGGATTTTAC
CTAAGCGCGGAGAGGTATTTACGCTTAGTTAAAATTAAAATTTATTTTGTTGTTTGTTT
TTATTGGGAGGAACATATGAAGTTCACTAAAAAATTTGCAGCAGTTGCAGTTGCTGCTTT
AGCCCTTGTAGGCACTACTGCTTGCTCAAATTCTAAGAGTAATGATAGCTCAAGTAGTAA
GTCAGCTGATATTCCAACTAAGATTACCAAGAAAACTACTGTAGTTTTCTGGCATGGTAT
GACTGGAACTCAAGAAAAGACTTTACAAAGCTTGACTAAGGATTTTGAAAAGAAGAATCC
TAACATTACTGTTAAGCTTGAAAACCAAGGTAAGTATACTGATTTACAAGCAAAGATTAA
CTCAACTTTGCAATCACCAAATAACTTGCCTACAATTACTCAAGCTTATCCTGGCTGGCT
TTTAAATGCCGCTAATAGCAAACGCTTGGTTGATATGACCCCTTACATTAACAATAGTAA
GATTGGTTGGGGCTCAGCTAAGAAGAGTGACATCCGTGAAGAATTATTAAAGGGTGCACA
AATTAAGGGTACTCAATATGGTATTCCATTTAATAAATCTGTTGAAATGTTAACTTATAA
CAAAGATTTATTAGATAAATATGGTGTTAAAGTACCAAAGACAATGGCTGAATTAAAGGC
AGCATCTAAGAAGATTTACGAAAAGAGTAACCACAAGGTTGTTGGTGCTGGATTCGACTC
ACTTTCAAACTACTACGTTTTAGGTATGAAGGATGAAGGCGTAAACTTCTCTAAGAACAT
CAACTTTACTGGTTCTAAGTCTAAGAAAGTAATCAACTACTATGCTGATGGTATTAAAGC
TGGTTACTTCAAGACTGCTGGTTCAGCTGGTTACCTTTCTGGTGACTTCTCTAACCAAAA
GGTTGCAATGTTTGTAGGTACTAGTGCTGGTGAAGGATTTGTTAAGATGGGTGTTGGTAA
TAAGTTCCAATACGGTGTTGCACCTCGTCCTTCTAAGTACAACATGCAACAAGGTACTGA
TATCTACATGTTCAACAAGGGTACTGCAGAACAAAAGGCAGCTGCCTTCATGTACATTAA
GTACTTGTTAGGTAAGAACCAACAATTGACTTGGGCTGATCAAACTGGATACATCCCTGT
AACTAACAATGTGATTAACTCTAGTGCTTACAAGAACTCAACTAAGTCTAAAGTTCCTGC
TCAATTAGACAAGGCAATGAAGAACTTATACAGTGTTCCTGTAGTTAAGAACAGCAACAC
TGCATACACTCAATTGAATTCAATTATGCAAAACATCTTTGCACAAGCAAAGAAGGGTCA
AAACTGGAATTCACAAATTGATGCCGGTAAGACTAAGTTCGAAAGTGCTTGGAATCAATA
AAAACAGAATATTAAGTAAAAAATGAGGACTGTCATTATGACAATCCTCATTTTTTTATA
GATGTTTTAGAAGAAAAGTAGAATGAAGGCAATTAATGCAGGTAGCATTTGAACAACAAA
AATCTTTTTACTTGCTGTAAAGCCCCCAAAAATAGCGACAACTACAACAAAGGCAAGCAA

AAGTTGCCATACAGTGATTAAAGTTGGCATTCTGAAGAAGAAAAATACAATTAAGATTAA
AAGACCTAGCATTCCATTAT

SEQ.ID. 16: LP 11 coding sequence only

ATGAAGTTCACTAAAAAATTTGCAGCAGTTGCAGTTGCTGCTTTAGCCCTTGTAGGCACT
ACTGCTTGCTCAAATTCTAAGAGTAATGATAGCTCAAGTAGTAAGTCAGCTGATATTCCA
ACTAAGATTACCAAGAAAACTACTGTAGTTTTCTGGCATGGTATGACTGGAACTCAAGAA
AAGACTTTACAAAGCTTGACTAAGGATTTTGAAAAGAAGAATCCTAACATTACTGTTAAG
CTTGAAAACCAAGGTAAGTATACTGATTTACAAGCAAAGATTAACTCAACTTTGCAATCA
CCAAATAACTTGCCTACAATTACTCAAGCTTATCCTGGCTGGCTTTTAAATGCCGCTAAT
AGCAAACGCTTGGTTGATATGACCCCTTACATTAACAATAGTAAGATTGGTTGGGCTCA
GCTAAGAAGAGTGACATCCGTGAAGAATTATTAAAGGGTGCACAAATTAAGGGTACTCAA
TATGGTATTCCATTTAATAAATCTGTTGAAATGTTAACTTATAACAAAGATTTATTAGAT
AAATATGGTGTTAAAGTACCAAAGACAATGGCTGAATTAAAGGCAGCATCTAAGAAGATT
TACGAAAAGAGTAACCACAAGGTTGTTGGTGCTGGATTCGACTCACTTTCAAACTACTAC
GTTTTAGGTATGAAGGATGAAGGCGTAAACTTCTCTAAGAACATCAACTTTACTGGTTCT
AAGTCTAAGAAAGTAATCAACTACTATGCTGATGGTATTAAAGCTGGTTACTTCAAGACT
GCTGGTTCAGCTGGTTACCTTTCTGGTGACTTCTCTAACCAAAAGGTTGCAATGTTTGTA
GGTACTAGTGCTGGTGAAGGATTTGTTAAGATGGGTGTTGGTAATAAGTTCCAATACGGT
GTTGCACCTCGTCCTTCTAAGTACAACATGCAACAAGGTACTGATATCTACATGTTCAAC
AAGGGTACTGCAGAACAAAAGGCAGCTGCCTTCATGTACATTAAGTACTTGTTAGGTAAG
AACCAACAATTGACTTGGGCTGATCAAACTGGATACATCCCTGTAACTAACAATGTGATT
AACTCTAGTGCTTACAAGAACTCAACTAAGTCTAAAGTTCCTGCTCAATTAGACAAGGCA
ATGAAGAACTTATACAGTGTTCCTGTAGTTAAGAACAGCAACACTGCATACACTCAATTG
AATTCAATTATGCAAAACATCTTTGCACAAGCAAAGAAGGGTCAAAACTGGAATTCACAA
ATTGATGCCGGTAAGACTAAGTTCGAAAGTGCTTGGAATCAATAA

SEQ.ID 17: LP 17 gene +/- 250 bp

GCTACAATAAATTCATAAGTTGGAGGTGTTACTATGCAAGAATTAAGAAACAATACTCTC
ATTTCTCTGATTGGGGGGGTAGAAAGCCATTCTATCAACGTTTCTAAGGATTTTCTAC
TTTAAATCCTTATTTTCTTGTACAGTACATAAAAAGCCAAGTTAAAGATGACTTTAATGG
GGTCATTCTTTAGCTTGGCTTTTTTGCGTCTTCAATTATTTTAATTCTAATAGCTCGAGT

GTAGGAGGAAATAGAGTGAAAAGTAAGTCTTATAAGTATGGATTGCTGCTTTAGTAGGT
GTAAGTGCCTTAGCATTAGCTGGTTGTCAAAGTAATGAAAACCAATCAAGTACATCTAAG
ACTAATGCCAAGTCGAAATCTTCAATAGTTAAGATTGAAAAGAAAAGTAAATCTAAGAGT
AGCAAGTCGGAAAAGCAAGAAGGTGAAGTGAAAAAACAGAGCAGTGAGAAGGTTAATAAG
GCAAGTAGTTCGAAGAAAGATGTAGAAAAAGTACCAACAGTTACTGAATTGATGAATAAT
GTTTCGGCCTTTAATGAAGGTAATTTTGATATTCGGCTTAATTATGCAACTGTTAAAGGT
TATGACACGGGAATAATTCAAGAAAATCCACGAGTAGTACATTATAAGTTCAACGGTGGG
ATGAACTCAGAGGTTTGGGCAACTGATCAATTTGTCTACAGTTTATCGGGTGATAAATGG
ACTAAAATTGGGCAAACTTATAAGATTGGAAGATTTGATCCGATGATTAGCCTTCCATCT
CCCCTTATTAATCAAATGAAAGTCTCAAAAACTAGTGATGGTTATCAAGCAAAATATGAG
GGAAACTCTGCTGAAGCTAAAGAATTAATGGAACATTATATTGTTTGGGTTTCAAGTGAA
AATAAATGGAATCCATTTGGAGACCAAGACTTTACATCAGCTACTCTAACTTATAGCTTT
AATAAACAAAAACAGTTAACTGAGATTCATTATGATTGGACCGCTGGTTCAAATAAAGGA
ACAGCTTCTTACACTAATATCAATCAACAAAAAATTGCAGTTCCAAATGATGTAATTCAA
GCAGCAGCTAAGAATTCTGACTCTATTGATAGTAACAATGATGATAATAGTGACGATGAA
TAGGGTCGAAAATAGATGGATAAAATAAAAGAAAATAAATTTGTTATTAAAGCTAAGAAA
ATTTGGTCTAAGCATAAGATCATTGTTTCGGTTCTAGGAGGCTTCTTGGTTCTGATATGT
TTATTTGGAATTAGAGAGGCTAGTCTTGTTGATCCATTAGATGGTGTTCAACAAGTGGGA
TGGAATTCGTATACGGGTTCTTGGTTTGTTCCGGATAAAGACCGAGATCGGATTGCAAGT
AAAATTAGAGCATCCATATT

SEQ.ID 18: LP 17 coding sequence only

GTGAAAAGTAAGTCTTATAAGTATGGGATTGCTGCTTTAGTAGGTGTAAGTGCCTTAGCA
TTAGCTGGTTGTCAAAGTAATGAAAACCAATCAAGTACATCTAAGACTAATGCCAAGTCG
AAATCTTCAATAGTTAAGATTGAAAAGAAAAGTAAATCTAAGAGTAGCAAGTCGGAAAAG
CAAGAAGGTGAAGTGAAAAAACAGAGCAGTGAGAAGGTTAATAAGGCAAGTAGTTCGAAG
AAAGATGTAGAAAAAGTACCAACAGTTACTGAATTGATGAATAATGTTTCGGCCTTTAAT
GAAGGTAATTTTGATATTCGGCTTAATTATGCAACTGTTAAAGGTTATGACACGGAATA
ATTCAAGAAAATCCACGAGTAGTACATTATAAGTTCAACGGTGGATGAACTCAGAGGTT
TGGGCAACTGATCAATTTGTCTACAGTTTATCGGGTGATAAATGGACTAAAATTGGGCAA
ACTTATAAGATTGGAAGATTTGATCCGATGATTAGCCTTCCATCTCCCCTTATTAATCAA
ATGAAAGTCTCAAAAACTAGTGATGGTTATCAAGCAAAATATGAGGAAACTCTGCTGAA
GCTAAAGAATTAATGGAACATTATATTGTTTGGGTTTCAAGTGAAAATAAATGGAATCCA

TTTGGAGACCAAGACTTTACATCAGCTACTCTAACTTATAGCTTTAATAAACAAAAACAG
TTAACTGAGATTCATTATGATTGGACCGCTGGTTCAAATAAAGGAACAGCTTCTTACACT
AATATCAATCAACAAAAAATTGCAGTTCCAAATGATGTAATTCAAGCAGCAGCTAAGAAT
TCTGACTCTATTGATAGTAACAATGATGATAATAGTGACGATGAATAG

SEQ.ID. 19: LP 29 gene +/- 250 bp

CAGATAATTTCTTTAAGGAAAAGACAGTAGTTCTAACTGGAAAATTATCTGCCTTTACTC
GAAGTGAATTTACTAAGAAGCTTCAGGATCTTGGTGCAAAAGTCACTGGGTCAGTTTCTA
AGAAGACAGATTATTTGATCTACGGAGCAGATGCGGGCTCTAAAAAAGATAAGGCTGAAA
AACTTCAAGTTCCAATGTTAACTGAACAAGAAGCAATTGCAAAAATTGAAAAATAAAGAA
TAAGAAGGAAAAATAAATTGAAAAGATTTTTGCAAATAGCACTACTACTAGCAACTGGGT
TAAGTTTAAGTGCATGTGGCAATTTAAAAAATTCTGATCTTGCCAATAACCCTACTACTT
CTACAACAAAGAAAAAAAGTTATCAAACTACCAGTACTAGTAAAAATGGGTATAATGTTC
TTTTAAAAGATGGAGAGTATGTAACTAGTCCAATAGAAGGAACAACTGAAAATACTAGTG
ATAATAATGTAGATAGTCGTGCTTTAGAATCTGGGTTAATTGATTTATCTCACAATACTT
TTTCAAGTAGTAAGTATGTTTTTCAAGAAGGACAAAAAATTTCGGTTGCTGATGCTACTG
ATTGGCTAGGAAGAAAGTCTAAAAGTAATCCAGAAGGTCTAAATGCGGAAAAAAGTAATA
AAAAGAATTCGTATAATCCTATTATTTTAGATCAAATTTTAGAACAAGATTTTTTGACTA
AATCTAATTCTAGTTACAAAATGGATGGTATGAGTATTGGGCTAGCTCTTAACTCGGTTG
ATTACTACCAAAAAGTTAAAGATGGTCCCCAATATCATAAAGATATTTCTCGTGCAGAAC
AGGAGACTTTTGGTAAAGAAGCAGCTAATAAGTTAGTTTCGTATTTACGTAAAAAGAAGG
GATTAAAAGATATCCCAATTTTGGTTGGACTTTTTAGTAAAACCAGTAAAGATTCTCTTG
TAGGTGGAAATTACTTTGCATATGGAATTGCTAATGCAAATAGTAGTAAAATAAATGATT
GGAAAACAGTCAATAATCGTAGTCAAGTGCTACCGACTGTTGGAAGTGAAAAAGCAATCA
ATTCTACTGATGCAGCTTCATTTAGTGATTTTAAAGCAGCTATTCAAGGTTACTTCCCTA
ATATTAGTGGGGTAACAGCGACTGTTCATTATCAAAATAAAACTTTGACACAAATGAACA
TTACTGTAACTACCCAATTCTTTGGTTATGCGCAGATTGAGAGTTTTTCAAGACTTGTAT
TGTCAGCTGCTAAGAAGTACTTACCTAAGGATGCACCAATAGAAATTAAGATTAATTCTG
TTAATGATACGCAGGCATTAATCGCTAAGAATTCCGCAGACGATAGTTATTATGTCCACG
TCTTTGGCGGTGAATAGGAGGCAAACGGTGAAAATTACAAAAGATGAAATCAATCACGTT
GCAGCACTATCTCGACTTGAGTTTGGTGAAGACGAAATTGATAAGTTTACTGAACAAATG
GGTGACATTATCAATATGGCACATCAATTAGCCGAAGTTGATACTGAAGGAGTTCCTGAA
ACTGTTCAAGTTGTTGATCGAGAGACTGACTTTAGAGAAGACAAGCCTGAACATTGGGAA

AGTCGCGCAGAATTAATGAAGAATGTTCCT

SEQ.ID. 20: LP 29 coding sequence only

TTGAAAAGATTTTTGCAAATAGCACTACTACTAGCAACTGGGTTAAGTTTAAGTGCATGTGG
CAATTTAAAAAATTCTGATCTTGCCAATAACCCTACTACTTCTACAACAAAGAAAAAAAGTT
ATCAAACTACCAGTACTAGTAAAAATGGGTATAATGTTCTTTTAAAAGATGGAGAGTATGTA
ACTAGTCCAATAGAAGGAACAACTGAAAATACTAGTGATAATAATGTAGATAGTCGTGCTTT
AGAATCTGGGTTAATTGATTTATCTCACAATACTTTTTCAAGTAGTAAGTATGTTTTTCAAG
AAGGACAAAAAATTTCGGTTGCTGATGCTACTGATTGGCTAGGAAGAAAGTCTAAAAGTAAT
CCAGAAGGTCTAAATGCGGAAAAAAGTAATAAAAAGAATTCGTATAATCCTATTATTTTAGA
TCAAATTTTAGAACAAGATTTTTTGACTAAATCTAATTCTAGTTACAAAATGGATGGTATGA
GTATTGGGCTAGCTCTTAACTCGGTTGATTACTACCAAAAAGTTAAAGATGGTCCCCAATAT
CATAAAGATATTTCTCGTGCAGAACAGGAGACTTTTGGTAAAGAAGCAGCTAATAAGTTAGT
TTCGTATTTACGTAAAAAGAAGGGATTAAAAGATATCCCAATTTTGGTTGGACTTTTTAGTA
AAACCAGTAAAGATTCTCTTGTAGGTGGAAATTACTTTGCATATGGAATTGCTAATGCAAAT
AGTAGTAAAATAAATGATTGGAAAACAGTCAATAATCGTAGTCAAGTGCTACCGACTGTTGG
AAGTGAAAAAGCAATCAATTCTACTGATGCAGCTTCATTTAGTGATTTTAAAGCAGCTATTC
AAGGTTACTTCCCTAATATTAGTGGGGTAACAGCGACTGTTCATTATCAAAATAAAACTTTG
ACACAAATGAACATTACTGTAACTACCCAATTCTTTGGTTATGCGCAGATTGAGAGTTTTTC
AAGACTTGTATTGTCAGCTGCTAAGAAGTACTTACCTAAGGATGCACCAATAGAAATTAAGA
TTAATTCTGTTAATGATACGCAGGCATTAATCGCTAAGAATTCCGCAGACGATAGTTATTAT
GTCCACGTCTTTGGCGGTGAATAG

SEQ.ID. 21:   CSP2 protein sequence

MLSKNNYQERLRKMDDKQERFSIRKFSVGAASVLVGTAVLTMQNVQTARADTTSNTTTKT
TDLSSEAEEQNKQNAYDKTLNEAQPTTKPADAPVESQDSKVASFSVPKNEGTFVEKTLTP
NITEETEAMQDKAELAQVNSEDKSAENNKTNVKEESQTVEENQMDKRSAEANISDTENTL
VSNTTLNVPKTTPATTRFSATALSESKFQVNAPRVGQDTPDTQVKKNNYKLVTNATALQQ
AINTGVAGVNIDRSIDASNVDLAIRNTFTIVGLNDAAALNLGQKSLNNSGNLTLQDIKIN
GSISGSGTVNIKGNVTSNVNSTNSSVPTQDQYNAQNYTGSRTNFKNSNIAARNVNIENGA
SLTINSSEINDGINLADGGTVKVGDNATLNVNLTNTSTTATRYHVAGVFAKNGGNFISGY
KSNVNFNTGLGQAIAIGATRPTSTDADRYGGYGTRSRNDGPTLVQLGDSSTFNFTGRDGI

ILGNNANFISGENSNVHFENKGRGVALDLANNSNIEISKHSITSFHSVGKTGTSGSFDGY
NYIGVNEGGNITVDEYATFRVILEGRGDNPWDDVISLDSRNANTNAAFTSKTGAIVDIRD
DNTNFYAELISFPLGSANSRIDIQDPLMLNLQRYSNGGATTGWMATGGDKINTTSAQYTA
NLIYMSGNKGVFSVSGGDYDPSNPNSSGFVVYQQIKSDGSKQIWLNVNSVDIPMNGFQTK
DIWNNQANPDVSIAGNGLTAGIKANQVHNFDGSPLTGPNAPYYGISTQRASHQIWIPHRT
SLEVTGEHKSTIKYVDENGKEIFPENTQSLDMKRSLILDITQDKIQDIQNYALSHTADET
LEYIKNSQAVVQDSGWDFTDANGKAVIDPYSTIDSPKLAGYAATIQSTNVSTLKVGADAS
AVTAKFMVEPSQDMVQNGELSTAYKQNGTTGIPADYVTVVVYNKQATYQAGKTDSKSVVR
DINYLDGKTGKKIPANLIKDNPVSQTVTMHRTEILDNSGKVIGYGTISEDGKSYTLNNDW
IIDGNWEAVKSPDLTTNGYKTPRFENGDIAKVVPAETVNENTPDTTVNVYYDHNEIPVGP
DTPDKHGVDLNELTKDVNETVHYVYSDGTKAAPDVVQTSKWTRTLTIDAVTNEVISDGQY
TTDWAIPKGEKTVYDQVDTPVIKGYHADKRQINATVVTQNNIEETVTYQPNGKIIPVDPN
GDPIPNVPTPTYPTDPTDPTKVVPDEPVPEIPGLIPSVPTVTPENPGKDTPVPYTPEVPA
KDQVAVVNYIDADDNNAIITSSGNLTGKAGEKIDYSTASTIEELENKGYVLVSDGFPAGA
TFDNDDNTTQIYTVVLKHGTVPVTPENPGKPGEPINPNDPDGPKWPEGTGENGVKRTGTQ
TIHYEGAGDKTPSDDVQTFDFTKKMLVDKVTGKIIDGGEWNVTSHTFGYKDTPVIEGYHA
DKRNAGGSVVTPDDLNKTITVSYQPNGKIIPVDPNGDPIPNVPTPTYPTDPTDPTKVVPD
EPVPEIPGLIPSVPTVTPENPGKDTPVPYTPEVPAKDQVAVVNYIDADDNNAIITSSDNL
TGKAGEKIDYSTASTIEELENKGYVLVSDGFPAGATFDNDDNTTQIYTVVLKHGTVPVTP
ENPGKPGEPINPNDPDGPKWPEGTGENGVKRTGTQTIHYEGAGDKTPSDDVQTFDFTKKM
LVDKVTGKIIDGGEWNVTSHTFGYKDTPVIEGYHADKRNAGGSVVTPDDLNKTITVSYQP
NGKIIPVDPNGDPIPNVPTPTYPTDPTDPTKVVPDEPVPEIPGLIPSVPTVTPENPGKDT
SVPYNPVKDPDKVTTVEGKQIIHFVDGDNNKPLKDPNIQTYEFKITNGVPNESSHTFTLV
NVPVIPGYVAEIKSAGGKTVTPDKPLAEVTVVYHKIGKIVPVDPNGNPIPNVPTPSYTND
PTDPTKVVPNEPVPAIPGKTPDKTTVTPVDPTKDTPVVYKDDKVPPAPNTQKAVVNFIDV
NTGKIISTSGILSGRPGEDINKLFSSAEVIKQLEAAGYEVVYNAFDGDGVTKYFDNDGNT
VQQFTIALKLKEKAKTPDPVLPDPEVPVEKETRSEQPVKQNVAVPTPEKPVEKKTTDNKE
VLPQTGADSNEAATILGAVATAIGMTSLIGAKRRKKDDK

SEQ.ID. 22:   Sequence of the CSP2 expressed protein

MANTNAAFTSKTGAIVDIRDDNTNFYAELISFPLGSANSRIDIQDPLMLNLQRYSNGGAT
TGWMATGGDKINTTSAQYTANLIYMSGNKGVFSVSGGDYDPSNPNSSGFVVYQQIKSDGS
KQIWLNVNSVDIPMNGFQTKDIWNNQANPDVSIAGNGLTAGIKANQVHNFDGSPLTGPNA

PYYGISTQRASHQIWIPHRTSLEVTGEHKSTIKYVDENGKEIFPENTQSLDMKRSLILDI
TQDKIQDIQNYALSHTADETLEYIKNSQAVVQDSGWDFTDANGKAVIDPYSTIDSPKLAG
YAATIQSTNVSTLKVGADASAVTAKFMVEPSQDMVQNGELSTAYKQNGTTGIPADYVTVV
VYNKQATYQAGKTDSKSVVRDINYLDGKTGKKIPANLIKDNPVSQTVTMHRTEILDNSGK
VIGYGTISEDGKSYTLNNDWIIDGNWEAVKSPDLTTNGYKTPRFENGDIAKVVPAETVNE
NTPDTTVNVYYDHNEIPVGPDTPDKHGVDLNELTKDVNETVHYVYSDGTKAAPDVVQTSK
WTRTLTIDAVTNEVISDGQYTTDWAIPKGEKTVYDQVDTPVIKGYHADKRQINATVVTQN
NIEETVTYQPNGKIIPVDPNGDPIPNVPTPTYPTDPTDPTKVVPDEPVPEIPGLIPSVPT
VTPENPGKDTPVPYTPEVPAKDQVAVVNYIDADDNNAIITSSGNLTGKAGEKIDYSTAST
IEELENKGYVLVRDPNSSSVDKLAAALEHHHHHH

SEQ.ID. 23:   CSP8 protein sequence

MKKKREKYTWLLLSTALITSGQILGGGEQIIKASVESQTNSVKKSKTAVEHSTTALSRQA
VEAQLAAQGVNFERLTPEEQQEVYVDVIVQLEALPASENGSIDSQTASRAEIEQASNKVI
AAQSGIKDEVQKITNQAIDKSYGYVVNGFATKAKVGDIKKLREIKGVKSVTLAKVYFAAD
TSANNMANVSTVWSNYQYKGEGTVVSIIDTGIDPNHKDLRLSDESKVKLTAKDIDGFTEN
SGYGRYFTSKVPFGHNYSDNNDIITDDDPKEQHGMHVAGIVAANGTGKNSASSVVGVAPE
AQLLAMKAFSNSDSSSTTDSTSVIGAVDDSAKLGADVLNMSLGSVSGEQTEDDPEIAAVE
KAVKHGTAAVISAGNSGTSTSNQEGVNKDFYGNPDMETIGSPGTSRSATTVASAENTKVT
TDGMTVSTADGKKIFGPSVTQLSPNTSHDAFDSKKFYIVKDASGKLGMGTPSQYTADVKG
KVAVVSRGEITFTDKQKYAQAAGAAGLIIVNNAGGNTPLTSVLYNEGFPTAGLSTDDGNK
LVAYVEAHPDELLRVNVEVQPLNNVIREEDLMSSFTSYGPVSDLSFKPDITAPGGNIWSL
QNNNGYINMSGTSMASPFIAGSQALLVQAMNDKTGKFYETYQKMSGSERAALIKNIQMNT
ANIEVDVDHGSVIESPRRQGAGLVNVEAAINAILHNPSTVSGSNGYPGVELKDFQDRQHQ
FTIKFTNRTNKDIEYGLNENGKFSDVYTSETDPKTGVLFEKKIDGASLTPSEKIVVPANS
TKEVTINLSLPDNFKENQYVEGFMAFTGSDNSHLKIPYMGFFGDWAAPAIFDGLNGLAFN
PGNNNLGTIVTAGNKNGAVGYAGLNQDEDGNYRVDPDAIALSTADGASVSWVRPQYFLFR
NANDVKAEILNQDGEVINTLVSLAHVTKSYWAASSQRYAKFNYAPAWDGTYFNQQTNKTE
KVPDGTYTYRVTGTVDGTKKQQHYDIKVKVDSVKPEVKNLKLGSHKDQTGKVSYVLKAEA
KDNFSGLNGQANTYVNGELNRSVAYDIVGSSSDGYQKIEVPLSDEQVKTLRAGKNDLAIA
VFDNATNAGTNSGTSNKPGEINFGLIIDNNLPQKITTVSDGYDMTDDSYTISGTYPEKVY
GTYTDKDGKEHDLNISYDEASERFVTKLPLSVSDYDTNVKFYADEEHETLITQKRINVSL
VPPKLESLKVDDQETYTGNEEAKLSQTSEDTVEVSGKVSDDTDKVAVKVAGKTYSAKPTK
EHTFKVKVPVSYGENTMNIVLTDKDGNSSSVKQIVKSSDRGKTVVSAKDVTFDNGIKFGT

TSVNTETENYDPKTGKLTLTGKVNRPTTTVRIGDHTVKVKADGTFKLVLDLGKHGAKVFP
VLIGDTTVNDTVQERLTFYVDSNNPELTLNQEKDQSGYVPVYTNKEEFKLQGTISDDYPY
YSLLINDNNVDANWDDVDYNGNKNLKKSFSHSVKLKEGKNTFNVVVVDNNDNRSEVQTLV
VYYKKAQKLASPQITATTASDKKSVTVTGKAKDGNVLYSTDNGNKYNVLPEDGVTVKNNG
KLLFKTVDKYGNESEVVEYDVKTIGKEESTVDKSVAQARKDLRKKLDQARALGNTGKYTH
ESAKKLAQARQEASKALKDKNATLQELKQASEQLEEAIKNLVEKPVDQNKDKDKVEDKDS
QVNALKEKLEETVKAGEKFDKDKYTDDSVEKVTKALDEAKVVLANKDANSTDVQDAIDSI
VNATKSLKEKQVSPEKTTQQEDTPKENKDTEEVLAAKNALKEKADKLSHLDTTKYTSESA
ENLSNALKKVNQVLTNKDANKAQVQEALDSLSQAEKDLVEKTESNKDVETAKEQLREELN
KHKDEDKSQYTDDSAKVKDNAEKISEGVLDSKDAQADEVNKAKDSLVEAEKGLVKKEENK
PAESDTQEVDKAREALEQEVNKNANVNLDGYTPESQDKFKEILNGVRDVLNDKNASASSL
EKAEKVLETATGVLTQVEHQVELPKVEQPVVTPEKKQTEQEEAKKSESSSVSTSKDEVKE
PEEKKQDAHSVSDKGTGTSVEKKGQTPADALSQVEHQAELSKNEQEEAKKSESHSASTSK
DEVKEPEEKKQDAESVSDKESSTLVGKKHQVARSAQESSKKNADNKVPTHKNSNKAQNTT
NSSTRSTKSSNNKELPKTGERETFFGLLVTGITALFASLGTVLRIKNKK

SEQ.ID. 24:   Sequence of the CSP8 expressed protein

MAYRVTGTVDGTKKQQHYDIKVKVDSVKPEVKNLKLGSHKDQTGKVSYVLKAEAKDNFSG
LNGQANTYVNGELNRSVAYDIVGSSSDGYQKIEVPLSDEQVKTLRAGKNDLAIAVFDNAT
NAGTNSGTSNKPGEINFGLIIDNNLPQKITTVSDGYDMTDDSYTISGTYPEKVYGTYTDK
DGKEHDLNISYDEASERFVTKLPLSVSDYDTNVKFYADEEHETLITQKRINVSLVPPKLE
SLKVDDQETYTGNEEAKLSQTSEDTVEVSGKVSDDTDKVAVKVAGKTYSAKPTKEHTFKV
KVPVSYGENTMNIVLTDKDGNSSSVKQIVKSSDRGKTVVSAKDVTFDNGIKFGTTSVNTE
TENYDPKTGKLTLTGKVNRPTTTVRIGDHTVKVKADGTFKLVLDLGKHGAKVFPVLIGDT
TVNDTVQERLTFYVDSNNPELTLNQEKDQSGYVPVYTNKEEFKLQGTISDDYPYYSLLIN
DNNVDANWDDVDYNGNKNLKKSFSHSVKLKEGKNTFNVVVVDNNDNRSEVQTLVVYYKKA
QKLASPQITATTASDKKSVTVTGKAKDGNVLYSTDNGNKYNVLPEDGVTVKNNGKLLFKT
VDKYGNESEVVEYDVKTIGKEESTVDKSVAQARKDLRKKLDQARALGNTGKYTHESAKKL
AQARQEASKALKDKNATLQELKQASEQLEEAIKNLVEKPVDQNKDKDKVEDKDSQVNALK
EKLEETVKAGEKFDKDKYTDDSVEKVTKALDEAKVVLANKDANSTDVQDAIDSIVNATKS
LKEKQVSPEKTTQQEDTPKENKDTEEVLAAKNALKEKADKLSHLDTTKYTSESAENLSNA
LKKVNQVLTNKDANKAQVQEALDSLSQAEKDLVEKTESNKDVETAKEQLREELNKHKDED
KSQYTDDSAKVKDNAEKISEGVLDSKDAQADEVNKAKDSLVEAEKGLVKKEENKPAESDT

QEVDKAREALEQEVNKNANVNLDGYTPESQDKFKEILNGVRDVLNDKNASASSLEKAEKV
LETATGVLTQVEHQVELPKVEQPVVTPEKKQTEQEEAKKSESSSVSTSKDEVKEPEEKKQ
DAHSVSDKGTGTSVEKKGQTPADALSQVEHQAELSKNEQEEAKKSESHSASTSKDEVKEP
EEKKQDAESVSDKESSTLVGKDPNSSSVDKLAAALEHHHHHH

SEQ.ID. 25:   CSP10 protein sequence

MVSKNNIEMKYKKMRDEKQRFSIRKFSVGAASVLIGLSFSLYNGQQAFADTVDNGNKSVV
ANAQDKEQTDNKQNTDTNPQNNEDKTNNNQTQNTVTADLDHSTVGASYKADTTNASEANK
SSDKSDEKQAATTETAKTEEAATKEVEAKNTANDDVAKKTEEAAAKTDTKVDVNKTVVEN
VKTEAATPNVETRNIETQTAKTDEEKVETAVDNTSSDLKNAVENANTEVSTNTFNVNKAV
RNALFLSANNLAERKVAAIALPAEAEDPNAVTVSDAKGFIDAIQNGTATTINVASDLNLA
DQTDKKYTEINIKNKRNIVIQSDNPEEKRTIDFSGYSFDMNTENSVTFKDLNIYARSYWG
LVYNAGGYTFNNVNFTGSQLIYTKPSINSTLTFKNNITANSVSSYVGPLDGKTRDTQQNG
GQQILQFEGGTNQIIFDENSNVTLTTEDANALEIDGGTTTIDVKDGANVAINPHSKGNPE
SRNGIGTGYVARAIAANAKMTINIDPNSTLTINTEKVDDDKDVAGALYLNSDAALNVNGK
LVINSNGTPSASKSNGVPVYINGSANINVGNGGSFVLDATNLGDYSSSLISVNGKGTVKL
DPHSTFKISGDGTGAVTAINLSSGSTFTSDQPDSFTIDLSANTSNGKSLIKNGTINFSRV
KTVTDGNESQPLGKIDVTYDRNGNATSYTITAQDKNTVNQVAEGLANKSLISLVKAGEDV
TLSNLHLSKNNVLTGTVASSGSDNPIYVTVTVGGVSTNVPVVGHYTVYTKTNKTVTSNNV
DYAAQTASTGGNFSIDLSKLASSLTNDAQVTVTATKDFVESAQTKSVAALRALNTTTLKE
LVDAAPAEEAKPSYYNATEEAQKAYTDAISKGNDILADPNNYDQVDVDNAVTAIQNAQKA
LTGKETDKKALQKAVDDASKVESSDNYTNADARLQNDYQAAIKAGQGILSKENATQTEVD
NALTTINNAKDALNGDAKKAASKEALQKAVDEAPTVKSDDAAYYNGSAEAKTAYDDAISA
GQIVLDNPDATATQITDALNAINTAKGNLKGEATDKAALQTAVDNSATVKESNNYTNADQ
TQKTAYDKAVTAAQTVLDKTNATQAEVNQALQDLETANRNLNGDAKTEAANKAALEAAVK
DAPNVRNTPAYYNGSEETQTAYNNAITAGQTVLNEANPSASEVKNALDAINAAKDNLKGK
ATNTEALETALTNANNAKETGNYTNADQANQEALNNAIIAGQEILKNTSATQAQVDSAAK
AITDAISGLNGDTNLANAKTAATEDIQKALDTKTTEITDATNIDQATKDQLIADAKKAAE
DANTAINQSTDTDTVNTAKTEGIANINKVTVPSLADAKTKAAKEIDQALTDKTKEINNAE
NIDQTTKDQLIKEATDAANTAKDAIEKSTTNDEATKAGQAGVDAITNVKVPSVTDSQNAA
KEVIDDALNAKTKEINDANNIDQTTKDQLIKEATDAANKAKEAIDKATTADAIKTAQDEG
TTNINNVTVPSLEDAKKAANKAVDDALTAQTEVINKANNLSDAEKKDLIDQVTAEANKAK
ENIETATTNNEAAQAGQAGVDAIKKIVPTSLDTVKSDANKAIDDALTKKLEEINSANDLT

TDEKTALTQEANTAADKAKEEITNATTNDAVIEAQNNGVTAIDGIKVPTESAVKEAAKQA
VADAATAKNQAIDASNLTDEEKAALKQKVTDAQNAADQAIDNATTNAAVTEAQTNGIKAI
NGIEVTTSTVKEAAKKAVADAATAKNNAIDASNLTDEEKAALKQKVTDAQNAADQAIDNA
TTNVAVTEAQTNGVNAINGIEVPTTSATKEHAITDLNAAVDDAKKAIDQDSNLTDEEKQA
AKDQIDRDATKAQEAINNAKTNDDVKKAGDAGTLAIDKDVANAAIDNAVAGKKAEISKTP
LTDEEKTALNNEVDQKAQEAKEAINNATTPEAVTTAQDSGVNNINETSVPSESAAKQAAK
EAVAKAVDEKNAAIDSSNLTEEEKEALKQKVTEAQTAADQAIDNATTNAGVTEAQTNGVN
AINGIEVPNKSDAKEQAITDLNTAVDNAKKAIDQDSNLTDEEKQAAKDQIDSDAKNAQDA
INNAKTNDDVKKAAYDGTLAIDKDVANAAIDNAVAGKKAEISNSSLTDEEKTALNNEVDQ
KANSAKDAINNATTPEAVTTAQGNGIKNINATSVPTTSTAKEAAKKAVAEAAEAKNSAID
SSNLTDEEKAALKQKVTEAQNGADHAIDNATTNAAVTEAKDNGISAIDGIEVPITSATKE
QAITDLNTAVDDAKKTIDQDNNLTDEQKQAAKDQIDSDAKTAQDAINNAKTDNDVNNAVN
SGKVAINKDVANAAIDNAVAGKLKEIQDPLTTEEKQAYTDLINSEATNAKQNIANATTVE
AVTTAQTNGVNEITNTEIPTTSSAKEKAIAAINDALQTKTDEINNASNINTQEKTDLINQ
ATEAANAAKNNINNATTNADVDTAQIKGEKAIADVTVPNLSDVKKESIDLINKALDAKTN
EINNASNLSQDEKQGLINDATNAATEAINNVNQAQTNDDAKAAATTGVQNIENITIPTLD
EAKKNANQAIDDALNSKVNEINNASNLNETEKQKLVDQANEAATTAKNNVEKATTNDDAR
DAANAGIDNIKGITFTSLEDAKNAANTAIDNALQVKTDEINNASNLSTEEKQDLINQASE
VAKNAKDNINNATTNDAVTEAQNKGIADIANVTVPSLDQVKQDAINAIKQVQDAKNKQIS
AASNLSTEEQKELSDQVDKIANDAIAQINDSSTTTNDAVTATRDDAIKQITDLFIPTLDG
AQTDALNAIESAKNAKLNDINNATHLTDQEKQALVDQTNKVADDATKEIKAAQTNDAVKS
AETAGLDNINNIAIPTLVQKQQEAIEELNVARDAKSSAIDNATDLTTDEKNSLKDKVQAE
YSNAVSNITSATTDEAVTTAKENGIAAIKDIQIPTKSPAKEQATSDLNTAVDEAKNAIDQ
DNNLTDEEKQAAKDQIDSDAKKAQEAIDNATTDDEVNSAVDNGKLAIDKDIANAAIDNAV
AGKKAEISKSSLTSEEKADLNNEVDQKAKDAKEAIKAATTPETVTTAQENGIKNINDTKV
PTESAAKEAAKKAVAEAAEAKNNAIDSSDLTNEEKAALKQEVADAQNAADRAIDAATTNT
AVTEAQDNGIKAIENVTVPTESAAKETAKKAVAEAAEAKNNAIDSSNLTSEEKAALKQEV
AEAQNAANTAIDNATTNAAVTEAEDNGIKAINSIEVPTKSDAKEQATSDLNSAVDEAKKA
IDQDSNLTDEEKQVAKDQIDSDAKKAQEAIDTAKTNDDVKKAIDDGTLAIDKDVANAAID
NAVAGKKAEISKSPLTDEEKTALNNEVDEKANSAKDAINKATTPEGVTEAQNSGIKSIDD
VNVPTESVAKEAAKKAVAEAAEAKNNAIDSSNLTDEEKAALKQEVSDAQTAANTAIDNAI
TNAEVTEAEDNGVKTINGIEVPTKSTTKEQATNDLNNEVENAKKAIDQDNNLTDEQKQAA
KDQIDSDAKKAQDAINNAKNNDDVKKAVDDGKLVIDKDVANAAIDNAVAGKKDEISKSPL
TDEEKAALNNEVDQKAEEAKEAIKAATTPGAVTTAQENGIKNINETEVPTESVAKEAAKR

AVAEAADAKNKAIDSSNLTDEEKAALKQEVSDAQTAANTAIDNATTNAAVTEAEDNGIKA
INSIEVPTKSDAKEQATSDLNSAVDEAKKAIDQDSNLTDEEKQVAKDQIDSDAKKAQEAI
DTAKTNDDVKKAIDDGTLAIDKDVANAAIDNAVAGKKAEISKSPLTDEEKTALNNEVDEK
ANSAKDAINKATTPEGVTEAQNSGIKSIDDVNVPTESVAKEAAKKAVAEAAEAKNNAIDS
SNLTDEEKAALKQEVSDAQTAANTAIDNAITNAEVTEAEDNGVKTINGIEVPTKSTTKEQ
ATNDLNNEVENAKKAIDQDSNLTDEEKQVAKDQIDSDAKKAQDAINNAKTNDDVKKAIDD
GTLAIDKDVANAAIDNAVAGKLKEINDSLTNEEKQAYTDLINNEADNAKQKIAEATTPEE
VTRAQEEGVKNINNINVPTTSPAKDAANAAIDQALKNKKDEINNATNISSEEKTDLIKQA
TEAANIAKDNINNATTNSEVETAQVDGEKAIADVTGPGLSDIKKESIDLINKALNEKQDE
INNASNLSQDESTELIDQAKKIATEAINEINNAQTNDEAKEAADTGVKNIENVSIPSIED
AKKNATQAIDDALNSKKNEINNASNLTDSEKTDLINQATEIANAAKDAINSATTNTAVEA
AEYKGVADINNIHFTNLDDSKKAANSAIEDALTTKKDEINNASNLSDSEKAKLINQATEI
ANAAKAAINNATTNSAVTAAENKGIEDIANINVPSLAETKQAAIDAIQQVQKAKNSQIEE
AKNLSADEQKNLIDQVNKIAQDAINKLNDPATTTNEVITDTRDKAIDQITNLFIPTLDSV
QKDAQEAINSAQETKIDEINKADNLTDQMKQNLIDQVDQVADKATKAINNAQTNDDVKEA
EIEGLEDIDSIKVPSLVESKDDAIKEINDALKKKTDEINAADLDQKQKDELISQITDIAT
ETKTKVFNATTNAEVDAEAEAGIKAIEAVKIPARTADNSNTESESKEQTVITNSVQPKRN
AVHHKNGTPVNKKATLPQTGKKDNLTLTLAGAALLGLAGVFSLFGLGDKRKKNK

SEQ.ID. 26:   Sequence of the CSP10 expressed protein

MVARAIAANAKMTINIDPNSTLTINTEKVDDDKDVAGALYLNSDAALNVNGKLVINSNGT
PSASKSNGVPVYINGSANINVGNGGSFVLDATNLGDYSSSLISVNGKGTVKLDPHSTFKI
SGDGTGAVTAINLSSGSTFTSDQPDSFTIDLSANTSNGKSLIKNGTINFSRVKTVTDGNE
SQPLGKIDVTYDRNGNATSYTITAQDKNTVNQVAEGLANKSLISLVKAGEDVTLSNLHLS
KNNVLTGTVASSGSDNPIYVTVTVGGVSTNVPVVGHYTVYTKTNKTVTSNNVDYAAQTAS
TGGNFSIDLSKLASSLTNDAQVTVTATKDFVESAQTKSVAALRALNTTTLKELVDAAPAE
EAKPSYYNATEEAQKAYTDAISKGNDILADPNNYDQVDVDNAVTAIQNAQKALTGKETDK
KALQKAVDDASKVESSDNYTNADARLQNDYQAAIKAGQGILSKENATQTEVDNALTTINN
AKDALNGDAKKAASKEALQKAVDEAPTVKSDDAAYYNGSAEAKTAYDDAISAGQIVLDNP
DATATQITDALNAINTAKGNLKGEATDKAALQTAVDNSATVKESNNYTNADQTQKTAYDK
AVTAAQTVLDKTNATQAEVNQALQDLETANRNLNGDAKTEAANKAALEAAVKDAPNVRNT
PAYYNGSEETQTAYNNAITAGQTVLNEANPSASEVKNALDAINAAKDNLKGKATNTEALE
TALTNANNAKETGNYTNADQANQEALNNAIIAGQEILKNTSATQAQVDSAAKAITDAISG

LNGDTNLANAKTAATEDIQKALDTKTTEITDATNIDQATKDQLIADAKKAAEDANTAINQ
STDTDTVNTAKTEGIANINKVTVPSLADAKTKAAKEIDQALTDKTKEINNAENIDQTTKD
QLIKEATDAANTAKDAIEKSTTNDEATKAGQAGVDAITNVKVPSVTDSQNAAKEVIDDAL
NAKTKEINDANNIDQTTKDQLIKEATDAANKAKEAIDKATTADAIKTAQDEGDPNSSSVD
KLAAALEHHHHHH

SEQ.ID. 27:   LP 1 protein sequence

MSLMMIILNHQLVPNRWRNITMKKAKLFGSLTLLSGVALTLAACGNNSNSKVDNPTKNFK
SATPKKAVKKGGTVSVALESDTPFTGIFLNELSDTQNDSDAMAPANEGLFDTDDTYQIND
KGPATLKLDNDNKTATIKIKNGVKWSDGKQVTAKDVEYSYEIIANKATKSSRYTDSLQNI
VGMSEYHDGKSNTISGIEMPDGENGRTVVIHFKEMKPGMKFSGNGYFWEAAAPYHYLKDI
PFDKLQSSDQVRKKPLFFGPYKMSKVVRGQAVTFVPNKYYWRGTPKLDKVTIQVLNPNSA
SQAIKSHKYDIAGVINVQWKNVANTNNVNWIAKIPLSYRYLGFKVGKWDAAKGENVMDKN
AKMNNKALRQAIAYGMNISAVNKRYTNGLTFHIPTLIPAQFGKYFDKNVKGYDYNIKKGN
ELLDKAGYKKKGKYRVQPNGKPLTIHFAAMSGDSTQEPIIQNYIQQWKKLGLNVKLTGGR
LMEMNSFYDKVQNDSKDVDMFMVAWSLSSEPSPNDLYNAKAPYNFSRFVTAKNTKLLQEM
DSQKAFNTNYRVKKFHEWQKYMDDEAYVVPVSNSYSIDAVNSKITGYSSKPSAANSLWYN
VGIAK

SEQ.ID. 28: Sequence of the LP1 expressed protein

MACGNNSNSKVDNPTKNFKSATPKKAVKKGGTVSVALESDTPFTGIFLNELSDTQNDSDA
MAPANEGLFDTDDTYQINDKGPATLKLDNDNKTATIKIKNGVKWSDGKQVTAKDVEYSYE
IIANKATKSSRYTDSLQNIVGMSEYHDGKSNTISGIEMPDGENGRTVVIHFKEMKPGMKF
SGNGYFWEAAAPYHYLKDIPFDKLQSSDQVRKKPLFFGPYKMSKVVRGQAVTFVPNKYYW
RGTPKLDKVTIQVLNPNSASQAIKSHKYDIAGVINVQWKNVANTNNVNWIAKIPLSYRYL
GFKVGKWDAAKGENVMDKNAKMNNKALRQAIAYGMNISAVNKRYTNGLTFHIPTLIPAQF
GKYFDKNVKGYDYNIKKGNELLDKAGYKKKGKYRVQPNGKPLTIHFAAMSGDSTQEPIIQ
NYIQQWKKLGLNVKLTGGRLMEMNSFYDKVQNDSKDVDMFMVAWSLSSEPSPNDLYNAKA
PYNFSRFVTAKNTKLLQEMDSQKAFNTNYRVKKFHEWQKYMDDEAYVVPVSNSYSIDAVN
SKITGYSSKPSAANSLWYNVGIAKDPNSSSVDKLAAALEHHHHHH

SEQ.ID. 29: LP 3 protein sequence

MLLEEKLMKKGKLIGTLTLLSGVALTLAACGNSNSNNTSHPNFKESTPKKTIKDGGNVSV
AVVTDTPFTGIFNDELSTNNTDSEVMQYGDESLFATNNTYKYVKGGAADIKINKDAKTAT
ITINPKVKWSDGQPLVAKDYEYAYEIIANKATHSQRYTSSLANLVGLEEYHDGKSNTISG
IEMPDGENGRTVVLHFKEMKPGMTQSGNGYIWEAAAPYHYLKDVPFDKLISSNKIRKNPL
FYGPYKVSKVVRGQSVSWVPNEHYYKGKPHLKKITASVITPASVAQSIKSNKFDVTQVSN
SQWPNIKGAKGVNFIANIPLAYSYLGFKVGKWDAAKGENVMNKDAKMNNRSLRQAIAYGM
NVDQVYKRYSSGLSFRIPTLIPKQFGDYFDKNVKGYTYNIKKGNELLDKAGYKKKGTYRV
QPNGKPLTIRLAAMSGSKVQEPIIQNYIQQWKKMGLNVKLTGGRLMEMNSFYDKVQNDSK
DVDMFIGAWSLSSEPSPQDLYGAKAPFNYSRFVTKENTDLLNDIDSQKAFNNSYRVKKFH
QWQAYMDKEAYVVPVANSYSIYAINNKLTGYSLEPSKSMGGGFPNWYYVGYAK

SEQ.ID. 30: Sequence of the LP3 protein expressed protein

MACGNSNSNNTSHPNFKESTPKKTIKDGGNVSVAVVTDTPFTGIFNDELSTNNTDSEVMQ
YGDESLFATNNTYKYVKGGAADIKINKDAKTATITINPKVKWSDGQPLVAKDYEYAYEII
ANKATHSQRYTSSLANLVGLEEYHDGKSNTISGIEMPDGENGRTVVLHFKEMKPGMTQSG
NGYIWEAAAPYHYLKDVPFDKLISSNKIRKNPLFYGPYKVSKVVRGQSVSWVPNEHYYKG
KPHLKKITASVITPASVAQSIKSNKFDVTQVSNSQWPNIKGAKGVNFIANIPLAYSYLGF
KVGKWDAAKGENVMNKDAKMNNRSLRQAIAYGMNVDQVYKRYSSGLSFRIPTLIPKQFGD
YFDKNVKGYTYNIKKGNELLDKAGYKKKGTYRVQPNGKPLTIRLAAMSGSKVQEPIIQNY
IQQWKKMGLNVKLTGGRLMEMNSFYDKVQNDSKDVDMFIGAWSLSSEPSPQDLYGAKAPF
NYSRFVTKENTDLLNDIDSQKAFNNSYRVKKFHQWQAYMDKEAYVVPVANSYSIYAINNK
LTGYSLEPSKSMGGGFPNWYYVGYAKDPNSSSVDKLAAALEHHHHHH

SEQ.ID. 31: LP 7 protein sequence

MKKKFSFLILLGVILVSLTTACSNKSSKQAKSTGPSASSLINAKFNSSFANGHFTQTTNSNE
MNQKSKSEGLFKDKGDVTNLTYTLTQKKKSQTEQMWLTKKDMYLLLEQNKGHWIKNSIDADS
FDSDQVKERFDPATFNKINKSLAQKATVQRKNGNYEISFDGTDANLWNAVNPLIIDAMNTPG
SQNMQVARLVKSAQVQNLKITYLIDPTTKNVESMTFKAQYTAGGKYNFTWNLTYDQLGQHSD
LAVPSDIQKNAISAEDIKKAQEEQNNQ

SEQ.ID. 32: Sequence of the LP7 expressed protein

MACSNKSSKQAKSTGPSASSLINAKFNSSFANGHFTQTTNSNEMNQKSKSEGLFKDKGDV
TNLTYTLTQKKKSQTEQMWLTKKDMYLLLEQNKGHWIKNSIDADSFDSDQVKERFDPATF
NKINKSLAQKATVQRKNGNYEISFDGTDANLWNAVNPLIIDAMNTPGSQNMQVARLVKSA
QVQNLKITYLIDPTTKNVESMTFKAQYTAGGKYNFTWNLTYDQLGQHSDLAVPSDIQKNA
ISAEDIKKAQEEQNNQDPNSSSVDKLAAALEHHHHHH

SEQ.ID. 33: LP 8 protein sequence

MRKHYQKIFAFFFAISSLVLILTGCSKKENSSNKISIVTSTNVYADIAQNVLGKYGKATA
IITNSATDPHDFEPTTADAKKVQNAKIVVANGLGYDSWLPKLAKSTNKSAVLVGEDLMNL
KNGANPHIWFDLNMPKKYVNYLVERLSKIDKKHAAYYKENGKEYLAKINKIQKIADSIDG
AKQKPVYVSEPVFDYALNATHFKIGDKAFEEAIENETDPSAKIIHQMNQTINNRGISFFV
KNSQVSSSTVNNFVKRAKSKNIPILQVRETIPNNTSYIKWMTENYQNLANINKKLD

SEQ.ID. 34: Sequence of the LP8 expressed protein

MACSKKENSSNKISIVTSTNVYADIAQNVLGKYGKATAIITNSATDPHDFEPTTADAKKV
QNAKIVVANGLGYDSWLPKLAKSTNKSAVLVGEDLMNLKNGANPHIWFDLNMPKKYVNYL
VERLSKIDKKHAAYYKENGKEYLAKINKIQKIADSIDGAKQKPVYVSEPVFDYALNATHF
KIGDKAFEEAIENETDPSAKIIHQMNQTINNRGISFFVKNSQVSSSTVNNFVKRAKSKNI
PILQVRETIPNNTSYIKWMTENYQNLANINKKLEDPNSSSVDKLAAALEHHHHHH

SEQ.ID. 35: LP 11 protein sequence

MKFTKKFAAVAVAALALVGTTACSNSKSNDSSSSKSADIPTKITKKTTVVFWHGMTGTQE
KTLQSLTKDFEKKNPNITVKLENQGKYTDLQAKINSTLQSPNNLPTITQAYPGWLLNAAN
SKRLVDMTPYINNSKIGWGSAKKSDIREELLKGAQIKGTQYGIPFNKSVEMLTYNKDLLD
KYGVKVPKTMAELKAASKKIYEKSNHKVVGAGFDSLSNYYVLGMKDEGVNFSKNINFTGS
KSKKVINYYADGIKAGYFKTAGSAGYLSGDFSNQKVAMFVGTSAGEGFVKMGVGNKFQYG
VAPRPSKYNMQQGTDIYMFNKGTAEQKAAAFMYIKYLLGKNQQLTWADQTGYIPVTNNVI
NSSAYKNSTKSKVPAQLDKAMKNLYSVPVVKNSNTAYTQLNSIMQNIFAQAKKGQNWNSQ
IDAGKTKFESAWNQ

SEQ.ID 36: Sequence of the LP11 expressed protein

MACSNSKSNDSSSSKSADIPTKITKKTTVVFWHGMTGTQEKTLQSLTKDFEKKNPNITVK
LENQGKYTDLQAKINSTLQSPNNLPTITQAYPGWLLNAANSKRLVDMTPYINNSKIGWGS
AKKSDIREELLKGAQIKGTQYGIPFNKSVEMLTYNKDLLDKYGVKVPKTMAELKAASKKI
YEKSNHKVVGAGFDSLSNYYVLGMKDEGVNFSKNINFTGSKSKKVINYYADGIKAGYFKT
AGSAGYLSGDFSNQKVAMFVGTSAGEGFVKMGVGNKFQYGVAPRPSKYNMQQGTDIYMFN
KGTAEQKAAAFMYIKYLLGKNQQLTWADQTGYIPVTNNVINSSAYKNSTKSKVPAQLDKA
MKNLYSVPVVKNSNTAYTQLNSIMQNIFAQAKKGQNWNSQIDAGKTKFESAWNQDPNSSS
VDKLAAALEHHHHHH

SEQ.ID 37: LP 17 protein sequence

VKSKSYKYGIAALVGVSALALAGCQSNENQSSTSKTNAKSKSSIVKIEKKSKSKSSKSEK
QEGEVKKQSSEKVNKASSSKKDVEKVPTVTELMNNVSAFNEGNFDIRLNYATVKGYDTGI
IQENPRVVHYKFNGGMNSEVWATDQFVYSLSGDKWTKIGQTYKIGRFDPMISLPSPLINQ
MKVSKTSDGYQAKYEGNSAEAKELMEHYIVWVSSENKWNPFGDQDFTSATLTYSFNKQKQ
LTEIHYDWTAGSNKGTASYTNINQQKIAVPNDVIQAAAKNSDSIDSNNDDNSDDE

SEQ.ID. 38: Sequence of the LP17 expressed protein

MGCQSNENQSSTSKTNAKSKSSIVKIEKKSKSKSSKSEKQEGEVKKQSSEKVNKASSSKK
DVEKVPTVTELMNNVSAFNEGNFDIRLNYATVKGYDTGIIQENPRVVHYKFNGGMNSEVW
ATDQFVYSLSGDKWTKIGQTYKIGRFDPMISLPSPLINQMKVSKTSDGYQAKYEGNSAEA
KELMEHYIVWVSSENKWNPFGDQDFTSATLTYSFNKQKQLTEIHYDWTAGSNKGTASYTN
INQQKIAVPNDVIQAAAKNSDSIDSNNDDNSDDEDPNSSSVDKLAAALEHHHHHH

SEQ.ID. 39: LP 29 protein sequence

LKRFLQIALLLATGLSLSACGNLKNSDLANNPTTSTTKKKSYQTTSTSKNGYNVLLKDGEYV
TSPIEGTTENTSDNNVDSRALESGLIDLSHNTFSSSKYVFQEGQKISVADATDWLGRKSKSN
PEGLNAEKSNKKNSYNPIILDQILEQDFLTKSNSSYKMDGMSIGLALNSVDYYQKVKDGPQY
HKDISRAEQETFGKEAANKLVSYLRKKKGLKDIPILVGLFSKTSKDSLVGGNYFAYGIANAN

SSKINDWKTVNNRSQVLPTVGSEKAINSTDAASFSDFKAAIQGYFPNISGVTATVHYQNKTL
TQMNITVTTQFFGYAQIESFSRLVLSAAKKYLPKDAPIEIKINSVNDTQALIAKNSADDSYY
VHVFGGE

SEQ.ID. 40:   Sequence of the LP29 expressed protein

MACGNLKNSDLANNPTTSTTKKKSYQTTSTSKNGYNVLLKDGEYVTSPIEGTTENTSDNN
VDSRALESGLIDLSHNTFSSSKYVFQEGQKISVADATDWLGRKSKSNPEGLNAEKSNKKN
SYNPIILDQILEQDFLTKSNSSYKMDGMSIGLALNSVDYYQKVKDGPQYHKDISRAEQET
FGKEAANKLVSYLRKKKGLKDIPILVGLFSKTSKDSLVGGNYFAYGIANANSSKINDWKT
VNNRSQVLPTVGSEKAINSTDAASFSDFKAAIQGYFPNISGVTATVHYQNKTLTQMNITV
TTQFFGYAQIESFSRLVLSAAKKYLPKDAPIEIKINSVNDTQALIAKNSADDSYYVHVFG
GEDPNSSSVDKLAAALEHHHHHH

## Claims

1. A polynucleotide selected from the group consisting of SEQ. ID. No. 1 to 20 or variants or fragments thereof, obtained by deleting, adding or substituting one or more nucleotides, having a degree of homology to SEQ. ID. No. 1 to 20 of at least 90 % and encoding a functional mucin binding polypeptide.

2. The polynucleotide according to claim 1, which has a degree of homology to any of SEQ. ID. No.1, 3, 5,7, 9, 11, 13, 15, 17 or 19 of at least 95 %, preferably 98 %.

3. The polynucleotide according to claim 1 or 2, which has a sequence as identified by any of SEQ. ID.. No. 2, 4, 6, 8, 10, 12, 14, 16, 18 or 20 or variants or fragments thereof, obtained by deleting adding or substituting one or more nucleotides, having a degree of homology to SEQ. ID. No. 2, 4, 6, 8, 10, 12, 14, 16, 18 or 20 of at least 90 % and encoding a functional mucin binding polypeptide.

4. A vector comprising a polynucleotide sequence according to any of the preceding claims.

5. The vector according to claim 4, which is an expression vector.

6. A host cell harboring a recombinant polynucleotide according to any of the claims 1 to 3 or a vector according to any of the claims 4 or 5.

7. The host cell according to claim 6, which is a procaryotic or an eucaryotic cell.

8. A polypeptide as identified by any of SEQ. ID.. Nos. 21 to 40, or variants thereof, obtained by deleting, adding or substituting one or more amino acids, which have a degree of homology to the polypeptide of SEQ. ID.. Nos. 21 to 40 of at least 90 % and are a functional, mucin binding polypeptide.

9. The polypeptide according to claim 8, which has a degree of homology to the polypeptide as identified by SEQ. ID. Nos. 21 to 40 of at least 95 %, preferably 98 %.

10. The polypeptide according to claim 8, which has a sequence as identified by SEQ. ID. No. 28.

11. A fusion-polypeptide containing a polypeptide according to any of the claims 8 to 10.

12. Use of a polynucleotide according to any of the claims 1 to 3 or a vector according to any of the claims 4 or 5 for the preparation of a polypeptide.

13. Use of a polynucleotide according to any of the claims 1 to 3 or a vector according to any of the claims 4 or 5 for the preparation of a micro-organism exhibiting improved mucin binding capacity.

14. The use according to claim 12, wherein the micro-organism is a probiotic microorganism.

15. A method for producing a polypeptide according to any of the claims 8 to 11, which comprises, culturing a host cell according to any of the claims 7 or 8 under conditions, which allow synthesis of the polypeptide and recovering the polypeptide.

16. An antibody, capable to bind to a polypeptide according to any of the claims 8 to 11.

17. A method for detecting a mucin binding protein in a biological sample, which comprises the steps
(a) providing a biological sample;
(a) optionally purifying the DNA contained in the biological sample; and
(b) detecting the presence of a polynucleotide according to any of the claims 1 to 3.

18. A method for detecting a mucin binding protein in a biological sample, which comprises the steps
(b) providing a biological sample;
(c) detecting the presence of a mucin binding polypeptide according to any of the claims 8 to 11.
